# EUROPEAN PATENT APPLICATION

(11) **EP 2 462 936 A1**
(43) Date of publication of application: **13.06.2012**
(21) Application number: 11194122.5
(22) Date of filing: 05.01.2004
(51) Int. Cl.: A61K 31/70, C07H 21/02, C07H 21/04, C12N 5/00, C12Q 1/68

(54) **SiRNA mediated post-transriptional gene-silencing of genes involved in alopecia**

(30) Priority: 03.01.2003 US 437842 P
(62) Divisional of application: 04700221.7
(71) Applicant: Gencia Corporation, Charlottesville, VA 30318 (US)
(72) Inventor: Kahn, Shaharyar, North Garden VA Virginia 22959 (US)
(74) Representative: Walker, Ross Thomson

(57) **Abstract**

Compositions and methods for the use of inhibitory nucleic acids, for example small inhibitory ribonucleic acids (SiRNA), to adjust, manipulate, prevent, inhibit, interfere, or block the androgen signal transduction pathway in a host cell, for example in a host's hair cell are provided. Aspects of the disclosure provide compositions and methods for interfering with the androgen signal transduction pathway by down regulating the expression of proteins involved in the androgen signal transduction pathway. Exemplary gene targets encoding proteins involved in the androgen signal transduction pathway include but are not limited to isozmes I and II OF 5-α reductase, the androgen receptor, aromatase, 3-α-hydroxysteroiddehydrogenase, 3-β-hydroxysteroiddehydrogenase, 3-β-hydroxysteroiddehydrogenase-4-5isomerase, 17-β-hydroxysteroidoxidoreductase, and steroid sulfatase. In some aspects, the inhibitory nucleic acids, for example siRNAs, interfere with the expression of targeted genes by preventing , reducing, or inhibiting the translation of mRNA transcribed from the target gene.

SEQUENCE LISTING

<110>  Gencia Corporation
       Khan, Shaharyar

<120>  siRNA Mediated Post-Transcriptional Gene Silencing of Genes
       Involved in Alopecia

<130>  60199.2JPWO

<140>  PCT/US2004/000128
<141>  2004-01-05

<150>  US 60/437,842
<151>  2003-01-03

<160>  1403

<170>  PatentIn version 3.3

<210>  1
<211>  636
<212>  DNA
<213>  Homo sapiens

<400>  1
atggcaacgg cgacgggggt ggcggaggag cgcctgctgg ccgcgctcgc ctacctgcag      60

tgcgccgtgg gctgcgcggt cttcccgcgt ctccgcagcg cgcccaactg catcctcctg     120

gccatgttcc tcgtccacta cgggcatcgg tgcttaattt acccatttct gatgcgagga     180

ggaaagccta tgccactgtt ggcgtgtaca atggcgatta tgttctgtac ctgtaacggc     240

tatttgcaaa gcagatactt gagccattgt gcagtgtatg ctgatgactg ggtaacagat     300

ccccgttttc taataggttt tggcttgtgg ttaacaggca tgttgataaa catccattca     360

gatcatatcc taaggaatct cagaaaacca ggagatactg gatacaaaat accaagggga     420

ggcttatttg aatacgtaac tgcagccaac tattttggag aaatcatgga gtggtgtggc     480

tatgccctgg ccagctggtc tgtccaaggc gcggcttttg ctttcttcac gttttgtttt     540

ttatctggta gagcaaaaga gcatcatgag tggtacctcc ggaaatttga agagtatcca     600

aagttcagaa aaattataat tccatttttg ttttaa                               636


<210>  2
<211>  21
<212>  DNA
<213>  Homo sapiens

<400>  2
aacggcgacg ggggtggcgg a                                                21


<210>  3
<211>  21
<212>  DNA
<213>  Homo sapiens

<400>  3
cggcgacggg gguggcggat t                                                21

<210>  4
<211>  21
<212>  DNA
<213>  Homo sapiens

<400>  4
uccgccaccc ccgucgccgt t                                                21


<210>  5
<211>  18
<212>  DNA
<213>  Homo sapiens

<400>  5
aactgcatcc tcctggcc                                                    18


<210>  6
<211>  18
<212>  DNA
<213>  Homo sapiens

<400>  6
cugcauccuc cuggcctt                                                    18


<210>  7
<211>  18
<212>  DNA
<213>  Homo sapiens

<400>  7
ggccaggagg augcagtt                                                    18


<210>  8
<211>  21
<212>  DNA
<213>  Homo sapiens

<400>  8
aatttaccca tttctgatgc g                                                21


<210>  9
<211>  21
<212>  DNA
<213>  Homo sapiens

<400>  9
uuuacccauu ucugaugcgt t                                                21


<210>  10
<211>  21
<212>  DNA
<213>  Homo sapiens

<400>  10
cgcaucagaa auggguaaat t                                                21

<210>  11
<211>  21
<212>  DNA
<213>  Homo sapiens

<400>  11
aaagcctatg ccactgttgg c                                                21


<210>  12
<211>  21
<212>  DNA
<213>  Homo sapiens

<400>  12
agccuaugcc acuguuggct t                                                21


<210>  13
<211>  21
<212>  DNA
<213>  Homo sapiens

<400>  13
gccaacagug gcauaggcut t                                                21


<210>  14
<211>  21
<212>  DNA
<213>  Homo sapiens

<400>  14
aatggcgatt atgttctgta c                                                21


<210>  15
<211>  21
<212>  DNA
<213>  Homo sapiens

<400>  15
uggcgauuau guucuguact t                                                21


<210>  16
<211>  21
<212>  DNA
<213>  Homo sapiens

<400>  16
guacagaaca uaaucgccat t                                                21


<210>  17
<211>  18
<212>  DNA
<213>  Homo sapiens

<400>  17
aacggctatt tgcaaagc                                                    18


<210>  18
<211>  18

<212>  DNA
<213>  Homo sapiens

<400>  18
cggcuauuug caaagctt                                                    18


<210>  19
<211>  18
<212>  DNA
<213>  Homo sapiens

<400>  19
gcuuugcaaa uagccgtt                                                    18


<210>  20
<211>  21
<212>  DNA
<213>  Homo sapiens

<400>  20
aaagcagata cttgagccat t                                                21


<210>  21
<211>  21
<212>  DNA
<213>  Homo sapiens

<400>  21
agcagauacu ugagccauut t                                                21


<210>  22
<211>  21
<212>  DNA
<213>  Homo sapiens

<400>  22
aauggcucaa guaucugcut t                                                21


<210>  23
<211>  18
<212>  DNA
<213>  Homo sapiens

<400>  23
aacagatccc cgttttct                                                    18


<210>  24
<211>  18
<212>  DNA
<213>  Homo sapiens

<400>  24
   cagauccccg uuuucutt                                                     18


<210>  25
<211>  18
<212>  DNA
<213>  Homo sapiens

<400>  25
agaaaacggg gaucugtt                                                    18


<210>  26
<211>  21
<212>  DNA
<213>  Homo sapiens

<400>  26
aataggtttt ggcttgtggt t                                                21


<210>  27
<211>  21
<212>  DNA
<213>  Homo sapiens

<400>  27
uagguuuugg cuugugguut t                                                21


<210>  28
<211>  21
<212>  DNA
<213>  Homo sapiens

<400>  28
aaccacaagc caaaaccuat t                                                21


<210>  29
<211>  21
<212>  DNA
<213>  Homo sapiens

<400>  29
aacaggcatg ttgataaaca t                                                21


<210>  30
<211>  21
<212>  DNA
<213>  Homo sapiens

<400>  30
caggcauguu gauaaacaut t                                                21


<210>  31
<211>  21
<212>  DNA
<213>  Homo sapiens

<400>  31
auguuuauca acaugccugt t                                                21


<210>  32
<211>  18
<212>  DNA
<213>  Homo sapiens

<400>  32

aaacatccat tcagatca                                                    18

<210>  33
<211>  18
<212>  DNA
<213>  Homo sapiens

<400>  33
acauccauuc agaucatt                                                    18

<210>  34
<211>  18
<212>  DNA
<213>  Homo sapiens

<400>  34
ugaucugaau ggaugutt                                                    18

<210>  35
<211>  21
<212>  DNA
<213>  Homo sapiens

<400>  35
aaggaatctc agaaaaccag g                                                21

<210>  36
<211>  21
<212>  DNA
<213>  Homo sapiens

<400>  36
ggaaucucag aaaaccaggt t                                                21

<210>  37
<211>  21
<212>  DNA
<213>  Homo sapiens

<400>  37
ccugguuuuc ugagauucct t                                                21

<210>  38
<211>  21
<212>  DNA
<213>  Homo sapiens

<400>  38
aatctcagaa aaccaggaga t                                                21

<210>  39
<211>  21
<212>  DNA
<213>  Homo sapiens

<400>  39
ucucagaaaa ccaggagaut t                                                21

<210>  40
<211>  21
<212>  DNA
<213>  Homo sapiens

<400>  40
aucuccuggu uuucugagat t                                                21


<210>  41
<211>  21
<212>  DNA
<213>  Homo sapiens

<400>  41
aaaaccagga gatactggat a                                                21


<210>  42
<211>  21
<212>  DNA
<213>  Homo sapiens

<400>  42
aaccaggaga uacuggauat t                                                21


<210>  43
<211>  21
<212>  DNA
<213>  Homo sapiens

<400>  43
uauccaguau cuccugguut t                                                21


<210>  44
<211>  21
<212>  DNA
<213>  Homo sapiens

<400>  44
aaccaggaga tactggatac a                                                21


<210>  45
<211>  21
<212>  DNA
<213>  Homo sapiens

<400>  45
ccaggagaua cuggauacat t                                                21


<210>  46
<211>  21
<212>  DNA
<213>  Homo sapiens

<400>  46
uguauccagu aucuccuggt t                                                21


<210>  47

<211>  18
<212>  DNA
<213>  Homo sapiens

<400>  47
aaaataccaa ggggaggc                                                    18


<210>  48
<211>  18
<212>  DNA
<213>  Homo sapiens

<400>  48
aauaccaagg ggaggctt                                                    18


<210>  49
<211>  18
<212>  DNA
<213>  Homo sapiens

<400>  49
gccuccccuu gguauutt                                                    18


<210>  50
<211>  18
<212>  DNA
<213>  Homo sapiens

<400>  50
aataccaagg ggaggctt                                                    18


<210>  51
<211>  18
<212>  DNA
<213>  Homo sapiens

<400>  51
uaccaagggg aggcuutt                                                    18


<210>  52
<211>  18
<212>  DNA
<213>  Homo sapiens

<400>  52
aagccucccc uugguatt                                                    18


<210>  53
<211>  18
<212>  DNA
<213>  Homo sapiens

<400>  53
aaggggaggc ttatttga                                                    18


<210>  54
<211>  18
<212>  DNA

<213>  Homo sapiens

<400>  54
ggggaggcuu auuugatt                                                    18


<210>  55
<211>  18
<212>  DNA
<213>  Homo sapiens

<400>  55
ucaaauaagc cucccctt                                                    18


<210>  56
<211>  21
<212>  DNA
<213>  Homo sapiens

<400>  56
aatacgtaac tgcagccaac t                                                21


<210>  57
<211>  21
<212>  DNA
<213>  Homo sapiens

<400>  57
uacguaacug cagccaacut t                                                21


<210>  58
<211>  21
<212>  DNA
<213>  Homo sapiens

<400>  58
aguuggcugc aguuacguat t                                                21


<210>  59
<211>  21
<212>  DNA
<213>  Homo sapiens

<400>  59
aactgcagcc aactattttg g                                                21


<210>  60
<211>  21
<212>  DNA
<213>  Homo sapiens

<400>  60
cugcagccaa cuauuuuggt t                                                21


<210>  61
<211>  21
<212>  DNA
<213>  Homo sapiens

<400>  61
ccaaaauagu uggcugcagt t                                                21


<210>  62
<211>  21
<212>  DNA
<213>  Homo sapiens

<400>  62
aactattttg gagaaatcat g                                                21


<210>  63
<211>  21
<212>  DNA
<213>  Homo sapiens

<400>  63
cuauuuugga gaaaucaugt t                                                21


<210>  64
<211>  21
<212>  DNA
<213>  Homo sapiens

<400>  64
caugauuucu ccaaaauagt t                                                21


<210>  65
<211>  20
<212>  DNA
<213>  Homo sapiens

<400>  65
aaatcatgga gtggtgtggc                                                  20


<210>  66
<211>  20
<212>  DNA
<213>  Homo sapiens

<400>  66
aucauggagu ggugugcctt                                                  20


<210>  67
<211>  20
<212>  DNA
<213>  Homo sapiens

<400>  67
gccacaccac uccaugautt                                                  20


<210>  68
<211>  21
<212>  DNA
<213>  Homo sapiens

<400>  68
aaggcgcggc ttttgctttc t                                                21

<210>  69
<211>  21
<212>  DNA
<213>  Homo sapiens

<400>  69
ggcgcggcuu uugcuuucut t                                                21


<210>  70
<211>  21
<212>  DNA
<213>  Homo sapiens

<400>  70
agaaagcaaa agccgcgcct t                                                21


<210>  71
<211>  21
<212>  DNA
<213>  Homo sapiens

<400>  71
aaaagagcat catgagtggt a                                                21


<210>  72
<211>  21
<212>  DNA
<213>  Homo sapiens

<400>  72
aagagcauca ugagugguat t                                                21


<210>  73
<211>  21
<212>  DNA
<213>  Homo sapiens

<400>  73
uaccacucau gaugcucuut t                                                21


<210>  74
<211>  21
<212>  DNA
<213>  Homo sapiens

<400>  74
aagagcatca tgagtggtac c                                                21


<210>  75
<211>  21
<212>  DNA
<213>  Homo sapiens

<400>  75
gagcaucaug agugguacct t                                                21

<210> 76
<211> 21
<212> DNA
<213> Homo sapiens

<400> 76
gguaccacuc augaugcuct t 21


<210> 77
<211> 18
<212> DNA
<213> Homo sapiens

<400> 77
aaatttgaag agtatcca 18


<210> 78
<211> 18
<212> DNA
<213> Homo sapiens

<400> 78
auuugaagag uauccatt 18


<210> 79
<211> 18
<212> DNA
<213> Homo sapiens

<400> 79
uggauacucu ucaaautt 18


<210> 80
<211> 18
<212> DNA
<213> Homo sapiens

<400> 80
aagagtatcc aaagttca 18


<210> 81
<211> 18
<212> DNA
<213> Homo sapiens

<400> 81
gaguauccaa aguucatt 18


<210> 82
<211> 18
<212> DNA
<213> Homo sapiens

<400> 82
ugaacuuugg auacuctt 18


<210> 83
<211> 21

<212>  DNA
<213>  Homo sapiens

<400>  83
aagttcagaa aaattataat t                                                21


<210>  84
<211>  21
<212>  DNA
<213>  Homo sapiens

<400>  84
guucagaaaa auuauaauut t                                                21


<210>  85
<211>  21
<212>  DNA
<213>  Homo sapiens

<400>  85
aauuauaauu uuucugaact t                                                21


<210>  86
<211>  21
<212>  DNA
<213>  Homo sapiens

<400>  86
aaaaattata attccatttt t                                                21


<210>  87
<211>  21
<212>  DNA
<213>  Homo sapiens

<400>  87
aaauuauaau uccauuuuut t                                                21


<210>  88
<211>  21
<212>  DNA
<213>  Homo sapiens

<400>  88
aaaaauggaa uuauaauuut t                                                21


<210>  89
<211>  21
<212>  DNA
<213>  Homo sapiens

<400>  89
aaattataat tccattttg t                                                21


<210>  90
<211>  21
<212>  DNA
<213>  Homo sapiens

<400>  90
auuauaauuc cauuuuugut t                                                21


<210>  91
<211>  21
<212>  DNA
<213>  Homo sapiens

<400>  91
acaaaaaugg aauuauaaut t                                                21


<210>  92
<211>  765
<212>  DNA
<213>  Homo sapiens

<400>  92
atgcaggttc agtgccagca gagcccagtg ctggcaggca gcgccacttt ggtcgccctt      60

ggggcactgg tcttgtacgt cgcgaagccc tccggctacg ggaagcacac ggagagcctg     120

aagcccgcgg ctacccgcct gccagcccgc gcagcctggt tcctgcagga gctgccctcc     180

ttcgcggtgc ccgcggggat cctcgctcgg cagcccctct ccctcttcgg gccacctggg     240

acggtgcttc tgggcctctt ctgcgtacat tacttccaca ggacatttgt gtactcactg     300

ctcaatcgag ggaggcctta tccagctgta ctcattttcc gaggcattgc cttctgcgct     360

ggaaatggat tccttcaaag ctactatctg atttactgtg ctgaataccc tgatgggtgg     420

tacacagaca tacggttttg cttgggtgtc ttcttattta ttttgggaat gggagtcaac     480

atccatagtg actatatatt gcgccagctc aggaagcctg gagaaatcac ctacaggatt     540

ccaaaaggtg gcttgtttac gtatgtttct ggagccaatt tccttggtga gatcattgaa     600

tggatcggct atgcgctggc cacttggtcc ctcccagcac ttgcatttgc atttttctca     660

gtttgtttcc ttgggctgcg agcttttcac caccataggt tctacctcaa gatgtttgag     720

gactacccca aatctcggaa agcccttatt ccattcatct tttaa                     765


<210>  93
<211>  21
<212>  DNA
<213>  Homo sapiens

<400>  93
aagccctccg gctacgggaa g                                                21


<210>  94
<211>  21
<212>  DNA
<213>  Homo sapiens

<400>  94
gcccuccggc uacgggaagt t                                                21

<210>  95
<211>  21
<212>  DNA
<213>  Homo sapiens

<400>  95
cuucccguag ccggagggct t                                                21


<210>  96
<211>  18
<212>  DNA
<213>  Homo sapiens

<400>  96
aagcacacgg agagcctg                                                    18


<210>  97
<211>  18
<212>  DNA
<213>  Homo sapiens

<400>  97
gcacacggag agccugtt                                                    18


<210>  98
<211>  18
<212>  DNA
<213>  Homo sapiens

<400>  98
caggcucucc gugugctt                                                    18


<210>  99
<211>  21
<212>  DNA
<213>  Homo sapiens

<400>  99
aagcccgcgg ctacccgcct g                                                21


<210>  100
<211>  21
<212>  DNA
<213>  Homo sapiens

<400>  100
gcccgcggcu acccgccugt t                                                21


<210>  101
<211>  21
<212>  DNA
<213>  Homo sapiens

<400>  101
caggcgggua gccgcgggct t                                                21


<210>  102
<211>  21

<212>  DNA
<213>  Homo sapiens

<400>  102
aatcgaggga ggccttatcc a                                                21


<210>  103
<211>  21
<212>  DNA
<213>  Homo sapiens

<400>  103
ucgagggagg ccuuauccat t                                                21


<210>  104
<211>  21
<212>  DNA
<213>  Homo sapiens

<400>  104
uggauaaggc cucccucgat t                                                21


<210>  105
<211>  21
<212>  DNA
<213>  Homo sapiens

<400>  105
aaatggattc cttcaaagct a                                                21


<210>  106
<211>  21
<212>  DNA
<213>  Homo sapiens

<400>  106
auggauuccu ucaaagcuat t                                                21


<210>  107
<211>  21
<212>  DNA
<213>  Homo sapiens

<400>  107
uagcuuugaa ggaauccaut t                                                21


<210>  108
<211>  21
<212>  DNA
<213>  Homo sapiens

<400>  108
aaagctacta tctgatttac t                                                21


<210>  109
<211>  21
<212>  DNA
<213>  Homo sapiens

<400>  109
agcuacuauc ugauuuacut t                                                21

<210>  110
<211>  21
<212>  DNA
<213>  Homo sapiens

<400>  110
aguaaaucag auaguagcut t                                                21

<210>  111
<211>  18
<212>  DNA
<213>  Homo sapiens

<400>  111
aataccctga tgggtggt                                                    18

<210>  112
<211>  18
<212>  DNA
<213>  Homo sapiens

<400>  112
uacccugaug gguggutt                                                    18

<210>  113
<211>  18
<212>  DNA
<213>  Homo sapiens

<400>  113
accacccauc aggguatt                                                    18

<210>  114
<211>  18
<212>  DNA
<213>  Homo sapiens

<400>  114
aatgggagtc aacatcca                                                    18

<210>  115
<211>  18
<212>  DNA
<213>  Homo sapiens

<400>  115
ugggagucaa cauccatt                                                    18

<210>  116
<211>  18
<212>  DNA
<213>  Homo sapiens

<400>  116

uggauguuga cucccatt                                                    18

<210>  117
<211>  18
<212>  DNA
<213>  Homo sapiens

<400>  117
aacatccata gtgactat                                                    18

<210>  118
<211>  18
<212>  DNA
<213>  Homo sapiens

<400>  118
cauccauagu gacuautt                                                    18

<210>  119
<211>  18
<212>  DNA
<213>  Homo sapiens

<400>  119
auagucacua uggaugtt                                                    18

<210>  120
<211>  21
<212>  DNA
<213>  Homo sapiens

<400>  120
aagcctggag aaatcaccta c                                                21

<210>  121
<211>  21
<212>  DNA
<213>  Homo sapiens

<400>  121
gccuggagaa aucaccuact t                                                21

<210>  122
<211>  21
<212>  DNA
<213>  Homo sapiens

<400>  122
guaggugauu ucuccaggct t                                                21

<210>  123
<211>  18
<212>  DNA
<213>  Homo sapiens

<400>  123
aaatcaccta caggattc                                                    18

<210> 124
<211> 18
<212> DNA
<213> Homo sapiens

<400> 124
aucaccuaca ggauuctt 18

<210> 125
<211> 18
<212> DNA
<213> Homo sapiens

<400> 125
gaauccugua ggugautt 18

<210> 126
<211> 21
<212> DNA
<213> Homo sapiens

<400> 126
aaaaggtggc ttgtttacgt a 21

<210> 127
<211> 21
<212> DNA
<213> Homo sapiens

<400> 127
aagguggcuu guuuacguat t 21

<210> 128
<211> 21
<212> DNA
<213> Homo sapiens

<400> 128
uacguaaaca agccaccuut t 21

<210> 129
<211> 21
<212> DNA
<213> Homo sapiens

<400> 129
aaggtggctt gtttacgtat g 21

<210> 130
<211> 21
<212> DNA
<213> Homo sapiens

<400> 130
gguggcuugu uuacguaugt t 21

<210> 131

<211>  21
<212>  DNA
<213>  Homo sapiens

<400>  131
cauacguaaa caagccacct t                                                21


<210>  132
<211>  21
<212>  DNA
<213>  Homo sapiens

<400>  132
aatttccttg gtgagatcat t                                                21


<210>  133
<211>  21
<212>  DNA
<213>  Homo sapiens

<400>  133
uuuccuuggu gagaucauut t                                                21


<210>  134
<211>  21
<212>  DNA
<213>  Homo sapiens

<400>  134
aaugaucuca ccaaggaaat t                                                21


<210>  135
<211>  18
<212>  DNA
<213>  Homo sapiens

<400>  135
aatggatcgg ctatgcgc                                                    18


<210>  136
<211>  18
<212>  DNA
<213>  Homo sapiens

<400>  136
uggaucggcu augcgctt                                                    18


<210>  137
<211>  18
<212>  DNA
<213>  Homo sapiens

<400>  137
gcgcauagcc gauccatt                                                    18


<210>  138
<211>  18
<212>  DNA

<213>  Homo sapiens

<400>  138
aagatgtttg aggactac                                                    18


<210>  139
<211>  18
<212>  DNA
<213>  Homo sapiens

<400>  139
gauguuugag gacuactt                                                    18


<210>  140
<211>  18
<212>  DNA
<213>  Homo sapiens

<400>  140
guaguccuca aacauctt                                                    18


<210>  141
<211>  21
<212>  DNA
<213>  Homo sapiens

<400>  141
aaatctcgga aagcccttat t                                                21


<210>  142
<211>  21
<212>  DNA
<213>  Homo sapiens

<400>  142
aucucggaaa gcccuuauut t                                                21


<210>  143
<211>  21
<212>  DNA
<213>  Homo sapiens

<400>  143
aauaagggcu uuccgagaut t                                                21


<210>  144
<211>  21
<212>  DNA
<213>  Homo sapiens

<400>  144
aaagccctta ttccattcat c                                                21


<210>  145
<211>  21
<212>  DNA
<213>  Homo sapiens

<400>  145
agcccuuauu ccauucauct t                                                21


<210>  146
<211>  21
<212>  DNA
<213>  Homo sapiens

<400>  146
gaugaaugga auaagggcut t                                                21


<210>  147
<211>  2763
<212>  DNA
<213>  Homo sapiens

<400>  147
atggaagtgc agttagggct gggaagggtc taccctcggc cgccgtccaa gacctaccga      60

ggagctttcc agaatctgtt ccagagcgtg cgcgaagtga tccagaaccc gggccccagg     120

cacccagagg ccgcgagcgc agcacctccc ggcgccagtt tgctgctgct gcagcagcag     180

cagcagcagc agcagcagca gcagcagcag cagcagcagc agcagcagca gcagcagcaa     240

gagactagcc ccaggcagca gcagcagcag cagggtgagg atggttctcc ccaagcccat     300

cgtagaggcc ccacaggcta cctggtcctg gatgaggaac agcaaccttc acagccgcag     360

tcggccctgg agtgccaccc cgagagaggt tgcgtcccag agcctggagc cgccgtggcc     420

gccagcaagg ggctgccgca gcagctgcca gcacctccgg acgaggatga ctcagctgcc     480

ccatccacgt tgtccctgct gggccccact ttccccggct taagcagctg ctccgctgac     540

cttaaagaca tcctgagcga ggccagcacc atgcaactcc ttcagcaaca gcagcaggaa     600

gcagtatccg aaggcagcag cagcgggaga gcgagggagg cctcgggggc tcccacttcc     660

tccaaggaca attacttagg gggcacttcg accatttctg acaacgccaa ggagttgtgt     720

aaggcagtgt cggtgtccat gggcctgggt gtggaggcgt tggagcatct gagtccaggg     780

gaacagcttc ggggggattg catgtacgcc ccacttttgg gagttccacc cgctgtgcgt     840

cccactcctt gtgccccatt ggccgaatgc aaaggttctc tgctagacga cagcgcaggc     900

aagagcactg aagatactgc tgagtattcc cctttcaagg gaggttacac caaagggcta     960

gaaggcgaga gcctaggctg ctctggcagc gctgcagcag ggagctccgg gacacttgaa    1020

ctgccgtcta ccctgtctct ctacaagtcc ggagcactgg acgaggcagc tgcgtaccag    1080

agtcgcgact actacaactt tccactggct ctggccggac cgccgccccc tccgccgcct    1140

ccccatcccc acgctcgcat caagctggag aacccgctgg actacggcag cgcctgggcg    1200

gctgcggcgg cgcagtgccg ctatggggac ctggcgagcc tgcatggcgc gggtgcagcg    1260

ggacccggtt ctgggtcacc ctcagccgcc gcttcctcat cctggcacac tctcttcaca    1320

gccgaagaag gccagttgta tggaccgtgt ggtggtggtg ggggtggtgg cggcggcggc    1380

ggcggcggcg gcggcggcgg cggcggcggc ggcggcggcg aggcgggagc tgtagccccc 1440

tacggctaca ctcggccccc tcaggggctg gcgggccagg aaagcgactt caccgcacct 1500

gatgtgtggt accctggcgg catggtgagc agagtgccct atcccagtcc cacttgtgtc 1560

aaaagcgaaa tgggcccctg gatggatagc tactccggac cttacgggga catgcgtttg 1620

gagactgcca gggaccatgt tttgcccatt gactattact ttccacccca gaagacctgc 1680

ctgatctgtg gagatgaagc ttctgggtgt cactatggag ctctcacatg tggaagctgc 1740

aaggtcttct tcaaaagagc cgctgaaggg aaacagaagt acctgtgcgc cagcagaaat 1800

gattgcacta ttgataaatt ccgaaggaaa aattgtccat cttgtcgtct tcggaaatgt 1860

tatgaagcag ggatgactct gggagcccgg aagctgaaga aacttggtaa tctgaaacta 1920

caggaggaag gagaggcttc cagcaccacc agccccactg aggagacaac ccagaagctg 1980

acagtgtcac acattgaagg ctatgaatgt cagcccatct ttctgaatgt cctggaagcc 2040

attgagccag gtgtagtgtg tgctggacac gacaacaacc agcccgactc ctttgcagcc 2100

ttgctctcta gcctcaatga actgggagag agacagcttg tacacgtggt caagtgggcc 2160

aaggccttgc ctggcttccg caacttacac gtggacgacc agatggctgt cattcagtac 2220

tcctggatgg ggctcatggt gtttgccatg ggctggcgat ccttcaccaa tgtcaactcc 2280

aggatgctct acttcgcccc tgatctggtt ttcaatgagt accgcatgca caagtcccgg 2340

atgtacagcc agtgtgtccg aatgaggcac ctctctcaag agtttggatg gctccaaatc 2400

accccccagg aattcctgtg catgaaagca ctgctactct tcagcattat tccagtggat 2460

gggctgaaaa atcaaaaatt ctttgatgaa cttcgaatga actacatcaa ggaactcgat 2520

cgtatcattg catgcaaaag aaaaaatccc acatcctgct caagacgctt ctaccagctc 2580

accaagctcc tggactccgt gcagcctatt gcgagagagc tgcatcagtt cacttttgac 2640

ctgctaatca agtcacacat ggtgagcgtg gactttccgg aaatgatggc agagatcatc 2700

tctgtgcaag tgcccaagat cctttctggg aaagtcaagc ccatctattt ccacacccag 2760

tga 2763

<210> 148
<211> 21
<212> DNA
<213> Homo sapiens

<400> 148
aagtgcagtt agggctggga a 21

<210> 149
<211> 21
<212> DNA
<213> Homo sapiens

<400> 149
gugcaguuag ggcugggaat t 21

<210> 150
<211> 21
<212> DNA
<213> Homo sapiens

<400> 150
uucccagccc uaacugcact t 21


<210> 151
<211> 21
<212> DNA
<213> Homo sapiens

<400> 151
aagggtctac cctcggccgc c 21


<210> 152
<211> 21
<212> DNA
<213> Homo sapiens

<400> 152
gggucuaccc ucggccgcct t 21


<210> 153
<211> 21
<212> DNA
<213> Homo sapiens

<400> 153
ggcggccgag gguagaccct t 21


<210> 154
<211> 19
<212> DNA
<213> Homo sapiens

<400> 154
aagacctacc gaggagctt 19


<210> 155
<211> 19
<212> DNA
<213> Homo sapiens

<400> 155
gaccuaccga ggagcuutt 19


<210> 156
<211> 19
<212> DNA
<213> Homo sapiens

<400> 156
aagcuccucg guagguctt 19

<210>  157
<211>  21
<212>  DNA
<213>  Homo sapiens

<400>  157
aatctgttcc agagcgtgcg c                                                21


<210>  158
<211>  21
<212>  DNA
<213>  Homo sapiens

<400>  158
ucuguuccag agcgugcgct t                                                21


<210>  159
<211>  21
<212>  DNA
<213>  Homo sapiens

<400>  159
gcgcacgcuc uggaacagat t                                                21


<210>  160
<211>  21
<212>  DNA
<213>  Homo sapiens

<400>  160
aagtgatcca gaacccgggc c                                                21


<210>  161
<211>  21
<212>  DNA
<213>  Homo sapiens

<400>  161
gugauccaga acccgggcct t                                                21


<210>  162
<211>  21
<212>  DNA
<213>  Homo sapiens

<400>  162
ggcccggguu cuggaucact t                                                21


<210>  163
<211>  18
<212>  DNA
<213>  Homo sapiens

<400>  163
aacccgggcc ccaggcac                                                    18


<210>  164
<211>  18

<212>  DNA
<213>  Homo sapiens

<400>  164
cccgggcccc aggcactt                                                    18


<210>  165
<211>  18
<212>  DNA
<213>  Homo sapiens

<400>  165
gugccugggg cccgggtt                                                    18


<210>  166
<211>  18
<212>  DNA
<213>  Homo sapiens

<400>  166
aagagactag ccccaggc                                                    18


<210>  167
<211>  18
<212>  DNA
<213>  Homo sapiens

<400>  167
gagacuagcc ccaggctt                                                    18


<210>  168
<211>  18
<212>  DNA
<213>  Homo sapiens

<400>  168
gccuggggcu agucuctt                                                    18


<210>  169
<211>  18
<212>  DNA
<213>  Homo sapiens

<400>  169
aagcccatcg tagaggcc                                                    18


<210>  170
<211>  18
<212>  DNA
<213>  Homo sapiens

<400>  170
gcccaucgua gaggcctt                                                    18


<210>  171
<211>  18
<212>  DNA
<213>  Homo sapiens

<400>  171
ggccucuacg augggctt                                                    18


<210>  172
<211>  21
<212>  DNA
<213>  Homo sapiens

<400>  172
aacagcaacc ttcacagccg c                                                21


<210>  173
<211>  21
<212>  DNA
<213>  Homo sapiens

<400>  173
cagcaaccuu cacagccgct t                                                21


<210>  174
<211>  21
<212>  DNA
<213>  Homo sapiens

<400>  174
gcggcuguga agguugcugt t                                                21


<210>  175
<211>  18
<212>  DNA
<213>  Homo sapiens

<400>  175
aaccttcaca gccgcagt                                                    18


<210>  176
<211>  18
<212>  DNA
<213>  Homo sapiens

<400>  176
ccuucacagc cgcagutt                                                    18


<210>  177
<211>  18
<212>  DNA
<213>  Homo sapiens

<400>  177
acugcggcug ugaaggtt                                                    18


<210>  178
<211>  21
<212>  DNA
<213>  Homo sapiens

<400>  178

aaggggctgc cgcagcagct g                                                21

<210>  179
<211>  21
<212>  DNA
<213>  Homo sapiens

<400>  179
ggggcugccg cagcagcugt t                                                21

<210>  180
<211>  21
<212>  DNA
<213>  Homo sapiens

<400>  180
cagcugcugc ggcagcccct t                                                21

<210>  181
<211>  19
<212>  DNA
<213>  Homo sapiens

<400>  181
aagcagctgc tccgctgac                                                   19

<210>  182
<211>  19
<212>  DNA
<213>  Homo sapiens

<400>  182
gcagcugcuc cgcugactt                                                   19

<210>  183
<211>  19
<212>  DNA
<213>  Homo sapiens

<400>  183
gucagcggag cagcugctt                                                   19

<210>  184
<211>  21
<212>  DNA
<213>  Homo sapiens

<400>  184
aaagacatcc tgagcgaggc c                                                21

<210>  185
<211>  21
<212>  DNA
<213>  Homo sapiens

<400>  185
agacauccug agcgaggcct t                                                21

<210>  186
<211>  21
<212>  DNA
<213>  Homo sapiens

<400>  186
ggccucgcuc aggaugucut t                                                21


<210>  187
<211>  21
<212>  DNA
<213>  Homo sapiens

<400>  187
aactccttca gcaacagcag c                                                21


<210>  188
<211>  21
<212>  DNA
<213>  Homo sapiens

<400>  188
cuccuucagc aacagcagct t                                                21


<210>  189
<211>  21
<212>  DNA
<213>  Homo sapiens

<400>  189
gcugcuguug cugaaggagt t                                                21


<210>  190
<211>  18
<212>  DNA
<213>  Homo sapiens

<400>  190
aacagcagca ggaagcag                                                    18


<210>  191
<211>  18
<212>  DNA
<213>  Homo sapiens

<400>  191
cagcagcagg aagcagtt                                                    18


<210>  192
<211>  18
<212>  DNA
<213>  Homo sapiens

<400>  192
cugcuuccug cugcugtt                                                    18


<210>  193

<211>  18
<212>  DNA
<213>  Homo sapiens

<400>  193
aagcagtatc cgaaggca                                                    18


<210>  194
<211>  18
<212>  DNA
<213>  Homo sapiens

<400>  194
gcaguauccg aaggcatt                                                    18


<210>  195
<211>  18
<212>  DNA
<213>  Homo sapiens

<400>  195
ugccuucgga uacugctt                                                    18


<210>  196
<211>  21
<212>  DNA
<213>  Homo sapiens

<400>  196
aaggcagcag cagcgggaga g                                                21


<210>  197
<211>  21
<212>  DNA
<213>  Homo sapiens

<400>  197
ggcagcagca gcgggagagt t                                                21


<210>  198
<211>  21
<212>  DNA
<213>  Homo sapiens

<400>  198
cucucccgcu gcugcugcct t                                                21


<210>  199
<211>  21
<212>  DNA
<213>  Homo sapiens

<400>  199
aaggacaatt acttaggggg c                                                21


<210>  200
<211>  21
<212>  DNA

<213>  Homo sapiens

<400>  200
ggacaauuac uuaggggct t                                                21


<210>  201
<211>  21
<212>  DNA
<213>  Homo sapiens

<400>  201
gcccccuaag uaauugucct t                                               21


<210>  202
<211>  21
<212>  DNA
<213>  Homo sapiens

<400>  202
aattacttag ggggcacttc g                                               21


<210>  203
<211>  21
<212>  DNA
<213>  Homo sapiens

<400>  203
uuacuuaggg ggcacuucgt t                                               21


<210>  204
<211>  21
<212>  DNA
<213>  Homo sapiens

<400>  204
cgaagugccc ccuaaguaat t                                               21


<210>  205
<211>  18
<212>  DNA
<213>  Homo sapiens

<400>  205
aacgccaagg agttgtgt                                                   18


<210>  206
<211>  18
<212>  DNA
<213>  Homo sapiens

<400>  206
cgccaaggag uugugutt                                                   18


<210>  207
<211>  18
<212>  DNA
<213>  Homo sapiens

<400>  207
acacaacucc uuggcgtt                                                    18


<210>  208
<211>  18
<212>  DNA
<213>  Homo sapiens

<400>  208
aaggagttgt gtaaggca                                                    18


<210>  209
<211>  18
<212>  DNA
<213>  Homo sapiens

<400>  209
ggaguugugu aaggcatt                                                    18


<210>  210
<211>  18
<212>  DNA
<213>  Homo sapiens

<400>  210
ugccuuacac aacucctt                                                    18


<210>  211
<211>  21
<212>  DNA
<213>  Homo sapiens

<400>  211
aaggcagtgt cggtgtccat g                                                21


<210>  212
<211>  21
<212>  DNA
<213>  Homo sapiens

<400>  212
ggcagugucg guguccaugt t                                                21


<210>  213
<211>  21
<212>  DNA
<213>  Homo sapiens

<400>  213
cauggacacc gacacugcct t                                                21


<210>  214
<211>  21
<212>  DNA
<213>  Homo sapiens

<400>  214
aacagcttcg gggggattgc a                                                21

<210>  215
<211>  21
<212>  DNA
<213>  Homo sapiens

<400>  215
cagcuucggg gggauugcat t                                                 21


<210>  216
<211>  21
<212>  DNA
<213>  Homo sapiens

<400>  216
ugcaaucccc ccgaagcugt t                                                 21


<210>  217
<211>  21
<212>  DNA
<213>  Homo sapiens

<400>  217
aatgcaaagg ttctctgcta g                                                 21


<210>  218
<211>  21
<212>  DNA
<213>  Homo sapiens

<400>  218
ugcaaagguu cucugcuagt t                                                 21


<210>  219
<211>  21
<212>  DNA
<213>  Homo sapiens

<400>  219
cuagcagaga accuuugcat t                                                 21


<210>  220
<211>  21
<212>  DNA
<213>  Homo sapiens

<400>  220
aaaggttctc tgctagacga c                                                 21


<210>  221
<211>  21
<212>  DNA
<213>  Homo sapiens

<400>  221
agguucucug cuagacgact t                                                 21

<210>  222
<211>  21
<212>  DNA
<213>  Homo sapiens

<400>  222
gucgucuagc agagaaccut t                                                21


<210>  223
<211>  21
<212>  DNA
<213>  Homo sapiens

<400>  223
aagagcactg aagatactgc t                                                21


<210>  224
<211>  21
<212>  DNA
<213>  Homo sapiens

<400>  224
gagcacugaa gauacugcut t                                                21


<210>  225
<211>  21
<212>  DNA
<213>  Homo sapiens

<400>  225
agcaguaucu ucagugcuct t                                                21


<210>  226
<211>  21
<212>  DNA
<213>  Homo sapiens

<400>  226
aagatactgc tgagtattcc c                                                21


<210>  227
<211>  21
<212>  DNA
<213>  Homo sapiens

<400>  227
gauacugcug aguauuccct t                                                21


<210>  228
<211>  21
<212>  DNA
<213>  Homo sapiens

<400>  228
gggaauacuc agcaguauct t                                                21


<210>  229
<211>  21

<212>  DNA
<213>  Homo sapiens

<400>  229
aagggaggtt acaccaaagg g                                                21


<210>  230
<211>  21
<212>  DNA
<213>  Homo sapiens

<400>  230
gggagguuac accaaagggt t                                                21


<210>  231
<211>  21
<212>  DNA
<213>  Homo sapiens

<400>  231
cccuuuggug uaaccuccct t                                                21


<210>  232
<211>  18
<212>  DNA
<213>  Homo sapiens

<400>  232
aaagggctag aaggcgag                                                    18


<210>  233
<211>  18
<212>  DNA
<213>  Homo sapiens

<400>  233
agggcuagaa ggcgagtt                                                    18


<210>  234
<211>  18
<212>  DNA
<213>  Homo sapiens

<400>  234
cucgccuucu agcccutt                                                    18


<210>  235
<211>  21
<212>  DNA
<213>  Homo sapiens

<400>  235
aaggcgagag cctaggctgc t                                                21


<210>  236
<211>  21
<212>  DNA
<213>  Homo sapiens

<400>  236
ggcgagagcc uaggcugcut t                                                21

<210>  237
<211>  21
<212>  DNA
<213>  Homo sapiens

<400>  237
agcagccuag gcucucgcct t                                                21

<210>  238
<211>  17
<212>  DNA
<213>  Homo sapiens

<400>  238
aactgccgtc taccctg                                                     17

<210>  239
<211>  17
<212>  DNA
<213>  Homo sapiens

<400>  239
cugccgucua cccugtt                                                     17

<210>  240
<211>  17
<212>  DNA
<213>  Homo sapiens

<400>  240
   caggguagac ggcagtt                                                    17

<210>  241
<211>  21
<212>  DNA
<213>  Homo sapiens

<400>  241
aagtccggag cactggacga g                                                21

<210>  242
<211>  21
<212>  DNA
<213>  Homo sapiens

<400>  242
guccggagca cuggacgagt t                                                21

<210>  243
<211>  21
<212>  DNA
<213>  Homo sapiens

<400>  243

cucguccagu gcuccggact t                                                21

<210>  244
<211>  21
<212>  DNA
<213>  Homo sapiens

<400>  244
aactttccac tggctctggc c                                                21

<210>  245
<211>  21
<212>  DNA
<213>  Homo sapiens

<400>  245
cuuuccacug gcucuggcct t                                                21

<210>  246
<211>  21
<212>  DNA
<213>  Homo sapiens

<400>  246
ggccagagcc aguggaaagt t                                                21

<210>  247
<211>  21
<212>  DNA
<213>  Homo sapiens

<400>  247
aagctggaga acccgctgga c                                                21

<210>  248
<211>  21
<212>  DNA
<213>  Homo sapiens

<400>  248
gcuggagaac ccgcuggact t                                                21

<210>  249
<211>  21
<212>  DNA
<213>  Homo sapiens

<400>  249
guccagcggg uucuccagct t                                                21

<210>  250
<211>  21
<212>  DNA
<213>  Homo sapiens

<400>  250
aacccgctgg actacggcag c                                                21

<210>  251
<211>  21
<212>  DNA
<213>  Homo sapiens

<400>  251
cccgcuggac uacggcagct t                                                21


<210>  252
<211>  21
<212>  DNA
<213>  Homo sapiens

<400>  252
gcugccguag uccagcgggt t                                                21


<210>  253
<211>  21
<212>  DNA
<213>  Homo sapiens

<400>  253
aagaaggcca gttgtatgga c                                                21


<210>  254
<211>  21
<212>  DNA
<213>  Homo sapiens

<400>  254
gaaggccagu uguauggact t                                                21


<210>  255
<211>  21
<212>  DNA
<213>  Homo sapiens

<400>  255
guccauacaa cuggccuuct t                                                21


<210>  256
<211>  21
<212>  DNA
<213>  Homo sapiens

<400>  256
aaggccagtt gtatggaccg t                                                21


<210>  257
<211>  21
<212>  DNA
<213>  Homo sapiens

<400>  257
ggccaguugu auggaccgut t                                                21


<210>  258

<211>  21
<212>  DNA
<213>  Homo sapiens

<400>  258
acgguccaua caacuggcct t                                                21


<210>  259
<211>  20
<212>  DNA
<213>  Homo sapiens

<400>  259
aaagcgactt caccgcacct                                                  20


<210>  260
<211>  20
<212>  DNA
<213>  Homo sapiens

<400>  260
agcgacuuca ccgcaccutt                                                  20


<210>  261
<211>  20
<212>  DNA
<213>  Homo sapiens

<400>  261
aggugcggug aagucgcutt                                                  20


<210>  262
<211>  21
<212>  DNA
<213>  Homo sapiens

<400>  262
aaaagcgaaa tgggcccctg g                                                21


<210>  263
<211>  21
<212>  DNA
<213>  Homo sapiens

<400>  263
aagcgaaaug ggccccuggt t                                                21


<210>  264
<211>  21
<212>  DNA
<213>  Homo sapiens

<400>  264
ccaggggccc auuucgcuut t                                                21


<210>  265
<211>  21
<212>  DNA

<213>  Homo sapiens

<400>  265
aagcgaaatg ggcccctgga t                                                21


<210>  266
<211>  21
<212>  DNA
<213>  Homo sapiens

<400>  266
gcgaaauggg ccccuggaut t                                                21


<210>  267
<211>  21
<212>  DNA
<213>  Homo sapiens

<400>  267
auccaggggc ccauuucgct t                                                21


<210>  268
<211>  21
<212>  DNA
<213>  Homo sapiens

<400>  268
aaatgggccc ctggatggat a                                                21


<210>  269
<211>  21
<212>  DNA
<213>  Homo sapiens

<400>  269
augggccccu ggauggauat t                                                21


<210>  270
<211>  21
<212>  DNA
<213>  Homo sapiens

<400>  270
uauccaucca ggggcccaut t                                                21


<210>  271
<211>  17
<212>  DNA
<213>  Homo sapiens

<400>  271
aagacctgcc tgatctg                                                     17


<210>  272
<211>  17
<212>  DNA
<213>  Homo sapiens

<400>  272
gaccugccug aucugtt                                                    17


<210>  273
<211>  17
<212>  DNA
<213>  Homo sapiens

<400>  273
cagaucaggc agguctt                                                    17


<210>  274
<211>  21
<212>  DNA
<213>  Homo sapiens

<400>  274
aagcttctgg gtgtcactat g                                               21


<210>  275
<211>  21
<212>  DNA
<213>  Homo sapiens

<400>  275
gcuucugggu gucacuaugt t                                               21


<210>  276
<211>  21
<212>  DNA
<213>  Homo sapiens

<400>  276
cauagugaca cccagaagct t                                               21


<210>  277
<211>  17
<212>  DNA
<213>  Homo sapiens

<400>  277
aagctgcaag gtcttct                                                    17


<210>  278
<211>  17
<212>  DNA
<213>  Homo sapiens

<400>  278
gcugcaaggu cuucutt                                                    17


<210>  279
<211>  17
<212>  DNA
<213>  Homo sapiens

<400>  279
agaagaccuu gcagctt                                                    17

<210>  280
<211>  21
<212>  DNA
<213>  Homo sapiens

<400>  280
aaggtcttct tcaaaagagc c                                                 21


<210>  281
<211>  21
<212>  DNA
<213>  Homo sapiens

<400>  281
ggucuucuuc aaaagagcct t                                                 21


<210>  282
<211>  21
<212>  DNA
<213>  Homo sapiens

<400>  282
ggcucuuuug aagaagacct t                                                 21


<210>  283
<211>  21
<212>  DNA
<213>  Homo sapiens

<400>  283
aaaagagccg ctgaagggaa a                                                 21


<210>  284
<211>  21
<212>  DNA
<213>  Homo sapiens

<400>  284
aagagccgcu gaagggaaat t                                                 21


<210>  285
<211>  21
<212>  DNA
<213>  Homo sapiens

<400>  285
uuucccuuca gcggcucuut t                                                 21


<210>  286
<211>  21
<212>  DNA
<213>  Homo sapiens

<400>  286
aagagccgct gaagggaaac a                                                 21

<210>  287
<211>  21
<212>  DNA
<213>  Homo sapiens

<400>  287
gagccgcuga agggaaacat t                                                21


<210>  288
<211>  21
<212>  DNA
<213>  Homo sapiens

<400>  288
uguuucccuu cagcggcuct t                                                21


<210>  289
<211>  21
<212>  DNA
<213>  Homo sapiens

<400>  289
aagggaaaca gaagtacctg t                                                21


<210>  290
<211>  21
<212>  DNA
<213>  Homo sapiens

<400>  290
gggaaacaga aguaccugut t                                                21


<210>  291
<211>  21
<212>  DNA
<213>  Homo sapiens

<400>  291
acagguacuu cuguuuccct t                                                21


<210>  292
<211>  21
<212>  DNA
<213>  Homo sapiens

<400>  292
aaacagaagt acctgtgcgc c                                                21


<210>  293
<211>  21
<212>  DNA
<213>  Homo sapiens

<400>  293
acagaaguac cugugcgcct t                                                21


<210>  294
<211>  21

<212>  DNA
<213>  Homo sapiens

<400>  294
ggcgcacagg uacuucugut t                                                21


<210>  295
<211>  21
<212>  DNA
<213>  Homo sapiens

<400>  295
aagtacctgt gcgccagcag a                                                21


<210>  296
<211>  21
<212>  DNA
<213>  Homo sapiens

<400>  296
guaccugugc gccagcagat t                                                21


<210>  297
<211>  21
<212>  DNA
<213>  Homo sapiens

<400>  297
ucugcuggcg cacagguact t                                                21


<210>  298
<211>  17
<212>  DNA
<213>  Homo sapiens

<400>  298
aaatgattgc actattg                                                     17


<210>  299
<211>  17
<212>  DNA
<213>  Homo sapiens

<400>  299
augauugcac uauugtt                                                     17


<210>  300
<211>  17
<212>  DNA
<213>  Homo sapiens

<400>  300
caauagugca aucautt                                                     17


<210>  301
<211>  21
<212>  DNA
<213>  Homo sapiens

<400>  301
aaattccgaa ggaaaaattg t                                                21

<210>  302
<211>  21
<212>  DNA
<213>  Homo sapiens

<400>  302
auuccgaagg aaaaauugut t                                                21

<210>  303
<211>  21
<212>  DNA
<213>  Homo sapiens

<400>  303
acaauuuuuc cuucggaaut t                                                21

<210>  304
<211>  21
<212>  DNA
<213>  Homo sapiens

<400>  304
aaggaaaaat tgtccatctt g                                                21

<210>  305
<211>  21
<212>  DNA
<213>  Homo sapiens

<400>  305
ggaaaaauug uccaucuugt t                                                21

<210>  306
<211>  21
<212>  DNA
<213>  Homo sapiens

<400>  306
caagauggac aauuuuucct t                                                21

<210>  307
<211>  21
<212>  DNA
<213>  Homo sapiens

<400>  307
aaaaattgtc catcttgtcg t                                                21

<210>  308
<211>  21
<212>  DNA
<213>  Homo sapiens

<400>  308

aaauugucca ucuugucgut t                                                21

<210>  309
<211>  21
<212>  DNA
<213>  Homo sapiens

<400>  309
acgacaagau ggacaauuut t                                                21

<210>  310
<211>  21
<212>  DNA
<213>  Homo sapiens

<400>  310
aaattgtcca tcttgtcgtc t                                                21

<210>  311
<211>  21
<212>  DNA
<213>  Homo sapiens

<400>  311
auuguccauc uugucgucut t                                                21

<210>  312
<211>  21
<212>  DNA
<213>  Homo sapiens

<400>  312
agacgacaag auggacaaut t                                                21

<210>  313
<211>  17
<212>  DNA
<213>  Homo sapiens

<400>  313
aaatgttatg aagcagg                                                     17

<210>  314
<211>  17
<212>  DNA
<213>  Homo sapiens

<400>  314
auguuaugaa gcaggtt                                                     17

<210>  315
<211>  17
<212>  DNA
<213>  Homo sapiens

<400>  315
ccugcuucau aacautt                                                     17

<210>  316
<211>  21
<212>  DNA
<213>  Homo sapiens

<400>  316
aagcagggat gactctggga g                                                21


<210>  317
<211>  21
<212>  DNA
<213>  Homo sapiens

<400>  317
gcagggauga cucugggagt t                                                21


<210>  318
<211>  21
<212>  DNA
<213>  Homo sapiens

<400>  318
cucccagagu caucccugct t                                                21


<210>  319
<211>  21
<212>  DNA
<213>  Homo sapiens

<400>  319
aagctgaaga aacttggtaa t                                                21


<210>  320
<211>  21
<212>  DNA
<213>  Homo sapiens

<400>  320
gcugaagaaa cuugguaaut t                                                21


<210>  321
<211>  21
<212>  DNA
<213>  Homo sapiens

<400>  321
auuaccaagu uucuucagct t                                                21


<210>  322
<211>  21
<212>  DNA
<213>  Homo sapiens

<400>  322
aagaaacttg gtaatctgaa a                                                21


<210>  323

<211>  21
<212>  DNA
<213>  Homo sapiens

<400>  323
gaaacuuggu aaucugaaat t                                                21


<210>  324
<211>  21
<212>  DNA
<213>  Homo sapiens

<400>  324
uuucagauua ccaaguuuct t                                                21


<210>  325
<211>  21
<212>  DNA
<213>  Homo sapiens

<400>  325
aaacttggta atctgaaact a                                                21


<210>  326
<211>  21
<212>  DNA
<213>  Homo sapiens

<400>  326
acuugguaau cugaaacuat t                                                21


<210>  327
<211>  21
<212>  DNA
<213>  Homo sapiens

<400>  327
uaguuucaga uuaccaagut t                                                21


<210>  328
<211>  17
<212>  DNA
<213>  Homo sapiens

<400>  328
aatctgaaac tacagga                                                     17


<210>  329
<211>  17
<212>  DNA
<213>  Homo sapiens

<400>  329
ucugaaacua caggatt                                                     17


<210>  330
<211>  17
<212>  DNA

<213>  Homo sapiens

<400>  330
uccuguaguu ucagatt                                                    17


<210>  331
<211>  17
<212>  DNA
<213>  Homo sapiens

<400>  331
aaactacagg aggaagg                                                    17


<210>  332
<211>  17
<212>  DNA
<213>  Homo sapiens

<400>  332
acuacaggag gaaggtt                                                    17


<210>  333
<211>  17
<212>  DNA
<213>  Homo sapiens

<400>  333
ccuuccuccu guagutt                                                    17


<210>  334
<211>  21
<212>  DNA
<213>  Homo sapiens

<400>  334
aaggagaggc ttccagcacc a                                               21


<210>  335
<211>  21
<212>  DNA
<213>  Homo sapiens

<400>  335
ggagaggcuu ccagcaccat t                                               21


<210>  336
<211>  21
<212>  DNA
<213>  Homo sapiens

<400>  336
uggugcugga agccucucct t                                               21


<210>  337
<211>  17
<212>  DNA
<213>  Homo sapiens

<400>  337
aacccagaag ctgacag                                                    17


<210>  338
<211>  17
<212>  DNA
<213>  Homo sapiens

<400>  338
cccagaagcu gacagtt                                                    17


<210>  339
<211>  17
<212>  DNA
<213>  Homo sapiens

<400>  339
cugucagcuu cugggtt                                                    17


<210>  340
<211>  17
<212>  DNA
<213>  Homo sapiens

<400>  340
aagctgacag tgtcaca                                                    17


<210>  341
<211>  17
<212>  DNA
<213>  Homo sapiens

<400>  341
gcugacagug ucacatt                                                    17


<210>  342
<211>  17
<212>  DNA
<213>  Homo sapiens

<400>  342
ugugacacug ucagctt                                                    17


<210>  343
<211>  21
<212>  DNA
<213>  Homo sapiens

<400>  343
aaggctatga atgtcagccc a                                               21


<210>  344
<211>  21
<212>  DNA
<213>  Homo sapiens

<400>  344
ggcuaugaau gucagcccat t                                               21

<210>  345
<211>  21
<212>  DNA
<213>  Homo sapiens

<400>  345
ugggcugaca uucauagcct t                                                21


<210>  346
<211>  21
<212>  DNA
<213>  Homo sapiens

<400>  346
aatgtcagcc catctttctg a                                                21


<210>  347
<211>  21
<212>  DNA
<213>  Homo sapiens

<400>  347
ugucagccca ucuuucugat t                                                21


<210>  348
<211>  21
<212>  DNA
<213>  Homo sapiens

<400>  348
ucagaaagau gggcugacat t                                                21


<210>  349
<211>  17
<212>  DNA
<213>  Homo sapiens

<400>  349
aatgtcctgg aagccat                                                     17


<210>  350
<211>  17
<212>  DNA
<213>  Homo sapiens

<400>  350
uguccuggaa gccautt                                                     17


<210>  351
<211>  17
<212>  DNA
<213>  Homo sapiens

<400>  351
auggcuucca ggacatt                                                     17

<210>  352
<211>  17
<212>  DNA
<213>  Homo sapiens

<400>  352
aagccattga gccaggt                                                    17


<210>  353
<211>  17
<212>  DNA
<213>  Homo sapiens

<400>  353
gccauugagc caggutt                                                    17


<210>  354
<211>  17
<212>  DNA
<213>  Homo sapiens

<400>  354
accuggcuca auggctt                                                    17


<210>  355
<211>  21
<212>  DNA
<213>  Homo sapiens

<400>  355
aacaaccagc ccgactcctt t                                               21


<210>  356
<211>  21
<212>  DNA
<213>  Homo sapiens

<400>  356
caaccagccc gacuccuuut t                                               21


<210>  357
<211>  21
<212>  DNA
<213>  Homo sapiens

<400>  357
aaaggagucg ggcugguugt t                                               21


<210>  358
<211>  21
<212>  DNA
<213>  Homo sapiens

<400>  358
aaccagcccg actcctttgc a                                               21


<210>  359
<211>  21

<212>  DNA
<213>  Homo sapiens

<400>  359
ccagcccgac uccuuugcat t                                                21


<210>  360
<211>  21
<212>  DNA
<213>  Homo sapiens

<400>  360
ugcaaaggag ucgggcuggt t                                                21


<210>  361
<211>  21
<212>  DNA
<213>  Homo sapiens

<400>  361
aatgaactgg gagagagaca g                                                21


<210>  362
<211>  21
<212>  DNA
<213>  Homo sapiens

<400>  362
ugaacuggga gagagacagt t                                                21


<210>  363
<211>  21
<212>  DNA
<213>  Homo sapiens

<400>  363
cugucucucu cccaguucat t                                                21


<210>  364
<211>  21
<212>  DNA
<213>  Homo sapiens

<400>  364
aactgggaga gagacagctt g                                                21


<210>  365
<211>  21
<212>  DNA
<213>  Homo sapiens

<400>  365
cugggagaga gacagcuugt t                                                21


<210>  366
<211>  21
<212>  DNA
<213>  Homo sapiens

<400>  366
caagcugucu cucucccagt t                                                21

<210>  367
<211>  17
<212>  DNA
<213>  Homo sapiens

<400>  367
aagtgggcca aggcctt                                                     17

<210>  368
<211>  17
<212>  DNA
<213>  Homo sapiens

<400>  368
gugggccaag gccuutt                                                     17

<210>  369
<211>  17
<212>  DNA
<213>  Homo sapiens

<400>  369
aaggccuugg cccactt                                                     17

<210>  370
<211>  21
<212>  DNA
<213>  Homo sapiens

<400>  370
aaggccttgc ctggcttccg c                                                21

<210>  371
<211>  21
<212>  DNA
<213>  Homo sapiens

<400>  371
ggccuugccu ggcuuccgct t                                                21

<210>  372
<211>  21
<212>  DNA
<213>  Homo sapiens

<400>  372
gcggaagcca ggcaaggcct t                                                21

<210>  373
<211>  21
<212>  DNA
<213>  Homo sapiens

<400>  373

aacttacacg tggacgacca g                                                21

<210>  374
<211>  21
<212>  DNA
<213>  Homo sapiens

<400>  374
cuuacacgug gacgaccagt t                                                21

<210>  375
<211>  21
<212>  DNA
<213>  Homo sapiens

<400>  375
cuggucgucc acguguaagt t                                                21

<210>  376
<211>  17
<212>  DNA
<213>  Homo sapiens

<400>  376
aatgtcaact ccaggat                                                     17

<210>  377
<211>  17
<212>  DNA
<213>  Homo sapiens

<400>  377
ugucaacucc aggautt                                                     17

<210>  378
<211>  17
<212>  DNA
<213>  Homo sapiens

<400>  378
auccuggagu ugacatt                                                     17

<210>  379
<211>  17
<212>  DNA
<213>  Homo sapiens

<400>  379
aactccagga tgctcta                                                     17

<210>  380
<211>  17
<212>  DNA
<213>  Homo sapiens

<400>  380
cuccaggaug cucuatt                                                     17

<210>  381
<211>  17
<212>  DNA
<213>  Homo sapiens

<400>  381
uagagcaucc uggagtt                                                    17


<210>  382
<211>  21
<212>  DNA
<213>  Homo sapiens

<400>  382
aatgagtacc gcatgcacaa g                                               21


<210>  383
<211>  21
<212>  DNA
<213>  Homo sapiens

<400>  383
ugaguaccgc augcacaagt t                                               21


<210>  384
<211>  21
<212>  DNA
<213>  Homo sapiens

<400>  384
cuugugcaug cgguacucat t                                               21


<210>  385
<211>  17
<212>  DNA
<213>  Homo sapiens

<400>  385
aagtcccgga tgtacag                                                    17


<210>  386
<211>  17
<212>  DNA
<213>  Homo sapiens

<400>  386
gucccggaug uacagtt                                                    17


<210>  387
<211>  17
<212>  DNA
<213>  Homo sapiens

<400>  387
cuguacaucc gggactt                                                    17


<210>  388

<211>  21
<212>  DNA
<213>  Homo sapiens

<400>  388
aatgaggcac ctctctcaag a                                                21


<210>  389
<211>  21
<212>  DNA
<213>  Homo sapiens

<400>  389
ugaggcaccu cucucaagat t                                                21


<210>  390
<211>  21
<212>  DNA
<213>  Homo sapiens

<400>  390
ucuugagaga ggugccucat t                                                21


<210>  391
<211>  21
<212>  DNA
<213>  Homo sapiens

<400>  391
aagagtttgg atggctccaa a                                                21


<210>  392
<211>  21
<212>  DNA
<213>  Homo sapiens

<400>  392
gaguuuggau ggcuccaaat t                                                21


<210>  393
<211>  21
<212>  DNA
<213>  Homo sapiens

<400>  393
uuuggagcca uccaaacuct t                                                21


<210>  394
<211>  17
<212>  DNA
<213>  Homo sapiens

<400>  394
aaatcacccc ccaggaa                                                     17


<210>  395
<211>  17
<212>  DNA

<213>  Homo sapiens

<400>  395
   aucacccccc aggaatt                                                        17


<210>  396
<211>  17
<212>  DNA
<213>  Homo sapiens

<400>  396
uuccuggggg gugautt                                                       17


<210>  397
<211>  21
<212>  DNA
<213>  Homo sapiens

<400>  397
aattcctgtg catgaaagca c                                                  21


<210>  398
<211>  21
<212>  DNA
<213>  Homo sapiens

<400>  398
uuccugugca ugaaagcact t                                                  21


<210>  399
<211>  21
<212>  DNA
<213>  Homo sapiens

<400>  399
gugcuuucau gcacaggaat t                                                  21


<210>  400
<211>  21
<212>  DNA
<213>  Homo sapiens

<400>  400
aaagcactgc tactcttcag c                                                  21


<210>  401
<211>  21
<212>  DNA
<213>  Homo sapiens

<400>  401
agcacugcua cucuucagct t                                                  21


<210>  402
<211>  21
<212>  DNA
<213>  Homo sapiens

<400>  402
gcugaagagu agcagugcut t                                                21


<210>  403
<211>  21
<212>  DNA
<213>  Homo sapiens

<400>  403
aaaaatcaaa aattctttga t                                                21


<210>  404
<211>  21
<212>  DNA
<213>  Homo sapiens

<400>  404
aaaucaaaaa uucuuugaut t                                                21


<210>  405
<211>  21
<212>  DNA
<213>  Homo sapiens

<400>  405
aucaaagaau uuuugauuut t                                                21


<210>  406
<211>  21
<212>  DNA
<213>  Homo sapiens

<400>  406
aaatcaaaaa ttctttgatg a                                                21


<210>  407
<211>  21
<212>  DNA
<213>  Homo sapiens

<400>  407
aucaaaaauu cuuugaugat t                                                21


<210>  408
<211>  21
<212>  DNA
<213>  Homo sapiens

<400>  408
ucaucaaaga auuuuugaut t                                                21


<210>  409
<211>  21
<212>  DNA
<213>  Homo sapiens

<400>  409
aaaaattctt tgatgaactt c                                                21

<210>  410
<211>  21
<212>  DNA
<213>  Homo sapiens

<400>  410
aaauucuuug augaacuuct t                                                21


<210>  411
<211>  21
<212>  DNA
<213>  Homo sapiens

<400>  411
gaaguucauc aaagaauuut t                                                21


<210>  412
<211>  21
<212>  DNA
<213>  Homo sapiens

<400>  412
aaattctttg atgaacttcg a                                                21


<210>  413
<211>  21
<212>  DNA
<213>  Homo sapiens

<400>  413
auucuuugau gaacuucgat t                                                21


<210>  414
<211>  21
<212>  DNA
<213>  Homo sapiens

<400>  414
ucgaaguuca ucaaagaaut t                                                21


<210>  415
<211>  21
<212>  DNA
<213>  Homo sapiens

<400>  415
aacttcgaat gaactacatc a                                                21


<210>  416
<211>  21
<212>  DNA
<213>  Homo sapiens

<400>  416
cuucgaauga acuacaucat t                                                21

<210>  417
<211>  21
<212>  DNA
<213>  Homo sapiens

<400>  417
ugauguaguu cauucgaagt t                                                21


<210>  418
<211>  21
<212>  DNA
<213>  Homo sapiens

<400>  418
aatgaactac atcaaggaac t                                                21


<210>  419
<211>  21
<212>  DNA
<213>  Homo sapiens

<400>  419
ugaacuacau caaggaacut t                                                21


<210>  420
<211>  21
<212>  DNA
<213>  Homo sapiens

<400>  420
aguuccuuga uguaguucat t                                                21


<210>  421
<211>  21
<212>  DNA
<213>  Homo sapiens

<400>  421
aactacatca aggaactcga t                                                21


<210>  422
<211>  21
<212>  DNA
<213>  Homo sapiens

<400>  422
cuacaucaag gaacucgaut t                                                21


<210>  423
<211>  21
<212>  DNA
<213>  Homo sapiens

<400>  423
aucgaguucc uugauguagt t                                                21


<210>  424
<211>  17

<212>  DNA
<213>  Homo sapiens

<400>  424
aaggaactcg atcgtat                                                    17


<210>  425
<211>  17
<212>  DNA
<213>  Homo sapiens

<400>  425
ggaacucgau cguautt                                                    17


<210>  426
<211>  17
<212>  DNA
<213>  Homo sapiens

<400>  426
auacgaucga guucctt                                                    17


<210>  427
<211>  17
<212>  DNA
<213>  Homo sapiens

<400>  427
aactcgatcg tatcatt                                                    17


<210>  428
<211>  17
<212>  DNA
<213>  Homo sapiens

<400>  428
cucgaucgua ucauutt                                                    17


<210>  429
<211>  17
<212>  DNA
<213>  Homo sapiens

<400>  429
aaugauacga ucgagtt                                                    17


<210>  430
<211>  21
<212>  DNA
<213>  Homo sapiens

<400>  430
aaaagaaaaa atcccacatc c                                               21


<210>  431
<211>  21
<212>  DNA
<213>  Homo sapiens

<400>  431
aagaaaaaau cccacauccu t                                                21


<210>  432
<211>  21
<212>  DNA
<213>  Homo sapiens

<400>  432
ggauguggga uuuuuucuut t                                                21


<210>  433
<211>  21
<212>  DNA
<213>  Homo sapiens

<400>  433
aagaaaaaat cccacatcct g                                                21


<210>  434
<211>  21
<212>  DNA
<213>  Homo sapiens

<400>  434
gaaaaaaucc cacauccugt t                                                21


<210>  435
<211>  21
<212>  DNA
<213>  Homo sapiens

<400>  435
caggaugugg gauuuuuuct t                                                21


<210>  436
<211>  21
<212>  DNA
<213>  Homo sapiens

<400>  436
aaaaaatccc acatcctgct c                                                21


<210>  437
<211>  21
<212>  DNA
<213>  Homo sapiens

<400>  437
aaaaucccac auccugcuct t                                                21


<210>  438
<211>  21
<212>  DNA
<213>  Homo sapiens

<400>  438

gagcaggaug ugggauuuut t                                                21

<210>  439
<211>  21
<212>  DNA
<213>  Homo sapiens

<400>  439
aaaatcccac atcctgctca a                                                21

<210>  440
<211>  21
<212>  DNA
<213>  Homo sapiens

<400>  440
aaucccacau ccugcucaat t                                                21

<210>  441
<211>  21
<212>  DNA
<213>  Homo sapiens

<400>  441
uugagcagga ugugggauut t                                                21

<210>  442
<211>  21
<212>  DNA
<213>  Homo sapiens

<400>  442
aatcccacat cctgctcaag a                                                21

<210>  443
<211>  21
<212>  DNA
<213>  Homo sapiens

<400>  443
ucccacaucc ugcucaagat t                                                21

<210>  444
<211>  21
<212>  DNA
<213>  Homo sapiens

<400>  444
ucuugagcag gaugugggat t                                                21

<210>  445
<211>  19
<212>  DNA
<213>  Homo sapiens

<400>  445
aagacgcttc taccagctc                                                   19

<210>  446
<211>  19
<212>  DNA
<213>  Homo sapiens

<400>  446
gacgcuucua ccagcuctt                                                    19


<210>  447
<211>  19
<212>  DNA
<213>  Homo sapiens

<400>  447
gagcugguag aagcguctt                                                    19


<210>  448
<211>  21
<212>  DNA
<213>  Homo sapiens

<400>  448
aagctcctgg actccgtgca g                                                 21


<210>  449
<211>  21
<212>  DNA
<213>  Homo sapiens

<400>  449
gcuccuggac uccgugcagt t                                                 21


<210>  450
<211>  21
<212>  DNA
<213>  Homo sapiens

<400>  450
cugcacggag uccaggagct t                                                 21


<210>  451
<211>  21
<212>  DNA
<213>  Homo sapiens

<400>  451
aatcaagtca cacatggtga g                                                 21


<210>  452
<211>  21
<212>  DNA
<213>  Homo sapiens

<400>  452
ucaagucaca cauggugagt t                                                 21


<210>  453

<211>  21
<212>  DNA
<213>  Homo sapiens

<400>  453
cucaccaugu gugacuugat t                                                 21


<210>  454
<211>  21
<212>  DNA
<213>  Homo sapiens

<400>  454
aagtcacaca tggtgagcgt g                                                 21


<210>  455
<211>  21
<212>  DNA
<213>  Homo sapiens

<400>  455
gucacacaug gugagcgugt t                                                 21


<210>  456
<211>  21
<212>  DNA
<213>  Homo sapiens

<400>  456
cacgcucacc augugugact t                                                 21


<210>  457
<211>  20
<212>  DNA
<213>  Homo sapiens

<400>  457
aaatgatggc agagatcatc                                                   20


<210>  458
<211>  20
<212>  DNA
<213>  Homo sapiens

<400>  458
augauggcag agaucauctt                                                   20


<210>  459
<211>  20
<212>  DNA
<213>  Homo sapiens

<400>  459
gaugaucucu gccaucautt                                                   20


<210>  460
<211>  21
<212>  DNA

<213>  Homo sapiens

<400>  460
aagtgcccaa gatcctttct g                                                21


<210>  461
<211>  21
<212>  DNA
<213>  Homo sapiens

<400>  461
gugcccaaga uccuuucugt t                                                21


<210>  462
<211>  21
<212>  DNA
<213>  Homo sapiens

<400>  462
cagaaaggau cuugggcact t                                                21


<210>  463
<211>  21
<212>  DNA
<213>  Homo sapiens

<400>  463
aagatccttt ctgggaaagt c                                                21


<210>  464
<211>  21
<212>  DNA
<213>  Homo sapiens

<400>  464
gauccuuucu gggaaaguct t                                                21


<210>  465
<211>  21
<212>  DNA
<213>  Homo sapiens

<400>  465
gacuuuccca gaaaggauct t                                                21


<210>  466
<211>  21
<212>  DNA
<213>  Homo sapiens

<400>  466
aaagtcaagc ccatctattt c                                                21


<210>  467
<211>  21
<212>  DNA
<213>  Homo sapiens

<400>  467
agucaagccc aucuauuuct t                                                21


<210>  468
<211>  21
<212>  DNA
<213>  Homo sapiens

<400>  468
gaaauagaug ggcuugacut t                                                21


<210>  469
<211>  21
<212>  DNA
<213>  Homo sapiens

<400>  469
aagcccatct atttccacac c                                                21


<210>  470
<211>  21
<212>  DNA
<213>  Homo sapiens

<400>  470
gcccaucuau uuccacacct t                                                21


<210>  471
<211>  21
<212>  DNA
<213>  Homo sapiens

<400>  471
ggugugggaaa uagaugggct t                                                21


<210>  472
<211>  1512
<212>  DNA
<213>  Homo sapiens

<400>  472
atggttttgg aaatgctgaa cccgatacat tataacatca ccagcatcgt gcctgaagcc      60

atgcctgctg ccaccatgcc agtcctgctc ctcactggcc tttttctctt ggtgtggaat     120

tatgagggca catcctcaat accaggtcct ggctactgca tgggaattgg acccctcatc     180

tcccacggca gattcctgtg gatggggatc ggcagtgcct gcaactacta caaccgggta     240

tatggagaat tcatgcgagt ctggatctct ggagaggaaa cactcattat cagcaagtcc     300

tcaagtatgt tccacataat gaagcacaat cattacagct ctcgattcgg cagcaaactt     360

gggctgcagt gcatcggtat gcatgagaaa ggcatcatat ttaacaacaa tccagagctc     420

tggaaaacaa ctcgaccctt ctttatgaaa gctctgtcag gccccggcct tgttcgtatg     480

gtcacagtct gtgctgaatc cctcaaaaca catctggaca ggttggagga ggtgaccaat     540

gaatcgggct atgtggacgt gttgaccctt ctgcgtcgtg tcatgctgga cacctctaac     600

acgctcttct tgaggatccc tttggacgaa agtgctatcg tggttaaaat ccaaggttat 660

tttgatgcat ggcaagctct cctcatcaaa ccagacatct tctttaagat ttcttggcta 720

tacaaaaagt atgagaagtc tgtcaaggat ttgaaagatg ccatagaagt tctgatagca 780

gaaaaaagac gcaggatttc cacagaagag aaactggaag aatgtatgga ctttgccact 840

gagttgattt tagcagagaa acgtggtgac ctgacaagag agaatgtgaa ccagtgcata 900

ttggaaatgc tgatcgcagc tcctgacacc atgtctgtct ctttgttctt catgctattt 960

ctcattgcaa agcaccctaa tgttgaagag gcaataataa aggaaatcca gactgttatt 1020

ggtgagagag acataaagat tgatgatata caaaaattaa aagtgatgga aaacttcatt 1080

tatgagagca tgcggtacca gcctgtcgtg gacttggtca tgcgcaaagc cttagaagat 1140

gatgtaatcg atggctaccc agtgaaaaag gggacaaaca ttatcctgaa tattggaagg 1200

atgcacagac tcgagttttt ccccaaaccc aatgaattta ctcttgaaaa ttttgcaaag 1260

aatgttcctt ataggtactt tcagccattt ggctttgggc cccgtggctg tgcaggaaag 1320

tacatcgcca tggtgatgat gaaagccatc ctcgttacac ttctgagacg attccacgtg 1380

aagacattgc aaggacagtg tgttgagagc atacagaaga tacacgactt gtccttgcac 1440

ccagatgaga ctaaaaacat gctggaaatg atctttaccc caagaagctc agacaggtgt 1500

ctggaacact ag 1512

<210> 473
<211> 21
<212> DNA
<213> Homo sapiens

<400> 473
aaatgctgaa cccgatacat t 21

<210> 474
<211> 21
<212> DNA
<213> Homo sapiens

<400> 474
augcugaacc cgauacauut t 21

<210> 475
<211> 21
<212> DNA
<213> Homo sapiens

<400> 475
aauguaucgg guucagcaut t 21

<210> 476
<211> 21
<212> DNA
<213> Homo sapiens

<400>  476
aacccgatac attataacat c                                                21

<210>  477
<211>  21
<212>  DNA
<213>  Homo sapiens

<400>  477
cccgauacau uauaacauct t                                                21

<210>  478
<211>  21
<212>  DNA
<213>  Homo sapiens

<400>  478
gauguuauaa uguaucgggt t                                                21

<210>  479
<211>  21
<212>  DNA
<213>  Homo sapiens

<400>  479
aacatcacca gcatcgtgcc t                                                21

<210>  480
<211>  21
<212>  DNA
<213>  Homo sapiens

<400>  480
caucaccagc aucgugccut t                                                21

<210>  481
<211>  21
<212>  DNA
<213>  Homo sapiens

<400>  481
aggcacgaug cuggugaugt t                                                21

<210>  482
<211>  19
<212>  DNA
<213>  Homo sapiens

<400>  482
aagccatgcc tgctgccac                                                   19

<210>  483
<211>  19
<212>  DNA
<213>  Homo sapiens

<400>  483

gccaugccug cugccactt                                                    19

<210>  484
<211>  19
<212>  DNA
<213>  Homo sapiens

<400>  484
guggcagcag gcauggctt                                                    19

<210>  485
<211>  18
<212>  DNA
<213>  Homo sapiens

<400>  485
aattatgagg gcacatcc                                                     18

<210>  486
<211>  18
<212>  DNA
<213>  Homo sapiens

<400>  486
   uuaugagggc acaucctt                                                    18

<210>  487
<211>  18
<212>  DNA
<213>  Homo sapiens

<400>  487
ggaugugccc ucauaatt                                                     18

<210>  488
<211>  21
<212>  DNA
<213>  Homo sapiens

<400>  488
aataccaggt cctggctact g                                                 21

<210>  489
<211>  21
<212>  DNA
<213>  Homo sapiens

<400>  489
uaccaggucc uggcuacugt t                                                 21

<210>  490
<211>  21
<212>  DNA
<213>  Homo sapiens

<400>  490
caguagccag gaccugguat t                                                 21

<210>  491
<211>  18
<212>  DNA
<213>  Homo sapiens

<400>  491
aattggaccc ctcatctc                                                    18


<210>  492
<211>  18
<212>  DNA
<213>  Homo sapiens

<400>  492
uuggaccccu caucuctt                                                    18


<210>  493
<211>  18
<212>  DNA
<213>  Homo sapiens

<400>  493
gagaugaggg guccaatt                                                    18


<210>  494
<211>  18
<212>  DNA
<213>  Homo sapiens

<400>  494
aactactaca accgggta                                                    18


<210>  495
<211>  18
<212>  DNA
<213>  Homo sapiens

<400>  495
cuacuacaac cggguatt                                                    18


<210>  496
<211>  18
<212>  DNA
<213>  Homo sapiens

<400>  496
uacccgguug uaguagtt                                                    18


<210>  497
<211>  18
<212>  DNA
<213>  Homo sapiens

<400>  497
aaccgggtat atggagaa                                                    18


<210>  498

<211>  18
<212>  DNA
<213>  Homo sapiens

<400>  498
ccggguauau ggagaatt                                                    18


<210>  499
<211>  18
<212>  DNA
<213>  Homo sapiens

<400>  499
uucuccauau acccggtt                                                    18


<210>  500
<211>  21
<212>  DNA
<213>  Homo sapiens

<400>  500
aattcatgcg agtctggatc t                                                21


<210>  501
<211>  21
<212>  DNA
<213>  Homo sapiens

<400>  501
uucaugcgag ucuggaucut t                                                21


<210>  502
<211>  21
<212>  DNA
<213>  Homo sapiens

<400>  502
agauccagac ucgcaugaat t                                                21


<210>  503
<211>  21
<212>  DNA
<213>  Homo sapiens

<400>  503
aaacactcat tatcagcaag t                                                21


<210>  504
<211>  21
<212>  DNA
<213>  Homo sapiens

<400>  504
acacucauua ucagcaagut t                                                21


<210>  505
<211>  21
<212>  DNA

<213>  Homo sapiens

<400>  505
acuugcugau aaugagugut t                                                21


<210>  506
<211>  18
<212>  DNA
<213>  Homo sapiens

<400>  506
aagtcctcaa gtatgttc                                                    18


<210>  507
<211>  18
<212>  DNA
<213>  Homo sapiens

<400>  507
guccucaagu auguuctt                                                    18


<210>  508
<211>  18
<212>  DNA
<213>  Homo sapiens

<400>  508
gaacauacuu gaggactt                                                    18


<210>  509
<211>  21
<212>  DNA
<213>  Homo sapiens

<400>  509
aagtatgttc cacataatga a                                                21


<210>  510
<211>  21
<212>  DNA
<213>  Homo sapiens

<400>  510
guauguucca cauaaugaat t                                                21


<210>  511
<211>  21
<212>  DNA
<213>  Homo sapiens

<400>  511
uucauuaugu ggaacauact t                                                21


<210>  512
<211>  21
<212>  DNA
<213>  Homo sapiens

<400>  512
aatgaagcac aatcattaca g                                                21


<210>  513
<211>  21
<212>  DNA
<213>  Homo sapiens

<400>  513
ugaagcacaa ucauuacagt t                                                21


<210>  514
<211>  21
<212>  DNA
<213>  Homo sapiens

<400>  514
cuguaaugau ugugcuucat t                                                21


<210>  515
<211>  21
<212>  DNA
<213>  Homo sapiens

<400>  515
aagcacaatc attacagctc t                                                21


<210>  516
<211>  21
<212>  DNA
<213>  Homo sapiens

<400>  516
gcacaaucau uacagcucut t                                                21


<210>  517
<211>  21
<212>  DNA
<213>  Homo sapiens

<400>  517
agagcuguaa ugauugugct t                                                21


<210>  518
<211>  21
<212>  DNA
<213>  Homo sapiens

<400>  518
aatcattaca gctctcgatt c                                                21


<210>  519
<211>  21
<212>  DNA
<213>  Homo sapiens

<400>  519
ucauuacagc ucucgauuct t                                                21

<210>  520
<211>  21
<212>  DNA
<213>  Homo sapiens

<400>  520
gaaucgagag cuguaaugat t                                                21


<210>  521
<211>  18
<212>  DNA
<213>  Homo sapiens

<400>  521
aaacttgggc tgcagtgc                                                    18


<210>  522
<211>  18
<212>  DNA
<213>  Homo sapiens

<400>  522
acuugggcug cagugctt                                                    18


<210>  523
<211>  18
<212>  DNA
<213>  Homo sapiens

<400>  523
gcacugcagc ccaagutt                                                    18


<210>  524
<211>  21
<212>  DNA
<213>  Homo sapiens

<400>  524
aaaggcatca tatttaacaa c                                                21


<210>  525
<211>  21
<212>  DNA
<213>  Homo sapiens

<400>  525
aggcaucaua uuuaacaact t                                                21


<210>  526
<211>  21
<212>  DNA
<213>  Homo sapiens

<400>  526
guuguuaaau augaugccut t                                                21

<210>  527
<211>  18
<212>  DNA
<213>  Homo sapiens

<400>  527
aacaacaatc cagagctc                                                    18


<210>  528
<211>  18
<212>  DNA
<213>  Homo sapiens

<400>  528
caacaaucca gagcuctt                                                    18


<210>  529
<211>  18
<212>  DNA
<213>  Homo sapiens

<400>  529
gagcucugga uuguugtt                                                    18


<210>  530
<211>  18
<212>  DNA
<213>  Homo sapiens

<400>  530
aacaatccag agctctgg                                                    18


<210>  531
<211>  18
<212>  DNA
<213>  Homo sapiens

<400>  531
caauccagag cucuggtt                                                    18


<210>  532
<211>  18
<212>  DNA
<213>  Homo sapiens

<400>  532
ccagagcucu ggauugtt                                                    18


<210>  533
<211>  18
<212>  DNA
<213>  Homo sapiens

<400>  533
aatccagagc tctggaaa                                                    18


<210>  534
<211>  18

<212>  DNA
<213>  Homo sapiens

<400>  534
uccagagcuc uggaaatt                                                    18


<210>  535
<211>  18
<212>  DNA
<213>  Homo sapiens

<400>  535
uuuccagagc ucuggatt                                                    18


<210>  536
<211>  21
<212>  DNA
<213>  Homo sapiens

<400>  536
aaaacaactc gacccttctt t                                                21


<210>  537
<211>  21
<212>  DNA
<213>  Homo sapiens

<400>  537
aacaacucga cccuucuuut t                                                21


<210>  538
<211>  21
<212>  DNA
<213>  Homo sapiens

<400>  538
aaagaagggu cgaguuguut t                                                21


<210>  539
<211>  21
<212>  DNA
<213>  Homo sapiens

<400>  539
aacaactcga cccttcttta t                                                21


<210>  540
<211>  21
<212>  DNA
<213>  Homo sapiens

<400>  540
caacucgacc cuucuuuaut t                                                21


<210>  541
<211>  21
<212>  DNA
<213>  Homo sapiens

<400>  541
auaaagaagg gucgaguugt t                                                21

<210>  542
<211>  21
<212>  DNA
<213>  Homo sapiens

<400>  542
aactcgaccc ttctttatga a                                                21

<210>  543
<211>  21
<212>  DNA
<213>  Homo sapiens

<400>  543
cucgacccuu cuuuaugaat t                                                21

<210>  544
<211>  21
<212>  DNA
<213>  Homo sapiens

<400>  544
uucauaaaga agggucgagt t                                                21

<210>  545
<211>  21
<212>  DNA
<213>  Homo sapiens

<400>  545
aaagctctgt caggccccgg c                                                21

<210>  546
<211>  21
<212>  DNA
<213>  Homo sapiens

<400>  546
agcucuguca ggccccggct t                                                21

<210>  547
<211>  21
<212>  DNA
<213>  Homo sapiens

<400>  547
gccggggccu gacagagcut t                                                21

<210>  548
<211>  21
<212>  DNA
<213>  Homo sapiens

<400>  548

aatccctcaa aacacatctg g                                                21

<210>  549
<211>  21
<212>  DNA
<213>  Homo sapiens

<400>  549
ucccucaaaa cacaucuggt t                                                21

<210>  550
<211>  21
<212>  DNA
<213>  Homo sapiens

<400>  550
ccagaugugu uuugagggat t                                                21

<210>  551
<211>  21
<212>  DNA
<213>  Homo sapiens

<400>  551
aaaacacatc tggacaggtt g                                                21

<210>  552
<211>  21
<212>  DNA
<213>  Homo sapiens

<400>  552
aacacaucug gacagguugt t                                                21

<210>  553
<211>  21
<212>  DNA
<213>  Homo sapiens

<400>  553
caaccugucc agauguguut t                                                21

<210>  554
<211>  21
<212>  DNA
<213>  Homo sapiens

<400>  554
aacacatctg gacaggttgg a                                                21

<210>  555
<211>  21
<212>  DNA
<213>  Homo sapiens

<400>  555
cacaucugga cagguuggat t                                                21

<210>  556
<211>  21
<212>  DNA
<213>  Homo sapiens

<400>  556
uccaaccugu ccagaugugt t                                                21


<210>  557
<211>  18
<212>  DNA
<213>  Homo sapiens

<400>  557
aatgaatcgg gctatgtg                                                    18


<210>  558
<211>  18
<212>  DNA
<213>  Homo sapiens

<400>  558
ugaaucgggc uaugugtt                                                    18


<210>  559
<211>  18
<212>  DNA
<213>  Homo sapiens

<400>  559
cacauagccc gauucatt                                                    18


<210>  560
<211>  21
<212>  DNA
<213>  Homo sapiens

<400>  560
aatcgggcta tgtggacgtg t                                                21


<210>  561
<211>  21
<212>  DNA
<213>  Homo sapiens

<400>  561
ucgggcuaug uggacgugut t                                                21


<210>  562
<211>  21
<212>  DNA
<213>  Homo sapiens

<400>  562
acacguccac auagcccgat t                                                21


<210>  563

<211>  18
<212>  DNA
<213>  Homo sapiens

<400>  563
aacacgctct tcttgagg                                                    18


<210>  564
<211>  18
<212>  DNA
<213>  Homo sapiens

<400>  564
cacgcucuuc uugaggtt                                                    18


<210>  565
<211>  18
<212>  DNA
<213>  Homo sapiens

<400>  565
ccucaagaag agcgugtt                                                    18


<210>  566
<211>  21
<212>  DNA
<213>  Homo sapiens

<400>  566
aaagtgctat cgtggttaaa a                                                21


<210>  567
<211>  21
<212>  DNA
<213>  Homo sapiens

<400>  567
agugcuaucg ugguuaaaat t                                                21


<210>  568
<211>  21
<212>  DNA
<213>  Homo sapiens

<400>  568
uuuuaaccac gauagcacut t                                                21


<210>  569
<211>  18
<212>  DNA
<213>  Homo sapiens

<400>  569
aaaatccaag gttatttt                                                    18


<210>  570
<211>  18
<212>  DNA

<213>  Homo sapiens

<400>  570
aauccaaggu uauuuutt                                                    18


<210>  571
<211>  18
<212>  DNA
<213>  Homo sapiens

<400>  571
aaaauaaccu uggauutt                                                    18


<210>  572
<211>  18
<212>  DNA
<213>  Homo sapiens

<400>  572
aatccaaggt tattttga                                                    18


<210>  573
<211>  18
<212>  DNA
<213>  Homo sapiens

<400>  573
uccaagguua uuuugatt                                                    18


<210>  574
<211>  18
<212>  DNA
<213>  Homo sapiens

<400>  574
ucaaaauaac cuuggatt                                                    18


<210>  575
<211>  18
<212>  DNA
<213>  Homo sapiens

<400>  575
aaggttattt tgatgcat                                                    18


<210>  576
<211>  18
<212>  DNA
<213>  Homo sapiens

<400>  576
gguuauuuug augcautt                                                    18


<210>  577
<211>  18
<212>  DNA
<213>  Homo sapiens

<400>  577
   augcaucaaa auaacctt                                                    18


<210>  578
<211>  21
<212>  DNA
<213>  Homo sapiens

<400>  578
aagctctcct catcaaacca g                                                 21


<210>  579
<211>  21
<212>  DNA
<213>  Homo sapiens

<400>  579
gcucuccuca ucaaaccagt t                                                 21


<210>  580
<211>  21
<212>  DNA
<213>  Homo sapiens

<400>  580
cugguuugau gaggagagct t                                                 21


<210>  581
<211>  21
<212>  DNA
<213>  Homo sapiens

<400>  581
aaaccagaca tcttctttaa g                                                 21


<210>  582
<211>  21
<212>  DNA
<213>  Homo sapiens

<400>  582
accagacauc uucuuuaagt t                                                 21


<210>  583
<211>  21
<212>  DNA
<213>  Homo sapiens

<400>  583
cuuaaagaag augucuggut t                                                 21


<210>  584
<211>  18
<212>  DNA
<213>  Homo sapiens

<400>  584
aagatttctt ggctatac                                                     18

<210>  585
<211>  18
<212>  DNA
<213>  Homo sapiens

<400>  585
gauuucuugg cuauactt                                                    18


<210>  586
<211>  18
<212>  DNA
<213>  Homo sapiens

<400>  586
guauagccaa gaaauctt                                                    18


<210>  587
<211>  21
<212>  DNA
<213>  Homo sapiens

<400>  587
aaaaagtatg agaagtctgt c                                                21


<210>  588
<211>  21
<212>  DNA
<213>  Homo sapiens

<400>  588
aaaguaugag aagucuguct t                                                21


<210>  589
<211>  21
<212>  DNA
<213>  Homo sapiens

<400>  589
gacagacuuc ucauacuuut t                                                21


<210>  590
<211>  21
<212>  DNA
<213>  Homo sapiens

<400>  590
aaagtatgag aagtctgtca a                                                21


<210>  591
<211>  21
<212>  DNA
<213>  Homo sapiens

<400>  591
aguaugagaa gucugucaat t                                                21

<210>  592
<211>  21
<212>  DNA
<213>  Homo sapiens

<400>  592
uugacagacu ucucauacut t                                                21


<210>  593
<211>  21
<212>  DNA
<213>  Homo sapiens

<400>  593
aagtctgtca aggatttgaa a                                                21


<210>  594
<211>  21
<212>  DNA
<213>  Homo sapiens

<400>  594
gucugucaag gauuugaaat t                                                21


<210>  595
<211>  21
<212>  DNA
<213>  Homo sapiens

<400>  595
uuucaaaucc uugacagact t                                                21


<210>  596
<211>  21
<212>  DNA
<213>  Homo sapiens

<400>  596
aaggatttga aagatgccat a                                                21


<210>  597
<211>  21
<212>  DNA
<213>  Homo sapiens

<400>  597
ggauuugaaa gaugccauat t                                                21


<210>  598
<211>  21
<212>  DNA
<213>  Homo sapiens

<400>  598
uauggcaucu uucaaaucct t                                                21


<210>  599
<211>  21

<212>  DNA
<213>  Homo sapiens

<400>  599
aaagatgcca tagaagttct g                                                21


<210>  600
<211>  21
<212>  DNA
<213>  Homo sapiens

<400>  600
agaugccaua gaaguucugt t                                                21


<210>  601
<211>  21
<212>  DNA
<213>  Homo sapiens

<400>  601
cagaacuucu auggcaucut t                                                21


<210>  602
<211>  18
<212>  DNA
<213>  Homo sapiens

<400>  602
aagttctgat agcagaaa                                                    18


<210>  603
<211>  18
<212>  DNA
<213>  Homo sapiens

<400>  603
guucugauag cagaaatt                                                    18


<210>  604
<211>  18
<212>  DNA
<213>  Homo sapiens

<400>  604
uuucugcuau cagaactt                                                    18


<210>  605
<211>  21
<212>  DNA
<213>  Homo sapiens

<400>  605
aaaaaagacg caggatttcc a                                                21


<210>  606
<211>  21
<212>  DNA
<213>  Homo sapiens

<400>  606
aaaagacgca ggauuuccat t                                                21

<210>  607
<211>  21
<212>  DNA
<213>  Homo sapiens

<400>  607
uggaaauccu gcgucuuuut t                                                21

<210>  608
<211>  21
<212>  DNA
<213>  Homo sapiens

<400>  608
aaaagacgca ggatttccac a                                                21

<210>  609
<211>  21
<212>  DNA
<213>  Homo sapiens

<400>  609
aagacgcagg auuuccacat t                                                21

<210>  610
<211>  21
<212>  DNA
<213>  Homo sapiens

<400>  610
uguggaaauc cugcgucuut t                                                21

<210>  611
<211>  21
<212>  DNA
<213>  Homo sapiens

<400>  611
aagacgcagg atttccacag a                                                21

<210>  612
<211>  21
<212>  DNA
<213>  Homo sapiens

<400>  612
gacgcaggau uuccacagat t                                                21

<210>  613
<211>  21
<212>  DNA
<213>  Homo sapiens

<400>  613

ucuguggaaa uccugcguct t                                                21

<210>  614
<211>  21
<212>  DNA
<213>  Homo sapiens

<400>  614
aagagaaact ggaagaatgt a                                                21

<210>  615
<211>  21
<212>  DNA
<213>  Homo sapiens

<400>  615
gagaaacugg aagaauguat t                                                21

<210>  616
<211>  21
<212>  DNA
<213>  Homo sapiens

<400>  616
uacauucuuc caguuucuct t                                                21

<210>  617
<211>  21
<212>  DNA
<213>  Homo sapiens

<400>  617
aaactggaag aatgtatgga c                                                21

<210>  618
<211>  21
<212>  DNA
<213>  Homo sapiens

<400>  618
acuggaagaa uguauggact t                                                21

<210>  619
<211>  21
<212>  DNA
<213>  Homo sapiens

<400>  619
guccauacau ucuuccagut t                                                21

<210>  620
<211>  21
<212>  DNA
<213>  Homo sapiens

<400>  620
aagaatgtat ggactttgcc a                                                21

<210> 621
<211> 21
<212> DNA
<213> Homo sapiens

<400> 621
gaauguaugg acuuugccat t 21


<210> 622
<211> 21
<212> DNA
<213> Homo sapiens

<400> 622
uggcaaaguc cauacauuct t 21


<210> 623
<211> 20
<212> DNA
<213> Homo sapiens

<400> 623
aatgtatgga ctttgccact 20


<210> 624
<211> 20
<212> DNA
<213> Homo sapiens

<400> 624
uguauggacu uugccacutt 20


<210> 625
<211> 20
<212> DNA
<213> Homo sapiens

<400> 625
aguggcaaag uccauacatt 20


<210> 626
<211> 21
<212> DNA
<213> Homo sapiens

<400> 626
aaacgtggtg acctgacaag a 21


<210> 627
<211> 21
<212> DNA
<213> Homo sapiens

<400> 627
acguggugac cugacaagat t 21


<210> 628

<211>  21
<212>  DNA
<213>  Homo sapiens

<400>  628
ucuugucagg ucaccacgut t                                                21


<210>  629
<211>  21
<212>  DNA
<213>  Homo sapiens

<400>  629
aagagagaat gtgaaccagt g                                                21


<210>  630
<211>  21
<212>  DNA
<213>  Homo sapiens

<400>  630
gagagaaugu gaaccagugt t                                                21


<210>  631
<211>  21
<212>  DNA
<213>  Homo sapiens

<400>  631
cacugguuca cauucucuct t                                                21


<210>  632
<211>  18
<212>  DNA
<213>  Homo sapiens

<400>  632
aatgtgaacc agtgcata                                                    18


<210>  633
<211>  18
<212>  DNA
<213>  Homo sapiens

<400>  633
ugugaaccag ugcauatt                                                    18


<210>  634
<211>  18
<212>  DNA
<213>  Homo sapiens

<400>  634
uaugcacugg uucacatt                                                    18


<210>  635
<211>  18
<212>  DNA

<213>  Homo sapiens

<400>  635
aaccagtgca tattggaa                                                    18


<210>  636
<211>  18
<212>  DNA
<213>  Homo sapiens

<400>  636
ccagugcaua uuggaatt                                                    18


<210>  637
<211>  18
<212>  DNA
<213>  Homo sapiens

<400>  637
uuccaauaug cacuggtt                                                    18


<210>  638
<211>  21
<212>  DNA
<213>  Homo sapiens

<400>  638
aaatgctgat cgcagctcct g                                                21


<210>  639
<211>  21
<212>  DNA
<213>  Homo sapiens

<400>  639
augcugaucg cagcuccugt t                                                21


<210>  640
<211>  21
<212>  DNA
<213>  Homo sapiens

<400>  640
caggagcugc gaucagcaut t                                                21


<210>  641
<211>  21
<212>  DNA
<213>  Homo sapiens

<400>  641
aaagcaccct aatgttgaag a                                                21


<210>  642
<211>  21
<212>  DNA
<213>  Homo sapiens

<400>  642
agcacccuaa uguugaagat t                                                21

<210>  643
<211>  21
<212>  DNA
<213>  Homo sapiens

<400>  643
ucuucaacau uagggugcut t                                                21

<210>  644
<211>  21
<212>  DNA
<213>  Homo sapiens

<400>  644
aatgttgaag aggcaataat a                                                21

<210>  645
<211>  21
<212>  DNA
<213>  Homo sapiens

<400>  645
uguugaagag gcaauaauat t                                                21

<210>  646
<211>  21
<212>  DNA
<213>  Homo sapiens

<400>  646
uauuauugcc ucuucaacat t                                                21

<210>  647
<211>  21
<212>  DNA
<213>  Homo sapiens

<400>  647
aagaggcaat aataaaggaa a                                                21

<210>  648
<211>  21
<212>  DNA
<213>  Homo sapiens

<400>  648
gaggcaauaa uaaaggaaat t                                                21

<210>  649
<211>  21
<212>  DNA
<213>  Homo sapiens

<400>  649
uuuccuuuau uauugccuct t                                                21

<210>  650
<211>  21
<212>  DNA
<213>  Homo sapiens

<400>  650
aataataaag gaaatccaga c                                                21


<210>  651
<211>  21
<212>  DNA
<213>  Homo sapiens

<400>  651
uaauaaagga aauccagact t                                                21


<210>  652
<211>  21
<212>  DNA
<213>  Homo sapiens

<400>  652
gucuggauuu ccuuuauuat t                                                21


<210>  653
<211>  21
<212>  DNA
<213>  Homo sapiens

<400>  653
aataaaggaa atccagactg t                                                21


<210>  654
<211>  21
<212>  DNA
<213>  Homo sapiens

<400>  654
uaaaggaaau ccagacugut t                                                21


<210>  655
<211>  21
<212>  DNA
<213>  Homo sapiens

<400>  655
acagucugga uuuccuuuat t                                                21


<210>  656
<211>  21
<212>  DNA
<213>  Homo sapiens

<400>  656
aaaggaaatc cagactgtta t                                                21

<210>  657
<211>  21
<212>  DNA
<213>  Homo sapiens

<400>  657
aggaaaucca gacuguuaut t                                                21


<210>  658
<211>  21
<212>  DNA
<213>  Homo sapiens

<400>  658
auaacagucu ggauuuccut t                                                21


<210>  659
<211>  17
<212>  DNA
<213>  Homo sapiens

<400>  659
aaatccagac tgttatt                                                     17


<210>  660
<211>  17
<212>  DNA
<213>  Homo sapiens

<400>  660
auccagacug uuauutt                                                     17


<210>  661
<211>  17
<212>  DNA
<213>  Homo sapiens

<400>  661
aauaacaguc uggautt                                                     17


<210>  662
<211>  21
<212>  DNA
<213>  Homo sapiens

<400>  662
aaagattgat gatatacaaa a                                                21


<210>  663
<211>  21
<212>  DNA
<213>  Homo sapiens

<400>  663
agauugauga uauacaaaat t                                                21


<210>  664
<211>  21

<212>  DNA
<213>  Homo sapiens

<400>  664
uuuuguauau caucaaucut t                                                21


<210>  665
<211>  21
<212>  DNA
<213>  Homo sapiens

<400>  665
aaaaattaaa agtgatggaa a                                                21


<210>  666
<211>  21
<212>  DNA
<213>  Homo sapiens

<400>  666
aaauuaaaag ugauggaaat t                                                21


<210>  667
<211>  21
<212>  DNA
<213>  Homo sapiens

<400>  667
uuuccaucac uuuuaauuut t                                                21


<210>  668
<211>  21
<212>  DNA
<213>  Homo sapiens

<400>  668
aaattaaaag tgatggaaaa c                                                21


<210>  669
<211>  21
<212>  DNA
<213>  Homo sapiens

<400>  669
auuaaaagug auggaaaact t                                                21


<210>  670
<211>  21
<212>  DNA
<213>  Homo sapiens

<400>  670
guuuuccauc acuuuuaaut t                                                21


<210>  671
<211>  21
<212>  DNA
<213>  Homo sapiens

<400>  671
aaaagtgatg gaaaacttca t                                                21

<210>  672
<211>  21
<212>  DNA
<213>  Homo sapiens

<400>  672
aagugaugga aaacuucaut t                                                21

<210>  673
<211>  21
<212>  DNA
<213>  Homo sapiens

<400>  673
augaaguuuu ccaucacuut t                                                21

<210>  674
<211>  20
<212>  DNA
<213>  Homo sapiens

<400>  674
aagtgatgga aaacttcatt                                                  20

<210>  675
<211>  20
<212>  DNA
<213>  Homo sapiens

<400>  675
gugauggaaa acuucauutt                                                  20

<210>  676
<211>  20
<212>  DNA
<213>  Homo sapiens

<400>  676
aaugaaguuu uccaucactt                                                  20

<210>  677
<211>  17
<212>  DNA
<213>  Homo sapiens

<400>  677
aaaacttcat ttatgag                                                     17

<210>  678
<211>  17
<212>  DNA
<213>  Homo sapiens

<400>  678

aacuucauuu augagtt                                                    17

<210>  679
<211>  17
<212>  DNA
<213>  Homo sapiens

<400>  679
cucauaaaug aaguutt                                                    17

<210>  680
<211>  17
<212>  DNA
<213>  Homo sapiens

<400>  680
aacttcattt atgagag                                                    17

<210>  681
<211>  17
<212>  DNA
<213>  Homo sapiens

<400>  681
cuucauuuau gagagtt                                                    17

<210>  682
<211>  17
<212>  DNA
<213>  Homo sapiens

<400>  682
cucucauaaa ugaagtt                                                    17

<210>  683
<211>  17
<212>  DNA
<213>  Homo sapiens

<400>  683
aaagccttag aagatga                                                    17

<210>  684
<211>  17
<212>  DNA
<213>  Homo sapiens

<400>  684
agccuuagaa gaugatt                                                    17

<210>  685
<211>  17
<212>  DNA
<213>  Homo sapiens

<400>  685
ucaucuucua aggcutt                                                    17

<210>  686
<211>  17
<212>  DNA
<213>  Homo sapiens

<400>  686
aagatgatgt aatcgat                                                      17


<210>  687
<211>  17
<212>  DNA
<213>  Homo sapiens

<400>  687
gaugauguaa ucgautt                                                      17


<210>  688
<211>  17
<212>  DNA
<213>  Homo sapiens

<400>  688
aucgauuaca ucauctt                                                      17


<210>  689
<211>  21
<212>  DNA
<213>  Homo sapiens

<400>  689
aatcgatggc tacccagtga a                                                 21


<210>  690
<211>  21
<212>  DNA
<213>  Homo sapiens

<400>  690
ucgauggcua cccagugaat t                                                 21


<210>  691
<211>  21
<212>  DNA
<213>  Homo sapiens

<400>  691
uucacugggu agccaucgat t                                                 21


<210>  692
<211>  21
<212>  DNA
<213>  Homo sapiens

<400>  692
aaaaagggga caaacattat c                                                 21


<210>  693

<211>  21
<212>  DNA
<213>  Homo sapiens

<400>  693
aaaggggaca aacauuauct t                                                21


<210>  694
<211>  21
<212>  DNA
<213>  Homo sapiens

<400>  694
gauaauguuu guccccuuut t                                                21


<210>  695
<211>  21
<212>  DNA
<213>  Homo sapiens

<400>  695
aaaggggaca aacattatcc t                                                21


<210>  696
<211>  21
<212>  DNA
<213>  Homo sapiens

<400>  696
aggggacaaa cauuauccut t                                                21


<210>  697
<211>  21
<212>  DNA
<213>  Homo sapiens

<400>  697
aggauaaugu uuguccccut t                                                21


<210>  698
<211>  21
<212>  DNA
<213>  Homo sapiens

<400>  698
aaacattatc ctgaatattg g                                                21


<210>  699
<211>  21
<212>  DNA
<213>  Homo sapiens

<400>  699
acauuauccu gaauauuggt t                                                21


<210>  700
<211>  21
<212>  DNA

<213>  Homo sapiens

<400>  700
ccaauauuca ggauaaugut t                                                21


<210>  701
<211>  17
<212>  DNA
<213>  Homo sapiens

<400>  701
aatattggaa ggatgca                                                     17


<210>  702
<211>  17
<212>  DNA
<213>  Homo sapiens

<400>  702
uauuggaagg augcatt                                                     17


<210>  703
<211>  17
<212>  DNA
<213>  Homo sapiens

<400>  703
ugcauccuuc caauatt                                                     17


<210>  704
<211>  17
<212>  DNA
<213>  Homo sapiens

<400>  704
aaggatgcac agactcg                                                     17


<210>  705
<211>  17
<212>  DNA
<213>  Homo sapiens

<400>  705
ggaugcacag acucgtt                                                     17


<210>  706
<211>  17
<212>  DNA
<213>  Homo sapiens

<400>  706
   cgagucugug caucctt                                                    17


<210>  707
<211>  21
<212>  DNA
<213>  Homo sapiens

<400>  707
aaacccaatg aatttactct t                                                21


<210>  708
<211>  21
<212>  DNA
<213>  Homo sapiens

<400>  708
acccaaugaa uuuacucuut t                                                21


<210>  709
<211>  21
<212>  DNA
<213>  Homo sapiens

<400>  709
aagaguaaau ucauugggut t                                                21


<210>  710
<211>  21
<212>  DNA
<213>  Homo sapiens

<400>  710
aatgaattta ctcttgaaaa t                                                21


<210>  711
<211>  21
<212>  DNA
<213>  Homo sapiens

<400>  711
ugaauuuacu cuugaaaaut t                                                21


<210>  712
<211>  21
<212>  DNA
<213>  Homo sapiens

<400>  712
auuuucaaga guaaauucat t                                                21


<210>  713
<211>  21
<212>  DNA
<213>  Homo sapiens

<400>  713
aatttactct tgaaaatttt g                                                21


<210>  714
<211>  21
<212>  DNA
<213>  Homo sapiens

<400>  714
uuuacucuug aaaauuuugt t                                                21

<210>  715
<211>  21
<212>  DNA
<213>  Homo sapiens

<400>  715
caaaauuuuc aagaguaaat t                                                21


<210>  716
<211>  17
<212>  DNA
<213>  Homo sapiens

<400>  716
aaaattttgc aaagaat                                                     17


<210>  717
<211>  17
<212>  DNA
<213>  Homo sapiens

<400>  717
aauuuugcaa agaautt                                                     17


<210>  718
<211>  17
<212>  DNA
<213>  Homo sapiens

<400>  718
auucuuugca aaauutt                                                     17


<210>  719
<211>  17
<212>  DNA
<213>  Homo sapiens

<400>  719
aattttgcaa agaatgt                                                     17


<210>  720
<211>  17
<212>  DNA
<213>  Homo sapiens

<400>  720
uuuugcaaag aaugutt                                                     17


<210>  721
<211>  17
<212>  DNA
<213>  Homo sapiens

<400>  721
acauucuuug caaaatt                                                     17

<210>  722
<211>  17
<212>  DNA
<213>  Homo sapiens

<400>  722
aaagaatgtt ccttata                                                    17


<210>  723
<211>  17
<212>  DNA
<213>  Homo sapiens

<400>  723
agaauguucc uuauatt                                                    17


<210>  724
<211>  17
<212>  DNA
<213>  Homo sapiens

<400>  724
uauaaggaac auucutt                                                    17


<210>  725
<211>  21
<212>  DNA
<213>  Homo sapiens

<400>  725
aatgttcctt ataggtactt t                                               21


<210>  726
<211>  21
<212>  DNA
<213>  Homo sapiens

<400>  726
uguuccuuau agguacuuut t                                               21


<210>  727
<211>  21
<212>  DNA
<213>  Homo sapiens

<400>  727
aaaguaccua uaaggaacat t                                               21


<210>  728
<211>  17
<212>  DNA
<213>  Homo sapiens

<400>  728
aaagtacatc gccatgg                                                    17


<210>  729
<211>  17

<212>  DNA
<213>  Homo sapiens

<400>  729
aguacaucgc cauggtt                                                    17


<210>  730
<211>  17
<212>  DNA
<213>  Homo sapiens

<400>  730
ccauggcgau guacutt                                                    17


<210>  731
<211>  21
<212>  DNA
<213>  Homo sapiens

<400>  731
aaagccatcc tcgttacact t                                               21


<210>  732
<211>  21
<212>  DNA
<213>  Homo sapiens

<400>  732
agccauccuc guuacacuut t                                               21


<210>  733
<211>  21
<212>  DNA
<213>  Homo sapiens

<400>  733
aaguguaacg aggauggcut t                                               21


<210>  734
<211>  21
<212>  DNA
<213>  Homo sapiens

<400>  734
aagacattgc aaggacagtg t                                               21


<210>  735
<211>  21
<212>  DNA
<213>  Homo sapiens

<400>  735
gacauugcaa ggacagugut t                                               21


<210>  736
<211>  21
<212>  DNA
<213>  Homo sapiens

<400>  736
acacuguccu ugcaauguct t                                                21

<210>  737
<211>  21
<212>  DNA
<213>  Homo sapiens

<400>  737
aaggacagtg tgttgagagc a                                                21

<210>  738
<211>  21
<212>  DNA
<213>  Homo sapiens

<400>  738
ggacagugug uugagagcat t                                                21

<210>  739
<211>  21
<212>  DNA
<213>  Homo sapiens

<400>  739
ugcucucaac acacugucct t                                                21

<210>  740
<211>  21
<212>  DNA
<213>  Homo sapiens

<400>  740
aagatacacg acttgtcctt g                                                21

<210>  741
<211>  21
<212>  DNA
<213>  Homo sapiens

<400>  741
gauacacgac uuguccuugt t                                                21

<210>  742
<211>  21
<212>  DNA
<213>  Homo sapiens

<400>  742
caaggacaag ucguguauct t                                                21

<210>  743
<211>  21
<212>  DNA
<213>  Homo sapiens

<400>  743

aaaaacatgc tggaaatgat c                                                21

<210>  744
<211>  21
<212>  DNA
<213>  Homo sapiens

<400>  744
aaacaugcug gaaaugauct t                                                21

<210>  745
<211>  21
<212>  DNA
<213>  Homo sapiens

<400>  745
gaucauuucc agcauguuut t                                                21

<210>  746
<211>  21
<212>  DNA
<213>  Homo sapiens

<400>  746
aaacatgctg gaaatgatct t                                                21

<210>  747
<211>  21
<212>  DNA
<213>  Homo sapiens

<400>  747
acaugcugga aaugaucuut t                                                21

<210>  748
<211>  21
<212>  DNA
<213>  Homo sapiens

<400>  748
aagaucauuu ccagcaugut t                                                21

<210>  749
<211>  21
<212>  DNA
<213>  Homo sapiens

<400>  749
aaatgatctt taccccaaga a                                                21

<210>  750
<211>  21
<212>  DNA
<213>  Homo sapiens

<400>  750
augaucuuua ccccaagaat t                                                21

<210>  751
<211>  21
<212>  DNA
<213>  Homo sapiens

<400>  751
uucuugggu aaagaucaut t                                                21


<210>  752
<211>  19
<212>  DNA
<213>  Homo sapiens

<400>  752
aagaagctca gacaggtgt                                                  19


<210>  753
<211>  19
<212>  DNA
<213>  Homo sapiens

<400>  753
gaagcucaga caggugutt                                                  19


<210>  754
<211>  19
<212>  DNA
<213>  Homo sapiens

<400>  754
acaccugucu gagcuuctt                                                  19


<210>  755
<211>  17
<212>  DNA
<213>  Homo sapiens

<400>  755
aagctcagac aggtgtc                                                    17


<210>  756
<211>  17
<212>  DNA
<213>  Homo sapiens

<400>  756
   gcucagacag guguctt                                                   17


<210>  757
<211>  17
<212>  DNA
<213>  Homo sapiens

<400>  757
gacaccuguc ugagctt                                                    17


<210>  758

<211>  972
<212>  DNA
<213>  Homo sapiens

<400>  758
atggatccca aatatcagcg tgtagagcta aatgatggtc atttcatgcc cgtattggga      60

tttggcacct atgcacctcc agaggttccg aggaacagag ctgtagaggt caccaaatta     120

gcaatagaag ctggcttccg ccatattgat tctgcttatt tatacaataa tgaggagcag     180

gttggactgg ccatccgaag caagattgca gatggcagtg tgaagagaga agacatattc     240

tacacttcaa agctttggtg cactttcttt caaccacaga tggtccaacc agccttggaa     300

agctcactga aaaaacttca actggactat gttgacctct atcttcttca tttcccaatg     360

gctctcaagc caggtgagac gccactacca aaagatgaaa atggaaaagt aatattcgac     420

acagtggatc tctgtgccac atgggaggtc atggagaagt gtaaggatgc aggattggcc     480

aagtccatcg gggtgtcaaa cttcaactgc aggcagctgg agatgatcct caacaagcca     540

ggactcaagt acaagcctgt ctgcaaccag gtagaatgtc atccttacct caaccagagc     600

aaactgctgg atttctgcaa gtcaaaagac attgttctgg ttgcccacag tgctctggga     660

acccaacgac ataaactatg ggtggaccca aactccccag ttcttttgga ggacccagtt     720

ctttgtgcct tagcaaagaa acacaaacga accccagccc tgattgccct gcgctaccag     780

ctgcagcgtg gggttgtggt cctggccaag agctacaatg agcagcggat cagagagaac     840

atccaggttt ttgaattcca gttgacatca gaggatatga aagttctaga tggtctaaac     900

agaaattatc gatatgttgt catggatttt gttatggacc atcctgatta tccattttca     960

gatgaatatt ag                                                         972


<210>  759
<211>  21
<212>  DNA
<213>  Homo sapiens

<400>  759
aaatatcagc gtgtagagct a                                                21


<210>  760
<211>  21
<212>  DNA
<213>  Homo sapiens

<400>  760
auaucagcgu guagagcuat t                                                21


<210>  761
<211>  21
<212>  DNA
<213>  Homo sapiens

<400>  761
uagcucuaca cgcugauaut t                                                21

<210> 762
<211> 21
<212> DNA
<213> Homo sapiens

<400> 762
aaatgatggt catttcatgc c 21


<210> 763
<211> 21
<212> DNA
<213> Homo sapiens

<400> 763
augaugguca uuucaugcct t 21


<210> 764
<211> 21
<212> DNA
<213> Homo sapiens

<400> 764
ggcaugaaau gaccaucaut t 21


<210> 765
<211> 21
<212> DNA
<213> Homo sapiens

<400> 765
aacagagctg tagaggtcac c 21


<210> 766
<211> 21
<212> DNA
<213> Homo sapiens

<400> 766
cagagcugua gaggucacct t 21


<210> 767
<211> 21
<212> DNA
<213> Homo sapiens

<400> 767
ggugaccucu acagcucugt t 21


<210> 768
<211> 18
<212> DNA
<213> Homo sapiens

<400> 768
aaattagcaa tagaagct 18

<210> 769
<211> 18
<212> DNA
<213> Homo sapiens

<400> 769
auuagcaaua gaagcutt 18


<210> 770
<211> 18
<212> DNA
<213> Homo sapiens

<400> 770
agcuucuauu gcuaautt 18


<210> 771
<211> 21
<212> DNA
<213> Homo sapiens

<400> 771
aatagaagct ggcttccgcc a 21


<210> 772
<211> 21
<212> DNA
<213> Homo sapiens

<400> 772
uagaagcugg cuuccgccat t 21


<210> 773
<211> 21
<212> DNA
<213> Homo sapiens

<400> 773
uggcggaagc cagcuucuat t 21


<210> 774
<211> 21
<212> DNA
<213> Homo sapiens

<400> 774
aagctggctt ccgccatatt g 21


<210> 775
<211> 21
<212> DNA
<213> Homo sapiens

<400> 775
gcuggcuucc gccauauugt t 21


<210> 776
<211> 21

<212>  DNA
<213>  Homo sapiens

<400>  776
caauauggcg gaagccagct t                                                21


<210>  777
<211>  18
<212>  DNA
<213>  Homo sapiens

<400>  777
aataatgagg agcaggtt                                                    18


<210>  778
<211>  18
<212>  DNA
<213>  Homo sapiens

<400>  778
uaaugaggag cagguutt                                                    18


<210>  779
<211>  18
<212>  DNA
<213>  Homo sapiens

<400>  779
aaccugcucc ucauuatt                                                    18


<210>  780
<211>  18
<212>  DNA
<213>  Homo sapiens

<400>  780
aatgaggagc aggttgga                                                    18


<210>  781
<211>  18
<212>  DNA
<213>  Homo sapiens

<400>  781
ugaggagcag guuggatt                                                    18


<210>  782
<211>  18
<212>  DNA
<213>  Homo sapiens

<400>  782
uccaaccugc uccucatt                                                    18


<210>  783
<211>  21
<212>  DNA
<213>  Homo sapiens

<400>  783
aagcaagatt gcagatggca g                                                21


<210>  784
<211>  21
<212>  DNA
<213>  Homo sapiens

<400>  784
gcaagauugc agauggcagt t                                                21


<210>  785
<211>  21
<212>  DNA
<213>  Homo sapiens

<400>  785
cugccaucug caaucuugct t                                                21


<210>  786
<211>  21
<212>  DNA
<213>  Homo sapiens

<400>  786
aagattgcag atggcagtgt g                                                21


<210>  787
<211>  21
<212>  DNA
<213>  Homo sapiens

<400>  787
gauugcagau ggcagugugt t                                                21


<210>  788
<211>  21
<212>  DNA
<213>  Homo sapiens

<400>  788
cacacugcca ucugcaauct t                                                21


<210>  789
<211>  18
<212>  DNA
<213>  Homo sapiens

<400>  789
aagagagaag acatattc                                                    18


<210>  790
<211>  18
<212>  DNA
<213>  Homo sapiens

<400>  790

gagagaagac auauuctt                                                    18

<210>  791
<211>  18
<212>  DNA
<213>  Homo sapiens

<400>  791
gaauaugucu ucucuctt                                                    18

<210>  792
<211>  18
<212>  DNA
<213>  Homo sapiens

<400>  792
aagacatatt ctacactt                                                    18

<210>  793
<211>  18
<212>  DNA
<213>  Homo sapiens

<400>  793
gacauauucu acacuutt                                                    18

<210>  794
<211>  18
<212>  DNA
<213>  Homo sapiens

<400>  794
aaguguagaa uaugucutt                                                   18

<210>  795
<211>  21
<212>  DNA
<213>  Homo sapiens

<400>  795
aaagctttgg tgcactttct t                                                21

<210>  796
<211>  21
<212>  DNA
<213>  Homo sapiens

<400>  796
agcuuuggug cacuuucuut t                                                21

<210>  797
<211>  21
<212>  DNA
<213>  Homo sapiens

<400>  797
aagaaagugc accaaagcut t                                                21

<210>  798
<211>  21
<212>  DNA
<213>  Homo sapiens

<400>  798
aaccacagat ggtccaacca g                                                21


<210>  799
<211>  21
<212>  DNA
<213>  Homo sapiens

<400>  799
ccacagaugg uccaaccagt t                                                21


<210>  800
<211>  21
<212>  DNA
<213>  Homo sapiens

<400>  800
cugguuggac caucuguggt t                                                21


<210>  801
<211>  18
<212>  DNA
<213>  Homo sapiens

<400>  801
aaccagcctt ggaaagct                                                    18


<210>  802
<211>  18
<212>  DNA
<213>  Homo sapiens

<400>  802
ccagccuugg aaagcutt                                                    18


<210>  803
<211>  18
<212>  DNA
<213>  Homo sapiens

<400>  803
agcuuuccaa ggcuggtt                                                    18


<210>  804
<211>  18
<212>  DNA
<213>  Homo sapiens

<400>  804
aaagctcact gaaaaaac                                                    18


<210>  805

<211>  18
<212>  DNA
<213>  Homo sapiens

<400>  805
agcucacuga aaaaactt                                                    18


<210>  806
<211>  18
<212>  DNA
<213>  Homo sapiens

<400>  806
guuuuucag ugagcutt                                                     18


<210>  807
<211>  21
<212>  DNA
<213>  Homo sapiens

<400>  807
aaaaaacttc aactggacta t                                                21


<210>  808
<211>  21
<212>  DNA
<213>  Homo sapiens

<400>  808
aaaacuucaa cuggacuaut t                                                21


<210>  809
<211>  21
<212>  DNA
<213>  Homo sapiens

<400>  809
auaguccagu ugaaguuuut t                                                21


<210>  810
<211>  21
<212>  DNA
<213>  Homo sapiens

<400>  810
aaaacttcaa ctggactatg t                                                21


<210>  811
<211>  21
<212>  DNA
<213>  Homo sapiens

<400>  811
aacuucaacu ggacuaugut t                                                21


<210>  812
<211>  21
<212>  DNA

<213>  Homo sapiens

<400>  812
acauagucca guugaaguut t                                                21


<210>  813
<211>  21
<212>  DNA
<213>  Homo sapiens

<400>  813
aacttcaact ggactatgtt g                                                21


<210>  814
<211>  21
<212>  DNA
<213>  Homo sapiens

<400>  814
cuucaacugg acuauguugt t                                                21


<210>  815
<211>  21
<212>  DNA
<213>  Homo sapiens

<400>  815
caacauaguc caguugaagt t                                                21


<210>  816
<211>  21
<212>  DNA
<213>  Homo sapiens

<400>  816
aactggacta tgttgacctc t                                                21


<210>  817
<211>  21
<212>  DNA
<213>  Homo sapiens

<400>  817
cuggacuaug uugaccucut t                                                21


<210>  818
<211>  21
<212>  DNA
<213>  Homo sapiens

<400>  818
agaggucaac auaguccagt t                                                21


<210>  819
<211>  18
<212>  DNA
<213>  Homo sapiens

<400>  819
aatggctctc aagccagg                                                    18


<210>  820
<211>  18
<212>  DNA
<213>  Homo sapiens

<400>  820
uggcucucaa gccaggtt                                                    18


<210>  821
<211>  18
<212>  DNA
<213>  Homo sapiens

<400>  821
ccuggcuuga gagccatt                                                    18


<210>  822
<211>  21
<212>  DNA
<213>  Homo sapiens

<400>  822
aagccaggtg agacgccact a                                                21


<210>  823
<211>  21
<212>  DNA
<213>  Homo sapiens

<400>  823
gccaggugag acgccacuat t                                                21


<210>  824
<211>  21
<212>  DNA
<213>  Homo sapiens

<400>  824
uaguggcguc ucaccuggct t                                                21


<210>  825
<211>  21
<212>  DNA
<213>  Homo sapiens

<400>  825
aaaagatgaa aatggaaaag t                                                21


<210>  826
<211>  21
<212>  DNA
<213>  Homo sapiens

<400>  826
aagaugaaaa uggaaaagut t                                                21

<210>  827
<211>  21
<212>  DNA
<213>  Homo sapiens

<400>  827
acuuuuccau uuucaucuut t                                                21


<210>  828
<211>  21
<212>  DNA
<213>  Homo sapiens

<400>  828
aagatgaaaa tggaaaagta a                                                21


<210>  829
<211>  21
<212>  DNA
<213>  Homo sapiens

<400>  829
gaugaaaaug gaaaaguaat t                                                21


<210>  830
<211>  21
<212>  DNA
<213>  Homo sapiens

<400>  830
uuacuuuucc auuuucauct t                                                21


<210>  831
<211>  21
<212>  DNA
<213>  Homo sapiens

<400>  831
aaaatggaaa agtaatattc g                                                21


<210>  832
<211>  21
<212>  DNA
<213>  Homo sapiens

<400>  832
aauggaaaag uaauauucgt t                                                21


<210>  833
<211>  21
<212>  DNA
<213>  Homo sapiens

<400>  833
cgaauauuac uuuuccauut t                                                21

<210> 834
<211> 21
<212> DNA
<213> Homo sapiens

<400> 834
aatggaaaag taatattcga c 21

<210> 835
<211> 21
<212> DNA
<213> Homo sapiens

<400> 835
uggaaaagua auauucgact t 21

<210> 836
<211> 21
<212> DNA
<213> Homo sapiens

<400> 836
gucgaauauu acuuuuccat t 21

<210> 837
<211> 18
<212> DNA
<213> Homo sapiens

<400> 837
aaaagtaata ttcgacac 18

<210> 838
<211> 18
<212> DNA
<213> Homo sapiens

<400> 838
aaguaauauu cgacactt 18

<210> 839
<211> 18
<212> DNA
<213> Homo sapiens

<400> 839
gugucgaaua uuacuutt 18

<210> 840
<211> 18
<212> DNA
<213> Homo sapiens

<400> 840
aagtaatatt cgacacag 18

<210> 841
<211> 18

<212>  DNA
<213>  Homo sapiens

<400>  841
guaauauucg acacagtt                                                    18


<210>  842
<211>  18
<212>  DNA
<213>  Homo sapiens

<400>  842
cugugucgaa uauuactt                                                    18


<210>  843
<211>  18
<212>  DNA
<213>  Homo sapiens

<400>  843
aatattcgac acagtgga                                                    18


<210>  844
<211>  18
<212>  DNA
<213>  Homo sapiens

<400>  844
uauucgacac aguggatt                                                    18


<210>  845
<211>  18
<212>  DNA
<213>  Homo sapiens

<400>  845
uccacugugu cgaauatt                                                    18


<210>  846
<211>  21
<212>  DNA
<213>  Homo sapiens

<400>  846
aagtgtaagg atgcaggatt g                                                21


<210>  847
<211>  21
<212>  DNA
<213>  Homo sapiens

<400>  847
guguaaggau gcaggauugt t                                                21


<210>  848
<211>  21
<212>  DNA
<213>  Homo sapiens

<400>  848
caauccugca uccuuacact t                                                21

<210>  849
<211>  18
<212>  DNA
<213>  Homo sapiens

<400>  849
aaggatgcag gattggcc                                                    18

<210>  850
<211>  18
<212>  DNA
<213>  Homo sapiens

<400>  850
ggaugcagga uuggcctt                                                    18

<210>  851
<211>  18
<212>  DNA
<213>  Homo sapiens

<400>  851
ggccaauccu gcauccttt                                                   18

<210>  852
<211>  21
<212>  DNA
<213>  Homo sapiens

<400>  852
   aagtccatcg gggtgtcaaa c                                                 21

<210>  853
<211>  21
<212>  DNA
<213>  Homo sapiens

<400>  853
guccaucggg gugucaaact t                                                21

<210>  854
<211>  21
<212>  DNA
<213>  Homo sapiens

<400>  854
guuugacacc ccgauggact t                                                21

<210>  855
<211>  21
<212>  DNA
<213>  Homo sapiens

<400>  855

aaacttcaac tgcaggcagc t                                                21

<210>  856
<211>  21
<212>  DNA
<213>  Homo sapiens

<400>  856
acuucaacug caggcagcut t                                                21

<210>  857
<211>  21
<212>  DNA
<213>  Homo sapiens

<400>  857
agcugccugc aguugaagut t                                                21

<210>  858
<211>  21
<212>  DNA
<213>  Homo sapiens

<400>  858
aactgcaggc agctggagat g                                                21

<210>  859
<211>  21
<212>  DNA
<213>  Homo sapiens

<400>  859
cugcaggcag cuggagaugt t                                                21

<210>  860
<211>  21
<212>  DNA
<213>  Homo sapiens

<400>  860
caucuccagc ugccugcagt t                                                21

<210>  861
<211>  18
<212>  DNA
<213>  Homo sapiens

<400>  861
aacaagccag gactcaag                                                    18

<210>  862
<211>  18
<212>  DNA
<213>  Homo sapiens

<400>  862
caagccagga cucaagtt                                                    18

<210> 863
<211> 18
<212> DNA
<213> Homo sapiens

<400> 863
cuugaguccu ggcuugtt 18

<210> 864
<211> 18
<212> DNA
<213> Homo sapiens

<400> 864
aagccaggac tcaagtac 18

<210> 865
<211> 18
<212> DNA
<213> Homo sapiens

<400> 865
gccaggacuc aaguactt 18

<210> 866
<211> 18
<212> DNA
<213> Homo sapiens

<400> 866
guacuugagu ccuggctt 18

<210> 867
<211> 21
<212> DNA
<213> Homo sapiens

<400> 867
aagtacaagc ctgtctgcaa c 21

<210> 868
<211> 21
<212> DNA
<213> Homo sapiens

<400> 868
guacaagccu gucugcaact t 21

<210> 869
<211> 21
<212> DNA
<213> Homo sapiens

<400> 869
guugcagaca ggcuuguact t 21

<210> 870

<211>  21
<212>  DNA
<213>  Homo sapiens

<400>  870
aagcctgtct gcaaccaggt a                                                21


<210>  871
<211>  21
<212>  DNA
<213>  Homo sapiens

<400>  871
gccugucugc aaccagguat t                                                21


<210>  872
<211>  21
<212>  DNA
<213>  Homo sapiens

<400>  872
uaccugguug cagacaggct t                                                21


<210>  873
<211>  21
<212>  DNA
<213>  Homo sapiens

<400>  873
aaccaggtag aatgtcatcc t                                                21


<210>  874
<211>  21
<212>  DNA
<213>  Homo sapiens

<400>  874
ccagguagaa ugucauccut t                                                21


<210>  875
<211>  21
<212>  DNA
<213>  Homo sapiens

<400>  875
aggaugacau ucuaccuggt t                                                21


<210>  876
<211>  21
<212>  DNA
<213>  Homo sapiens

<400>  876
aatgtcatcc ttacctcaac c                                                21


<210>  877
<211>  21
<212>  DNA

<213>  Homo sapiens

<400>  877
ugucauccuu accucaacct t                                                21


<210>  878
<211>  21
<212>  DNA
<213>  Homo sapiens

<400>  878
gguugaggua aggaugacat t                                                21


<210>  879
<211>  18
<212>  DNA
<213>  Homo sapiens

<400>  879
aaccagagca aactgctg                                                    18


<210>  880
<211>  18
<212>  DNA
<213>  Homo sapiens

<400>  880
ccagagcaaa cugcugtt                                                    18


<210>  881
<211>  18
<212>  DNA
<213>  Homo sapiens

<400>  881
cagcaguuug cucuggtt                                                    18


<210>  882
<211>  21
<212>  DNA
<213>  Homo sapiens

<400>  882
aaactgctgg atttctgcaa g                                                21


<210>  883
<211>  21
<212>  DNA
<213>  Homo sapiens

<400>  883
acugcuggau uucugcaagt t                                                21


<210>  884
<211>  21
<212>  DNA
<213>  Homo sapiens

<400>  884
cuugcagaaa uccagcagut t                                                21

<210>  885
<211>  21
<212>  DNA
<213>  Homo sapiens

<400>  885
aagtcaaaag acattgttct g                                                21

<210>  886
<211>  21
<212>  DNA
<213>  Homo sapiens

<400>  886
gucaaaagac auuguucugt t                                                21

<210>  887
<211>  21
<212>  DNA
<213>  Homo sapiens

<400>  887
cagaacaaug ucuuuugact t                                                21

<210>  888
<211>  21
<212>  DNA
<213>  Homo sapiens

<400>  888
aaaagacatt gttctggttg c                                                21

<210>  889
<211>  21
<212>  DNA
<213>  Homo sapiens

<400>  889
aagacauugu ucugguugct t                                                21

<210>  890
<211>  21
<212>  DNA
<213>  Homo sapiens

<400>  890
gcaaccagaa caaugucuut t                                                21

<210>  891
<211>  21
<212>  DNA
<213>  Homo sapiens

<400>  891
aagacattgt tctggttgcc c                                                21

<210>  892
<211>  21
<212>  DNA
<213>  Homo sapiens

<400>  892
gacauuguuc ugguugccct t                                                21


<210>  893
<211>  21
<212>  DNA
<213>  Homo sapiens

<400>  893
gggcaaccag aacaauguct t                                                21


<210>  894
<211>  21
<212>  DNA
<213>  Homo sapiens

<400>  894
aacgacataa actatgggtg g                                                21


<210>  895
<211>  21
<212>  DNA
<213>  Homo sapiens

<400>  895
cgacauaaac uauggguggt t                                                21


<210>  896
<211>  21
<212>  DNA
<213>  Homo sapiens

<400>  896
ccacccauag uuuaugucgt t                                                21


<210>  897
<211>  21
<212>  DNA
<213>  Homo sapiens

<400>  897
aaactatggg tggacccaaa c                                                21


<210>  898
<211>  21
<212>  DNA
<213>  Homo sapiens

<400>  898
acuaugggug gacccaaact t                                                21

<210>  899
<211>  21
<212>  DNA
<213>  Homo sapiens

<400>  899
guuugggucc acccauagut t                                                21


<210>  900
<211>  21
<212>  DNA
<213>  Homo sapiens

<400>  900
aaactcccca gttcttttgg a                                                21


<210>  901
<211>  21
<212>  DNA
<213>  Homo sapiens

<400>  901
acuccccagu ucuuuuggat t                                                21


<210>  902
<211>  21
<212>  DNA
<213>  Homo sapiens

<400>  902
uccaaaagaa cuggggagut t                                                21


<210>  903
<211>  21
<212>  DNA
<213>  Homo sapiens

<400>  903
aaagaaacac aaacgaaccc c                                                21


<210>  904
<211>  21
<212>  DNA
<213>  Homo sapiens

<400>  904
agaaacacaa acgaacccct t                                                21


<210>  905
<211>  21
<212>  DNA
<213>  Homo sapiens

<400>  905
gggguucguu uguguuucut t                                                21


<210>  906
<211>  21

<212>  DNA
<213>  Homo sapiens

<400>  906
aaacacaaac gaaccccagc c                                                21


<210>  907
<211>  21
<212>  DNA
<213>  Homo sapiens

<400>  907
acacaaacga accccagcct t                                                21


<210>  908
<211>  21
<212>  DNA
<213>  Homo sapiens

<400>  908
ggcugggguu cguuugugut t                                                21


<210>  909
<211>  21
<212>  DNA
<213>  Homo sapiens

<400>  909
aaacgaaccc cagccctgat t                                                21


<210>  910
<211>  21
<212>  DNA
<213>  Homo sapiens

<400>  910
acgaacccca gcccugauut t                                                21


<210>  911
<211>  21
<212>  DNA
<213>  Homo sapiens

<400>  911
aaucagggcu gggguucgut t                                                21


<210>  912
<211>  21
<212>  DNA
<213>  Homo sapiens

<400>  912
aaccccagcc ctgattgccc t                                                21


<210>  913
<211>  21
<212>  DNA
<213>  Homo sapiens

<400>  913
ccccagcccu gauugcccut t                                                21

<210>  914
<211>  21
<212>  DNA
<213>  Homo sapiens

<400>  914
agggcaauca gggcuggggt t                                                21

<210>  915
<211>  21
<212>  DNA
<213>  Homo sapiens

<400>  915
aagagctaca atgagcagcg g                                                21

<210>  916
<211>  21
<212>  DNA
<213>  Homo sapiens

<400>  916
gagcuacaau gagcagcggt t                                                21

<210>  917
<211>  21
<212>  DNA
<213>  Homo sapiens

<400>  917
ccgcugcuca uuguagcuct t                                                21

<210>  918
<211>  21
<212>  DNA
<213>  Homo sapiens

<400>  918
aatgagcagc ggatcagaga g                                                21

<210>  919
<211>  21
<212>  DNA
<213>  Homo sapiens

<400>  919
ugagcagcgg aucagagagt t                                                21

<210>  920
<211>  21
<212>  DNA
<213>  Homo sapiens

<400>  920

cucucugauc cgcugcucat t                                                21

<210>  921
<211>  18
<212>  DNA
<213>  Homo sapiens

<400>  921
aacatccagg tttttgaa                                                    18

<210>  922
<211>  18
<212>  DNA
<213>  Homo sapiens

<400>  922
cauccagguu uuugaatt                                                    18

<210>  923
<211>  18
<212>  DNA
<213>  Homo sapiens

<400>  923
uucaaaaacc uggaugtt                                                    18

<210>  924
<211>  21
<212>  DNA
<213>  Homo sapiens

<400>  924
aattccagtt gacatcagag g                                                21

<210>  925
<211>  21
<212>  DNA
<213>  Homo sapiens

<400>  925
uuccaguuga caucagaggt t                                                21

<210>  926
<211>  21
<212>  DNA
<213>  Homo sapiens

<400>  926
ccucugaugu caacuggaat t                                                21

<210>  927
<211>  21
<212>  DNA
<213>  Homo sapiens

<400>  927
aaagttctag atggtctaaa c                                                21

<210>  928
<211>  21
<212>  DNA
<213>  Homo sapiens

<400>  928
aguucuagau ggucuaaact t                                                21


<210>  929
<211>  21
<212>  DNA
<213>  Homo sapiens

<400>  929
guuuagacca ucuagaacut t                                                21


<210>  930
<211>  18
<212>  DNA
<213>  Homo sapiens

<400>  930
aaacagaaat tatcgata                                                    18


<210>  931
<211>  18
<212>  DNA
<213>  Homo sapiens

<400>  931
acagaaauua ucgauatt                                                    18


<210>  932
<211>  18
<212>  DNA
<213>  Homo sapiens

<400>  932
uaucgauaau uucugutt                                                    18


<210>  933
<211>  21
<212>  DNA
<213>  Homo sapiens

<400>  933
aaattatcga tatgttgtca t                                                21


<210>  934
<211>  21
<212>  DNA
<213>  Homo sapiens

<400>  934
auuaucgaua uguugucaut t                                                21


<210>  935

<211>  21
<212>  DNA
<213>  Homo sapiens

<400>  935
augacaacau aucgauaaut t                                                21


<210>  936
<211>  1122
<212>  DNA
<213>  Homo sapiens

<400>  936
atgacgggct ggagctgcct tgtgacagga gcaggagggt ttctgggaca gaggatcatc      60

cgcctcttgg tgaaggagaa ggagctgaag gagatcaggg tcttggacaa ggccttcgga     120

ccagaattga gagaggaatt ttctaaactc cagaacaaga ccaagctgac agtgctggaa     180

ggagacattc tggatgagcc attcctgaag agagcctgcc aggacgtctc ggtcatcatc     240

cacaccgcct gtatcattga tgtcttcggt gtcactcaca gagagtctat catgaatgtc     300

aatgtgaaag gtacccagct cctgttagag gcctgtgtcc aagctagtgt gccagtcttc     360

atctacacca gtagcataga ggtagccggg cccaactcct acaaggaaat catccagaat     420

ggccatgaag aagagcctct ggaaaacaca tggcccgctc catacccaca cagcaaaaag     480

cttgctgaga aggctgtact ggcggctaac gggtggaatc tgaaaaacgg cggcaccctg     540

tacacttgtg ccttacgacc catgtatatc tatggggaag gaagccgatt cctttctgct     600

agtataaacg aggccctgaa caacaatggg atcctgtcaa gtgttggaaa gttctccact     660

gttaacccag tctatgttgg caatgtggcc tgggcccaca ttctggcctt gagggccctg     720

caggacccca agaaggcccc aagcatccga ggacagttct actatatctc agatgacacg     780

cctcaccaaa gctatgataa ccttaattac accctgagca aagagttcgg cctccgcctt     840

gattccagat ggagctttcc tttatccctg atgtattgga ttggcttcct gctggaaata     900

gtgagcttcc tactcaggcc aatttacacc tatcgaccgc ccttcaaccg ccacatagtc     960

acattgtcaa atagcgtatt caccttctct tataagaagg ctcagcgaga tctggcgtat    1020

aagccactct acagctggga ggaagccaag cagaaaacgg tggagtgggt tggttccctt    1080

gtggaccggc acaaggagac cctgaagtcc aagactcagt ga                       1122


<210>  937
<211>  21
<212>  DNA
<213>  Homo sapiens

<400>  937
aaggagaagg agctgaagga g                                                21


<210>  938
<211>  21
<212>  DNA

<213>  Homo sapiens

<400>  938
ggagaaggag cugaaggagt t                                                21


<210>  939
<211>  21
<212>  DNA
<213>  Homo sapiens

<400>  939
cuccuucagc uccuucucct t                                                21


<210>  940
<211>  21
<212>  DNA
<213>  Homo sapiens

<400>  940
aaggagctga aggagatcag g                                                21


<210>  941
<211>  21
<212>  DNA
<213>  Homo sapiens

<400>  941
ggagcugaag gagaucaggt t                                                21


<210>  942
<211>  21
<212>  DNA
<213>  Homo sapiens

<400>  942
ccugaucucc uucagcucct t                                                21


<210>  943
<211>  21
<212>  DNA
<213>  Homo sapiens

<400>  943
aaggagatca gggtcttgga c                                                21


<210>  944
<211>  21
<212>  DNA
<213>  Homo sapiens

<400>  944
ggagaucagg gucuuggact t                                                21


<210>  945
<211>  21
<212>  DNA
<213>  Homo sapiens

<400> 945
guccaagacc cugaucucct t 21

<210> 946
<211> 18
<212> DNA
<213> Homo sapiens

<400> 946
aaggccttcg gaccagaa 18

<210> 947
<211> 18
<212> DNA
<213> Homo sapiens

<400> 947
ggccuucgga ccagaatt 18

<210> 948
<211> 18
<212> DNA
<213> Homo sapiens

<400> 948
uucugguccg aaggcctt 18

<210> 949
<211> 21
<212> DNA
<213> Homo sapiens

<400> 949
aattgagaga ggaattttct a 21

<210> 950
<211> 21
<212> DNA
<213> Homo sapiens

<400> 950
uugagagagg aauuuucuat t 21

<210> 951
<211> 21
<212> DNA
<213> Homo sapiens

<400> 951
uagaaaauuc cucucucaat t 21

<210> 952
<211> 21
<212> DNA
<213> Homo sapiens

<400> 952
aattttctaa actccagaac a 21

<210>  953
<211>  21
<212>  DNA
<213>  Homo sapiens

<400>  953
uuuucuaaac uccagaacat t                                                21


<210>  954
<211>  21
<212>  DNA
<213>  Homo sapiens

<400>  954
uguucuggag uuuagaaaat t                                                21


<210>  955
<211>  21
<212>  DNA
<213>  Homo sapiens

<400>  955
aaactccaga acaagaccaa g                                                21


<210>  956
<211>  21
<212>  DNA
<213>  Homo sapiens

<400>  956
acuccagaac aagaccaagt t                                                21


<210>  957
<211>  21
<212>  DNA
<213>  Homo sapiens

<400>  957
cuuggucuug uucuggagut t                                                21


<210>  958
<211>  21
<212>  DNA
<213>  Homo sapiens

<400>  958
aacaagacca agctgacagt g                                                21


<210>  959
<211>  21
<212>  DNA
<213>  Homo sapiens

<400>  959
caagaccaag cugacagugt t                                                21

<210>  960
<211>  21
<212>  DNA
<213>  Homo sapiens

<400>  960
cacugucagc uuggucuugt t                                                21


<210>  961
<211>  21
<212>  DNA
<213>  Homo sapiens

<400>  961
aagaccaagc tgacagtgct g                                                21


<210>  962
<211>  21
<212>  DNA
<213>  Homo sapiens

<400>  962
gaccaagcug acagugcugt t                                                21


<210>  963
<211>  21
<212>  DNA
<213>  Homo sapiens

<400>  963
cagcacuguc agcuugguct t                                                21


<210>  964
<211>  18
<212>  DNA
<213>  Homo sapiens

<400>  964
aagctgacag tgctggaa                                                    18


<210>  965
<211>  18
<212>  DNA
<213>  Homo sapiens

<400>  965
gcugacagug cuggaatt                                                    18


<210>  966
<211>  18
<212>  DNA
<213>  Homo sapiens

<400>  966
uuccagcacu gucagctt                                                    18


<210>  967
<211>  18

<212>  DNA
<213>  Homo sapiens

<400>  967
aaggagacat tctggatg                                                    18


<210>  968
<211>  18
<212>  DNA
<213>  Homo sapiens

<400>  968
ggagacauuc uggaugtt                                                    18


<210>  969
<211>  18
<212>  DNA
<213>  Homo sapiens

<400>  969
cauccagaau gucucctt                                                    18


<210>  970
<211>  21
<212>  DNA
<213>  Homo sapiens

<400>  970
aagagagcct gccaggacgt c                                                21


<210>  971
<211>  21
<212>  DNA
<213>  Homo sapiens

<400>  971
gagagccugc caggacguct t                                                21


<210>  972
<211>  21
<212>  DNA
<213>  Homo sapiens

<400>  972
gacguccugg caggcucuct t                                                21


<210>  973
<211>  18
<212>  DNA
<213>  Homo sapiens

<400>  973
aatgtcaatg tgaaaggt                                                    18


<210>  974
<211>  18
<212>  DNA
<213>  Homo sapiens

<400>  974
ugucaaugug aaaggutt                                                    18


<210>  975
<211>  18
<212>  DNA
<213>  Homo sapiens

<400>  975
accuuucaca uugacatt                                                    18


<210>  976
<211>  21
<212>  DNA
<213>  Homo sapiens

<400>  976
aatgtgaaag gtacccagct c                                                21


<210>  977
<211>  21
<212>  DNA
<213>  Homo sapiens

<400>  977
ugugaaaggu acccagcuct t                                                21


<210>  978
<211>  21
<212>  DNA
<213>  Homo sapiens

<400>  978
gagcugggua ccuuucacat t                                                21


<210>  979
<211>  21
<212>  DNA
<213>  Homo sapiens

<400>  979
aaaggtaccc agctcctgtt a                                                21


<210>  980
<211>  21
<212>  DNA
<213>  Homo sapiens

<400>  980
agguacccag cuccuguuat t                                                21


<210>  981
<211>  21
<212>  DNA
<213>  Homo sapiens

<400>  981
   uaacaggagc uggguaccut t                                                21

<210>  982
<211>  20
<212>  DNA
<213>  Homo sapiens

<400>  982
aagctagtgt gccagtcttc                                                 20

<210>  983
<211>  20
<212>  DNA
<213>  Homo sapiens

<400>  983
gcuagugugc cagucuuctt                                                 20

<210>  984
<211>  20
<212>  DNA
<213>  Homo sapiens

<400>  984
gaagacuggc acacuagctt                                                 20

<210>  985
<211>  21
<212>  DNA
<213>  Homo sapiens

<400>  985
aactcctaca aggaaatcat c                                               21

<210>  986
<211>  21
<212>  DNA
<213>  Homo sapiens

<400>  986
cuccuacaag gaaaucauct t                                               21

<210>  987
<211>  21
<212>  DNA
<213>  Homo sapiens

<400>  987
gaugauuucc uuguaggagt t                                               21

<210>  988
<211>  18
<212>  DNA
<213>  Homo sapiens

<400>  988
aaggaaatca tccagaat                                                   18

<210>  989
<211>  18
<212>  DNA
<213>  Homo sapiens

<400>  989
ggaaaucauc cagaautt                                                    18


<210>  990
<211>  18
<212>  DNA
<213>  Homo sapiens

<400>  990
auucuggaug auuucctt                                                    18


<210>  991
<211>  18
<212>  DNA
<213>  Homo sapiens

<400>  991
aaatcatcca gaatggcc                                                    18


<210>  992
<211>  18
<212>  DNA
<213>  Homo sapiens

<400>  992
aucauccaga auggcctt                                                    18


<210>  993
<211>  18
<212>  DNA
<213>  Homo sapiens

<400>  993
ggccauucug gaugautt                                                    18


<210>  994
<211>  18
<212>  DNA
<213>  Homo sapiens

<400>  994
aatggccatg aagaagag                                                    18


<210>  995
<211>  18
<212>  DNA
<213>  Homo sapiens

<400>  995
uggccaugaa gaagagtt                                                    18


<210>  996

<211>  18
<212>  DNA
<213>  Homo sapiens

<400>  996
cucuucuuca uggccatt                                                    18


<210>  997
<211>  21
<212>  DNA
<213>  Homo sapiens

<400>  997
aagaagagcc tctggaaaac a                                                21


<210>  998
<211>  21
<212>  DNA
<213>  Homo sapiens

<400>  998
gaagagccuc uggaaaacat t                                                21


<210>  999
<211>  21
<212>  DNA
<213>  Homo sapiens

<400>  999
uguuuuccag aggcucuuct t                                                21


<210>  1000
<211>  21
<212>  DNA
<213>  Homo sapiens

<400>  1000
aagagcctct ggaaaacaca t                                                21


<210>  1001
<211>  21
<212>  DNA
<213>  Homo sapiens

<400>  1001
gagccucugg aaaacacaut t                                                21


<210>  1002
<211>  21
<212>  DNA
<213>  Homo sapiens

<400>  1002
auguguuuuc cagaggcuct t                                                21


<210>  1003
<211>  21
<212>  DNA

<213>  Homo sapiens

<400>  1003
aaaacacatg gcccgctcca t                                                21


<210>  1004
<211>  21
<212>  DNA
<213>  Homo sapiens

<400>  1004
aacacauggc ccgcuccaut t                                                21


<210>  1005
<211>  21
<212>  DNA
<213>  Homo sapiens

<400>  1005
auggagcggg ccauguguut t                                                21


<210>  1006
<211>  21
<212>  DNA
<213>  Homo sapiens

<400>  1006
aacacatggc ccgctccata c                                                21


<210>  1007
<211>  21
<212>  DNA
<213>  Homo sapiens

<400>  1007
cacauggccc gcuccauact t                                                21


<210>  1008
<211>  21
<212>  DNA
<213>  Homo sapiens

<400>  1008
guauggagcg ggccaugugt t                                                21


<210>  1009
<211>  18
<212>  DNA
<213>  Homo sapiens

<400>  1009
aaaaagcttg ctgagaag                                                    18


<210>  1010
<211>  18
<212>  DNA
<213>  Homo sapiens

<400>  1010
aaagcuugcu gagaagtt                                                    18


<210>  1011
<211>  18
<212>  DNA
<213>  Homo sapiens

<400>  1011
cuucucagca agcuuutt                                                    18


<210>  1012
<211>  18
<212>  DNA
<213>  Homo sapiens

<400>  1012
aaagcttgct gagaaggc                                                    18


<210>  1013
<211>  18
<212>  DNA
<213>  Homo sapiens

<400>  1013
agcuugcuga gaaggctt                                                    18


<210>  1014
<211>  18
<212>  DNA
<213>  Homo sapiens

<400>  1014
gccuucucag caagcutt                                                    18


<210>  1015
<211>  21
<212>  DNA
<213>  Homo sapiens

<400>  1015
aaggctgtac tggcggctaa c                                                21


<210>  1016
<211>  21
<212>  DNA
<213>  Homo sapiens

<400>  1016
ggcuguacug gcggcuaact t                                                21


<210>  1017
<211>  21
<212>  DNA
<213>  Homo sapiens

<400>  1017
guuagccgcc aguacagcct t                                                21

<210>  1018
<211>  21
<212>  DNA
<213>  Homo sapiens

<400>  1018
aacgggtgga atctgaaaaa c                                                21


<210>  1019
<211>  21
<212>  DNA
<213>  Homo sapiens

<400>  1019
cggguggaau cugaaaaact t                                                21


<210>  1020
<211>  21
<212>  DNA
<213>  Homo sapiens

<400>  1020
guuuuucaga uuccacccgt t                                                21


<210>  1021
<211>  21
<212>  DNA
<213>  Homo sapiens

<400>  1021
aatctgaaaa acggcggcac c                                                21


<210>  1022
<211>  21
<212>  DNA
<213>  Homo sapiens

<400>  1022
ucugaaaaac ggcggcacct t                                                21


<210>  1023
<211>  21
<212>  DNA
<213>  Homo sapiens

<400>  1023
ggugccgccg uuuuucagat t                                                21


<210>  1024
<211>  18
<212>  DNA
<213>  Homo sapiens

<400>  1024
aaaaacggcg gcaccctg                                                    18

<210> 1025
<211> 18
<212> DNA
<213> Homo sapiens

<400> 1025
aaacggcggc acccugtt 18


<210> 1026
<211> 18
<212> DNA
<213> Homo sapiens

<400> 1026
cagggugccg ccguuutt 18


<210> 1027
<211> 18
<212> DNA
<213> Homo sapiens

<400> 1027
aaacggcggc accctgta 18


<210> 1028
<211> 18
<212> DNA
<213> Homo sapiens

<400> 1028
acggcggcac ccuguatt 18


<210> 1029
<211> 18
<212> DNA
<213> Homo sapiens

<400> 1029
uacagggugc cgccgutt 18


<210> 1030
<211> 21
<212> DNA
<213> Homo sapiens

<400> 1030
aaggaagccg attcctttct g 21


<210> 1031
<211> 21
<212> DNA
<213> Homo sapiens

<400> 1031
ggaagccgau uccuuucugt t 21


<210> 1032
<211> 21

<212>  DNA
<213>  Homo sapiens

<400>  1032
cagaaaggaa ucggcuucct t                                                21


<210>  1033
<211>  19
<212>  DNA
<213>  Homo sapiens

<400>  1033
aagccgattc ctttctgct                                                   19


<210>  1034
<211>  19
<212>  DNA
<213>  Homo sapiens

<400>  1034
gccgauuccu uucugcutt                                                   19


<210>  1035
<211>  19
<212>  DNA
<213>  Homo sapiens

<400>  1035
agcagaaagg aaucggctt                                                   19


<210>  1036
<211>  21
<212>  DNA
<213>  Homo sapiens

<400>  1036
aaacgaggcc ctgaacaaca a                                                21


<210>  1037
<211>  21
<212>  DNA
<213>  Homo sapiens

<400>  1037
acgaggcccu gaacaacaat t                                                21


<210>  1038
<211>  21
<212>  DNA
<213>  Homo sapiens

<400>  1038
uuguuguuca gggccucgut t                                                21


<210>  1039
<211>  21
<212>  DNA
<213>  Homo sapiens

<400>  1039
aacaacaatg ggatcctgtc a                                                21


<210>  1040
<211>  21
<212>  DNA
<213>  Homo sapiens

<400>  1040
caacaauggg auccugucat t                                                21


<210>  1041
<211>  21
<212>  DNA
<213>  Homo sapiens

<400>  1041
ugacaggauc ccauuguugt t                                                21


<210>  1042
<211>  21
<212>  DNA
<213>  Homo sapiens

<400>  1042
aacaatggga tcctgtcaag t                                                21


<210>  1043
<211>  21
<212>  DNA
<213>  Homo sapiens

<400>  1043
caaugggauc cugucaagut t                                                21


<210>  1044
<211>  21
<212>  DNA
<213>  Homo sapiens

<400>  1044
acuugacagg aucccauugt t                                                21


<210>  1045
<211>  21
<212>  DNA
<213>  Homo sapiens

<400>  1045
aatgggatcc tgtcaagtgt t                                                21


<210>  1046
<211>  21
<212>  DNA
<213>  Homo sapiens

<400>  1046

ugggauccug ucaaguguut t                                                21

<210>  1047
<211>  21
<212>  DNA
<213>  Homo sapiens

<400>  1047
aacacuugac aggaucccat t                                                21

<210>  1048
<211>  21
<212>  DNA
<213>  Homo sapiens

<400>  1048
aagtgttgga aagttctcca c                                                21

<210>  1049
<211>  21
<212>  DNA
<213>  Homo sapiens

<400>  1049
guguuggaaa guucuccact t                                                21

<210>  1050
<211>  21
<212>  DNA
<213>  Homo sapiens

<400>  1050
guggagaacu uuccaacact t                                                21

<210>  1051
<211>  18
<212>  DNA
<213>  Homo sapiens

<400>  1051
aaagttctcc actgttaa                                                    18

<210>  1052
<211>  18
<212>  DNA
<213>  Homo sapiens

<400>  1052
aguucuccac uguuaatt                                                    18

<210>  1053
<211>  18
<212>  DNA
<213>  Homo sapiens

<400>  1053
uuaacagugg agaacutt                                                    18

<210>  1054
<211>  21
<212>  DNA
<213>  Homo sapiens

<400>  1054
aacccagtct atgttggcaa t                                                21


<210>  1055
<211>  21
<212>  DNA
<213>  Homo sapiens

<400>  1055
cccagucuau guuggcaaut t                                                21


<210>  1056
<211>  21
<212>  DNA
<213>  Homo sapiens

<400>  1056
auugccaaca uagacugggt t                                                21


<210>  1057
<211>  21
<212>  DNA
<213>  Homo sapiens

<400>  1057
aatgtggcct gggcccacat t                                                21


<210>  1058
<211>  21
<212>  DNA
<213>  Homo sapiens

<400>  1058
uguggccugg gcccacauut t                                                21


<210>  1059
<211>  21
<212>  DNA
<213>  Homo sapiens

<400>  1059
aaugugggcc caggccacat t                                                21


<210>  1060
<211>  21
<212>  DNA
<213>  Homo sapiens

<400>  1060
aagaaggccc caagcatccg a                                                21


<210>  1061

<211>  21
<212>  DNA
<213>  Homo sapiens

<400>  1061
gaaggcccca agcauccgat t                                                21


<210>  1062
<211>  21
<212>  DNA
<213>  Homo sapiens

<400>  1062
ucggaugcuu ggggccuuct t                                                21


<210>  1063
<211>  21
<212>  DNA
<213>  Homo sapiens

<400>  1063
aaggccccaa gcatccgagg a                                                21


<210>  1064
<211>  21
<212>  DNA
<213>  Homo sapiens

<400>  1064
ggccccaagc auccgaggat t                                                21


<210>  1065
<211>  21
<212>  DNA
<213>  Homo sapiens

<400>  1065
uccucggaug cuuggggcct t                                                21


<210>  1066
<211>  21
<212>  DNA
<213>  Homo sapiens

<400>  1066
aagcatccga ggacagttct a                                                21


<210>  1067
<211>  21
<212>  DNA
<213>  Homo sapiens

<400>  1067
gcauccgagg acaguucuat t                                                21


<210>  1068
<211>  21
<212>  DNA

<213>  Homo sapiens

<400>  1068
uagaacuguc cucggaugct t                                                21


<210>  1069
<211>  21
<212>  DNA
<213>  Homo sapiens

<400>  1069
aaagctatga taaccttaat t                                                21


<210>  1070
<211>  21
<212>  DNA
<213>  Homo sapiens

<400>  1070
agcuaugaua accuuaauut t                                                21


<210>  1071
<211>  21
<212>  DNA
<213>  Homo sapiens

<400>  1071
aauuaagguu aucauagcut t                                                21


<210>  1072
<211>  21
<212>  DNA
<213>  Homo sapiens

<400>  1072
aaccttaatt acaccctgag c                                                21


<210>  1073
<211>  21
<212>  DNA
<213>  Homo sapiens

<400>  1073
ccuuaauuac acccugagct t                                                21


<210>  1074
<211>  21
<212>  DNA
<213>  Homo sapiens

<400>  1074
gcucagggug uaauuaaggt t                                                21


<210>  1075
<211>  21
<212>  DNA
<213>  Homo sapiens

<400>  1075
aattacaccc tgagcaaaga g                                                21


<210>  1076
<211>  21
<212>  DNA
<213>  Homo sapiens

<400>  1076
uuacacccug agcaaagagt t                                                21


<210>  1077
<211>  21
<212>  DNA
<213>  Homo sapiens

<400>  1077
cucuuugcuc aggguguaat t                                                21


<210>  1078
<211>  21
<212>  DNA
<213>  Homo sapiens

<400>  1078
aaagagttcg gcctccgcct t                                                21


<210>  1079
<211>  21
<212>  DNA
<213>  Homo sapiens

<400>  1079
agaguucggc cuccgccuut t                                                21


<210>  1080
<211>  21
<212>  DNA
<213>  Homo sapiens

<400>  1080
aaggcggagg ccgaacucut t                                                21


<210>  1081
<211>  18
<212>  DNA
<213>  Homo sapiens

<400>  1081
aaatagtgag cttcctac                                                    18


<210>  1082
<211>  18
<212>  DNA
<213>  Homo sapiens

<400>  1082
auagugagcu uccuactt                                                    18

<210>  1083
<211>  18
<212>  DNA
<213>  Homo sapiens

<400>  1083
guaggaagcu cacuautt                                                    18


<210>  1084
<211>  21
<212>  DNA
<213>  Homo sapiens

<400>  1084
aatttacacc tatcgaccgc c                                                21


<210>  1085
<211>  21
<212>  DNA
<213>  Homo sapiens

<400>  1085
uuuacaccua ucgaccgcct t                                                21


<210>  1086
<211>  21
<212>  DNA
<213>  Homo sapiens

<400>  1086
ggcggucgau agguguaaat t                                                21


<210>  1087
<211>  18
<212>  DNA
<213>  Homo sapiens

<400>  1087
aaccgccaca tagtcaca                                                    18


<210>  1088
<211>  18
<212>  DNA
<213>  Homo sapiens

<400>  1088
ccgccacaua gucacatt                                                    18


<210>  1089
<211>  18
<212>  DNA
<213>  Homo sapiens

<400>  1089
ugugacuaug uggcggtt                                                    18

<210>  1090
<211>  21
<212>  DNA
<213>  Homo sapiens

<400>  1090
aaatagcgta ttcaccttct c                                                21


<210>  1091
<211>  21
<212>  DNA
<213>  Homo sapiens

<400>  1091
auagcguauu caccuucuct t                                                21


<210>  1092
<211>  21
<212>  DNA
<213>  Homo sapiens

<400>  1092
gagaagguga auacgcuaut t                                                21


<210>  1093
<211>  21
<212>  DNA
<213>  Homo sapiens

<400>  1093
aagaaggctc agcgagatct g                                                21


<210>  1094
<211>  21
<212>  DNA
<213>  Homo sapiens

<400>  1094
gaaggcucag cgagaucugt t                                                21


<210>  1095
<211>  21
<212>  DNA
<213>  Homo sapiens

<400>  1095
cagaucucgc ugagccuuct t                                                21


<210>  1096
<211>  21
<212>  DNA
<213>  Homo sapiens

<400>  1096
aaggctcagc gagatctggc g                                                21


<210>  1097
<211>  21

<212>  DNA
<213>  Homo sapiens

<400>  1097
ggcucagcga gaucuggcgt t                                                21


<210>  1098
<211>  21
<212>  DNA
<213>  Homo sapiens

<400>  1098
cgccagaucu cgcugagcct t                                                21


<210>  1099
<211>  21
<212>  DNA
<213>  Homo sapiens

<400>  1099
aagccactct acagctggga g                                                21


<210>  1100
<211>  21
<212>  DNA
<213>  Homo sapiens

<400>  1100
gccacucuac agcugggagt t                                                21


<210>  1101
<211>  21
<212>  DNA
<213>  Homo sapiens

<400>  1101
cucccagcug uagaguggct t                                                21


<210>  1102
<211>  21
<212>  DNA
<213>  Homo sapiens

<400>  1102
aagccaagca gaaaacggtg g                                                21


<210>  1103
<211>  21
<212>  DNA
<213>  Homo sapiens

<400>  1103
   gccaagcaga aaacgguggt t                                                21


<210>  1104
<211>  21
<212>  DNA
<213>  Homo sapiens

<400>  1104
ccaccguuuu cugcuuggct t                                                21

<210>  1105
<211>  21
<212>  DNA
<213>  Homo sapiens

<400>  1105
aagcagaaaa cggtggagtg g                                                21

<210>  1106
<211>  21
<212>  DNA
<213>  Homo sapiens

<400>  1106
gcagaaaacg guggaguggt t                                                21

<210>  1107
<211>  21
<212>  DNA
<213>  Homo sapiens

<400>  1107
ccacuccacc guuuucugct t                                                21

<210>  1108
<211>  21
<212>  DNA
<213>  Homo sapiens

<400>  1108
aaaacggtgg agtgggttgg t                                                21

<210>  1109
<211>  21
<212>  DNA
<213>  Homo sapiens

<400>  1109
aacgguggag uggguuggut t                                                21

<210>  1110
<211>  21
<212>  DNA
<213>  Homo sapiens

<400>  1110
accaacccac uccaccguut t                                                21

<210>  1111
<211>  21
<212>  DNA
<213>  Homo sapiens

<400>  1111

aacggtggag tgggttggtt c                                              21


<210>  1112
<211>  21
<212>  DNA
<213>  Homo sapiens

<400>  1112
cgguggagug gguugguuct t                                              21


<210>  1113
<211>  21
<212>  DNA
<213>  Homo sapiens

<400>  1113
gaaccaaccc acuccaccgt t                                              21


<210>  1114
<211>  21
<212>  DNA
<213>  Homo sapiens

<400>  1114
aaggagaccc tgaagtccaa g                                              21


<210>  1115
<211>  21
<212>  DNA
<213>  Homo sapiens

<400>  1115
ggagacccug aaguccaagt t                                              21


<210>  1116
<211>  21
<212>  DNA
<213>  Homo sapiens

<400>  1116
cuuggacuuc agggucucct t                                              21


<210>  1117
<211>  1122
<212>  DNA
<213>  Homo sapiens

<400>  1117
atgacgggct ggagctgcct tgtgacagga gcaggagggt ttctgggaca gaggatcatc      60

cgcctcttgg tgaaggagaa ggagctgaag gagatcaggg tcttggacaa ggccttcgga     120

ccagaattga gagaggaatt ttctaaactc cagaacaaga ccaagctgac agtgctggaa     180

ggagacattc tggatgagcc attcctgaag agagcctgcc aggacgtctc ggtcatcatc     240

cacaccgcct gtatcattga tgtcttcggt gtcactcaca gagagtctat catgaatgtc     300

aatgtgaaag gtacccagct cctgttagag gcctgtgtcc aagctagtgt gccagtcttc     360

atctacacca gtagcataga ggtagccggg cccaactcct acaaggaaat catccagaat 420

ggccatgaag aagagcctct ggaaaacaca tggcccgctc catacccaca cagcaaaaag 480

cttgctgaga aggctgtact ggcggctaac gggtggaatc tgaaaaacgg cggcaccctg 540

tacacttgtg ccttacgacc catgtatatc tatggggaag gaagccgatt cctttctgct 600

agtataaacg aggccctgaa caacaatggg atcctgtcaa gtgttggaaa gttctccact 660

gttaacccag tctatgttgg caatgtggcc tgggcccaca ttctggcctt gagggccctg 720

caggacccca agaaggcccc aagcatccga ggacagttct actatatctc agatgacacg 780

cctcaccaaa gctatgataa ccttaattac accctgagca aagagttcgg cctccgcctt 840

gattccagat ggagctttcc tttatccctg atgtattgga ttggcttcct gctggaaata 900

gtgagcttcc tactcaggcc aatttacacc tatcgaccgc ccttcaaccg ccacatagtc 960

acattgtcaa atagcgtatt caccttctct tataagaagg ctcagcgaga tctggcgtat 1020

aagccactct acagctggga ggaagccaag cagaaaacgg tggagtgggt tggttccctt 1080

gtggaccggc acaaggagac cctgaagtcc aagactcagt ga 1122

<210> 1118
<211> 21
<212> DNA
<213> Homo sapiens

<400> 1118
aaggagaagg agctgaagga g 21

<210> 1119
<211> 21
<212> DNA
<213> Homo sapiens

<400> 1119
ggagaaggag cugaaggagt t 21

<210> 1120
<211> 21
<212> DNA
<213> Homo sapiens

<400> 1120
cuccuucagc uccuucucct t 21

<210> 1121
<211> 21
<212> DNA
<213> Homo sapiens

<400> 1121
aaggagctga aggagatcag g 21

<210> 1122

<211>  21
<212>  DNA
<213>  Homo sapiens

<400>  1122
ggagcugaag gagaucaggt t                                                21


<210>  1123
<211>  21
<212>  DNA
<213>  Homo sapiens

<400>  1123
ccugaucucc uucagcucct t                                                21


<210>  1124
<211>  21
<212>  DNA
<213>  Homo sapiens

<400>  1124
aaggagatca gggtcttgga c                                                21


<210>  1125
<211>  21
<212>  DNA
<213>  Homo sapiens

<400>  1125
ggagaucagg gucuuggact t                                                21


<210>  1126
<211>  21
<212>  DNA
<213>  Homo sapiens

<400>  1126
guccaagacc cugaucucct t                                                21


<210>  1127
<211>  18
<212>  DNA
<213>  Homo sapiens

<400>  1127
aaggccttcg gaccagaa                                                    18


<210>  1128
<211>  18
<212>  DNA
<213>  Homo sapiens

<400>  1128
ggccuucgga ccagaatt                                                    18


<210>  1129
<211>  18
<212>  DNA

<213>  Homo sapiens

<400>  1129
uucugguccg aaggcctt                                                    18


<210>  1130
<211>  21
<212>  DNA
<213>  Homo sapiens

<400>  1130
aattgagaga ggaattttct a                                                21


<210>  1131
<211>  21
<212>  DNA
<213>  Homo sapiens

<400>  1131
uugagagagg aauuuucuat t                                                21


<210>  1132
<211>  21
<212>  DNA
<213>  Homo sapiens

<400>  1132
uagaaaauuc cucucucaat t                                                21


<210>  1133
<211>  21
<212>  DNA
<213>  Homo sapiens

<400>  1133
aattttctaa actccagaac a                                                21


<210>  1134
<211>  21
<212>  DNA
<213>  Homo sapiens

<400>  1134
uuuucuaaac uccagaacat t                                                21


<210>  1135
<211>  21
<212>  DNA
<213>  Homo sapiens

<400>  1135
uguucuggag uuuagaaaat t                                                21


<210>  1136
<211>  21
<212>  DNA
<213>  Homo sapiens

<400>  1136
aaactccaga acaagaccaa g                                                21


<210>  1137
<211>  21
<212>  DNA
<213>  Homo sapiens

<400>  1137
acuccagaac aagaccaagt t                                                21


<210>  1138
<211>  21
<212>  DNA
<213>  Homo sapiens

<400>  1138
cuuggucuug uucuggagut t                                                21


<210>  1139
<211>  21
<212>  DNA
<213>  Homo sapiens

<400>  1139
aacaagacca agctgacagt g                                                21


<210>  1140
<211>  21
<212>  DNA
<213>  Homo sapiens

<400>  1140
caagaccaag cugacagugt t                                                21


<210>  1141
<211>  21
<212>  DNA
<213>  Homo sapiens

<400>  1141
cacugucagc uuggucuugt t                                                21


<210>  1142
<211>  21
<212>  DNA
<213>  Homo sapiens

<400>  1142
aagaccaagc tgacagtgct g                                                21


<210>  1143
<211>  21
<212>  DNA
<213>  Homo sapiens

<400>  1143
gaccaagcug acagugcugt t                                                21

<210>  1144
<211>  21
<212>  DNA
<213>  Homo sapiens

<400>  1144
cagcacuguc agcuugguct t                                                21


<210>  1145
<211>  18
<212>  DNA
<213>  Homo sapiens

<400>  1145
aagctgacag tgctggaa                                                    18


<210>  1146
<211>  18
<212>  DNA
<213>  Homo sapiens

<400>  1146
gcugacagug cuggaatt                                                    18


<210>  1147
<211>  18
<212>  DNA
<213>  Homo sapiens

<400>  1147
uuccagcacu gucagctt                                                    18


<210>  1148
<211>  18
<212>  DNA
<213>  Homo sapiens

<400>  1148
aaggagacat tctggatg                                                    18


<210>  1149
<211>  18
<212>  DNA
<213>  Homo sapiens

<400>  1149
ggagacauuc uggaugtt                                                    18


<210>  1150
<211>  18
<212>  DNA
<213>  Homo sapiens

<400>  1150
cauccagaau gucucctt                                                    18

<210> 1151
<211> 21
<212> DNA
<213> Homo sapiens

<400> 1151
aagagagcct gccaggacgt c 21


<210> 1152
<211> 21
<212> DNA
<213> Homo sapiens

<400> 1152
gagagccugc caggacguct t 21


<210> 1153
<211> 21
<212> DNA
<213> Homo sapiens

<400> 1153
gacguccugg caggcucuct t 21


<210> 1154
<211> 18
<212> DNA
<213> Homo sapiens

<400> 1154
aatgtcaatg tgaaaggt 18


<210> 1155
<211> 18
<212> DNA
<213> Homo sapiens

<400> 1155
ugucaaugug aaaggutt 18


<210> 1156
<211> 18
<212> DNA
<213> Homo sapiens

<400> 1156
accuuucaca uugacatt 18


<210> 1157
<211> 21
<212> DNA
<213> Homo sapiens

<400> 1157
aatgtgaaag gtacccagct c 21


<210> 1158
<211> 21

<212>  DNA
<213>  Homo sapiens

<400>  1158
ugugaaaggu acccagcuct t                                                21


<210>  1159
<211>  21
<212>  DNA
<213>  Homo sapiens

<400>  1159
gagcugggua ccuuucacat t                                                21


<210>  1160
<211>  21
<212>  DNA
<213>  Homo sapiens

<400>  1160
aaaggtaccc agctcctgtt a                                                21


<210>  1161
<211>  21
<212>  DNA
<213>  Homo sapiens

<400>  1161
agguacccag cuccuguuat t                                                21


<210>  1162
<211>  21
<212>  DNA
<213>  Homo sapiens

<400>  1162
   uaacaggagc uggguaccut t                                               21


<210>  1163
<211>  20
<212>  DNA
<213>  Homo sapiens

<400>  1163
aagctagtgt gccagtcttc                                                  20


<210>  1164
<211>  20
<212>  DNA
<213>  Homo sapiens

<400>  1164
gcuagugugc cagucuuctt                                                  20


<210>  1165
<211>  20
<212>  DNA
<213>  Homo sapiens

<400>  1165
gaagacuggc acacuagctt                                                    20


<210>  1166
<211>  21
<212>  DNA
<213>  Homo sapiens

<400>  1166
aactcctaca aggaaatcat c                                                  21


<210>  1167
<211>  21
<212>  DNA
<213>  Homo sapiens

<400>  1167
cuccuacaag gaaaucauct t                                                  21


<210>  1168
<211>  21
<212>  DNA
<213>  Homo sapiens

<400>  1168
gaugauuucc uuguaggagt t                                                  21


<210>  1169
<211>  18
<212>  DNA
<213>  Homo sapiens

<400>  1169
aaggaaatca tccagaat                                                      18


<210>  1170
<211>  18
<212>  DNA
<213>  Homo sapiens

<400>  1170
ggaaaucauc cagaautt                                                      18


<210>  1171
<211>  18
<212>  DNA
<213>  Homo sapiens

<400>  1171
auucuggaug auuucctt                                                      18


<210>  1172
<211>  18
<212>  DNA
<213>  Homo sapiens

<400>  1172

aaatcatcca gaatggcc                                                    18

<210>  1173
<211>  18
<212>  DNA
<213>  Homo sapiens

<400>  1173
aucauccaga auggcctt                                                    18

<210>  1174
<211>  18
<212>  DNA
<213>  Homo sapiens

<400>  1174
ggccauucug gaugautt                                                    18

<210>  1175
<211>  18
<212>  DNA
<213>  Homo sapiens

<400>  1175
aatggccatg aagaagag                                                    18

<210>  1176
<211>  18
<212>  DNA
<213>  Homo sapiens

<400>  1176
uggccaugaa gaagagtt                                                    18

<210>  1177
<211>  18
<212>  DNA
<213>  Homo sapiens

<400>  1177
cucuucuuca uggccatt                                                    18

<210>  1178
<211>  21
<212>  DNA
<213>  Homo sapiens

<400>  1178
aagaagagcc tctggaaaac a                                                21

<210>  1179
<211>  21
<212>  DNA
<213>  Homo sapiens

<400>  1179
gaagagccuc uggaaaacat t                                                21

<210>  1180
<211>  21
<212>  DNA
<213>  Homo sapiens

<400>  1180
uguuuuccag aggcucuuct t                                                21


<210>  1181
<211>  21
<212>  DNA
<213>  Homo sapiens

<400>  1181
aagagcctct ggaaaacaca t                                                21


<210>  1182
<211>  21
<212>  DNA
<213>  Homo sapiens

<400>  1182
gagccucugg aaaacacaut t                                                21


<210>  1183
<211>  21
<212>  DNA
<213>  Homo sapiens

<400>  1183
auguguuuuc cagaggcuct t                                                21


<210>  1184
<211>  21
<212>  DNA
<213>  Homo sapiens

<400>  1184
aaaacacatg gcccgctcca t                                                21


<210>  1185
<211>  21
<212>  DNA
<213>  Homo sapiens

<400>  1185
aacacauggc ccgcuccaut t                                                21


<210>  1186
<211>  21
<212>  DNA
<213>  Homo sapiens

<400>  1186
auggagcggg ccauguguut t                                                21


<210>  1187

<211>  21
<212>  DNA
<213>  Homo sapiens

<400>  1187
aacacatggc ccgctccata c                                                21


<210>  1188
<211>  21
<212>  DNA
<213>  Homo sapiens

<400>  1188
cacauggccc gcuccauact t                                                21


<210>  1189
<211>  21
<212>  DNA
<213>  Homo sapiens

<400>  1189
guauggagcg ggccaugugt t                                                21


<210>  1190
<211>  18
<212>  DNA
<213>  Homo sapiens

<400>  1190
aaaaagcttg ctgagaag                                                    18


<210>  1191
<211>  18
<212>  DNA
<213>  Homo sapiens

<400>  1191
aaagcuugcu gagaagtt                                                    18


<210>  1192
<211>  18
<212>  DNA
<213>  Homo sapiens

<400>  1192
cuucucagca agcuuutt                                                    18


<210>  1193
<211>  18
<212>  DNA
<213>  Homo sapiens

<400>  1193
aaagcttgct gagaaggc                                                    18


<210>  1194
<211>  18
<212>  DNA

<213>  Homo sapiens

<400>  1194
agcuugcuga gaaggctt                                                    18


<210>  1195
<211>  18
<212>  DNA
<213>  Homo sapiens

<400>  1195
gccuucucag caagcutt                                                    18


<210>  1196
<211>  21
<212>  DNA
<213>  Homo sapiens

<400>  1196
aaggctgtac tggcggctaa c                                                21


<210>  1197
<211>  21
<212>  DNA
<213>  Homo sapiens

<400>  1197
ggcuguacug gcggcuaact t                                                21


<210>  1198
<211>  21
<212>  DNA
<213>  Homo sapiens

<400>  1198
guuagccgcc aguacagcct t                                                21


<210>  1199
<211>  21
<212>  DNA
<213>  Homo sapiens

<400>  1199
aacgggtgga atctgaaaaa c                                                21


<210>  1200
<211>  21
<212>  DNA
<213>  Homo sapiens

<400>  1200
cggguggaau cugaaaaact t                                                21


<210>  1201
<211>  21
<212>  DNA
<213>  Homo sapiens

<400>  1201
guuuuucaga uuccacccgt t                                                21


<210>  1202
<211>  21
<212>  DNA
<213>  Homo sapiens

<400>  1202
aatctgaaaa acggcggcac c                                                21


<210>  1203
<211>  21
<212>  DNA
<213>  Homo sapiens

<400>  1203
ucugaaaaac ggcggcacct t                                                21


<210>  1204
<211>  21
<212>  DNA
<213>  Homo sapiens

<400>  1204
ggugccgccg uuuuucagat t                                                21


<210>  1205
<211>  18
<212>  DNA
<213>  Homo sapiens

<400>  1205
aaaaacggcg gcaccctg                                                    18


<210>  1206
<211>  18
<212>  DNA
<213>  Homo sapiens

<400>  1206
aaacggcggc acccugtt                                                    18


<210>  1207
<211>  18
<212>  DNA
<213>  Homo sapiens

<400>  1207
cagggugccg ccguuutt                                                    18


<210>  1208
<211>  18
<212>  DNA
<213>  Homo sapiens

<400>  1208
aaacggcggc accctgta                                                    18

<210>  1209
<211>  18
<212>  DNA
<213>  Homo sapiens

<400>  1209
acggcggcac ccuguatt                                                    18


<210>  1210
<211>  18
<212>  DNA
<213>  Homo sapiens

<400>  1210
uacagggugc cgccgutt                                                    18


<210>  1211
<211>  21
<212>  DNA
<213>  Homo sapiens

<400>  1211
aaggaagccg attcctttct g                                                21


<210>  1212
<211>  21
<212>  DNA
<213>  Homo sapiens

<400>  1212
ggaagccgau uccuuucugt t                                                21


<210>  1213
<211>  21
<212>  DNA
<213>  Homo sapiens

<400>  1213
cagaaaggaa ucggcuucct t                                                21


<210>  1214
<211>  19
<212>  DNA
<213>  Homo sapiens

<400>  1214
aagccgattc ctttctgct                                                   19


<210>  1215
<211>  19
<212>  DNA
<213>  Homo sapiens

<400>  1215
gccgauuccu uucugcutt                                                   19

<210>  1216
<211>  19
<212>  DNA
<213>  Homo sapiens

<400>  1216
agcagaaagg aaucggctt                                                    19


<210>  1217
<211>  21
<212>  DNA
<213>  Homo sapiens

<400>  1217
aaacgaggcc ctgaacaaca a                                                 21


<210>  1218
<211>  21
<212>  DNA
<213>  Homo sapiens

<400>  1218
acgaggcccu gaacaacaat t                                                 21


<210>  1219
<211>  21
<212>  DNA
<213>  Homo sapiens

<400>  1219
uuguuguuca gggccucgut t                                                 21


<210>  1220
<211>  21
<212>  DNA
<213>  Homo sapiens

<400>  1220
aacaacaatg ggatcctgtc a                                                 21


<210>  1221
<211>  21
<212>  DNA
<213>  Homo sapiens

<400>  1221
caacaauggg auccugucat t                                                 21


<210>  1222
<211>  21
<212>  DNA
<213>  Homo sapiens

<400>  1222
ugacaggauc ccauuguugt t                                                 21


<210>  1223
<211>  21

<212>  DNA
<213>  Homo sapiens

<400>  1223
aacaatggga tcctgtcaag t                                                21


<210>  1224
<211>  21
<212>  DNA
<213>  Homo sapiens

<400>  1224
caaugggauc cugucaagut t                                                21


<210>  1225
<211>  21
<212>  DNA
<213>  Homo sapiens

<400>  1225
acuugacagg aucccauugt t                                                21


<210>  1226
<211>  21
<212>  DNA
<213>  Homo sapiens

<400>  1226
aatgggatcc tgtcaagtgt t                                                21


<210>  1227
<211>  21
<212>  DNA
<213>  Homo sapiens

<400>  1227
ugggauccug ucaaguguut t                                                21


<210>  1228
<211>  21
<212>  DNA
<213>  Homo sapiens

<400>  1228
aacacuugac aggaucccat t                                                21


<210>  1229
<211>  21
<212>  DNA
<213>  Homo sapiens

<400>  1229
aagtgttgga aagttctcca c                                                21


<210>  1230
<211>  21
<212>  DNA
<213>  Homo sapiens

<400>  1230
guguuggaaa guucuccact t                                                21

<210>  1231
<211>  21
<212>  DNA
<213>  Homo sapiens

<400>  1231
guggagaacu uuccaacact t                                                21

<210>  1232
<211>  18
<212>  DNA
<213>  Homo sapiens

<400>  1232
aaagttctcc actgttaa                                                    18

<210>  1233
<211>  18
<212>  DNA
<213>  Homo sapiens

<400>  1233
aguucuccac uguuaatt                                                    18

<210>  1234
<211>  18
<212>  DNA
<213>  Homo sapiens

<400>  1234
uuaacagugg agaacutt                                                    18

<210>  1235
<211>  21
<212>  DNA
<213>  Homo sapiens

<400>  1235
aacccagtct atgttggcaa t                                                21

<210>  1236
<211>  21
<212>  DNA
<213>  Homo sapiens

<400>  1236
cccagucuau guuggcaaut t                                                21

<210>  1237
<211>  21
<212>  DNA
<213>  Homo sapiens

<400>  1237

auugccaaca uagacugggt t 21

<210> 1238
<211> 21
<212> DNA
<213> Homo sapiens

<400> 1238
aatgtggcct gggcccacat t 21

<210> 1239
<211> 21
<212> DNA
<213> Homo sapiens

<400> 1239
uguggccugg gcccacauut t 21

<210> 1240
<211> 21
<212> DNA
<213> Homo sapiens

<400> 1240
aaugugggcc caggccacat t 21

<210> 1241
<211> 21
<212> DNA
<213> Homo sapiens

<400> 1241
aagaaggccc caagcatccg a 21

<210> 1242
<211> 21
<212> DNA
<213> Homo sapiens

<400> 1242
gaaggcccca agcauccgat t 21

<210> 1243
<211> 21
<212> DNA
<213> Homo sapiens

<400> 1243
ucggaugcuu ggggccuuct t 21

<210> 1244
<211> 21
<212> DNA
<213> Homo sapiens

<400> 1244
aaggccccaa gcatccgagg a 21

<210>  1245
<211>  21
<212>  DNA
<213>  Homo sapiens

<400>  1245
ggccccaagc auccgaggat t                                                21


<210>  1246
<211>  21
<212>  DNA
<213>  Homo sapiens

<400>  1246
uccucggaug cuuggggcct t                                                21


<210>  1247
<211>  21
<212>  DNA
<213>  Homo sapiens

<400>  1247
aagcatccga ggacagttct a                                                21


<210>  1248
<211>  21
<212>  DNA
<213>  Homo sapiens

<400>  1248
gcauccgagg acaguucuat t                                                21


<210>  1249
<211>  21
<212>  DNA
<213>  Homo sapiens

<400>  1249
uagaacuguc cucggaugct t                                                21


<210>  1250
<211>  21
<212>  DNA
<213>  Homo sapiens

<400>  1250
aaagctatga taaccttaat t                                                21


<210>  1251
<211>  21
<212>  DNA
<213>  Homo sapiens

<400>  1251
agcuaugaua accuuaauut t                                                21


<210>  1252

<211>  21
<212>  DNA
<213>  Homo sapiens

<400>  1252
aauuaagguu aucauagcut t                                                21


<210>  1253
<211>  21
<212>  DNA
<213>  Homo sapiens

<400>  1253
aaccttaatt acaccctgag c                                                21


<210>  1254
<211>  21
<212>  DNA
<213>  Homo sapiens

<400>  1254
ccuuaauuac acccugagct t                                                21


<210>  1255
<211>  21
<212>  DNA
<213>  Homo sapiens

<400>  1255
gcucagggug uaauuaaggt t                                                21


<210>  1256
<211>  21
<212>  DNA
<213>  Homo sapiens

<400>  1256
aattacaccc tgagcaaaga g                                                21


<210>  1257
<211>  21
<212>  DNA
<213>  Homo sapiens

<400>  1257
uuacacccug agcaaagagt t                                                21


<210>  1258
<211>  21
<212>  DNA
<213>  Homo sapiens

<400>  1258
cucuuugcuc aggguguaat t                                                21


<210>  1259
<211>  21
<212>  DNA

<213>  Homo sapiens

<400>  1259
aaagagttcg gcctccgcct t                                                21


<210>  1260
<211>  21
<212>  DNA
<213>  Homo sapiens

<400>  1260
agaguucggc cuccgccuut t                                                21


<210>  1261
<211>  21
<212>  DNA
<213>  Homo sapiens

<400>  1261
aaggcggagg ccgaacucut t                                                21


<210>  1262
<211>  18
<212>  DNA
<213>  Homo sapiens

<400>  1262
aaatagtgag cttcctac                                                    18


<210>  1263
<211>  18
<212>  DNA
<213>  Homo sapiens

<400>  1263
auagugagcu uccuactt                                                    18


<210>  1264
<211>  18
<212>  DNA
<213>  Homo sapiens

<400>  1264
guaggaagcu cacuautt                                                    18


<210>  1265
<211>  21
<212>  DNA
<213>  Homo sapiens

<400>  1265
aatttacacc tatcgaccgc c                                                21


<210>  1266
<211>  21
<212>  DNA
<213>  Homo sapiens

<400>  1266
uuuacaccua ucgaccgcct t                                                21

<210>  1267
<211>  21
<212>  DNA
<213>  Homo sapiens

<400>  1267
ggcggucgau agguguaaat t                                                21

<210>  1268
<211>  18
<212>  DNA
<213>  Homo sapiens

<400>  1268
aaccgccaca tagtcaca                                                    18

<210>  1269
<211>  18
<212>  DNA
<213>  Homo sapiens

<400>  1269
ccgccacaua gucacatt                                                    18

<210>  1270
<211>  18
<212>  DNA
<213>  Homo sapiens

<400>  1270
ugugacuaug uggcggtt                                                    18

<210>  1271
<211>  21
<212>  DNA
<213>  Homo sapiens

<400>  1271
aaatagcgta ttcaccttct c                                                21

<210>  1272
<211>  21
<212>  DNA
<213>  Homo sapiens

<400>  1272
auagcguauu caccuucuct t                                                21

<210>  1273
<211>  21
<212>  DNA
<213>  Homo sapiens

<400>  1273
gagaagguga auacgcuaut t                                                21

<210>  1274
<211>  21
<212>  DNA
<213>  Homo sapiens

<400>  1274
aagaaggctc agcgagatct g                                                21


<210>  1275
<211>  21
<212>  DNA
<213>  Homo sapiens

<400>  1275
gaaggcucag cgagaucugt t                                                21


<210>  1276
<211>  21
<212>  DNA
<213>  Homo sapiens

<400>  1276
cagaucucgc ugagccuuct t                                                21


<210>  1277
<211>  21
<212>  DNA
<213>  Homo sapiens

<400>  1277
aaggctcagc gagatctggc g                                                21


<210>  1278
<211>  21
<212>  DNA
<213>  Homo sapiens

<400>  1278
ggcucagcga gaucuggcgt t                                                21


<210>  1279
<211>  21
<212>  DNA
<213>  Homo sapiens

<400>  1279
cgccagaucu cgcugagcct t                                                21


<210>  1280
<211>  21
<212>  DNA
<213>  Homo sapiens

<400>  1280
aagccactct acagctggga g                                                21

<210> 1281
<211> 21
<212> DNA
<213> Homo sapiens

<400> 1281
gccacucuac agcugggagt t 21


<210> 1282
<211> 21
<212> DNA
<213> Homo sapiens

<400> 1282
cucccagcug uagaguggct t 21


<210> 1283
<211> 21
<212> DNA
<213> Homo sapiens

<400> 1283
aagccaagca gaaaacggtg g 21


<210> 1284
<211> 21
<212> DNA
<213> Homo sapiens

<400> 1284
gccaagcaga aaacgguggt t 21


<210> 1285
<211> 21
<212> DNA
<213> Homo sapiens

<400> 1285
ccaccguuuu cugcuuggct t 21


<210> 1286
<211> 21
<212> DNA
<213> Homo sapiens

<400> 1286
aagcagaaaa cggtggagtg g 21


<210> 1287
<211> 21
<212> DNA
<213> Homo sapiens

<400> 1287
gcagaaaacg guggaguggt t 21


<210> 1288
<211> 21

<212>  DNA
<213>  Homo sapiens

<400>  1288
ccacuccacc guuuucugct t                                                21


<210>  1289
<211>  21
<212>  DNA
<213>  Homo sapiens

<400>  1289
aaaacggtgg agtgggttgg t                                                21


<210>  1290
<211>  21
<212>  DNA
<213>  Homo sapiens

<400>  1290
   aacgguggag uggguuggut t                                                 21


<210>  1291
<211>  21
<212>  DNA
<213>  Homo sapiens

<400>  1291
accaacccac uccaccguut t                                                21


<210>  1292
<211>  21
<212>  DNA
<213>  Homo sapiens

<400>  1292
aacggtggag tgggttggtt c                                                21


<210>  1293
<211>  21
<212>  DNA
<213>  Homo sapiens

<400>  1293
cgguggagug gguugguuct t                                                21


<210>  1294
<211>  21
<212>  DNA
<213>  Homo sapiens

<400>  1294
gaaccaaccc acuccaccgt t                                                21


<210>  1295
<211>  21
<212>  DNA
<213>  Homo sapiens

<400>  1295
aaggagaccc tgaagtccaa g                                                21

<210>  1296
<211>  21
<212>  DNA
<213>  Homo sapiens

<400>  1296
ggagacccug aaguccaagt t                                                21

<210>  1297
<211>  21
<212>  DNA
<213>  Homo sapiens

<400>  1297
cuuggacuuc agggucucct t                                                21

<210>  1298
<211>  987
<212>  DNA
<213>  Homo sapiens

<400>  1298
atggcccgca ccgtggtgct catcaccggc tgttcctcgg gcatcggcct gcacttggcc      60

gtacgtctgg cttcagatcc atcccagagc ttcaaagtgt atgccacgtt gagggacctg     120

aaaacacagg gccggctgtg ggaggcggcc cgggccctgg catgccctcc gggatccctg     180

gagacgttgc agctggacgt aagggactca aaatccgtgg ccgctgcccg ggaacgcgtg     240

actgagggcc gcgtggacgt gctggtgtgt aacgcaggcc tgggcctgct ggggccgctg     300

gaggcgctgg gggaggacgc cgtggcctct gtgctggacg tgaatgtagt agggactgtg     360

cggatgctgc aggccttcct gccagacatg aagaggcgcg gttcgggacg cgtgttggtg     420

accgggagcg tgggaggatt gatggggctg cctttcaatg acgtttattg cgccagcaag     480

ttcgcgctcg aaggcttatg cgagagtctg gcggttctgc tgctgccctt tggggtccac     540

ttgagcctga tcgagtgcgg cccagtgcac accgccttca tggagaaggt gttgggcagc     600

ccagaggagg tgctggaccg cacggacatc cacaccttcc accgcttcta ccaatacctc     660

gcccacagca agcaagtctt tcgcgaggcg gcgcagaacc ctgaggaggt ggcggaggtc     720

ttcctcaccg ctttgcgcgc cccgaagccg accctgcgct acttcaccac cgagcgcttc     780

ctgcccctgc tgcggatgcg cctggacgac cccagcggct ccaactacgt caccgccatg     840

caccgggaag tgttcggcga cgttccggca aaggccgagg ctggggccga ggctgggggc     900

ggggccgggc ctggggcaga ggacgaggcc gggcgcagtg cggtggggga ccctgagctc     960

ggcgatcctc cggccgcccc gcagtaa                                         987

<210>  1299

<211>  21
<212>  DNA
<213>  Homo sapiens

<400>  1299
aaagtgtatg ccacgttgag g                                                21


<210>  1300
<211>  21
<212>  DNA
<213>  Homo sapiens

<400>  1300
aguguaugcc acguugaggt t                                                21


<210>  1301
<211>  21
<212>  DNA
<213>  Homo sapiens

<400>  1301
ccucaacgug gcauacacut t                                                21


<210>  1302
<211>  21
<212>  DNA
<213>  Homo sapiens

<400>  1302
aaaacacagg gccggctgtg g                                                21


<210>  1303
<211>  21
<212>  DNA
<213>  Homo sapiens

<400>  1303
aacacagggc cggcuguggt t                                                21


<210>  1304
<211>  21
<212>  DNA
<213>  Homo sapiens

<400>  1304
ccacagccgg cccuguguut t                                                21


<210>  1305
<211>  21
<212>  DNA
<213>  Homo sapiens

<400>  1305
aacacagggc cggctgtggg a                                                21


<210>  1306
<211>  21
<212>  DNA

<213>  Homo sapiens

<400>  1306
cacagggccg gcugugggat t                                                21


<210>  1307
<211>  21
<212>  DNA
<213>  Homo sapiens

<400>  1307
ucccacagcc ggcccugugt t                                                21


<210>  1308
<211>  21
<212>  DNA
<213>  Homo sapiens

<400>  1308
aagggactca aaatccgtgg c                                                21


<210>  1309
<211>  21
<212>  DNA
<213>  Homo sapiens

<400>  1309
gggacucaaa auccguggct t                                                21


<210>  1310
<211>  21
<212>  DNA
<213>  Homo sapiens

<400>  1310
gccacggauu uugaguccct t                                                21


<210>  1311
<211>  21
<212>  DNA
<213>  Homo sapiens

<400>  1311
aaaatccgtg gccgctgccc g                                                21


<210>  1312
<211>  21
<212>  DNA
<213>  Homo sapiens

<400>  1312
aauccguggc cgcugcccgt t                                                21


<210>  1313
<211>  21
<212>  DNA
<213>  Homo sapiens

<400>  1313
cgggcagcgg ccacggauut t                                                21

<210>  1314
<211>  21
<212>  DNA
<213>  Homo sapiens

<400>  1314
aatccgtggc cgctgcccgg g                                                21

<210>  1315
<211>  21
<212>  DNA
<213>  Homo sapiens

<400>  1315
uccguggccg cugcccgggt t                                                21

<210>  1316
<211>  21
<212>  DNA
<213>  Homo sapiens

<400>  1316
cccgggcagc ggccacggat t                                                21

<210>  1317
<211>  18
<212>  DNA
<213>  Homo sapiens

<400>  1317
aacgcgtgac tgagggcc                                                    18

<210>  1318
<211>  18
<212>  DNA
<213>  Homo sapiens

<400>  1318
cgcgugacug agggcctt                                                    18

<210>  1319
<211>  18
<212>  DNA
<213>  Homo sapiens

<400>  1319
ggcccucagu cacgcgtt                                                    18

<210>  1320
<211>  21
<212>  DNA
<213>  Homo sapiens

<400>  1320
aacgcaggcc tgggcctgct g                                                21

<210>  1321
<211>  21
<212>  DNA
<213>  Homo sapiens

<400>  1321
cgcaggccug ggccugcugt t                                                21


<210>  1322
<211>  21
<212>  DNA
<213>  Homo sapiens

<400>  1322
cagcaggccc aggccugcgt t                                                21


<210>  1323
<211>  18
<212>  DNA
<213>  Homo sapiens

<400>  1323
aatgtagtag ggactgtg                                                    18


<210>  1324
<211>  18
<212>  DNA
<213>  Homo sapiens

<400>  1324
uguaguaggg acugugtt                                                    18


<210>  1325
<211>  18
<212>  DNA
<213>  Homo sapiens

<400>  1325
cacagucccu acuacatt                                                    18


<210>  1326
<211>  21
<212>  DNA
<213>  Homo sapiens

<400>  1326
aagaggcgcg gttcgggacg c                                                21


<210>  1327
<211>  21
<212>  DNA
<213>  Homo sapiens

<400>  1327
gaggcgcggu ucgggacgct t                                                21

<210> 1328
<211> 21
<212> DNA
<213> Homo sapiens

<400> 1328
gcgucccgaa ccgcgccuct t 21


<210> 1329
<211> 21
<212> DNA
<213> Homo sapiens

<400> 1329
aatgacgttt attgcgccag c 21


<210> 1330
<211> 21
<212> DNA
<213> Homo sapiens

<400> 1330
ugacguuuau ugcgccagct t 21


<210> 1331
<211> 21
<212> DNA
<213> Homo sapiens

<400> 1331
gcuggcgcaa uaaacgucat t 21


<210> 1332
<211> 18
<212> DNA
<213> Homo sapiens

<400> 1332
aagttcgcgc tcgaaggc 18


<210> 1333
<211> 18
<212> DNA
<213> Homo sapiens

<400> 1333
guucgcgcuc gaaggctt 18


<210> 1334
<211> 18
<212> DNA
<213> Homo sapiens

<400> 1334
gccuucgagc gcgaactt 18


<210> 1335
<211> 21

<212>  DNA
<213>  Homo sapiens

<400>  1335
aaggcttatg cgagagtctg g                                                21


<210>  1336
<211>  21
<212>  DNA
<213>  Homo sapiens

<400>  1336
ggcuuaugcg agagucuggt t                                                21


<210>  1337
<211>  21
<212>  DNA
<213>  Homo sapiens

<400>  1337
ccagacucuc gcauaagcct t                                                21


<210>  1338
<211>  18
<212>  DNA
<213>  Homo sapiens

<400>  1338
aaggtgttgg gcagccca                                                    18


<210>  1339
<211>  18
<212>  DNA
<213>  Homo sapiens

<400>  1339
gguguugggc agcccatt                                                    18


<210>  1340
<211>  18
<212>  DNA
<213>  Homo sapiens

<400>  1340
ugggcugccc aacacctt                                                    18


<210>  1341
<211>  18
<212>  DNA
<213>  Homo sapiens

<400>  1341
aatacctcgc ccacagca                                                    18


<210>  1342
<211>  18
<212>  DNA
<213>  Homo sapiens

<400>  1342
uaccucgccc acagcatt                                                    18

<210>  1343
<211>  18
<212>  DNA
<213>  Homo sapiens

<400>  1343
ugcugugggc gagguatt                                                    18

<210>  1344
<211>  21
<212>  DNA
<213>  Homo sapiens

<400>  1344
aagcaagtct ttcgcgaggc g                                                21

<210>  1345
<211>  21
<212>  DNA
<213>  Homo sapiens

<400>  1345
gcaagucuuu cgcgaggcgt t                                                21

<210>  1346
<211>  21
<212>  DNA
<213>  Homo sapiens

<400>  1346
cgccucgcga aagacuugct t                                                21

<210>  1347
<211>  21
<212>  DNA
<213>  Homo sapiens

<400>  1347
aagtctttcg cgaggcggcg c                                                21

<210>  1348
<211>  21
<212>  DNA
<213>  Homo sapiens

<400>  1348
gucuuucgcg aggcggcgct t                                                21

<210>  1349
<211>  21
<212>  DNA
<213>  Homo sapiens

<400>  1349

gcgccgccuc gcgaaagact t                                                21

<210>  1350
<211>  21
<212>  DNA
<213>  Homo sapiens

<400>  1350
aaccctgagg aggtggcgga g                                                21

<210>  1351
<211>  21
<212>  DNA
<213>  Homo sapiens

<400>  1351
cccugaggag guggcggagt t                                                21

<210>  1352
<211>  21
<212>  DNA
<213>  Homo sapiens

<400>  1352
cuccgccacc uccucagggt t                                                21

<210>  1353
<211>  21
<212>  DNA
<213>  Homo sapiens

<400>  1353
aagccgaccc tgcgctactt c                                                21

<210>  1354
<211>  21
<212>  DNA
<213>  Homo sapiens

<400>  1354
gccgacccug cgcuacuuct t                                                21

<210>  1355
<211>  21
<212>  DNA
<213>  Homo sapiens

<400>  1355
gaaguagcgc agggucggct t                                                21

<210>  1356
<211>  18
<212>  DNA
<213>  Homo sapiens

<400>  1356
aactacgtca ccgccatg                                                    18

<210>  1357
<211>  18
<212>  DNA
<213>  Homo sapiens

<400>  1357
cuacgucacc gccaugtt                                                    18


<210>  1358
<211>  18
<212>  DNA
<213>  Homo sapiens

<400>  1358
cauggcggug acguagtt                                                    18


<210>  1359
<211>  21
<212>  DNA
<213>  Homo sapiens

<400>  1359
aagtgttcgg cgacgttccg g                                                21


<210>  1360
<211>  21
<212>  DNA
<213>  Homo sapiens

<400>  1360
guguucggcg acguuccggt t                                                21


<210>  1361
<211>  21
<212>  DNA
<213>  Homo sapiens

<400>  1361
ccggaacguc gccgaacact t                                                21


<210>  1362
<211>  21
<212>  DNA
<213>  Homo sapiens

<400>  1362
aaaggccgag gctggggccg a                                                21


<210>  1363
<211>  21
<212>  DNA
<213>  Homo sapiens

<400>  1363
aggccgaggc uggggccgat t                                                21


<210>  1364

<211>  21
<212>  DNA
<213>  Homo sapiens

<400>  1364
ucggccccag ccucggccut t                                                21


<210>  1365
<211>  424
<212>  DNA
<213>  Homo sapiens

<400>  1365
ggaaatggca ccttgggatg agctgtcaca gcaagactga cttctgtcac caccctttac      60

atcacggctt caattatttc tatgggatct ctttgaccaa tctgagagac tgcaagcccg     120

gagagggcag tgtcttcacc acgggcttca agaggctggt cttcctcccc ctgcagatcg     180

tcggggtcac cctccttacc cttgctgcac tcaattgtct ggggctactc cacgtgcctc     240

taggcgtttt tttcagcctt ctcttcctag cagccctaat cctgaccctt ttcttgggct     300

tccttcatta cttccggccc ctgaactgct tcatgatgag gaactacgag atcattcagc     360

agcccatgtc ctatgacaat ctcacccaga ggctaacggt ggaggcggcc cagttcatac     420

agcg                                                                  424


<210>  1366
<211>  21
<212>  DNA
<213>  Homo sapiens

<400>  1366
aaatggcacc ttgggatgag c                                                21


<210>  1367
<211>  21
<212>  DNA
<213>  Homo sapiens

<400>  1367
auggcaccuu gggaugagct t                                                21


<210>  1368
<211>  21
<212>  DNA
<213>  Homo sapiens

<400>  1368
gcucauccca aggugccaut t                                                21


<210>  1369
<211>  21
<212>  DNA
<213>  Homo sapiens

<400>  1369
aagactgact tctgtcacca c                                                21

<210>  1370
<211>  21
<212>  DNA
<213>  Homo sapiens

<400>  1370
gacugacuuc ugucaccact t                                                21


<210>  1371
<211>  21
<212>  DNA
<213>  Homo sapiens

<400>  1371
guggugacag aagucaguct t                                                21


<210>  1372
<211>  21
<212>  DNA
<213>  Homo sapiens

<400>  1372
aattatttct atgggatctc t                                                21


<210>  1373
<211>  21
<212>  DNA
<213>  Homo sapiens

<400>  1373
uuauuucuau gggaucucut t                                                21


<210>  1374
<211>  21
<212>  DNA
<213>  Homo sapiens

<400>  1374
agagauccca uagaaauaat t                                                21


<210>  1375
<211>  21
<212>  DNA
<213>  Homo sapiens

<400>  1375
aatctgagag actgcaagcc c                                                21


<210>  1376
<211>  21
<212>  DNA
<213>  Homo sapiens

<400>  1376
ucugagagac ugcaagccct t                                                21

<210> 1377
<211> 21
<212> DNA
<213> Homo sapiens

<400> 1377
gggcuugcag ucucucagat t 21


<210> 1378
<211> 18
<212> DNA
<213> Homo sapiens

<400> 1378
aagcccggag agggcagt 18


<210> 1379
<211> 18
<212> DNA
<213> Homo sapiens

<400> 1379
gcccggagag ggcagutt 18


<210> 1380
<211> 18
<212> DNA
<213> Homo sapiens

<400> 1380
acugcccucu ccgggctt 18


<210> 1381
<211> 21
<212> DNA
<213> Homo sapiens

<400> 1381
aagaggctgg tcttcctccc c 21


<210> 1382
<211> 21
<212> DNA
<213> Homo sapiens

<400> 1382
gaggcugguc uuccucccct t 21


<210> 1383
<211> 21
<212> DNA
<213> Homo sapiens

<400> 1383
ggggaggaag accagccuct t 21


<210> 1384
<211> 21

<212>  DNA
<213>  Homo sapiens

<400>  1384
aattgtctgg ggctactcca c                                                21


<210>  1385
<211>  21
<212>  DNA
<213>  Homo sapiens

<400>  1385
uugucugggg cuacuccact t                                                21


<210>  1386
<211>  21
<212>  DNA
<213>  Homo sapiens

<400>  1386
guggaguagc cccagacaat t                                                21


<210>  1387
<211>  21
<212>  DNA
<213>  Homo sapiens

<400>  1387
aatcctgacc cttttcttgg g                                                21


<210>  1388
<211>  21
<212>  DNA
<213>  Homo sapiens

<400>  1388
uccugacccu uuucuugggt t                                                21


<210>  1389
<211>  21
<212>  DNA
<213>  Homo sapiens

<400>  1389
cccaagaaaa gggucaggat t                                                21


<210>  1390
<211>  21
<212>  DNA
<213>  Homo sapiens

<400>  1390
aactgcttca tgatgaggaa c                                                21


<210>  1391
<211>  21
<212>  DNA
<213>  Homo sapiens

<400>  1391
cugcuucaug augaggaact t                                                21

<210>  1392
<211>  21
<212>  DNA
<213>  Homo sapiens

<400>  1392
guuccucauc augaagcagt t                                                21

<210>  1393
<211>  19
<212>  DNA
<213>  Homo sapiens

<400>  1393
aactacgaga tcattcagc                                                   19

<210>  1394
<211>  19
<212>  DNA
<213>  Homo sapiens

<400>  1394
cuacgagauc auucagctt                                                   19

<210>  1395
<211>  19
<212>  DNA
<213>  Homo sapiens

<400>  1395
gcugaaugau cucguagtt                                                   19

<210>  1396
<211>  21
<212>  DNA
<213>  Homo sapiens

<400>  1396
aatctcaccc agaggctaac g                                                21

<210>  1397
<211>  21
<212>  DNA
<213>  Homo sapiens

<400>  1397
ucucacccag aggcuaacgt t                                                21

<210>  1398
<211>  21
<212>  DNA
<213>  Homo sapiens

<400>  1398

cguuagccuc ugggugagat t                                                21

<210>  1399
<211>  21
<212>  DNA
<213>  Homo sapiens

<400>  1399
aacggtggag gcggcccagt t                                                21

<210>  1400
<211>  21
<212>  DNA
<213>  Homo sapiens

<400>  1400
cgguggaggc ggcccaguut t                                                21

<210>  1401
<211>  21
<212>  DNA
<213>  Homo sapiens

<400>  1401
aacugggccg ccuccaccgt t                                                21

<210>  1402
<211>  20
<212>  DNA
<213>  Artificial Sequence

<220>
<223>  Synthetic

<400>  1402
atgcaggttc agtgccagca                                                  20

<210>  1403
<211>  22
<212>  DNA
<213>  Artificial Sequence

<220>
<223>  Synthetic

<400>  1403
ttaaaaagat gaatggaata ag                                               22

## Description

### BACKGROUND

### 1. Technical Field

The present disclosure relates generally to the methods and compositions for manipulating hair growth or hair loss. In particular, aspects of the present disclosure are directed to methods and compositions that interfere with genetic expression of genes involved in or related to hair loss or hair growth, for example using inhibitory nucleic acids in cases of androgenic alopecia.

### 2. Related Art

Hair loss in both men and women has garnered much attention from the medical and pharmaceutical industry because of the high demand for effective and reliable treatments by those suffering from this condition. In human beings, hair growth and its renewal are mainly determined by the activity of the hair follicles. Their activity is cyclical and comprises essentially three phases, namely the anagenic phase, the catagenic phase and the telegenic phase. The active anagenic phase or growth phase, which lasts several years and during which the hair grows longer, is followed by a very short and transitory catagenic or involution phase, which lasts a few weeks, and then by a resting phase, known as the telogenic phase, which lasts a few months. At the end of the resting period, the hair falls out and another cycle recommences. The head of hair is thus constantly renewed and, of the approximately 150,000 hairs, which a head of hair contains, at each instant, approximately 10% of them are at rest and will therefore be replaced in a few months.

In a significant number of cases, early hair loss takes place in subjects who are genetically predisposed to the condition. Hair loss affects men in particular and is more particularly androgenetic in character. Hair loss is also referred to as androgenic alopecia or alternatively androgeno-genetic alopecia (AGA).

Because of the link between androgens and hair loss, most hair loss therapies are designed to interfere with androgenic action or to include some form of hormone therapy. Although therapies for hair loss do exist, these therapies are not effective in many individuals, and in some cases are only available to specific genders of suffers.

For example, U.S. Pat. Nos. 5,571,817; 5,670,643 and 6,12,362 disclose a composition and method for producing and using 17β-N-t butylcarbamoyl-4-aza-5α-androst-1-en-3-one known generically as finesteride. The efficacy of compounds such as finesteride relies upon their ability to inhibit the action of various proteins involved in androgen mediated hair loss. Finesteride is commercially disturbed under the tradename Propecia^{®} and is a testosterone-5α-reductase inhibitor. 5α-reductase is an enzyme that coverts testosterone into dihydroxy testosterone (DHT). Thus, finesteride decreases the amount of DHT. Finesteride is reported to be less effective in women and may potentially cause serious birth defects if used by pregnant women.

Another well known treatment for hair loss uses a compound known as minoxidil. U.S. Pat. No. 4,596,812 discloses methods of using minoxidil, 6-amino-1,2-dihydro-1-hydroxy-2-imino-4-piperodinopyrimidine, for treating male pattern baldness. Minoxidil is a vasodilator developed for the treatment of high blood pressure. Minoxidil is believed to increase hair growth by increasing the delivery of blood to a desired area. It is generally recognized that not all individuals will respond to minoxidil, and those who do respond require treatment application for life to maintain efficacy. Additionally, minoxidil may cause itchiness and redness of the scalp.

Thus, there is a need for new and effective treatments for androgen related disorders, including hair loss, for both men and women.

There is also a need for new and effective compositions for the treatment of hair loss including the prevention of hair loss or the induction of hair growth.

### SUMMARY

Aspects of the present disclosure are directed to compositions and methods for the use of inhibitory nucleic acids, for example small inhibitory ribonucleic acids (siRNA), to adjust, manipulate, prevent, inhibit, interfere, or block the androgen signal transduction pathway in a host cell, for example in a host's hair cell. Other aspects of the disclosure provide compositions and methods for interfering with the androgen signal transduction pathway by down regulating the expression of proteins involved in the androgen signal transduction pathway. Exemplary gene targets encoding proteins involved in the androgen signal transduction pathway include but are not limited to isozymes I and II of 5-α reductase, the androgen receptor, aromatase, 3-α-hydroxysteroiddehydrogenase, 3-β-hydroxysteroiddehydrogenase, 3-β-hydroxysteroiddehydrogenase-4-5-isomerase, 17-β-hydroxysteroidoxidoreductase and steroid sulfatase. In some aspects, the inhibitory nucleic acids, for example siRNAs, interfere with the expression of targeted genes by preventing, reducing, or inhibiting the translation of mRNA transcribed from the targeted gene.

Another aspect of the disclosure provides compositions and methods for the treatment of androgen related diseases by decreasing the sensitivity of androgens in a host or host cell. The sensitivity of a cell to androgens can be reduced or regulated using siRNAs that interfere with or interrupt the androgen signal transduction pathway, for example by interfering or blocking the expression or function of any protein in the androgen signal transduction cascade. An exemplary aspect provides methods and compositions for treating hair loss using siRNAs, for example, to regulate the cell cycle of hair cells by manipulating the androgen signal transduction pathway.

Still another aspect or the disclosure provides compositions and methods for treating hair loss in a mammal, for example a human, by interfering with or inhibiting, the androgen signal transduction pathway of a hair cell. For example, siRNAs can be used to inhibit or reduce the production of DHT in a host or host cell using siRNAs targeted to enzymes involved in the production of DHT, for example 5-α reductase. Contacting mRNAs encoding 5α-reductase with a corresponding siRNA interferes with the translation of the mRNA and thereby interferes with the expression of the 5α-reductase. Some aspects provide siRNAs that induce the degradation, typically enzymatic degradation, of the target mRNA. The less 5α-reductase, the less DHT or ability to produce DHT. By reducing or inhibiting the production of DHT, the cycle of hair growth and renewal can be manipulated resulting in reduced hair loss or promoting new hair growth. Accordingly, other aspects of the present disclosure are directed to compositions and methods that inhibit the production of proteins that mediate the effects of androgens in androgenetic alopecia. Exemplary proteins included those proteins encoded by the genes for both isozymes I and II of 5-α reductase, the androgen receptor, aromatase, 3-α-hydroxysteroiddehydrogenase, 3-β-hydroxysteroiddehydrogenase,3-β-hydroxysteroiddehydrogenase-4-5-isomerase, 17-β-hydroxysteroidoxidoreductase, and steroid sulfatase.

Still other aspects of the disclosure provide compositions and methods for manipulating the androgen receptor signal transduction pathway in a host cell to reduce, inhibit, or prevent hair loss or to induce hair growth. In one aspect, inhibitory nucleic acids, typically ribonucleic acids, and more typically double stranded ribonucleic acids are provided that interfere with, inhibit, or interrupt the expression of proteins involved in the androgen signal transduction pathway. In particular, inhibitory ribonucleic acids (iRNA) or small inhibitory ribonucleic acids (siRNA) that comprise at least a partial sequence of nucleic acids encoding proteins involved with or related to the androgen signal transduction pathway are disclosed that inhibit or interfere with the expression of these proteins. The active siRNAs can be from about 10 to about 25 nucleotides in length although it will be appreciated that longer RNAs can be employed that are processed to result in siRNAs of smaller sizes, typically from about 10 to about 25 nucleotides in length.

Accordingly, aspects of the present disclsoure describe a method of treating the hyperandrogenic conditions of androgenic alopecia, including male pattern alopecia, acne vulgaris, seborrhea, and female hirsutism by topical administration, and a method of treating all of the above conditions as well as benign prostatic hypertrophy, by systemic administration, of the novel compounds of the present disclosure. In one method, an siRNA is administered to a host, wherein the siRNA interrupts or interferes with the expression of a gene involved in the androgen signal transduction pathway that results in the reduction or inhibition of DHT production or androgen receptor expression.

Still another aspect provides a method of screening siRNAs to identify siRNAs that inhibit, interfere or otherwise disrupt the androgen signal transduction pathway, for example in hair cells. The screening can be accomplished using in vitro cell lines. Alternatively, the screening method can be used to identify compounds that mimic or synergize with siRNAs.

Yet another aspect provides a vector, typically a viral vector, that encodes a siRNA that interferes with or inhibits the androgen signal transduction pathway. The vector can be designed to express double stranded siRNA directly or indirectly wherein the siRNA comprises at least a portion of the target mRNA nucleic acid sequence, typically a mRNA encoding a protein in the androgen signal transduction pathway. Alternatively, the vector can encode a nucleic acid sequence containing specific cleavage sites such that the vector expresses a nucleic acid that can be cleaved to produce inhibitory nucleic acids of less than about 30 nucleotides in length. The expressed inhibitory nucleic acids are typically RNA, and more typically are double-stranded RNA. An exemplary vector includes a promoter for human RNA polymerase III, flanked on the 3' end by a termination signal composed of five T residues. Between start and stop signals is a 21-nucleotide DNA template and its inverted repeat separated by a short non-homologous nucleotide spacer.

The present disclosure also encompasses a cell comprising a siRNA targeted to a mRNA encoding a protein in the androgen signal transduction pathway, for example isozymes I and II of 5-α reductase, the androgen receptor, aromatase, 3-α-hydroxysteroiddehydrogenase, 3-β-hydroxysteroiddehydrogenase, 3-β-hydroxysteroiddehydrogenase-4-5-isomerase, 17-β-hydroxysteroidoxidoreductase, and steroid sulfatase. Alternatively, the cell comprises a vector that when expressed, produces a siRNA that interferes with or inhibits the expression of a protein in the androgen signal transduction pathway. Suitable vectors containing functional elements such as promoters and other regulatory elements are known in the art. The sequence of representative siRNAs is provided in Table 1.

The inhibitory nucleic acids of the present disclosure can be formulated for administration by conventional means including topical, oral, and intravenous administration. These topical pharmaceutical compositions containing the compounds of the present disclosure ordinarily include about 0.1% to 15%, preferably about 0.1 to 5%, and more preferably about 0.1 % to. 2%, of the active compound, in admixture with a pharmaceutically acceptable carrier.

The compositions according to the disclosure may be in any form suitable for application to hair and scalp, especially in the form of an aqueous, alcoholic or aqueous-alcoholic preparation, such as a solution, gel, cream, emulsion or dispersion.

### BRIEF DESCRIPTION OF THE DRAWINGS

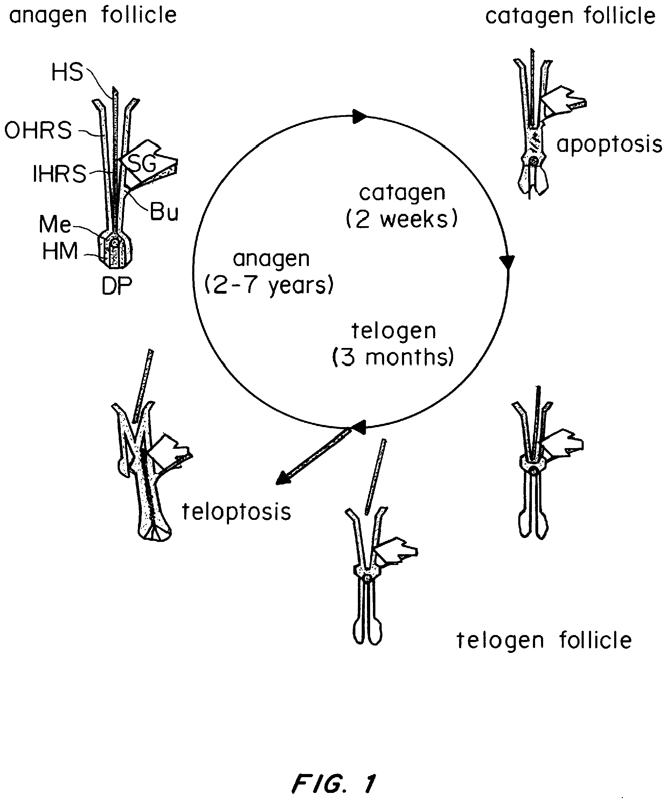

**Figure 1** is a diagram showing the cycle of hair growth.

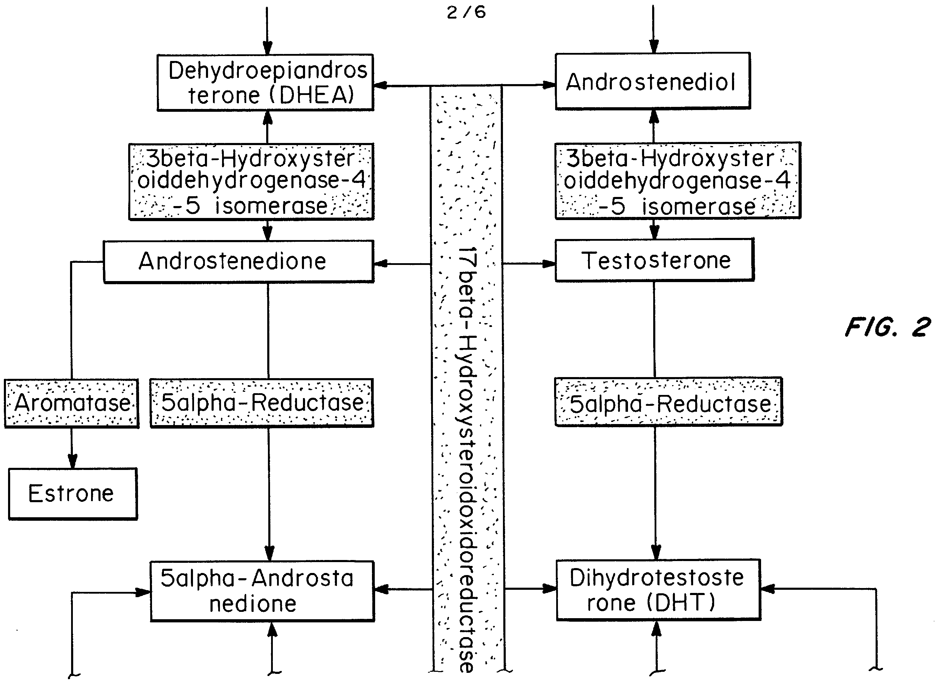

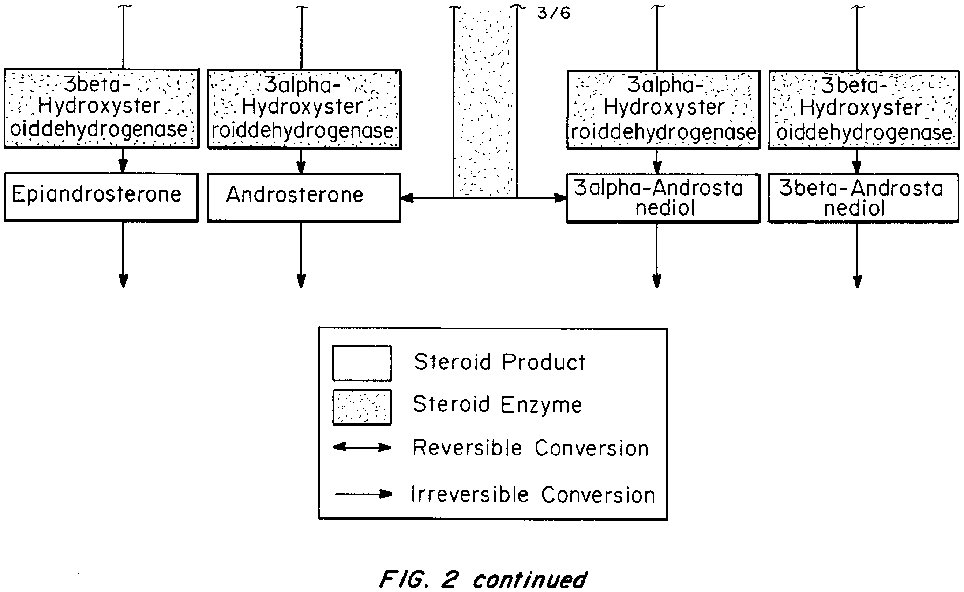

**Figure 2** is a flow diagram illustrating the genes involved in en exemplary androgen signal transduction pathway.
**Figure 3** is an autoradiograph showing the full length clone of steroid 5-alpha-reductase.
**Figure 4** is a diagram of the PCR amplicon that was TA cloned (Invitrogen) to generate pcDNA 3.1/ SRD5a-GFP.
**Figures 5A-C** are fluorescence micrographs of Sy5y cells transfected to express with GFP-tagged steroid 5-alpha-reductase and rhodamine labelled dsRNA specific for the reporter protein.

### DETAILED DESCRIPTION

### Definitions

Unless otherwise indicated the following terms used in the specification and claims have the meanings discussed below:
The term "organism" refers to any living entity comprised of at least one cell. A living organism can be as simple as, for example, a single eukaryotic cell or as complex as a mammal, including a human being.

The term "therapeutically effective amount" as used herein refers to that amount of the compound being administered which will relieve to some extent one or more of the symptoms of the disorder being treated. In reference to hair loss, a therapeutically effective amount refers to that amount which has the effect of (1) reducing the amount of hair loss, (2) inhibiting (that is, slowing to some extent, preferably stopping) androgen sensitivity, (3) inducing the grow of hair, and/or, (4) relieving to some extent (or, preferably, eliminating) one or more symptoms associated with the androgen related disease including but not limited to hair loss.

"Pharmaceutically acceptable salt" refers to those salts which retain the biological effectiveness and properties of the free bases and which are obtained by reaction with inorganic or organic acids such as hydrochloric acid, hydrobromic acid, sulfuric acid, nitric acid, phosphoric acid, methanesulfonic acid, ethanesulfonic acid, p-toluenesulfonic acid, salicylic acid, malic acid, maleic acid, succinic acid, tartaric acid, citric acid, and the like.

A "pharmaceutical composition" refers to a mixture of one or more of the compounds described herein, or a pharmaceutically acceptable salts thereof, with other chemical components, such as physiologically acceptable carriers and excipients. The purpose of a pharmaceutical composition is to facilitate administration of a compound to an organism.

As used herein, a "pharmaceutically acceptable carrier" refers to a carrier or diluent that does not cause significant irritation to an organism and does not abrogate the biological activity and properties of the administered compound.

An "excipient" refers to an inert substance added to a pharmaceutical composition to further facilitate administration of a compound. Examples, without limitation, of excipients include calcium carbonate, calcium phosphate, various sugars and types of starch, cellulose derivatives, gelatin, vegetable oils and polyethylene glycols.

"Treating" or "treatment" of a disease includes preventing the disease from occurring in an animal that may be predisposed to the disease but does not yet experience or exhibit symptoms of the disease (prophylactic treatment), inhibiting the disease (slowing or arresting its development), providing relief from the symptoms or side-effects of the disease (including palliative treatment), and relieving the disease (causing regression of the disease). With regard to cancer, these terms simply mean that the life expectancy of an individual affected with a cancer will be increased or that one or more of the symptoms of the disease will be reduced.

The term "prodrug" refers to an agent, including nucleic acids and proteins, which is converted into a biologically active form *in vivo.* Prodrugs are often useful because, in some situations, they may be easier to administer than the parent compound. They may, for instance, be bioavailable by oral administration whereas the parent compound is not. The prodrug may also have improved solubility in pharmaceutical compositions over the parent drug. A prodrug may be converted into the parent drug by various mechanisms, including enzymatic processes and metabolic hydrolysis. Harper, N.J. (1962). Drug Latentiation in Tucker, ed. Progress in Drug Research, 4:221-294; Morozowich et al. (1977). Application of Physical Organic Principles to Prodrug Design in E. B. Roche ed. Design of Biopharmaceutical Properties through Prodrugs and Analogs, APhA; Acad. Pharm. Sci.; E. B. Roche, ed. (1977). Bioreversible Carriers in Drug in Drug Design, Theory and Application, APhA; H. Bundgaard, ed. (1985) Design of Prodrugs, Elsevier; Wang et al. (1999) Prodrug approaches to the improved delivery of peptide drug, Curr. Pharm. Design. 5(4):265-287; Pauletti et al. (1997), Improvement in peptide bioavailability: Peptidomimetics and Prodrug Strategies, Adv. Drug. Delivery Rev. 27:235-256; Mizen et al. (1998). The Use of Esters as Prodrugs for Oral Delivery of β-Lactam antibiotics, Pharm. Biotech, 11,:345-365; Gaignault et al. (1996). Designing Prodrugs and Bioprecursors I. Carrier Prodrugs, Pract. Med. Chem. 671-696; M. Asgharnejad (2000). Improving Oral Drug Transport Via Prodrugs, in G. L. Amidon, P. 1. Lee and E. M. Topp, Eds., Transport Processes in Pharmaceutical Systems, Marcell Dekker, p. 185-218; Balant et al. (1990) Prodrugs for the improvement of drug absorption via different routes of administration, Eur. J. Drug Metab. Pharmacokinet., 15(2): 143-53; Balimane and Sinko (1999). Involvement of multiple transporters in the oral absorption of nucleoside analogues, Adv. Drug Delivery Rev., 39(1-3):183-209; Browne (1997). Fosphenytoin (Cerebyx), Clin. Neuropharmacol. 20(1): 1-12; Bundgaard (1979). Bioreversible derivatization of drugs--principle and applicability to improve the therapeutic effects of drugs, Arch. Pharm. Chemi. 86(1): 1-39; H. Bundgaard, ed. (1985) Design of Prodrugs, New York: Elsevier; Fleisher et al. (1996). Improved oral drug delivery: solubility limitations overcome by the use of prodrugs, Adv. Drug Delivery Rev. 19(2): 115-130; Fleisher et al. (1985). Design of prodrugs for improved gastrointestinal absorption by intestinal enzyme targeting, Methods Enzymol. 112: 360-81; Farquhar D, et al. (1983). Biologically Reversible Phosphate-Protective Groups, J. Pharm. Sci., 72(3): 324-325; Han, H.K. et al. (2000). Targeted prodrug design to optimize drug delivery, AAPS PharmSci., 2(1): E6; Sadzuka Y. (2000). Effective prodrug liposome and conversion to active metabolite, Cur.r Drug Metab., 1(1):31-48; D.M. Lambert (2000) Rationale and applications of lipids as prodrug carriers, Eur. J. Pharm. Sci., 11 Suppl 2:S15-27; Wang, W. et al. (1999) Prodrug approaches to the improved delivery of peptide drugs. Curr. Pharm. Des., 5(4):265-87,

As used herein, the term "topically active agents" refers to compositions of the present disclosure that elicit pharmacological responses at the site of application (contact) to a host

As used herein, the term "topically" refers to application of the compositions of the present disclosure to the surface of the skin and mucosal cells and tissues.

As used herein, the term "surface" is used in its broadest sense. In one sense, the term refers to the outermost boundaries of an organism or inanimate object (e.g., vehicles, buildings, and food processing equipment, etc.) that are capable of being contacted by the compositions of the present disclosure (e.g., for animals: the skin, hair, and fur, etc., and for plants: the leaves, stems, flowering parts, and fruiting bodies, etc.). In another sense, the term also refers to the inner membranes and surfaces of animals and plants (e.g., for animals: the digestive tract, vascular tissues, and the like, and for plants: the vascular tissues, etc.) capable of being contacted by compositions by any of a number of transdermal delivery routes (e.g., injection, ingestion, transdermal delivery, inhalation, and the like).

Androgen signal transduction pathway means the physiological and or biological sequence of events and the proteins and genes involved therein, from the production of androgen through the binding to the androgen receptor and the subsequent binding and activation of the androgen receptor and associated proteins and cofactors resulting in the induction of gene transcription.

The term "nucleic acid" is a term of art that refers to a string of at least two base-sugar-phosphate combinations. For naked DNA delivery, a polynucleotide contains more than 120 monomeric units since it must be distinguished from an oligonucleotide. However, for purposes of delivering RNA, RNAi and siRNA, either single or double stranded, a polynucleotide contains 2 or more monomeric units. Nucleotides are the monomeric units of nucleic acid polymers. The term includes deoxyribonucleic acid (DNA) and ribonucleic acid (RNA) in the form of a messenger RNA, anti-sense, plasmid DNA, parts of a plasmid DNA or genetic material derived from a virus. Anti-sense is a polynucleotide that interferes with the function of DNA and/or RNA. The term nucleic acids--refers to a string of at least two base-sugar-phosphate combinations. Natural nucleic acids have a phosphate backbone, artificial nucleic acids may contain other types of backbones, but contain the same bases. Nucleotides are the monomeric units of nucleic acid polymers. The term includes deoxyribonucleic acid (DNA) and ribonucleic acid (RNA). RNA may be in the form of an tRNA (transfer RNA), siRNA (small nuclear RNA), rRNA (ribosomal RNA), mRNA (messenger RNA), anti-sense RNA, RNAi, siRNA, and ribozymes. The term also includes PNAs (peptide nucleic acids), phosphorothioates, and other variants of the phosphate backbone of native nucleic acids.

The term "siRNA" means a small inhibitory ribonucleic acid. The siRNA are typically less than 30 nucleotides in length and can be single or double stranded. The ribonucleotides can be natural or artificial and can be chemically modified. Longer siRNAs can comprise cleavage sites that can be enzymatically or chemically cleaved to produce siRNAs having lengths less than 30 nucleotides, typically 21 to 23 nucleotides. siRNAs share sequence homology with corresponding target mRNAs. The sequence homology can be 100 percent or less but sufficient to result is sequence specific association between the siRNA and the targeted mRNA. Exemplary siRNAs do not activate the interferon signal transduction pathway.

The term "inhibitory nucleic acid" means an RNA, DNA, or combination thereof that interferes or interrupts the translation of mRNA. Inhibitory nucleic acids can be single or double stranded. The nucleotides of the inhibitory nucleic acid can be chemically modified, natural or artificial.

### Embodiments

Embodiments of present disclosure are directed, in part, to preventing, reducing, or inhibiting hair loss in a host, for example a mammal, using compositions comprising inhibitory nucleic acids such as siRNAs or salts or prodrugs thereof. The siRNAs of the present disclosure are designed to inhibit or interfere with the translation of mRNA encoding proteins involved in the androgen signal transduction pathway. In some aspects, the siRNAs induce the enzymatic cleavage of target mRNAs. Proteins involved in the androgen signal transduction pathway include but are not limited to: the androgen receptor, 5-α reductase, aromatase, 3-α-hydroxysteroiddehydrogenase, 3-β-hydroxysteroiddehydrogenase, 3-β-hydroxysteroiddehydrogenase-4-5-isomerase, 17-β-hydroxysteroidoxidoreductase, and steroid sulfatase, Alternatively, aspects of the disclosure is also directed to inducing or increasing the growth of hair, for example using compositions comprising inhibitory nucleic acids such as siRNAs. The sequence of exemplary siRNAs and their targets is provided in Table 1.

In one embodiment, the expression of androgen receptor in a host cell, typically a hair cell, is reduced or inhibited by contacting the hair cell with an siRNA. siRNAs of the present disclosure comprise at least a partial sequence of the target mRNAs, for example mRNA encoding the androgen receptor. The sequence homology between an siRNA and a target mRNA can be 100 percent or less but greater than about 50 percent, and typically 90 percent or greater. The percentage of sequence homology between siRNA and a target mRNA should be sufficient to result in sequence specific association between the siRNA and the target mRNA under cytoplasmic conditions.

### The Physiology of Androgen related Hair Loss

One exemplary embodiment of the present disclosure is directed to the treatment or prevention of androgen related hair loss in an organism, for example the treatment or prevention of alopecia using inhibitory nucleic acids to interfere or interrupt the androgen receptor signal transduction pathway. Alopecia is essentially due to a disturbance in hair renewal which results, at first, in an acceleration in the frequency of the cycles at the expense of the quality of the hair and then of its amount. A progressive thinning of the head of hair takes place by regression of the so-called "terminal" hairs to the downy stage. Regions are preferentially affected, in particular the temple or frontal bulbs in men and, in women, a diffuse alopecia of the vertex is observed. The term alopecia covers a whole family of complaints of the hair follicle, whose final consequence is the partial or general permanent loss of the hair.

Accordingly, the present disclosure encompasses the treatment of hair loss in men and women related including alopecia, pattern baldness, or androgen related hair loss. In one embodiment, inhibitory nucleic acids prevent hair loss by down-regulating, inhibiting, or interrupting the expression of the androgen receptor in a host cell, for example a hair cell. By down-regulating the androgen receptor in hair cells, the hair cells are desensitized to androgens and the cell cycle of the hair cell is unaffected by androgens. Thus, another embodiment of the present disclosure provides a hair cell desensitized to androgens by inhibitory nucleic acids, for example siRNAs homologous to the androgen receptor that inhibit or reduce the expression of the androgen receptor in a host hair cell. It will be appreciated by those of skill in the art that the present disclosure encompasses siRNAs designed to interrupt or interfere with the expression or function of any protein in the androgen signal transduction pathway, either directly or indirectly, for the treatment, prevention, or reduction of hair loss in an organism.

### Inhibitory Nucleic Acids

The inhibitory nucleic acids of certain embodiments of the present disclosure are directed to inhibiting or interfering with the expression of proteins involved in the androgen signal transduction pathway. The inhibitory nucleic acids disclosed herein include small inhibitory ribonucleic acids (siRNAs) that are typically less than 30 nucleotides in length, more typically 21 to 23 nucleotides in length, and can be single or double stranded. One strand of a double-stranded siRNA comprises at least a partial sequence complementary to a target RNA. The ribonucleotides of the siRNA can be natural or artificial and can be chemically modified. Longer siRNAs can comprise cleavage sites that can be enzymatically or chemically cleaved to produce siRNAs having lengths less than 30 nucleotides. siRNAs share sequence homology with corresponding target mRNAs. The phosphate backbones of the siRNAs can be chemically modified to resist enzymatic degradation. The sequence homology can be about 100 percent or less, but sufficient to result is sequence specific association between the siRNA and the targeted mRNA.

Nucleic acids, in particular RNA, are known to participate in a form of post-transcriptional gene silencing termed "RNA interference" or RNAi. First observed in plants, reduction of expression of specific mRNA sequences was found to be inducible in *Drosophilia melanogaster* and *Caenorhabditis elegant* by introduction of double-stranded RNA (dsRNA) molecules mimicking the sequence of the mRNA. The effect was found to be potent and extremely long-lived in these experimental model organisms, generally extending to the F1 progeny of a treated adult specimen. Additionally, the effect was found to be exquisitely sequence-specific; discrepancy of even a few base pairs between the dsRNA and the target mRNA virtually abolished the silencing. RNAi has been used experimentally in these non-mammalian systems to generate transient silencing of specific genes of interest, especially those which are not amenable to more traditional gene knockout methods (e.g., those that produce embryonic lethality and thus cannot be studied in the adult animal).

The first evidence that dsRNA could lead to gene silencing came from work in the nematode *Caenorhabditis elegans.* Researchers Guo and Kemphues used antisense RNA to shut down expression of the par-1 gene in order to assess its function. As expected, injection of the antisense RNA disrupted expression of par-1, but quizzically, injection of the sense-strand control did too. This result was a puzzle until three years later. It was then that Fire and Mello first injected dsRNA — a mixture of both sense and antisense strands — into *C. elegans.* This injection resulted in much more efficient silencing than injection of either the sense or the antisense strands alone. Injection of just a few molecules of dsRNA per cell was sufficient to completely silence the homologous gene's expression. Furthermore, injection of dsRNA into the gut of the worm caused gene silencing not only throughout the worm, but also in its first generation offspring. The potency of RNAi inspired Fire and Timmons to try feeding nematodes bacteria that had been engineered to express dsRNA homologous to the C. elegans unc-22 gene. Surprisingly, these worms developed an unc-22 null-like phenotype. Further work showed that soaking worms in dsRNA was also able to induce silencing. These strategies, whereby large numbers of nematodes are exposed to dsRNA, have enabled large-scale screens to select for RNAi-defective *C. elegans* mutants and have led to large numbers of gene knockout studies within this organism. Thus, one embodiment of the present disclosure provides siRNAs comprising a sense strand and an anti-sense strand, wherein the sense strand comprises at least a partial sequence of a target mRNA.

RNAi has also been observed in *Drosophila melanogaster.* Although a strategy in which yeast were engineered to produce dsRNA and then fed to fruit flies failed to work, microinjecting *Drosophila* embryos with dsRNA does induce silencing. Silencing can also be induced by biolistic techniques in which dsRNA is "shot" into *Drosophila* embryos, or by engineering flies to carry DNA containing an inverted repeat of the gene to be silenced. Over the last few years, these RNAi strategies have been used as reverse genetics tools in *Drosophila* organisms, embryo lysates, and cells to characterize various loss-of-function phenotypes. Zamore and colleagues found that dsRNA added to *Drosophila* embryo lysates was processed to 21-23 nucleotide species. They also found that the homologous endogenous mRNA was cleaved only in the region corresponding to the introduced dsRNA and that cleavage occurred at 21-23 nucleotide intervals.

Current models of RNAi divide the process of inhibition into broad "initiation" and "effector" stages. In the initiation step, input dsRNA is digested into 21-23 nucleotide small interfering RNAs (siRNAs), which have also been called "guide RNAs." Evidence indicates that siRNAs are produced when the enzyme Dicer, a member of the RNase III family of dsRNA-specific ribonucleases, processively cleaves dsRNA in an ATP-dependent, processive manner. Successive cleavage events degrade the RNA to 19-21 bp duplexes (siRNAs), each with 2-nucleotide 3' overhangs. Inhibitory nucleic acids of the present disclosure can be enzymatically cleaved for example in vivo, to produce siRNAs from 10 to about 30 nucleotides, typically about 19 to about 23 nucleotides.

In the effector step, the siRNA duplexes bind to a nuclease complex to form what is known as the RNA-induced silencing complex, or RISC. An ATP-depending unwinding of the siRNA duplex is required for activation of the RISC. The active RISC then targets the homologous transcript by base pairing interactions and cleaves the mRNA ∼12 nucleotides from the 3' terminus of the siRNA. Although the mechanism of cleavage is at this date unclear, research indicates that each RISC contains a single siRNA and an RNase that appears to be distinct from Dicer. Because of the remarkable potency of RNAi in some organisms, an amplification step within the RNAi pathway has also been proposed. Amplification could occur by copying of the input dsRNAs, which would generate more siRNAs, or by replication of the siRNAs themselves. Alternatively or in addition, amplification could be effected by multiple turnover events of the RISC. One embodiment encompasses the in vivo amplification of the siRNAs disclosed herein. Additionally, the siRNAs described herein can form a complex with additional proteins and/or cofactors to enzymatically cleave a target mRNA.

### Androgens and the Androgen Receptor

Androgens, specifically testosterone (T) and dihydroxytestosterone (DHT), mediate hair loss including AGA. The present disclosure provides siRNAs that inhibit the expression of androgen receptors or 5α reductase thereby preventing or reducing hair loss or stimulating hair growth. Testosterone taken up by hair cells in the scalp is converted to DHT by the enzyme 5-α reductase. Inhibiting the expression of 5α reductase with the siRNAs of the present disclosure reduces the levels of DHT. In one embodiment, DHT can be reduced locally or systemically using the inhibitory nucleic acids described herein.

DHT binds to its receptor, the Androgen Receptor (AR), which acts to alter nuclear gene expression. The AR is an intracellular receptor residing in the nucleus or cytoplasm and is a member of the steroid hormone receptor family. The change in nuclear gene expression of androgen responsive genes in response to androgen forces the hair cell to progressively shorten the duration of anagen with successive hair cycles, causing reduced hair renewal and hair loss.

The AR is typically bound to heat shock protein 90 that maintains the AR inactive state and the AR hormone binding affinity (Fang, Y. et al. (1996). J Biol Chem. 271 (45), 28697-28702). Upon binding however, direct androgen action is initiated as inhibitory heat shock proteins are released from the androgen receptor. The AR is then phosphorylated and undergoes a conformational change necessary for translocation and dimerization (Grino, P.B. et al. (1987). Endocrinol. 120, 1914-1920). Although in the wild-type receptor, this ligand binding is necessary for transcriptional activity, one *in* vivo receptor with a deleted ligand binding domain does possess transcriptional activity. This may indicate that the unliganded binding domain is actually a repressor of receptor action due to conformational constraints in the unbound receptor possessing the ligand binding domain (Jenster, G. et al. Mol Endocrinol. (1991). 5, 1396-1404).

Once in the nucleus (either by direct binding there or by translocation), the phosphorylated receptor is dimerized and binds to a DNA androgen response element (ARE). The hormone response element, which is also bound by other hormone receptors from this family, is a 15 base pair sequence responsible for transcription initiation. Once bound, other transcription regulating proteins or co-activators may also bind the AR-ARE complex to stabilize the promoter of the regulated gene (Shibata, H. (1997). Recent Progress Horm Research. 52, 141-164; Kang, H.Y. (1999). J. Biol. Chem. 274 (13), 8570-8576). Such co-activators include proteins such as ARA 54, ARA 55, ARA 70, ARA 160 (Yeh, S. et al. (1996). Proc. Natl. Acad. Sci. USA. 93 (11), 5517-5521; Hsiao, P.W. (1999). J. Biol. Chem. 32, 22373-22379). This binding of such co-factors ultimately results in the regulation of transcription rate. Thus, one embodiment provides inhibitory nucleic acids, for example siRNAs, comprising at least a partial sequence of a target mRNA encoding a protein that associates with the androgen receptor, for example the AR-ARE. These siRNAs can inhibit or interfere with the expression of the AR associated proteins and thereby inhibit or interfere with the transcription acitivity of the AR. Exemplary AR associated proteins include but are not limited to ARA 54, ARA 55, ARA 70, ARA 160. The resultant mRNA from androgen dependent transcription is then processed and transported to ribosomes where it is translated into proteins that can alter cellular function. Thus, exemplary embodiments of the present disclosure encompass siRNAs that inhibit or interfere with the translation of mRNA from androgen dependent transcription.

In some tissues there is evidence for a ligand-independent dependent activation of transcriptional activity via the AR. An unliganded receptor with a deletion of the ligand binding domain may possess activity. This indicates activity in the absence of ligand binding. In addition, growth factors (insulin-like growth factor, keratinocyte growth factor, and epidermial growth factor) as well as protein kinase A activators might be able to induce a transcriptionally active AR in the absence of ligand binding. Some of these ligand independent transcription activators may act via influencing the AR phosphorylation state. Thus, one embodiment discloses siRNAs targeted to ligand independent transcription activators of AR, for example growth factors and protein kinase A activators for the treatment of androgen related diseases such as hair loss.

Ligand binding, for example DHT, alters the protein conformation of the AR to allow binding of coactivator molecules that can amplify the hormone signal and mediate transcriptional initiation of androgen responsive genes. The AR can also undergo intramolecular interactions that influence its activity and interaction with cofactors. The AR protein has several domains or motifs similar to the modular structure of other steroid hormone receptors. There are two characterized forms of the androgen receptor. The first, and predominant form, is a 110-114 kDa protein of 910-919 amino acids. The second is a smaller 87 kDa protein of about 720-729 amino acids in length that makes up only about 4-26% of the detectible androgen receptors located in varying tissues.

### 5-α reductase

Exemplary siRNAs of the present disclosure can also be targeted to enzymes that mediate the production or modification of androgens, for ezample 5-α reductase, The enzyme 5-α reductase converts testosterone into DHT and it is DHT that is most potent in promoting AGA. There are at least two 5-α reductase isoenzymes, type I and type II. They are active in different regions of the body, but they both produce DHT that can circulate throughout the body and exert effects in organs other than where DHT was produced. Type II 5-α reductase is the most active enzyme form. It produces 60% to 70% of the total DHT human beings. Type I produces the remaining 30% to 40% of DHT. 5-α reductase inhibitors, promoted as treatments for AGA, should ideally block both types of isoenzyme to have maximum effect. Finasteride is a type II 5-α reductase inhibitor and so reduces DHT levels by at most 70%. However, the use of finesteride has been associated with reduced libido, teratagenic effects and other side effects in certain individuals thus prompting a more specific and safer approach to treating AGA.

### Existing Therapies

All androgen-responsive genes are activated by androgen receptor (AR) bound to either T or DHT and it is believed that AR is more transcriptionally active when bound to DHT than T. Thus, therapies for treating androgen related diseases, including alopecia, encompass anti-androgens. The present disclosure encompasses therapies for hair loss, in particular androgen related hair loss, by using the inhibitory nucleic acids disclosed herein, alone, or in combination with existing hair loss therapies. For example the two classes of anti-androgens, presently available, are the steroidal derivatives, all of which possess mixed agonistic and antagonistic activities, and the pure non-steroidal anti-androgens of the class of flutamide and its derivatives. Flutamide has a structure similar to the male sex hormone testosterone. It works by blocking and preventing the binding of testosterone to the receptors on the surface of cells. The non-steroidal flutamide and its derivatives display pure anti-androgenic activity, without exerting agonistic or any other hormonal activity. Flutamide and its derivatives, Casodex and Anandron, are highly effective in the treatment of prostate cancer.

Exemplary anti-androgens with a steroid structure include but are not limited to (17 alpha-acetoxy-6-chloro-2-oxa-4, 6-pregnadiene-3, 20-dione). This compound inhibits 5- αdihydrotestosterone (5-α-DHT) and testosterone receptors activity by blocking androgen receptors and in this way decreasing the nuclear androgen receptors fraction, Representative examples of steroidal blocking agents include spironolactone, cyproterone acetate, trimethyltrienolone (available from Roussel Uclaf under the designation RU 2956), canrenone and canrenoic acid. Examples of suitable non-steroidal blocking agents include flutamide (α, α,.α.-trifluoro-2-methyl-4'-nitro-m-propionotoluidide) and hydroxy-flutamide (α, α,.α.-trifluoro-2-methyl-4'-nitro-m-lactoluidide), both available from Schering Corp., and RU 23908 (5,5-dimethyl-3-[4-nitro-3(trifluoromethyl)phenyl]-2,4-imidazolidinedione) and RU 22930 (5,6-dihydro-2-methyl-4-[4-nitro-3-(trifluromethyl)phenyl]-2H-1,2,4-oxadio zin-3-(4H)-one), available from Roussel Uclaf.

RU 56187 and RU 58841 are additional examples of non-steroidal anti-androgens. They are N-substituted arylthiohydantoins, and inhibit 5 α-dihydrotestosterone (5-α-DHT) and testosterone receptors activity by blocking androgen receptors. RU 58841 and RU 56187 are eliminated from blood stream relatively quickly, but they form a common metabolite, the N-desalkyl derivative, RU 56279 (also anti-androgen), which is eliminated much more slowly and is responsible for general adverse anti-androgenic effects.

Other compounds for the treatment of hair loss are disclosed in U.S. Pat. No. 6,451,777 which is incorporated by reference in its entirety and include but are not limited to lupane triterpenes, derivatives of lupane triterpenes, derivatives of oleanane triterpenes, derivatives of ursane triterpenes, and salts and mixtures thereof.

Additional therapeutic agents for the treatment of hair loss can be used in combination with the present disclosure, including formulations in which a minoxidil solution, typically comprising about 5% minoxidil, is combined with azelaic acid, typically about 5%. Azelaic acid is a topically effective inhibitor of 5-α reductase. Another part of this formulation is the anti-inflammatory agent betamethasone valerate. Additionally, retinoic acid can be added, typically about 0.025%.

Spironolactone is another suitable second therapeutic agent for use with the present disclosure. Spironolactone is a medication that has been used for many years to treat hypertension and fluid retention. Its effect is local at blocking DHT and there are no systemic effects from its use on the scalp. Spironolactone is a potent anti-androgen. It competes with DHT for the receptor sites on the hair follicles

According to another aspect of the present disclosure, a method is provided for hormonal therapy in a patient (i.e., suffering from an androgen-dependent condition) which includes contacting a nucleic acid encoding an 5-α reductase of a patient with an inhibitory RNA under conditions effective to bind the inhibitory RNA compound to the nucleic acid encoding an 5-α reductase and effect a change in an androgen-dependent condition, such as alopecia. Androgen-dependent conditions which may be treated according to the present disclosure include those conditions which are associated hyperandrogenic conditions.

### Pharmaceutical Compositions

Exemplary embodiments of the present disclosure include pharmaceutical compositions that can be manufactured by processes well known in the art, e.g., by means of conventional mixing, dissolving, granulating, dragee-making, levigating, emulsifying, encapsulating, entrapping, lyophilizing processes or spray drying. Moreover, in certain embodiments, the compositions of the present disclosure may be formulated for horticultural or agricultural use. Such formulations include dips, sprays, seed dressings, stem injections, sprays, and mists.

The compositions of the present disclosure can be liquids or lyophilized or otherwise dried formulation and include diluents of various buffer content (e.g., Tris-HCl, acetate, phosphate), pH and ionic strength, additives such as albumin or gelatin to prevent absorption to surfaces, a surfactant such as a polysorbate surfactant (e.g., TWEEN 20, TWEEN 40, TWEEN 60, and TWEEN 80), a pheoxypolyethoxyethanol surfactant (e.g., TRITON X-100, X-301, X-165, X-102, and X-200, and TYLOXAPOL) Pluronic F68, or sodium dodecyl sulfate, solubilizing agents (e.g., glycerol, polyethylene glycerol), anti-oxidants (e.g., ascorbic acid, sodium metabisulfite), preservatives (e.g., Thimerosal, benzyl alcohol, parabens), bulking substances or tonicity modifiers (e.g., lactose, mannitol), covalent attachment of polymers such as polyethylene glycol to the protein, complexation with metal ions, or incorporation of the material into or onto particulate preparations of polymeric compounds such as polylactic acid, polglycolic acid, hydrogels, etc, or onto liposomes, microemulsions, micelles, unilamellar or multilamellar vesicles, erythrocyte ghosts, or spheroplasts. Such compositions will influence the physical state, solubility, stability, rate of in vivo release, and rate of in vivo clearance, controlled or sustained release compositions include formulation in lipophilic depots (e.g., fatty acids, waxes, oils).

The present disclosure contemplates formulations that may be employed in pharmaceutical and therapeutic compositions and applications suitable for inhibiting the action of androgens relating to hair loss. Such compositions may be employed to inhibit expression of proteins related to hair growth or loss, interfere with signal transduction mechanisms of the androgen receptor, interfere with the formation, dissemination of androgens, or increase the growth of hair.

For in vivo applications, the compositions can be administered in any effective pharmaceutically acceptable form to warm blooded animals having hair, including human and animal subjects. Generally, this entails preparing compositions that are essentially free of pyrogens, as well as other impurities that could be harmful to humans or animals.

Other embodiments of the disclosure provide particulate compositions coated with polymers (e.g., poloxamers or poloxamines). Still other embodiments of the compositions of the disclosure incorporate particulate forms protective coatings, protease inhibitors or permeation enhancers for various routes of administration, including parenteral, pulmonary, nasal and oral. In one embodiment the pharmaceutical composition is administered buccally, rectally, vaginally, topically, nasally, parenterally, paracancerally, transmucosally, transdermally, intramuscularly, intravenously, intradermally, subcutaneously, intraperitonealy, intraventricularly, intracranially, intratumorally, spray or in any other form effective to deliver active compositions.

For topical applications, the pharmaceutically acceptable carrier may take the form of a liquid, cream, foam, lotion, or gel, and may additionally comprise organic solvents, emulsifiers, gelling agents, moisturizers, stabilizers, surfactants, wetting agents, preservatives, time release agents, and minor amounts of humectants, sequestering agents, dyes, perfumes, and other components commonly employed in pharmaceutical compositions for topical administration.

Further, as used herein "pharmaceutically acceptable carrier" are well known to those skilled in the art and include, but are not limited to, 0.01-0.1M and preferably 0.05M phosphate buffer or 0.8% saline. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media.

Parenteral vehicles include sodium chloride solution, Ringer's dextrose, dextrose and sodium chloride, lactated Ringer's or fixed oils. Intravenous vehicles include fluid and nutrient replenishers, electrolyte replenishers such as those based on Ringer's dextrose, and the like. Preservatives and other additives may also be present, such as, for example, antimicrobials, antioxidants, collating agents, inert gases and the like.

Controlled or sustained release compositions include formulation in lipophilic depots (e.g, fatty acids, waxes, oils). Also comprehended by the disclosure are particulate compositions coated with polymers (e.g. poloxamers or poloxamines) and the compound coupled to antibodies directed against tissue-specific receptors, ligands or antigens or coupled to ligands of tissue-specific receptors.

Tablet and dosage forms of the compositions in which the emulsions are formulated for oral or topical administration include liquid capsules, and suppositories. In solid dosage forms for oral administration, the compositions may be admixed with one or more substantially inert diluent (e.g., sucrose, lactose, or starch, and the like) and may additionally comprise lubricating agents, buffering agents, enteric coatings, and other components well known to those skilled in the art.

Compounds modified by the covalent attachment of water-soluble polymers such as polyethylene glycol, copolymers of polyethylene glycol and polypropylene glycol, carboxymethyl cellulose, dextran, polyvinyl alcohol, polyvinylpyrrolidone or polyproline are known to exhibit substantially longer half-lives in blood following intravenous injection than do the corresponding unmodified compounds. Such modifications may also increase the compound's solubility in aqueous solution, eliminate aggregation, enhance the physical and chemical stability of the compound, and greatly reduce the immunogenicity and reactivity of the compound. As a result, the desired in vivo biological activity may be achieved by the administration of such polymer-compound abducts less frequently or in lower doses than with the unmodified compound.

In yet another embodiment, the pharmaceutical composition can be delivered in a controlled release system. For example, the agent may be administered using intravenous infusion, an implantable osmotic pump, a transdermal patch, liposomes, or other modes of administration. In one embodiment, a pump may be used (Sefton (1987). CRC Crit. Ref. Biomed. Eng. 14:201; Buchwald et al. (1980). Surgery 88:507; Saudek et al. (1989). N. Engl. J. Med. 321:574). In another embodiment, polymeric materials can be used. In yet another embodiment a controlled release system can be placed in proximity of the therapeutic target, i.e., the brain, thus requiring only a fraction of the systemic. Preferably, a controlled release device is introduced into a subject in proximity of the site of inappropriate immune activation or a tumor. Other controlled release systems are discussed in the review by Langer (1990). Science 249:1527-1533.

In other embodiments, the compositions may be impregnated into absorptive materials, such as sutures, bandages, and gauze, or coated onto the surface of solid phase materials, such as surgical staples, zippers and catheters to deliver the compositions to a site for the prevention of microbial infection. Other delivery systems of this type will be readily apparent to those skilled in the art.

Examples of suitable oily vehicles or solvents for use with the present disclosure are vegetable or animal oils such as sunflower oil or fish-liver oil. Preparations can be effected both as dry and as wet granules. For parenteral administration (subcutaneous, intravenous, intra-arterial, or intramuscular injection), the compositions or their physiologically tolerated derivatives such as salts, esters, N-oxides, and the like are converted into a solution, suspension, or emulsion, if desired with the substances customary and suitable for this purpose, for example, solubilizers or other auxiliaries. Examples are: sterile liquids such as water and oils, with or without the addition of a surfactant and other pharmaceutically acceptable adjuvants. Illustrative oils are those of petroleum, animal, vegetable, or synthetic origin, for example, peanut oil, soybean oil, or mineral oil. In general, water, saline, aqueous dextrose and related sugar solutions, and glycols such as propylene glycols or polyethylene glycol are preferred liquid carriers, particularly for injectable solutions.

In addition, if desired, the composition can contain minor amounts of auxiliary substances such as wetting or emulsifying agents, pH buffering agents which enhance the effectiveness of the active ingredient.

An active component can be formulated into the composition as neutralized pharmaceutically acceptable salt forms. Pharmaceutically acceptable salts include the acid addition salts (formed with the free amino groups of the polypeptide or antibody molecule) and which are formed with inorganic acids such as, for example, hydrochloric or phosphoric acids, or such organic acids as acetic, oxalic, tartaric, mandelic, and the like. Salts formed from the free carboxyl groups can also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

For topical administration to body surfaces using, for example, creams gels, drops, and the like, the inhibitory nucleic acids and there prodrugs or their physiologically tolerated derivatives such as salts, esters, N-oxides, and the like are prepared and applied as solutions, suspensions, or emulsions in a physiologically acceptable diluent with or without a pharmaceutical carrier.

In another embodiment, the active compound can be delivered in a vesicle, in particular a liposome (see Langer (1990). Science, 249:1527-1533; Treat et al. (1989). in Lopez-Berestein and Fidler (eds.), Liposomes in the Therapy of Infectious Disease and Cancer, Liss, N.Y., pp. 353-365)..

Suitable salts of the compositions disclosed herein include pharmaceutically acceptable salts. Other salts may, however, be useful in the preparation of the compounds according to the disclosure or of their pharmaceutically acceptable salts. Suitable pharmaceutically acceptable salts of the compounds of this disclosure include acid addition salts which may, for example, be formed by mixing a solution of the compound according to the disclosure with a solution of a pharmaceutically acceptable acid such as hydrochloric acid, sulphuric acid, methanesulphonic acids fumaric acid, maleic acid, succinic acid, acetic acid, benzoic: acid, oxalic acid, citric acid, tartaric acid, carbonic acid or phosphoric acid.

### Combination Therapy

It is also an aspect of this disclosure that a composition described herein, or its prodrug, salt, or derivative might be combined with other therapeurtic agents for the treatment of the diseases and disorders discussed above. Exemplary second agents include but are not limited to zinc salts of carboxylic acids, saponins, tritapenes, crataegolic acid, celastrol, asiatic acid, inhibitors of 5-α-reductase, 1,4-methyl-4-azasteroids, androgen receptor antagonists, Minoxidil, azelaic acid and its derivatives, cyclosporin, triiodothyronine, diazoxide and potassium channel openers, and combinations thereof.

### Dosage

Compositions disclosed here can be administered in a therapuetically acceptable amount. Generally, a therapeutically effective amount means an amount of compound effective to prevent, alleviate or ameliorate symptoms of disease such as inducing hair growth or preventing hair loss. Therapeutically effective amounts of compositions described herein can be approximately 10 mg/m² to 400 mg/m², preferably 50 mg/m² to 300 mg/m², more preferably 100 mg/m² to 220 mg/m², even more preferably 195 mg/m². For any compound used in the methods of the disclosure, the therapeutically effective amount or dose can be estimated initially from cell culture assays. Then, the dosage can be formulated for use in animal models so as to achieve a circulating concentration range that includes the IC₅₀ as determined in cell culture (i.e., the concentration of the test compound which achieves a half-maximal inhibition of the androgen signal transduction pathway). Such information can then be used to more accurately determine useful doses in humans.

Toxicity and therapeutic efficacy of the compounds described herein can be determined by standard pharmaceutical procedures in cell cultures or experimental animals, e.g., by determining the IC₅₀ and the LD₅₀ for a subject composition. The data obtained from these cell culture assays and animal studies can be used in formulating a range of dosage for use in humans. The dosage may vary depending upon the dosage form employed and the route of administration utilized. The exact formulation, route of administration and dosage can be chosen by the individual physician in view of the patient's condition. (See e.g., Hardman (2001) in The Pharmacological Basis of Therapeutics (10th edition) McGraw Hill).

Dosage amount and interval may be adjusted individually to provide plasma levels of the active species which are sufficient regulate the androgen signal transduction pathway to achieve the desired result, for example induction of hair growth or prevention of hair loss. The minimal effective plasma concentration levels will vary for each compound but can be estimated from *in vitro* data, e.g., the concentration necessary to achieve 50-90% inhibition of an androgen signal transduction pathway may be ascertained using the assays described herein. Preferably, the dosages necessary to achieve the minimal effective plasma concentration levels will depend on individual characteristics and route of administration. HPLC assays or bioassays can be used to determine plasma concentrations.

Dosage intervals can also be determined using minimal effective plasma concentration levels. Compounds should be administered using a regimen that maintains plasma levels above the minimal effective plasma concentration level for 10-90% of the time, preferably between 30-90% and most preferably between 50-90%. In cases of local administration or selective uptake, the effective local concentration of the compositions may not be related to plasma concentration and other procedures known in the art may be employed to determine the correct dosage amount and interval.

The amount of a composition administered will, of course, be dependent on the subject being treated, the severity of the affliction, the manner of administration, the judgment of the prescribing physician, etc.

### Packaging

The compositions may, if desired, be presented in a pack or dispenser device, such as an FDA approved kit, which may contain one or more unit dosage forms containing the compositions described herein. The pack may for example comprise metal or plastic foil, such as a blister pack. The pack or dispenser device may be accompanied by instructions for administration. The pack or dispenser may also be accompanied by a notice associated with the container in a form prescribed by a governmental agency regulating the manufacture, use or sale of pharmaceuticals, which notice is reflective of approval by the agency of the form of the compositions or of human or veterinary administration. Such notice, for example, may be of the labeling approved by the U.S. Food and Drug Administration for prescription drugs or of an approved product insert. Compositions comprising a compound of the disclosure formulated in a compatible pharmaceutical carrier may also be prepared, placed in an appropriate container, and labeled for treatment of an indicated condition. Suitable conditions indicated on the label may include treatment of a tumor, inhibition of angiogenesis, treatment of fibrosis, diabetes, and the like.

### In vivo assays

The effect of an inhibitory nucleic acid can be tested by assaying the telogen period in C3H mice (Harlan Sprague Dawley, Inc., Indianapolis, Ind.) from approximately 40 days of age until about 75 days of age, when hair growth is synchronized. It is believed that after 75 days of age, hair growth is no longer synchronized. Wherein about 40 day-old mice with dark fur (brown or black) are used in hair growth experiments, melanogenesis occurs along with hair (fur) growth wherein the topical application of hair growth promoters are evaluated. The Telogen Conversion Assay herein below can be used to screen compounds for potential hair growth by measuring melanogenesis.

For example, three groups of 44 day-old C3H mice can be utilized: a vehicle control group, a positive control group, and a test compound group, wherein the test compound group is administered a compound of the present disclosure. The length of the assay is at least 19 days with 15 treatment days (wherein the treatment days occur Mondays through Fridays). Day 1 is the first day of treatment. Most studies will end on Day 19, but a few may be carried out to Day 24 if the melanogenesis response looks positive, but occurs slowly.

Typically, the mice are treated topically Monday through Friday on their lower back (base of tail to the lower rib). A pipettor and tip are used to deliver 100-400 µL to each mouse's back. The 400 µL application is applied slowly while moving hair on the mouse to allow the application to reach the skin.

While each treatment is being applied to the mouse topically, a visual grade of from 0 to 4 can be given to the skin color in the application area of each animal. As the mice convert from telogen to anagen their skin color will become more bluish-black. The grades 0 to 4 represent the following visual observations as the skin progresses from white to bluish-black: Whitish Skin Color-0; Skin is light gray (indication of initiation of anagen)-1; Appearance of Blue Spots-2; Blue Spots are aggregating to form one large blue area-3; Skin is dark blue (almost black) with color covering majority of treatment area (indication of mouse in full anagen)-4.

### EXAMPLES

### Example 1: Synthesis of siRNA

Single-stranded, gene-specific sense and antisense RNA oligomers optionally with overhanging 3' deoxynucleotides are prepared and purified by PAGE using the sequences listed in Tables 1-9 below. The two oligomers, 40 µM each, are annealed in 250 µl of buffer containing 50 mM Tris-HCl, pH 8.0 and 100 mM NaCl, by heating to 94° C for 2 minutes, cooling to 90° C for 1 minute, then cooling to 20° C at a rate of 1 ° C per minute. The resulting siRNA is stored at -20° C prior to use.

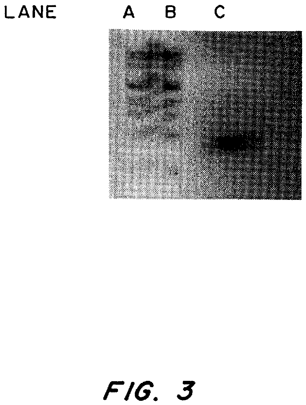

The steroid 5-alpha-reductase (SRD5a Locus Link id: 6715) was PCR amplified from the full-length cDNA clone (clone MGC:12396 IMAGE:3683274). Primers were designed to generate the full-length clone for TA-cloning into pcDNA 3.1 CT-GFP. As such, the reverse primer lacked a stop codon (Forward Primer: atgcaggttcagtgccagca [Seq ID No.: 1401]; Reverse Primer: ttaaaaagatgaatggaataag [Seq ID No.: 1402]) The 800 base pair product is shown in Figure 3.

### Example 2: In vitro assays

To determine the inhibition of androgen signal transduction proteins with siRNAs prepared above, the siRNAs are administered to cell culture cells expressing androgen signal transduction pathway proteins such as the androgen receptor or 5α reductase. Exemplary cell lines expressing androgen signal transduction pathway proteins are found in the catalogue for American Tissue Type Culture which is incorporated herein in its entirety. Briefly, cell lines are maintained in RPMI 1640 media (GibcoBRL, Gaithersburg, Md.) containing 10% BCS. Varying amounts (150-350 µL/ml) of siRNA were added to the culture media. Cells are incubated under the same conditions, at 37° C, in 5% CO₂ for 1-4 days. At the end of the incubation period, the cells are washed with PBS (phosphate buffered saline) and detached from the culture vessels using versene. The cells are then assayed for expression of androgen signal transduction proteins such as androgen receptor and or 5α reductase.

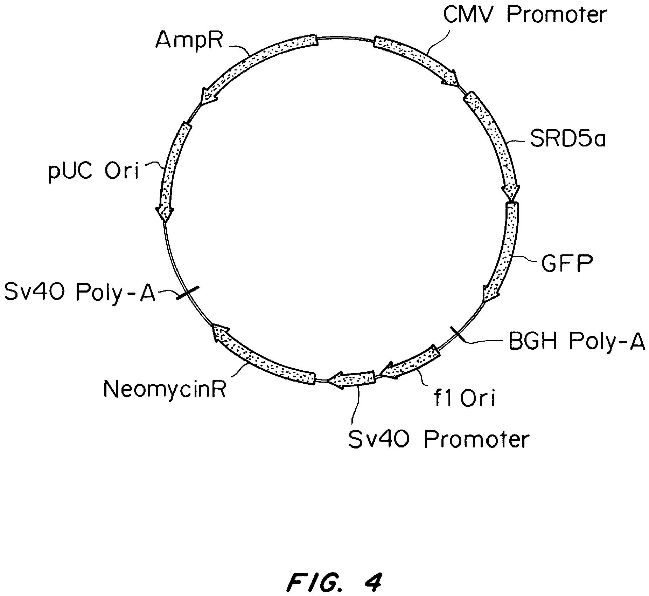

Figure 4 shows the PCR amplicon that was TA cloned (Invitrogen) according to manufacture's instructions to generate pcDNA 3.1/ SRD5a-GFP. This plasmid generates the SRD5a protein linked to the GFP (green fluorescent protein) reporter in mammalian cells. The pcDNA 3.1/SRD5a-GFP was transfected into Sy5y cells and confocal images were taken 24 hours later.

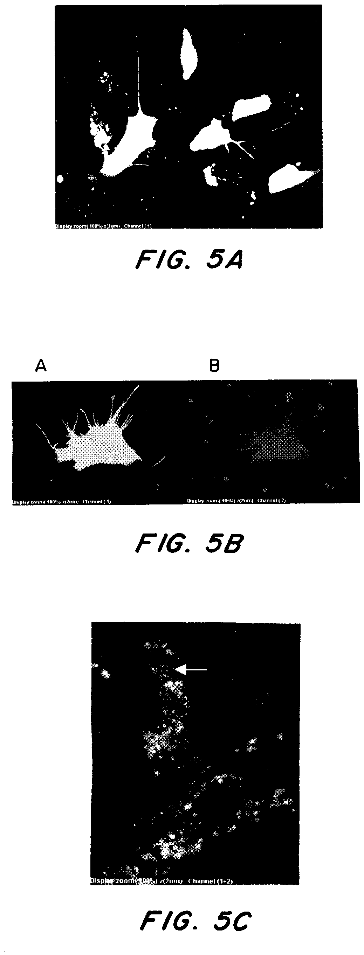

Figure 5A shows abundant reporter gene expression. The SRD5a amplicon was ligated 5' and 3' with T7 primers. An in vitro transcription reaction (Ambion) was conducted according to manufacturer's instructions to generate full-length double stranded RNA (dsRNA) encoding for the SRD5a protein. RNase III digestion was carried out according to manufacturer's (Ambion) directions to generate several 19-21 nucleotide dsRNAs. These dsRNA species were rhodamine labeled (Molecular Probes) and transfected (Qiagen) into Sy5y cells expressing the SRD5a-GFP protein. Procedures were preformed according to the respective manufacturer's instructions.

Figure 5B shows confocal micrographs of GFP reporter cells containing rhodamine labeled dsRNA for knockdown of the SRD5a protein 4 hours post-transfection of Rhodamine labeled dsRNA. Panel A shows green fluorescent protein fluorescence in transfected cells, and panel B shows the same cells with rhodamine labeled dsRNA fluorescence.

Figure 5C is a confocal fluorescence micrograph taken 24 hrs after transfection with the dsRNA. The arrow indicates puntate green fluoresence in a field of predominant rhodamine fluorescence. Thus, the data show that transfection with dsRNA inhibits the expression of the the SRD5a-GFP protein.

The present disclosure is not to be limited in scope by the exemplified embodiments which are intended as illustrations of single aspects of the disclosure, and any clones, nucleic or amino acid sequences which are functionally equivalent are within the scope of the disclosure. Indeed, various modifications of the disclosure in addition to those described herein will become apparent to those skilled in the art from the foregoing description and accompanying drawings. Such modifications are intended to fall within the scope of the appended claims.

All references cited herein are hereby incorporated by reference in their entirety.

| **Table 1: 5-alpha reductase Type I** |
|---|
| **5-alpha reductase Type I - P18405 (gi:134151) [SEQ ID NO.1]** |
| 1 atggcaacgg cgacgggggt ggcggaggag cgcctgctgg ccgcgctcgc ctacctgcag 61 tgcgccgtgg gctgcgcggt cttcccgcgt ctccgcagcg cgcccaactg catcctcctg 121 gccatgttcc tcgtccacta cgggcatcgg tgcttaattt acccatttct gatgcgagga 181 ggaaagccta tgccactgtt ggcgtgtaca atggcgatta tgttctgtac ctgtaacggc 241 tatttgcaaa gcagatactt gagccattgt gcagtgtatg ctgatgactg ggtaacagat 301 ccccgttttc taataggttt tggcttgtgg ttaacaggca tgttgataaa catccattca 361 gatcatatcc taaggaatct cagaaaacca ggagatactg gatacaaaat accaagggga 421 ggcttatttg aatacgtaac tgcagccaac tattttggag aaatcatgga gtggtgtggc 481 tatgccctgg ccagctggtc tgtccaaggc gcggcttttg ctttcttcac gttttgtttt 541 ttatctggta gagcaaaaga gcatcatgag tggtacctcc ggaaatttga agagtatcca 601 aagttcagaa aaattataat tccatttttg ttttaa |
| **Target sequence 1:** AACGGCGACGGGGGTGGCGGA **[SEQ ID NO. 2]** |
| Position in gene sequence: 6 |
| GC content: 76.2% |
| Sense strand siRNA: CGGCGACGGGGGUGGCGGAtt **[SEQ ID NO 3.]** |
| Antisense strand siRNA: UCCGCCACCCCCGUCGCCGtt **[SEQ ID NO. 4]** |
| **Target sequence 2:** AACTGCATCCTCCTG121GCC **[SEQ ID NO. 5]** |
| Position in gene sequence: 108 |
| GC content: 52.4% |
| Sense strand siRNA: CUGCAUCCUCCUG121GCCtt **[SEQ ID NO. 6]** |
| Antisense strand siRNA: GGC 121 CAGGAGGAUGCAGtt **[SEQ ID NO. 7]** |
| **Target sequence 3:** AATTTACCCATTTCTGATGCG **[SEQ ID NO. 8]** |
| Position in gene sequence: 161 |
| GC content: 38.1% |
| Sense strand siRNA: UUUACCCAUUUCUGAUGCGtt **[SEQ TD NO. 9]** |
| Antisense strand siRNA; CGCAUCAGAAAUGGGUAAAtt **[SEQ ID NO. 10]** |
| **Target sequence 4:** AAAGCCTATGCCACTGTTGGC**[SEQ ID NO. 11]** |
| Position in gene sequence: 191 |
| GC content: 52.4% |
| Sense strand siRNA: AGCCUAUGCCACUGUUGGCtt **[SEQ ID NO. 12]** |
| Antisense strand siRNA: GCCAACAGUGGCAUAGGCUtt **[SEQ ID NO. 13]** |
| **Target sequence 5:** AATGGCGATTATGTTCTGTAC **[SEQ ID NO. 14]** |
| Position in gene sequence: 218 |
| GC content: 38.1% |
| Sense strand siRNA: UGGCGAUUAUGUUCUGUACtt **[SEQ ID NO. 15]** |
| Antisense strand siRNA: GUACAGAACAUAAUCGCCAtt **[SEQ ID NO. 16]** |
| **Target sequence 6:** AACGGC241TATTTGCAAAGC **[SEQ ID NO. 17]** |
| Position in gene sequence: 243 |
| GC content: 38.1% |
| Sense strand siRNA: CGGC241UAUUUGCAAAGCtt **[SEQ ID NO. 18]** |
| Antisense strand siRNA: GCUUUGCAAAUA142GCCGtt **[SEQ ID NO. 19]** |
| **Target sequence 7:** AAAGCAGATACTTGAGCCATT **[SEQ ID NO. 20]** |
| Position in gene sequence: 259 |
| GC content: 38.1% |
| Sense strand siRNA: AGCAGAUACUUGAGCCAUUtt **[SEQ ID NO. 21]** |
| Antisense strand siRNA: AAUGGCUCAAGUAUCUGCUtt **[SEQ ID NO. 22]** |
| **Target sequence 8:** AACAGAT301CCCCGTTTTCT **[SEQ ID NO. 23]** |
| Position in gene sequence: 305 |
| GC content: 38.1% |
| Sense strand siRNA: CAGAU301CCCCGUUUUCUtt **[SEQ ID NO. 24]** |
| Antisense strand siRNA: AGAAAACGGGG103AUCUGtt **[SEQ ID NO. 25]** |
| **Target sequence 9:** AATAGGTTTTGGCTTGTGGTT **[SEQ ID NO. 26]** |
| Position in gene sequence: 326 |
| GC content: 38.1% |
| Sense strand siRNA: UAGGUUUUGGCUUGUGGUUtt **[SEQ ID NO. 27]** |
| Antisense strand siRNA: AACCACAAGCCAAAACCUAtt **[SEQ ID NO. 28]** |
| **Target sequence 10:** AACAGGCATGTTGATAAACAT **[SEQ NO. 29]** |
| Position in gene sequence: 347 |
| GC content: 33.3% |
| Sense strand siRNA: CAGGCAUGUUGAUAAACAUtt **[SEQ ID NO. 30]** |
| Antisense strand siRNA: AUGUUUAUCAACAUGCCUGtt **[SEQ ID NO. 31]** |
| **Target sequence 11:** AAACATCCATTCA361GATCA **[SEQ ID NO. 32]** |
| Position in gene sequence: 362 |
| GC content: 28.6% |
| Sense strand siRNA: ACAUCCAUUCA361GAUCAtt **[SEQ ID NO. 33]** |
| Antisense strand siRNA: UGAUC163UGAAUGGAUGUtt**[SEQ ID NO. 34]** |
| **Target sequence 12:** AAGGAATCTCAGAAAACCAGG **[SEQ ID NO. 35]** |
| Position in gene sequence: 389 |
| GC content: 42.9% |
| Sense strand siRNA: GGAAUCUCAGAAAACCAGGtt **[SEQ ID NO. 36]** |
| Antisense strand siRNA: CCUGGUUUUCUGAGAUUCCtt **[SEQ ID NO. 37]** |
| **Target sequence 13:** AATCTCAGAAAACCAGGAGAT **[SEQ ID NO.38]** |
| Position in gene sequence: 393 |
| GC content: 38.1% |
| Sense strand siRNA: UCUCAGAAAACCAGGAGAUtt **[SEQ ID NO. 39]** |
| Antisense strand siRNA: AUCUCCUGGUUUUCUGAGAtt **[SEQ ID NO. 40]** |
| **Target sequence 14:** AAAACCAGGAGATACTGGATA **[SEQ ID NO. 41]** |
| Position in gene sequence: 401 |
| GC content: 38.1% |
| Sense strand siRNA: AACCAGGAGAUACUGGAUAtt**[SEQ ID NO. 42]** |
| Antisense strand siRNA: UAUCCAGUAUCUCCUGGUUtt **[SEQ ID NO. 43]** |
| **Target sequence 15:** AACCAGGAGATACTGGATACA **[SEQ ID NO. 44]** |
| Position in gene sequence: 403 |
| GC content: 42.9% |
| Sense strand siRNA: CCAGGAGAUACUGGAUACAtt **[SEQ ID NO. 45]** |
| Antisense strand siRNA: UGUAUCCAGUAUCUCCUGGtt **[SEQ ID NO. 46]** |
| **Target sequence 16:** AAAATACCAAGGGGA421GGC **[SEQ ID NO. 47]** |
| Position in gene sequence: 423 |
| GC content: 42.9% |
| Sense strand siRNA: AAUACCAAGGGGA421GGCtt **[SEQ ID NO. 48]** |
| Antisense strand siRNA: GCC124UCCCCUUGGUAUUtt **[SEQ ID NO. 49]** |
| **Target sequence 17:** AATACCAAGGGGA421 GGCTT **[SEQ ID NO. 50]** |
| Position in gene sequence: 425 |
| GC content: 42.9% |
| Sense strand siRNA: UACCAAGGGGA421GGCUUtt **[SEQ ID NO. 51]** |
| Antisense strand siRNA: AAGCC124UCCCCUUGGUAtt **[SEQ ID NO. 52]** |
| **Target sequence 18:** AAGGGGA421GGCTTATTTGA **[SEQ ID NO. 53]** |
| Position in gene sequence: 431 |
| GC content: 38.1% |
| Sense strand siRNA: GGGGA421GGCUUAUWGAtt **[SEQ ID NO. 54]** |
| Antisense strand siRNA: UCAAAUAAGCC124UCCCCtt **[SEQ ID NO. 55]** |
| **Target sequence 19:** AATACGTAACTGCAGCCAACT **[SEQ ID NO. 56]** |
| Position in gene sequence: 451 |
| GC content: 42.9% |
| Sense strand siRNA: UACGUAACUGCAGCCAACUtt **[SEQ ID NO. 57]** |
| Antisense strand siRNA: AGUUGGCUGCAGUUACGUAtt **[SEQ ID NO. 58]** |
| **Target sequence 20:** AACTGCAGCCAACTATTTTGG **[SEQ ID NO. 59]** |
| Position in gene sequence: 458 |
| content: 42.9% |
| Sense strand siRNA: CUGCAGCCAACUAUUUUGGtt **[SEQ ID NO. 60]** |
| Antisense strand siRNA: CCAAAAUAGUUGGCUGCAGtt **[SEQ ID NO. 61]** |
| **Sequence 21:** AACTATTTTGGAGAAATCATG **[SEQ ID NO. 62]** |
| Position in gene sequence: 468 |
| GC content: 28.6% |
| Sense strand siRNA: CUAUUUUGGAGAAAUCAUGtt **[SEQ ID NO. 63]** |
| Antisense strand siRNA: CAUGAUUUCUCCAAAAUAGtt **[SEQ ID NO. 64]** |
| **Target sequence 22:** AAATCATGGAGTGGTGTGGC4 **[SEQ ID NO. 65]** |
| Position in gene sequence: 481 |
| GC content: 47.6% |
| Sense strand siRNA: AUCAUGGAGUGGUGUGGC4tt **[SEQ ID NO. 66]** |
| Antisense strand siRNA: 4GCCACACCACUCCAUGAUtt **[SEQ ID NO. 67]** |
| **Target sequence 23:** AAGGCGCGGCTTTTGCTTTCT **[SEQ ID NO. 68]** |
| Position in gene sequence: 529 |
| GC content: 52.4% |
| Sense strand siRNA: GGCGCGGCUUUUGCUUUCUtt **[SEQ ID NO. 69]** |
| Antisense strand siRNA: AGAAAGCAAAAGCCGCGCCtt **[SEQ ID NO. 70]** |
| **Target sequence 24:** AAAAGAGCATCATGAGTGGTA **[SEQ ID NO. 71]** |
| Position in gene sequence: 581 |
| GC content: 38.1% |
| Sense strand siRNA: AAGAGCAUCAUGAGUGGUAtt **[SEQ ID NO. 72]** |
| Antisense strand siRNA: UACCACUCAUGAUGCUCWtt **(SEQ ID NO. 73]** |
| **Target sequence 25:** AAGAGCATCATGAGTGGTACC **[SEQ ID NO. 74]** |
| Position in gene sequence: 583 |
| GC content: 47.6% |
| Sense strand siRNA: GAGCAUCAUGAGUGGUACCtt **[SEQ ID NO. 75]** |
| Antisense strand siRNA: GGUACCACUCAUGAUGCUCtt **[SEQ ID NO. 76]** |
| **Target sequence 26:** AAATTTGAAGAGTATCCA601 **[SEQ ID NO. 77]** |
| Position in gene sequence: 609 |
| GC content: 23.8% |
| Sense strand siRNA: AUUUGAAGAGUAUCCA601tt **[SEQ ID NO. 78]** |
| Antisense strand siRNA: 106UGGAUACUCUUCAAAUtt **[SEQ ID NO. 79]** |
| **Target sequence 27:** AAGAGTATCCA601AAGTTCA **[SEQ ID NO. 80]** |
| Position in gene sequence: 616 |
| GC content: 28.6% |
| Sense strand siRNA: GAGUAUCCA601AAGUUCAtt **[SEQ ID NO. 81]** |
| Antisense strand siRNA: UGAACUU106UGGAUACUCtt **[SEQ ID NO. 82]** |
| **Target sequence 28:** AAGTTCAGAAAAATTATAATT **[SEQ ID NO. 83]** |
| Position in gene sequence: 630 |
| GC content: 14.3% |
| Sense strand siRNA: GUUCAGAAAAAUUAUAAUUtt **[SEQ ID NO. 84]** |
| Antisense strand siRNA: AAUUAUAAUUUUUCUGAACtt **[SEQ ID NO. 85]** |
| **Target sequence 29:** AAAAATTATAATTCCATTTTT **[SEQ ID NO. 86]** |
| Position in gene sequence: 638 |
| GC content: 9.5% |
| Sense strand siRNA: AAAUUAUAAUUCCAWUUUtt **[SEQ ID NO. 87]** |
| Antisense strand siRNA: AAAAAUGGAAUUAUAAUUUtt **[SEQ ID NO. 88]** |
| **Target sequence 30:** AAATTATAATTCCATTTTTGT **[SEQ ID NO. 89]** |
| Position in gene sequence: 640 |
| GC content: 14.3% |
| Sense strand siRNA: AUUAUAAUUCCAUCTUUUGUtt **[SEQ ID NO. 90]** |
| Antisense strand siRNA: ACAAAAAUGGAAUUAUAAUtt **[SEQ ID NO. 91]** |

| **Table 2: 5-alpha reductase Type II** |
|---|
| **5-alpha reductase Type II - Q28892 (gi: 2498888) [SEQ ID NO. 92]** |
| 1 atgcaggttc agtgccagca gagcccagtg ctggcaggca gcgccacttt ggtcgccctt 61 ggggcactgg tcttgtacgt cgcgaagccc tccggctacg ggaagcacac ggagagcctg 121 aagcccgcgg ctacccgcct gccagcccgc gcagcctggt tcctgcagga gctgccctcc 181 ttcgcggtgc ccgcggggat cctcgctcgg cagcccctct ccctcttcgg gccacctggg 241 acggtgcttc tgggcctctt ctgcgtacat tacttccaca ggacatttgt gtactcactg 301 ctcaatcgag ggaggcctta tccagctgta ctcattttcc gaggcattgc cttctgcgct 361 ggaaatggat tccttcaaag ctactatctg atttactgtg ctgaataccc tgatgggtgg 421 tacacagaca tacggttttg cttgggtgtc ttcttattta ttttgggaat gggagtcaac 481 atccatagtg actatatatt gcgccagctc aggaagcctg gagaaatcac ctacaggatt 541 ccaaaaggtg gcttgtttac gtatgtttct ggagccaatt tccttggtga gatcattgaa 601 tggatcggct atgcgctggc cacttggtcc ctcccagcac ttgcatttgc atttttctca 661 gtttgtttcc ttgggctgcg agcttttcac caccataggt tctacctcaa gatgtttgag 721 gactacccca aatctcggaa agcccttatt ccattcatct tttaa |
| **Target sequence 1:** AAGCCCTCCGGCTACGGGAAG **[SEQ ID NO. 93]** |
| Position in gene sequence: 88 |
| GC content: 66.7% |
| Sense strand siRNA: GCCCUCCGGCUACGGGAAGtt **[SEQ ID NO. 94]** |
| Antisense strand siRNA: CUUCCCGUAGCCGGAGGGCtt **[SEQ ID NO. 95]** |
| **Target sequence 2:** AAGCACACGGAGAGCCTG121 **[SEQ ID NO. 96]** |
| Position in gene sequence: 106 |
| GC content: 52.4% |
| Sense strand siRNA: GCACACGGAGAGCCUG121tt **[SEQ ID NO. 97]** |
| Antisense strand siRNA: 121CAGGCUCUCCGUGUGCtt **[SEQ ID NO. 98]** |
| **Target sequence 3:** AAGCCCGCGGCTACCCGCCTG **[SEQ ID NO. 99]** |
| Position in gene sequence: 127 |
| GC content: 76.2% |
| Sense strand siRNA: GCCCGCGGCUACCCGCCUGtt **[SEQ ID NO. 100]** |
| Antisense strand siRNA: CAGGCGGGUAGCCGCGGGCtt **[SEQ ID NO. 101]** |
| **Target sequence 4:** AATCGAGGGAGGCCTTATCCA **[SEQ ID NO. 102]** |
| Position in gene sequence: 319 |
| GC content: 52.4% |
| Sense strand siRNA: UCGAGGGAGGCCUUAUCCAtt **[SEQ ID NO. 103]** |
| Antisense strand siRNA: UGGAUAAGGCCUCCCUCGAtt **[SEQ ID NO. 104]** |
| **Target sequence 5:** AAATGGATTCCTTCAAAGCTA **[SEQ ID NO. 105]** |
| Position in gene sequence: 381 |
| GC content: 33.3% |
| Sense strand siRNA: AUGGAUUCCUUCAAAGCUAtt **[SEQ ID NO. 106]** |
| Antisense strand siRNA: UAGCUUUGAAGGAAUCCAUtt **[SEQ ID NO. 107]** |
| **Target sequence 6:** AAAGCTACTATCTGATTTACT **[SEQ ID NO. 108]** |
| Position in gene sequence: 395 |
| GC content: 28.6% |
| Sense strand siRNA: AGCUACUAUCUGAUUUACUtt **[SEQ ID NO. 109]** |
| Antisense strand siRNA: AGUAAAUCAGAUAGUAGCUtt **[SEQ ID NO. 110]** |
| **Target sequence 7:** AATACCCTGATGGGTGG421T **[SEQ ID NO. 111]** |
| Position in gene sequence: 422 |
| GC content: 42.9% |
| Sense strand siRNA: UACCCUGAUGGGUGG421Utt **[SEQ ID NO.112]** |
| Antisense strand siRNA: A124CCACCCAUCAGGGUAtt **[SEQ ID NO. 113]** |
| **Target sequence 8:** AATGGGAGTCAAC481ATCCA **[SEQ ID NO. 114]** |
| Position in gene sequence: 489 |
| GC content: 38.1 % |
| Sense strand siRNA: UGGGAGUCAAC481AUCCAtt **[SEQ ID NO. 115]** |
| Antisense strand siRNA: UGGAU184GUUGACUCCCAtt **[SEQ ID NO. 116]** |
| **Target sequence 9:** AAC481ATCCATAGTGACTAT **[SEQ ID NO. 117]** |
| Position in gene sequence: 499 |
| GC content: 28.6% |
| Sense strand siRNA: C481AUCCAUAGUGACUAUtt **[SEQ ID NO. 118]** |
| Antisense strand siRNA: AUAGUCACUAUGGAU184Gtt **[SEQ ID NO. 119]** |
| **Target sequence 10:** AAGCCTGGAGAAATCACCTAC **[SEQ ID NO. 120]** |
| Position in gene sequence: 538 |
| GC content: 47.6% |
| Sense strand siRNA: GCCUGGAGAAAUCACCUACtt **[SEQ ID NO. 121]** |
| Antisense strand siRNA: GUAGGUGAUUUCUCCAGGCtt **[SEQ ID NO. 122]** |
| **Target sequence 11:** AAATCACCTACAGGATT541C **[SEQ ID NO. 123]** |
| Position in gene sequence: 548 |
| GC content: 33.3% |
| Sense strand siRNA: AUCACCUACAGGAUU541Ctt **[SEQ ID NO. 124]** |
| Antisense strand siRNA: G145AAUCCUGUAGGUGAUtt **[SEQ ID NO. 125]** |
| **Target sequence 12:** AAAAGGTGGCTTGTTTACGTA **[SEQ ID NO. 126]** |
| Position in gene sequence: 570 |
| GC content: 38.1% |
| Sense strand siRNA: AAGGUGGCUUGUUUACGUAtt **[SEQ ID NO. 127]** |
| Antisense strand siRNA: UACGUAAACAAGCCACCUUtt **[SEQ ID NO. 128]** |
| **Target sequence 13:** AAGGTGGCTTGTTTACGTATG **[SEQ ID NO. 129]** |
| Position in gene sequence: 572 |
| GC content: 42.9% |
| Sense strand siRNA: GGUGGCUUGUUUACGUAUGtt **[SEQ ID NO. 130]** |
| Antisense strand siRNA: CAUACGUAAACAAGCCACCtt **[SEQ ID NO. 131]** |
| **Target sequence 14:** AATTTCCTTGGTGAGATCATT **[SEQ ID NO. 132]** |
| Position in gene sequence: 604 |
| GC content: 33.3% |
| Sense strand siRNA: UUUCCUUGGUGAGAUCAUUtt **[SEQ ID NO. 133]** |
| Antisense strand siRNA: AAUGAUCUCACCAAGGAAAtt **[SEQ ID NO. 134]** |
| **Target sequence 15:** AA601TGGATCGGCTATGCGC **[SEQ ID NO. 135]** |
| Position in gene sequence: 626 |
| GC content: 47.6% |
| Sense strand siRNA: 601UGGAUCGGCUAUGCGCtt **[SEQ ID NO. 136]** |
| Antisense strand siRNA: GCGCAUAGCCGAUCCA106tt **[SEQ ID NO. 137]** |
| **Target sequence 16:** AAGATGTTTGAG721GACTAC **[SEQ ID NO. 138]** |
| Position in gene sequence: 742 |
| GC content: 33.3% |
| Sense strand siRNA: GAUGUUUGAG721GACUACtt **[SEQ ID NO. 139]** |
| Antisense strand siRNA: GUAGUC 127CUCAAACAUCtt **[SEQ ID NO. 140]** |
| **Target sequence 17:** AAATCTCGGAAAGCCCTTATT **[SEQ ID NO. 141]** |
| Position in gene sequence: 766 |
| GC content: 38.1% |
| Sense strand siRNA: AUCUCGGAAAGCCCUUAUUtt **[SEQ ID NO. 142]** |
| Antisense strand siRNA; AAUAAGGGCUUUCCGAGAUtt **[SEQ ID NO. 143]** |
| **Target sequence 18:** AAAGCCCTTATTCCATTCATC **[SEQ ID NO. 144]** |
| Position in gene sequence: 775 |
| GC content: 38.1% |
| Sense strand siRNA: AGCCCUUAUUCCAUUCAUCtt **[SEQ ID NO. 145]** |
| Antisense strand siRNA: GAUGAAUGGAAUAAGGGCUtt **[SEQ ID NO. 1461** |

| **Table 3: Androgen Receptor** |
|---|
| **Androgen Receptor - P10275 (gi:2132251) [SEQ ID NO. 147]** |
| 1 atggaagtgc agttagggct gggaagggtc taccctcggc cgccgtccaa gacctaccga 61 ggagctttcc agaatctgtt ccagagcgtg cgcgaagtga tccagaaccc gggccccagg 121 cacccagagg ccgcgagcgc agcacctccc ggcgccagtt tgctgctgct gcagcagcag 181 cagcagcagc agcagcagca gcagcagcag cagcagcagc agcagcagca gcagcagca 241 gagactagcc ccaggcagca gcagcagcag cagggtgagg atggttctcc ccaagcccat 301 cgtagaggcc ccacaggcta cctggtcctg gatgaggaac agcaaccttc acagccgcag 361 tcggccctgg agtgccaccc cgagagaggt tgcgtcccag agcctggagc cgccgtggcc 421 gccagcaagg ggctgccgca gcagctgcca gcacctccgg acgaggatga ctcagctgcc 481 ccatccacgt tgtccctgct gggccccact ttccccggct taagcagctg ctccgctgac 541 cttaaagaca tcctgagcga ggccagcacc atgcaactcc ttcagcaaca gcagcaggaa 601 gcagtatccg aaggcagcag cagcgggaga gcgagggagg cctcgggggc tcccacttcc 661 tccaaggaca attacttagg gggcacttcg accatttctg acaacgccaa ggagttgtgt 721 aaggcagtgt cggtgtccat gggcctgggt gtggaggcgt tggagcatct gagtccaggg 781 gaacagcttc ggggggattg catgtacgcc ccacttttgg gagttccacc cgctgtgcgt 841 cccactcctt gtgccccatt ggccgaatgc aaaggttctc tgctagacga cagcgcaggc 901 aagagcactg aagatactgc tgagtattcc cctttcaagg gaggttacac caaagggcta 961 gaaggcgaga gcctaggctg ctctggcagc gctgcagcag ggagctccgg gacacttgaa 1021 ctgccgtcta ccctgtctct ctacaagtcc ggagcactgg acgaggcagc tgcgtaccag 1081 agtcgcgact actacaactt tccactggct ctggccggac cgccgccccc tccgccgcct 1141 ccccatcccc acgctcgcat caagctggag aacccgctgg actacggcag cgcctgggcg 1201 gctgcggcgg cgcagtgccg ctatggggac ctggcgagcc tgcatggcgc gggtgcagcg 1261 ggacccggtt ctgggtcacc ctcagccgcc gcttcctcat cctggcacac tctcttcaca 1321 gccgaagaag gccagttgta tggaccgtgt ggtggtggtg ggggtggtgg cggcggcggc 1381 ggcggcggcg gcggcggcgg cggcggcggc ggcggcggcg aggcgggagc tgtagccccc 1441 tacggctaca ctcggccccc tcaggggctg gcgggccagg aaagcgactt caccgcacct 1501 gatgtgtggt accctggcgg catggtgagc agagtgccct atcccagtcc cacttgtgtc 1561 aaaagcgaaa tgggcccctg gatggatagc tactccggac cttacgggga catgcgtttg 1621 gagactgcca gggaccatgt tttgcccatt gactattact ttccacccca gaagacctgc 1681 ctgatctgtg gagatgaagc ttctgggtgt cactatggag ctctcacatg tggaagctgc 1741 aaggtcttct tcaaaagagc cgctgaaggg aaacagaagt acctgtgcgc cagcagaaat 1801 gattgcacta ttgataaatt ccgaaggaaa aattgtccat cttgtcgtct tcggaaatgt 1861 tatgaagcag ggatgactct gggagcccgg aagctgaaga aacttggtaa tctgaaacta 1921 caggaggaag gagaggcttc cagcaccacc agccccactg aggagacaac ccagaagctg 1981 acagtgtcac acattgaagg ctatgaatgt cagcccatct ttctgaatgt cctggaagcc 2041 attgagccag gtgtagtgtg tgctggacac gacaacaacc agcccgactc ctttgcagcc 2101 ttgctctcta gcctcaatga actgggagag agacagcttg tacacgtggt caagtgggcc 2161 aaggccttgc ctggcttccg caacttacac gtggacgacc agatggctgt cattcagtac 2221 tcctggatgg ggctcatggt gtttgccatg ggctggcgat ccttcaccaa tgtcaactcc 2281 aggatgctct acttcgcccc tgatctggtt ttcaatgagt accgcatgca caagtcccgg 2341 atgtacagcc agtgtgtccg aatgaggcac ctctctcaag agtttggatg gctccaaatc 2401 accccccagg aattcctgtg catgaaagca ctgctactct tcagcattat tccagtggat 2461 gggctgaaaa atcaaaaatt ctttgatgaa cttcgaatga actacatcaa ggaactcgat 2521 cgtatcattg catgcaaaag aaaaaatccc acatcctgct caagacgctt ctaccagctc 2581 accaagctcc tggactccgt gcagcctatt gcgagagagc tgcatcagtt cacttttgac 2641 ctgctaatca agtcacacat ggtgagcgtg gactttccgg aaatgatggc agagatcatc 2701 tctgtgcaag tgcccaagat cctttctggg aaagtcaagc ccatctattt ccacacccag |
| 2761 tga |
| **Target sequence 1:** AAGTGCAGTTAGGGCTGGGAA **[SEQ ID NO. 148]** |
| Position in gene sequence: 5 |
| GC content: 52.4% |
| Sense strand siRNA: GUGCAGUUAGGGCUGGGAAtt **[SEQ ID NO. 149]** |
| Antisense strand siRNA: UUCCCAGCCCUAACUGCACtt **[SEQ ID NO. 150]** |
| **Target sequence 2:** AAGGGTCTACCCTCGGCCGCC **[SEQ ID NO. 151]** |
| Position in gene sequence: 24 |
| GC content: 71.4% |
| Sense strand siRNA: GGGUCUACCCUCGGCCGCCtt **[SEQ ID NO. 152].** |
| Antisense strand siRNA: GGCGGCCGAGGGUAGACCCtt **[SEQ ID NO. 153]** |
| **Target sequence 3:** AAGACCTACCGA61GGAGCTT **[SEQ ID NO. 154]** |
| Position in gene sequence: 49 |
| Content: 47.6% |
| Sense strand siRNA: GACCUACCGA61GGAGCUUtt **[SEQ ID NO. 155]** |
| Antisense strand siRNA: AAGCUCC16UCGGUAGGUCtt **[SEQ ID NO. 156]** |
| **Target sequence 4:** AATCTGTTCCAGAGCGTGCGC **[SEQ ID NO. 157]** |
| Position in gene sequence: 75 |
| GC content; 57.1% |
| sense strand siRNA: UCUGUUCCAGAGCGUGCGCtt **[SEQ ID NO. 158]** |
| Antisense strand siRNA: GCGCACGCUCUGGAACAGAtt **[SEQ ID NO. 159]** |
| **Target sequence 5:** AAGTGATCCAGAACCCGGGCC **[SEQ ID NO. 160]** |
| Position in gene sequence: 97 |
| GC content: 61.9% |
| Sense strand siRNA: GUGAUCCAGAACCCGGGCCtt **[SEQ ID NO. 161]** |
| Antisense strand siRNA: GGCCCGGGUUCUGGAUCACtt **[SEQ ID NO. 162]** |
| **Target sequence 6:** AACCCGGGCCCCAGG12ICAC **[SEQ ID NO. 163]** |
| Position in gene sequence: 108 |
| GC content: 66.7% |
| Sense strand siRNA: CCCGGGCCCCAGG121CACtt **[SEQ ID NO. 164]** |
| Antisense strand siRNA: GUG121CCUGGGGCCCGGGtt **[SEQ ID NO. 165]** |
| **Target sequence 7:** AA241GAGACTAGCCCCAGGC **[SEQ ID NO. 166]** |
| Position in gene sequence: 247 |
| GC content: 52.4% |
| Sense strand siRNA: 241 GAGACUAGCCCCAGGCtt **[SEQ ID NO. 167]** |
| Antisense strand siRNA: GCCUGGGGCUAGUCUC142tt **[SEQ ID NO. 168]** |
| **Target sequence 8:** AAGCCCAT301CGTAGAGGCC **[SEQ ID NO. 169]** |
| Position in gene sequence: 304 |
| GC content: 52.4% |
| Sense strand siRNA: GCCCAU301CGUAGAGGCCtt **[SEQ ID NO. 170]** |
| Antisense strand siRNA: GGCCUCUACG103AUGGGCtt **[SEQ ID NO. 171]** |
| **Target sequence 9:** AACAGCAACCTTCACAGCCGC **[SEQ ID NO. 172]** |
| Position in gene sequence: 352 |
| GC content: 57.1% |
| Sense strand siRNA: CAGCAACCUUCACAGCCGCtt **[SEQ ID NO. 173]** |
| Antisense strand siRNA: GCGGCUGUGAAGGUUGCUGtt **[SEQ ID NO. 174]** |
| **Target sequence 10:** AACCTTCACACCCGCAG361T **[SEQ ID NO. 175]** |
| Position in gene sequence: 358 |
| GC content: 47.6% |
| Sense strand siRNA: CCUUCACAGCCGCAG361Utt **[SEQ ID NO.** 176] |
| Antisense strand siRNA: A163CUGCGGCUGUGAAGGtt **[SEQ ID NO. 177]** |
| **Target sequence 11:** AAGGGGCTGCCGCAGCAGCTG **[SEQ ID NO. 178]** |
| Position in gene sequence: 447 |
| GC content: 71.4% |
| Sense strand siRNA: GGGGCUGCCGCAGCAGCUGtt **[SEQ ID NO. 179]** |
| Antisense strand siRNA: CAGCUGCUGCGGCAGCCCCtt **[SEQ ID NO. 180]** |
| **Target sequence 12:** AAGCAGCTGCTCCGCTGAC54 **[SEQ ID NO. 181]** |
| Position in gene sequence: 545 |
| GC content: 57.1 % |
| Sense strand siRNA: GCAGCUGCUCCGCUGAC54tt **[SEQ ID NO. 182]** |
| Antisense strand siRNA: 45GUCAGCGGAGCAGCUGCtt **[SEQ ID NO. 183]** |
| **Target sequence 13:** AAAGACATCCTGAGCGAGGCC **[SEQ ID NO. 184]** |
| Position in gene sequence: 570 |
| GC content: 57.1% |
| Sense strand siRNA: AGACAUCCUGAGCGAGGCCtt **[SEQ ID NO. 185]** |
| Antisense strand siRNA: GGCCUCGCUCAGGAUGUCUtt **[SEQ ID NO. 186]** |
| **Target sequence 14:** AACTCCTTCAGCAACAGCAGC **[SEQ ID NO. 187]** |
| Position in gene sequence: 601 |
| GC content: 52.4% |
| Sense strand siRNA: CUCCUUCAGCAACAGCAGCtt **[SEQ ID NO. 188]** |
| Antisense strand siRNA: GCUGCUGUUGCUGAAGGAGtt **[SEQ ID NO. 189]** |
| **Target sequence 15:** AACAGCAGCAGGAA601GCAG **[SEQ ID NO. 190]** |
| Position in gene sequence: 613 |
| GC content: 47.6% |
| Sense strand siRNA: CAGCAGCAGGAA601GCAGtt **[SEQ ID NO. 191]** |
| Antisense strand siRNA: CUGC106UUCCUGCUGCUGtt **[SEQ ID NO. 192]** |
| **Target sequence 16:** AA601GCAGTATCCGAAGGCA **[SEQ ID NO. 193]** |
| Position in gene sequence: 625 |
| GC content: 42.9% |
| Sense strand siRNA: 601 GCAGUAUCCGAAGGCAtt **[SEQ ID NO. 194]** |
| Antisense strand siRNA: UGCCUUCGGAUACUGC106tt **[SEQ ID NO. 195]** |
| **Target sequence 17:** AAGGCAGCAGCAGCGGGAGAG **[SEQ ID NO. 196]** |
| Position in gene sequence: 640 |
| GC content: 66.7% |
| Sense strand siRNA: GGCAGCAGCAGCGGGAGAGtt **[SEQ ID NO. 197]** |
| Antisense strand siRNA: CUCUCCCGCUGCUGCUGCCtt **[SEQ ID NO. 198]** |
| **Target sequence 18:** AAGGACAATTACTTAGGGGGC **[SEQ ID NO. 199]** |
| Position in gene sequence: 696 |
| GC content: 47.6% |
| Sense strand siRNA: GGACAAUUACUUAGGGGGCtt **[SEQ ID NO. 200]** |
| Antisense strand siRNA: GCCCCCUAAGUAAUUGUCCtt **[SEQ ID NO. 201]** |
| **Target sequence 19:** AATTACTTAGGGGGCACTTCG **[SEQ ID NO. 202]** |
| Position in gene sequence: 702 |
| GC content: 47.6% |
| Sense strand siRNA: UUACUUAGGGGGCACUUCGtt **[SEQ ID NO. 203]** |
| Antisense strand siRNA: CGAAGUGCCCCCUAAGUAAtt **[SEQ ID NO. 204]** |
| **Target sequence 20:** AACGCCAAGGAGTTGTGT721 **[SEQ ID NO. 205]** |
| Position in gene sequence: 735 |
| GC content: 42.9% |
| Sense strand siRNA: CGCCAAGGAGUUGUGU721tt **[SEQ ID NO. 206]** |
| Antisense strand siRNA: 127ACACAACUCCUUGGCGtt **[SEQ ID NO. 207]** |
| **Target sequence 21:** AAGGAGTTGTGT721AAGGCA **[SEQ ID NO. 208]** |
| Position in gene sequence: 741 |
| GC content: 38,1% |
| Sense strand siRNA: GGAGUUGUGU721AAGGCAtt **[SEQ ID NO. 209]** |
| Antisense strand siRNA: UGCCUU127ACACAACUCCtt **[SEQ ID NO. 210]** |
| **Target sequence 22:** AAGGCAGTGTCGGTGTCCATG **[SEQ ID NO. 211]** |
| Position in gene sequence: 756 |
| GC content: 57.1 % |
| Sense strand siRNA: GGCAGUGUCGGUGUCCAUGtt **[SEQ ID NO. 212]** |
| Antisense strand siRNA: CAUGGACACCGACACUGCCtt **[SEQ ID NO. 213]** |
| **Target sequence 23:** AACAGCTTCGGGGGGATTGCA **[SEQ ID NO. 214]** |
| Position in gene sequence: 820 |
| GC content: 57.1% |
| Sense strand siRNA: CAGCUUCGGGGGGAUUGCAtt **[SEQ ID NO. 215]** |
| Antisense strand siRNA: UGCAAUCCCCCCGAAGCUGtt **[SEQ ID NO. 216]** |
| **Target sequence 24:** AATGCAAAGGTTCTCTGCTAG **[SEQ ID NO. 217]** |
| Position in gene sequence: 907 |
| GC content: 42.9% |
| Sense strand siRNA: UGCAAAGGUUCUCUGCUAGtt **[SEQ ID NO. 218]** |
| Antisense strand siRNA: CUAGCAGAGAACCUUUGCAtt **[SEQ ID NO. 219]** |
| **Target sequence 25:** AAAGGTTCTCTGCTAGACGAC **[SEQ ID NO. 220]** |
| Position in gene sequence: 912 |
| GC content: 47.6% |
| Sense strand siRNA: AGGUUCUCUGCUAGACGACtt **[SEQ ID NO. 221]** |
| Antisense strand siRNA: GUCGUCUAGCAGAGAACCUtt **[SEQ ID NO. 222]** |
| **Target sequence 26:** AAGAGCACTGAAGATACTGCT **[SEQ ID NO. 223]** |
| Position in gene sequence: 945 |
| GC content: 42.9% |
| Sense strand siRNA: GAGCACUGAAGAUACUGCUtt **[SEQ ID NO. 224]** |
| Antisense strand siRNA: AGCAGUAUCUUCAGUGCUCtt **[SEQ ID NO. 225]** |
| **Target sequence 27:** AAGATACTGCTGAGTATTCCC **[SEQ ID NO. 226]** |
| Position in gene sequence: 955 |
| GC content: 42.9% |
| Sense strand siRNA: GAUACUGCUGAGUAUUCCCtt **[SEQ ID NO. 227]** |
| Antisense strand siRNA: GGGAAUACUCAGCAGUAUCtt **[SEQ ID NO. 228]** |
| **Target sequence 28:** AAGGGAGGTTACACCAAAGGG **[SEQ ID NO. 229]** |
| Position in gene sequence: 981 |
| GC content: 52.4% |
| Sense strand siRNA: GGGAGGUUACACCAAAGGGtt **[SEQ ID NO. 230]** |
| Antisense strand siRNA: CCCUUUGGUGUAACCUCCCtt **[SEQ ID NO. 231]** |
| **Target sequence 29:** AAAGGGCTA961 GAAGGCGAG **[SEQ ID NO. 232]** |
| Position in gene sequence: 996 |
| GC content: 47.6% |
| Sense strand siRNA: AGGGCUA961 GAAGGCGAGtt **[SEQ ID NO. 233]** |
| Antisense strand siRNA: CUCGCCUUC169UAGCCCUtt **[SEQ ID NO. 234]** |
| **Target sequence 30:** AAGGCGAGAGCCTAGGCTGCT **[SEQ ID NO. 235]** |
| Position in gene sequence: 1009 |
| GC content: 61.9% |
| Sense strand siRNA: GGCGAGAGCCUAGGCUGCUtt **[SEQ ID NO. 236]** |
| Antisense strand siRNA: AGCAGCCUAGGCUCUCGCCtt **[SEQ ID NO. 237**] |
| **Target sequence 31:** AA1021CTGCCGTCTACCCTG **[SEQ ID NO. 238]** |
| Position in gene sequence: 1066 |
| GC content: 47.6% |
| Sense strand siRNA: 1021CUGCCGUCUACCCUGtt **[SEQ ID NO. 239]** |
| Antisense strand siRNA: CAGGGUAGACGGCAG1201tt **[SEQ ID NO. 240]** |
| **Target sequence 32:** AAGTCCGGAGCACTGGACGAG **[SEQ ID NO. 241]** |
| Position in gene sequence: 1096 |
| GC content: 61.9% |
| Sense strand siRNA: GUCCGGAGCACUGGACGAGtt **[SEQ ID NO. 242]** |
| Antisense strand siRNA: CUCGUCCAGUGCUCCGGACtt **[SEQ ID NO. 243]** |
| **Target sequence 33:** AACTTTCCACTGGCTCTGGCC **[SEQ ID NO. 244]** |
| Position in gene sequence: 1151 |
| GC content: 57.1 % |
| Sense strand siRNA: CUUUCCACUGGCUCUGGCCtt **[SEQ ID NO. 245]** |
| Antisense strand siRNA: GGCCAGAGCCAGUGGAAAGtt **[SEQ ID NO. 246]** |
| **Target sequence 34:** AAGCTGGAGAACCCGCTGGAC **[SEQ ID NO. 247]** |
| Position in gene sequence: 1221 |
| GC content: 61.9% |
| Sense strand siRNA: GCUGGAGAACCCGCUGGACtt **[SEQ ID NO. 248]** |
| Antisense strand siRNA: GUCCAGCGGGUUCUCCAGCtt **[SEQ ID NO. 249]** |
| **Target sequence 35:** AACCCGCTGGACTACGGCAGC **[SEQ ID NO. 250]** |
| Position in gene sequence: 1230 |
| GC content: 66.7% |
| Sense strand siRNA: CCCGCUGGACUACGGCAGCtt[SEQ **ID NO. 251]** |
| Antisense strand siRNA: GCUGCCGUAGUCCAGCGGGtt **[SEQ ID NO. 252]** |
| **Target sequence 36:** AAGAAGGCCAGTTGTATGGAC **[SEQ ID NO. 253]** |
| Position in gene sequence: 1396 |
| GC content: 47.6% |
| Sense strand siRNA: GAAGGCCAGUUGUAUGGACtt **[SEQ ID NO. 254]** |
| Antisense strand siRNA: GUCCAUACAACUGGCCUUCtt **[SEQ ID NO. 255]** |
| **Target sequence 37:** AAGGCCAGTTGTATGGACCGT **[SEQ ID NO. 256]** |
| Position in gene sequence: 1399 |
| GC content: 52.4% |
| Sense strand siRNA: GGCCAGUUGUAUGGACCGUtt **[SEQ ID NO. 257]** |
| Antisense strand siRNA: ACGGUCCAUACAACUGGCCtt **[SEQ ID NO. 258]** |
| **Target sequence 38:** AAAGCGACTTCACCGCACCT1 **[SEQ ID NO. 259]** |
| Position in gene sequence: 1560 |
| GC content: 52.4% |
| Sense strand siRNA: AGCGACUUCACCGCACCU1tt **[SEQ ID NO. 260]** |
| Antisense strand siRNA: 1AGGUGCGGUGAAGUCGCUtt **[SEQ ID NO. 261]** |
| **Target sequence 39:** AAAAGCGAAATGGGCCCCTGG **[SEQ ID NO. 262]** |
| Position in gene sequence: 1648 |
| GC content: 57.1 % |
| Sense strand siRNA: AAGCGAAAUGGGCCCCUGGtt **[SEQ ID NO. 263]** |
| Antisense strand siRNA: CCAGGGGCCCAUUUCGCUUtt **[SEQ ID NO. 264]** |
| **Target sequence 40:** AAGCGAAATGGGCCCCTGGAT **[SEQ ID NO. 265]** |
| Position in gene sequence: 1650 |
| GC content: 57.1% |
| Sense strand siRNA: GCGAAAUGGGCCCCUGGAUtt **[SEQ ID NO. 266]** |
| Antisense strand siRNA: AUCCAGGGGCCCAUUUCGCtt **[SEQ ID NO. 267]** |
| **Target sequence 41:** AAATGGGCCCCTGGATGGATA **[SEQ ID NO. 268]** |
| Position in gene sequence: 1655 |
| GC content: 52.4% |
| Sense strand siRNA: AUGGGCCCCUGGAUGGAUAtt **[SEQ ID NO. 269]** |
| Antisense strand siRNA: UAUCCAUCCAGGGGCCCAUtt **[SEQ ID NO. 270]** |
| **Target sequence 42:** AAGACCTGC1681CTGATCTG **[SEQ ID NO. 271]** |
| Position in gene sequence: 1763 |
| GC content: 42.9% |
| Sense strand siRNA: GACCUGC1681CUGAUCUGtt **[SEQ ID NO. 272]** |
| Antisense strand siRNA: CAGAUCAG1861GCAGGUCtt **[SEQ ID NO. 273]** |
| **Target sequence 43:** AAGCTTCTGGGTGTCACTATG **[SEQ ID NO. 274]** |
| Position in gene sequence: 1792 |
| GC content: 47.6% |
| Sense strand siRNA: GCUUCUGGGUGUCACUAUGtt **[SEQ ID NO. 275]** |
| Antisense strand siRNA: CAUAGUGACACCCAGAAGCtt **[SEQ ID NO. 276]** |
| **Target sequence 44:** AAGCTGC1741AAGGTCTTCT **[SEQ ID NO. 277]** |
| Position in gene sequence: 1829 |
| GC content: 38.1% |
| Sense strand siRNA: GCUGC1741AAGGUCUUCUtt **[SEQ ID NO. 278]** |
| Antisense strand siRNA: AGAAGACCUU147IGCAGCtt **[SEQ ID NO. 279]** |
| **Target sequence 45:** AAGGTCTTCTTCAAAAGAGCC **[SEQ ID NO. 280]** |
| Position in gene sequence: 1840 |
| GC content: 42.9% |
| Sense strand siRNA: GGUCUUCUUCAAAAGAGCCtt **[SEQ ID NO. 281]** |
| Antisense strand siRNA: GGCUCUUUUGAAGAAGACCtt **[SEQ ID NO. 282]** |
| **Target sequence 46:** AAAAGAGCCGCTGAAGGGAAA **[SEQ ID NO. 283]** |
| Position in gene sequence: 1852 |
| GC content: 47.6% |
| Sense strand siRNA: AAGAGCCGCUGAAGGGAAAtt **[SEQ ID NO. 284**] |
| Antisense strand siRNA: UUUCCCUUCAGCGGCUCUUtt **[SEQ ID NO. 285]** |
| **Target sequence 47:** AAGAGCCGCTGAAGGGAAACA **[SEQ ID NO. 286]** |
| Position in gene sequence: 1854 |
| GC content: 52.4% |
| Sense strand siRNA: GAGCCGCUGAAGGGAAACAtt **[SEQ ID NO. 287]** Antisense |
| strand siRNA: UGUUUCCCUUCAGCGGCUCtt **[SEQ ID NO. 288]** |
| **Target sequence 48:** AAGGGAAACAGAAGTACCTGT **[SEQ ID NO. 289]** |
| Position in gene sequence: 1865 |
| GC content: 42.9% |
| Sense strand siRNA: GGGAAACAGAAGUACCUGUtt **[SEQ ID NO. 290]** |
| Antisense strand siRNA: ACAGGUACUUCUGUUUCCCtt **[SEQ ID NO. 291]** |
| **Target sequence 49:** AAACAGAAGTACCTGTGCGCC **[SEQ ID NO. 292]** |
| Position in gene sequence: 1870 |
| GC content: 52.4% |
| Sense strand siRNA: ACAGAAGUACCUGUGCGCCtt **[SEQ ID NO. 293]** |
| Antisense strand siRNA: GGCGCACAGGUACUUCUGUtt **[SEQ ID NO. 294]** |
| **Target sequence 50:** AAGTACCTGTGCGCCAGCAGA **[SEQ ID NO. 295]** |
| Position in gene sequence: 1876 |
| GC content: 57.1% |
| Sense strand siRNA: GUACCUGUGCGCCAGCAGAtt **[SEQ ID NO. 296]** |
| Antisense strand siRNA: UCUGCUGGCGCACAGGUACtt **[SEQ ID NO. 297]** |
| **Target sequence 51:** AAAT1801GATTGCACTATTG **[SEQ ID NO. 298]** |
| Position in gene sequence: 1896 |
| GC content: 23.8% |
| Sense strand siRNA: AU1801GAUUGCACUAUUGtt **[SEQ ID NO. 299]** |
| Antisense strand siRNA: CAAUAGUGCAAUC1081Autt **[SEQ ID NO. 300]** |
| **Target sequence 52:** AAATTCCGAAGGAAAAATTGT **[SEQ ID NO. 301]** |
| Position in gene sequence: 1919 |
| GC content: 28.6% |
| Sense strand siRNA: AUUCCGAAGGAAAAAUUGUtt **[SEQ ID NO. 302]** |
| Antisense strand siRNA: ACAAUUUUUCCUUCGGAAUtt **[SEQ ID NO. 303]** |
| **Target sequence 53:** AAGGAAAAATTGTCCATCTTG **[SEQ ID NO. 304]** |
| Position in gene sequence: 1927 |
| GC content: 33.3% |
| Sense strand siRNA: GGAAAAAUUGUCCAUCUUGtt **[SEQ ID NO. 305]** |
| Antisense strand siRNA: CAAGAUGGACAAUUUUUCCtt **[SEQ ID NO. 306]** |
| **Target sequence 54:** AAAAATTGTCCATCTTGTCGT **[SEQ ID NO. 307]** |
| Position in gene sequence: 1931 |
| GC content: 33.3% |
| Sense strand siRNA: AAAUUGUCCAUCUUGUCGUtt **[SEQ ID NO. 308]** |
| Antisense strand siRNA: ACGACAAGAUGGACAAUUUtt **[SEQ ID NO. 309]** |
| **Target sequence 55:** AAATTGTCCATCTTGTCGTCT **[SEQ ID NO. 310]** |
| Position in gene sequence: 1933 |
| GC content: 38.1% |
| Sense strand siRNA: AUUGUCCAUCUUGUCGUCUtt **[SEQ ID NO. 311]** |
| Antisense strand siRNA: AGACGACAAGAUGGACAAUtt [SEQ ID NO. 312] |
| **Target sequence 56:** AAATGT1861TATGAAGCAGG **[SEQ ID NO. 313]** |
| Position in gene sequence: 1958 |
| GC content: 28.6% |
| Sense strand siRNA: AUGU1861UAUGAAGCAGGtt **[SEQ ID NO. 314]** |
| Antisense strand siRNA: CCUGCUUCAUA1681ACAUtt **[SEQ ID NO. 315]** |
| **Target sequence 57:** AAGCAGGGATGACTCTGGGAG **[SEQ ID NO. 316]** |
| Position in gene sequence: 1972 |
| GC content: 57.1 % |
| Sense strand siRNA: GCAGGGAUGACUCUGGGAGtt **[SEQ ID NO. 317]** |
| Antisense strand siRNA: CUCCCAGAGUCAUCCCUGCtt **[SEQ ID NO. 318]** |
| **Target sequence 58:** AAGCTGAAGAAACTTGGTAAT **[SEQ ID NO. 319]** |
| Position in gene sequence: 1998 |
| GC content: 33.3% |
| Sense strand siRNA: GCUGAAGAAACUUGGUAAUtt **[SEQ ID NO. 320]** |
| Antisense strand siRNA: AUUACCAAGUUUCUUCAGCtt **[SEQ ID NO. 321]** |
| **Target sequence 59:** AAGAAACTTGGTAATCTGAAA **[SEQ ID NO. 322]** |
| Position in gene sequence: 2004 |
| GC content: 28.6% |
| Sense strand siRNA: GAAACUUGGUAAUCUGAAAtt **[SEQ ID NO. 323]** |
| Antisense strand siRNA: UUUCAGAUUACCAAGUUUCtt **[SEQ ID NO. 324]** |
| **Target sequence 60:** AAACTTGGTAATCTGAAACTA **[SEQ ID NO. 325]** |
| Position in gene sequence: 2007 |
| GC content: 28.6% |
| Sense strand siRNA: ACUUGGUAAUCUGAAACUAtt **[SEQ ID NO. 326]** |
| Antisense strand siRNA: UAGUUUCAGAUUACCAAGUtt **[SEQ ID NO. 327]** |
| **Target sequence 61:** AATCTGAAACTA192ICAGGA **[SEQ ID NO. 328]** |
| Position in gene sequence: 2016 |
| GC content: 28.6% |
| Sense strand siRNA: UCUGAAACUA1921CAGGAtt **[SEQ ID NO. 329]** |
| Antisense strand siRNA: UCCUG1291UAGUUUCAGAtt **[SEQ ID NO. 330]** |
| **Target sequence 62:** AAACTA1921CAGGAGGAAGG **[SEQ ID NO. 331]** |
| Position in gene sequence: 2022 |
| GC content: 38,1% |
| Sense strand siRNA: ACUA1921CAGGAGGAAGGtt **[SEQ ID NO. 332]** |
| Antisense strand siRNA: CCUUCCUCCUG1291UAGUtt **[SEQ ID NO. 333]** |
| **Target sequence 63:** AAGGAGAGGCTTCCAGCACCA **[SEQ ID NO. 334]** |
| Position in gene sequence: 2039 |
| GC content: 57.1% |
| Sense strand siRNA: GGAGAGGCUUCCAGCACCAtt **[SEQ ID NO. 335]** |
| Antisense strand siRNA: UGGUGCUGGAAGCCUCUCCtt **[SEQ ID NO. 336]** |
| **Target sequence 64:** AACCCAGAAGCTG1981ACAG **[SEQ ID NO. 337]** |
| Position in gene sequence: 2079 |
| GC content: 42.9% |
| Sense strand siRNA: CCCAGAAGCUG1981ACAGtt **[SEQ ID NO. 338]** |
| Antisense strand siRNA: CUGU1891CAGCUUCUGGGtt **[SEQ ID NO. 339]** |
| **Target sequence 65:** AAGCTG1981ACAGTGTCACA **[SEQ ID NO. 340]** |
| Position in gene sequence: 2086 |
| GC content: 38.1% |
| Sense strand siRNA: GCUG1981ACAGUGUCACAtt **[SEQ ID NO. 341]** |
| Antisense strand siRNA: UGUGACACUGU1891CAGCtt **[SEQ ID NO. 342]** |
| **Target sequence 66:** AAGGCTATGAATGTCAGCCCA [**SEQ ID NO. 343]** |
| Position in gene sequence: 2112 |
| GC content: 47.6% |
| Sense strand siRNA: GGCUAUGAAUGUCAGCCCAtt **[SEQ ID NO. 344]** |
| Antisense strand siRNA: UGGGCUGACAUUCAUAGCCtt **[SEQ ID NO. 345]** |
| **Target sequence 67:** AATGTCAGCCCATCTTTCTGA **[SEQ ID NO. 346]** |
| Position in gene sequence: 2121 |
| GC content: 42.9% |
| Sense strand siRNA: UGUCAGCCCAUCUUUCUGAtt **[SEQ ID NO. 347]** |
| Antisense strand siRNA: UCAGAAAGAUGGGCUGACAtt **[SEQ ID NO. 348]** |
| **Target sequence 68:** AATGTCCTGGAAGCC2041AT **[SEQ ID NO. 349]** |
| Position in gene sequence: 2141 |
| GC content: 38.1% |
| Sense strand siRNA: UGUCCUGGAAGCC2041Autt **[SEQ ID NO. 350]** |
| Antisense strand siRNA: AU1402GGCUUCCAGGACAtt **[SEQ ID NO. 351]** |
| **Target sequence 69:** AAGCC2041ATTGAGCCAGGT **[SEQ ID NO. 352]** |
| Position in gene sequence: 2151 |
| GC content: 42.9% |
| Sense strand siRNA: GCC2041AUUGAGCCAGGUtt **[SEQ ID NO. 353]** |
| Antisense strand siRNA: ACCUGGCUCAAU1402GGCtt **[SEQ ID NO. 354]** |
| **Target sequence 70:** AACAACCAGCCCGACTCCTTT **[SEQ ID NO. 355]** |
| Position in gene sequence: 2193 |
| GC content: 52.4% |
| Sense strand siRNA: CAACCAGCCCGACUCCUUUtt **[SEQ ID NO. 356]** |
| Antisense strand siRNA: AAAGGAGUCGGGCUGGUUGtt **[SEQ ID NO. 357]** |
| **Target sequence 71:** AACCAGCCCGACTCCTTTGCA **[SEQ ID NO. 358]** |
| Position in gene sequence: 2196 |
| GC content: 57.1% |
| Sense strand siRNA: CCAGCCCGACUCCUUUGCAtt **[SEQ ID NO. 359]** |
| Antisense strand siRNA: UGCAAAGGAGUCGGGCUGGtt **[SEQ ID NO. 360]** |
| **Target sequence 72:** AATGAACTGGGAGAGAGACAG **[SEQ ID NO. 361]** |
| Position in gene sequence: 2239 |
| GC content: 47.6% |
| Sense strand siRNA: UGAACUGGGAGAGAGACAGtt **[SEQ ID NO. 362]** |
| Antisense strand siRNA; CUGUCUCUCUCCCAGUUCAtt **[SEQ ID NO. 363]** |
| **Target sequence 73:** AACTGGGAGAGAGACAGCTTG **[SEQ ID NO. 364]** |
| Position in gene sequence: 2243 |
| GC content: 52.4% |
| Sense strand siRNA: CUGGGAGAGAGACAGCUUGtt **[SEQ ID NO. 365]** |
| Antisense strand siRNA: CAAGCUGUCUCUCUCCCAGtt **[SEQ ID NO. 366]** |
| **Target sequence 74:** AAGTGGGCC2161AAGGCCTT **[SEQ ID NO. 367]** |
| Position in gene sequence: 2275 |
| GC content: 47.6% |
| Sense strand siRNA: GUGGGCC2161AAGGCCUUtt **[SEQ ID NO. 368]** |
| Antisense strand siRNA: AAGGCCUUI612GGCCCACtt **[SEQ ID NO. 369]** |
| **Target sequence 75:** AAGGCCTTGCCTGGCTTCCGC **[SEQ ID NO. 370]** |
| Position in gene sequence: 2288 |
| GC content: 66.7% |
| Sense strand siRNA: GGCCUUGCCUGGCUUCCGCtt **[SEQ ID NO. 371]** |
| Antisense strand siRNA: GCGGAAGCCAGGCAAGGCCtt **[SEQ ID NO. 372]** |
| **Target sequence 76:** AACTTACACGTGGACGACCAG **[SEQ ID NO. 373]** |
| Position in gene sequence: 2309 |
| GC content: 52.4% |
| Sense strand siRNA: CUUACACGUGGACGACCAGtt **[SEQ ID NO. 374]** |
| Antisense strand siRNA: CUGGUCGUCCACGUGUAAGtt **[SEQ ID NO. 375]** |
| **Target sequence 77:** AATGTCAACTCC2281AGGAT **[SEQ ID NO. 376]** |
| Position in gene sequence: 2400 |
| GC content: 33.3% |
| Sense strand siRNA: UGUCAACUCC2281AGGAUtt **[SEQ ID NO. 377]** |
| Antisense strand siRNA: AUCCU1822GGAGUUGACAtt **[SEQ ID NO. 378]** |
| **Target sequence 78:** AACTCC2281AGGATGCTCTA **[SEQ ID NO. 379]** |
| Position in gene sequence: 2406 |
| GC content: 38.1% |
| Sense strand siRNA: CUCC2281AGGAUGCUCUAtt **[SEQ ID NO. 380]** |
| Antisense strand siRNA: UAGAGCAUCCU1822GGAGtt **[SEQ ID NO. 381]** |
| **Target sequence 79:** AATGAGTACCGCATGCACAAG **[SEQ ID NO. 382]** |
| Position in gene sequence: 2449 |
| GC content: 47.6% |
| Sense strand siRNA: UGAGUACCGCAUGCACAAGtt **[SEQ ID NO. 383]** |
| Antisense strand siRNA: CUUGUGCAUGCGGUACUCAtt **[SEQ ID NO. 384]** |
| **Target sequence 80:** AAGTCCCGG2341ATGTACAG [SEQ **ID NO. 385]** |
| Position in gene sequence: 2467 |
| GC content: 42.9% |
| Sense strand siRNA: GUCCCGG2341AUGUACAGtt **[SEQ ID NO. 386]** |
| Antisense strand siRNA: CUGUACAU1432CCGGGACtt **[SEQ ID NO. 387]** |
| **Target sequence 81:** AATGAGGCACCTCTCTCAAGA **[SEQ ID NO. 388]** |
| Position in gene sequence: 2500 |
| GC content: 47.6% |
| Sense strand siRNA: UGAGGCACCUCUCUCAAGAtt [SEQ **ID NO. 389]** |
| Antisense strand siRNA: UCUUGAGAGAGGUGCCUCAtt **[SEQ ID NO. 390]** |
| **Target sequence 82:** AAGAGTTTGGATGGCTCCAAA **[SEQ ID NO. 391]** |
| Position in gene sequence: 2517 |
| GC content: 42.9% |
| Sense strand siRNA: GAGUUUGGAUGGCUCCAAAtt **[SEQ ID NO. 392]** |
| Antisense strand siRNA: UUUGGAGCCAUCCAAACUCtt **[SEQ ID NO. 393]** |
| **Target sequence 83:** AAATC2401ACCCCCCAGGAA **[SEQ ID NO. 394]** |
| Position in gene sequence: 2535 |
| GC content: 42.9% |
| Sense strand siRNA: AUC2401ACCCCCCAGGAAtt **[SEQ ID NO. 395]** |
| Antisense strand siRNA: UUCCUGGGGGGU1042GAUtt **[SEQ ID NO. 396]** |
| **Target sequence 84:** AATTCCTGTGCATGAAAGCAC **[SEQ ID NO. 397]** |
| Position in gene sequence: 2554 |
| GC content: 42.9% |
| Sense strand siRNA: UUCCUGUGCAUGAAAGCACtt **[SEQ ID NO. 398]** |
| Antisense strand siRNA: GUGCUUUCAUGCACAGGAAtt **[SEQ ID NO. 399]** |
| **Target sequence 85:** AAAGCACTGCTACTCTTCAGC **[SEQ ID NO. 400]** |
| Position in gene sequence: 2568 |
| GC content: 47.6% |
| Sense strand siRNA: AGCACUGCUACUCUUCAGCtt **[SEQ ID NO. 401]** |
| Antisense strand siRNA: GCUGAAGAGUAGCAGUGCUtt **[SEQ ID NO. 402]** |
| **Target sequence 86:** AAAAATCAAAAATTCTTTGAT **[SEQ ID NO. 403]** |
| Position in gene sequence: 2614 |
| GC content: 14.3% |
| Sense strand siRNA: AAAUCAAAAAUUCUUUGAUtt **[SEQ ID NO. 404]** |
| Antisense strand siRNA: AUCAAAGAAUUUUUGAUUUtt **[SEQ ID NO. 405]** |
| **Target sequence 87:** AAATCAAAAATTCTTTGATGA **[SEQ ID NO. 406]** |
| Position in gene sequence: 2616 |
| GC content: 19% |
| Sense strand siRNA: AUCAAAAAUUCUUUGAUGAtt **[SEQ ID NO. 407]** |
| Antisense strand siRNA: UCAUCAAAGAAUUUUUGAUtt **[SEQ ID NO. 408]** |
| **Target sequence 88:** AAAAATTCTTTGATGAACTTC **[SEQ ID NO. 409]** |
| Position in gene sequence: 2621 |
| GC content: 23.8% |
| Sense strand siRNA: AAAUUCUUUGAUGAACUUCtt **[SEQ ID NO. 410]** |
| Antisense strand siRNA: GAAGUUCAUCAAAGAAUUUtt **[SEQ ID NO. 411]** |
| **Target sequence 89:** AAATTCTTTGATGAACTTCGA **[SEQ ID NO. 412]** |
| Position in gene sequence: 2623 |
| GC content: 28.6% |
| Sense strand siRNA: AUUCUUUGAUGAACUUCGAtt **[SEQ ID NO. 413]** |
| Antisense strand siRNA: UCGAAGUUCAUCAAAGAAUtt **[SEQ ID NO. 414]** |
| **Target sequence 90:** AACTTCGAATGAACTACATCA **[SEQ ID NO. 415]** |
| Position in gene sequence: 2636 |
| GC content: 33.3% |
| Sense strand siRNA: CUUCGAAUGAACUACAUCAtt **[SEQ ID NO. 416]** |
| Antisense strand siRNA: UGAUGUAGUUCAUUCGAAGtt **[SEQ ID NO. 417]** |
| **Target sequence 91:** AATGAACTACATCAAGGAACT **[SEQ ID NO. 418]** |
| Position in gene sequence: 2643 |
| GC content: 33.3% |
| Sense strand siRNA: UGAACUACAUCAAGGAACUtt **[SEQ ID NO. 419]** |
| Antisense strand siRNA: AGUUCCUUGAUGUAGUUCAtt **[SEQ ID NO. 420]** |
| **Target sequence 92:** AACTACATCAAGGAACTCGAT **[SEQ ID NO. 421]** |
| Position in gene sequence: 2647 |
| GC content: 38.1% |
| Sense strand siRNA: CUACAUCAAGGAACUCGAUtt **[SEQ ID NO. 422]** |
| Antisense strand siRNA: AUCGAGUUCCUUGAUGUAGtt **[SEQ ID NO. 423]** |
| **Target sequence 93:** AAGGAACTCGAT2521CGTAT **[SEQ ID NO. 424]** |
| Position in gene sequence: 2656 |
| GC content: 33.3% |
| Sense strand siRNA: GGAACUCGAU2521 CGUAUtt **[SEQ ID NO. 425]** |
| Antisense strand siRNA: AUACG1252AUCGAGUUCCtt **[SEQ ID NO. 426]** |
| **Target sequence 94:** AACTCGAT2521CGTATCATT **[SEQ ID NO. 427]** |
| Position in gene sequence: 2660 |
| GC content: 28.6% |
| Sense strand siRNA: CUCGAU2521 CGUAUCAUUtt **[SEQ ID NO. 428]** |
| Antisense strand siRNA: AAUGAUACG1252AUCGAGtt **[SEQ ID NO. 429]** |
| **Target sequence 95:** AAAAGAAAAAATCCCACATCC **[SEQ ID NO. 430]** |
| Position in gene sequence: 2687 |
| GC content: 33.3% |
| Sense strand siRNA: AAGAAAAAAUCCCACAUCCtt **[SEQ ID NO. 431]** |
| Antisense strand siRNA: GGAUGUGGGAUUUUUUCUUtt **[SEQ ID NO. 432]** |
| **Target sequence 96:** AAGAAAAAATCCCACATCCTG **[SEQ ID NO. 433]** |
| Position in gene sequence: 2689 |
| GC content: 38.1% |
| Sense strand siRNA: GAAAAAAUCCCACAUCCUGtt **[SEQ ID NO. 434]** |
| Antisense strand siRNA: CAGGAUGUGGGAUUUUUUCtt **[SEQ ID NO. 435]** |
| **Target sequence 97:** AAAAAATCCCACATCCTGCTC **[SEQ ID NO. 436]** |
| Position in gene sequence: 2692 |
| GC content: 42.9% |
| Sense strand siRNA: AAAAUCCCACAUCCUGCUCtt **[SEQ ID NO. 437]** |
| Antisense strand siRNA: GAGCAGGAUGUGGGAUUUUtt **[SEQ ID NO. 438]** |
| **Target sequence 98:** AAAATCCCACATCCTGCTCAA **[SEQ ID NO. 439]** |
| Position in gene sequence: 2694 |
| GC content: 42.9% |
| Sense strand siRNA: AAUCCCACAUCCUGCUCAAtt **[SEQ ID NO. 440]** |
| Antisense strand siRNA: UUGAGCAGGAUGUGGGAUUtt **[SEQ ID NO. 441]** |
| **Target sequence 99:** AATCCCACATCCTGCTCAAGA **[SEQ ID NO. 442]** |
| Position in gene sequence: 2696 |
| GC content: 47.6% |
| Sense strand siRNA: UCCCACAUCCUGCUCAAGAtt **[SEQ ID NO. 443]** |
| Antisense strand siRNA: UCUUGAGCAGGAUGUGGGAtt **[SEQ ID NO. 444]** |
| **Target sequence 100:** AAGACGCTTCTACCAGCTC25 **[SEQ ID NO. 445]** |
| Position in gene sequence: 2713 |
| GC content: 47.6% |
| Sense strand siRNA: GACGCUUCUACCAGCUC25tt **[SEQ ID NO. 446]** |
| Antisense strand siRNA: 52GAGCUGGUAGAAGCGUCtt **[SEQ ID NO. 447]** |
| **Target sequence 101:** AAGCTCCTGGACTCCGTGCAG **[SEQ ID NO. 448]** |
| Position in gene sequence: 2739 |
| GC content: 61.9% |
| Sense strand siRNA: GCUCCUGGACUCCGUGCAGtt **[SEQ ID NO. 449]** |
| Antisense strand siRNA: CUGCACGGAGUCCAGGAGCtt **[SEQ ID NO. 450]** |
| **Target sequence 102:** AATCAAGTCACACATGGTGAG **[SEQ ID NO. 451]** |
| Position in gene sequence: 2805 |
| GC content: 42.9% |
| Sense strand siRNA: UCAAGUCACACAUGGUGAGtt **[SEQ ID NO. 452]** |
| Antisense strand siRNA: CUCACCAUGUGUGACUUGAtt **[SEQ ID NO. 453]** |
| **Target sequence 103:** AAGTCACACATGGTGAGCGTG **[SEQ ID NO. 454]** |
| Position in gene sequence: 2809 |
| GC content: 52.4% |
| Sense strand siRNA: GUCACACAUGGUGAGCGUGtt **[SEQ ID NO. 455]** |
| Antisense strand siRNA: CACGCUCACCAUGUGUGACtt **[SEQ ID NO. 456]** |
| **Target sequence 104:** AAATGATGGCAGAGATCATC2 **[SEQ ID NO. 457]** |
| Position in gene sequence: 2840 |
| GC content: 38.1% |
| Sense strand siRNA: AUGAUGGCAGAGAUCAUC2tt **[SEQ ID NO. 458]** |
| Antisense strand siRNA: 2GAUGAUCUCUGCCAUCAUtt **[SEQ ID NO. 459]** |
| **Target sequence 105:** AAGTGCCCAAGATCCTTTCTG **[SEQ ID NO. 460]** |
| Position in gene sequence: 2871 |
| GC content: 47.6% |
| Sense strand siRNA: GUGCCCAAGAUCCUUUCUGtt **[SEQ ID NO. 461]** |
| Antisense strand siRNA: CAGAAAGGAUCUUGGGCACtt **[SEQ ID NO. 462]** |
| **Target sequence 106:** AAGATCCTTTCTGGGAAAGTC **[SEQ ID NO. 463]** |
| Position in gene sequence: 2879 |
| GC content: 42.9% |
| Sense strand siRNA: GAUCCUUUCUGGGAAAGUCtt **[SEQ ID NO. 464]** |
| Antisense strand siRNA: GACUUUCCCAGAAAGGAUCtt **[SEQ ID NO. 465]** |
| **Target sequence 107:** AAAGTCAAGCCCATCTATTTC **[SEQ ID NO. 466]** |
| Position in gene sequence: 2894 |
| GC content: 38.1% |
| Sense strand siRNA: AGUCAAGCCCAUCUAUUUCtt **[SEQ ID NO. 467]** |
| Antisense strand siRNA: GAAAUAGAUGGGCUUGACUtt **[SEQ ID NO. 468]** |
| **Target sequence 108:** AAGCCCATCTATTTCCACACC **[SEQ ID NO. 469]** |
| Position in gene sequence: 2900 |
| GC content: 47.6% |
| Sense strand siRNA: GCCCAUCUAUUUCCACACCtt **[SEQ ID NO. 470]** |
| Antisense strand siRNA: GGUGUGGAAAUAGAUGGGCtt **[SEQ ID NO. 471]** |

| **Table 4: Aromatase** |
|---|
| **Aromatase - P11511 (gi: 117293) [SEQ ID NO. 472]** |
| 1 atggttttgg aaatgctgaa cccgatacat tataacatca ccagcatcgt gcctgaagcc 61 atgcctgctg ccaccatgcc agtcctgctc ctcactggcc tttttctctt ggtgtggaat 121 tatgagggca catcctcaat accaggtcct ggctactgca tgggaattgg acccctcatc 181 tcccacggca gattcctgtg gatggggatc ggcagtgcct gcaactacta caaccgggta 241 tatggagaat tcatgcgagt ctggatctct ggagaggaaa cactcattat cagcaagtcc 301 tcaagtatgt tccacataat gaagcacaat cattacagct ctcgattcgg cagcaaactt 361 gggctgcagt gcatcggtat gcatgagaaa ggcatcatat ttaacaacaa tccagagctc 421 tggaaaacaa ctcgaccctt ctttatgaaa gctctgtcag gccccggcct tgttcgtatg 481 gtcacagtct gtgctgaatc cctcaaaaca catctggaca ggttggagga ggtgaccaat 541 gaatcgggct atgtggacgt gttgaccctt ctgcgtcgtg tcatgctgga cacctctaac 601 acgctcttct tgaggatccc tttggacgaa agtgctatcg tggttaaaat ccaaggttat 661 tttgatgcat ggcaagctct cctcatcaaa ccagacatct tctttaagat ttcttggcta 721 tacaaaaagt atgagaagtc tgtcaaggat ttgaaagatg ccatagaagt tctgatagca 781 gaaaaaagac gcaggatttc cacagaagag aaactggaag aatgtatgga ctttgccact 841 gagttgattt tagcagagaa acgtggtgac ctgacaagag agaatgtgaa ccagtgcata 901 ttggaaatgc tgatcgcagc tcctgacacc atgtctgtct ctttgttctt catgctattt 961 ctcattgcaa agcaccctaa tgttgaagag gcaataataa aggaaatcca gactgttatt 1021 ggtgagagag acataaagat tgatgatata caaaaattaa aagtgatgga aaacttcatt 1081 tatgagagca tgcggtacca gcctgtcgtg gacttggtca tgcgcaaagc cttagaagat 1141 gatgtaatcg atggctaccc agtgaaaaag gggacaaaca ttatcctgaa tattggaagg 1201 atgcacagac tcgagttttt ccccaaaccc aatgaattta ctcttgaaaa ttttgcaaag 1261 aatgttcctt ataggtactt tcagccattt ggctttgggc cccgtggctg tgcaggaaag 1321 tacatcgcca tggtgatgat gaaagccatc ctcgttacac ttctgagacg attccacgtg 1381 aagacattgc aaggacagtg tgttgagagc atacagaaga tacacgactt gtccttgcac 1441 ccagatgaga ctaaaaacat gctggaaatg atctttaccc caagaagctc agacaggtgt 1501 ctggaacact ag |
| **Target sequence 1:** AAATGCTGAACCCGATACATT **[SEQ ID NO. 473]** |
| Position in gene sequence: 12 |
| GC content: 38.1% |
| Sense strand siRNA: AUGCUGAACCCGAUACAUUtt **[SEQ ID NO. 474]** |
| Antisense strand siRNA: AAUGUAUCGGGUUCAGCAUtt **[SEQ ID NO. 475]** |
| **Target sequence 2:** AACCCGATACATTATAACATC **[SEQ ID NO. 476]** |
| Position in gene sequence: 20 |
| GC content: 33.3% |
| Sense strand siRNA: CCCGAUACAUUAUAACAUCtt **[SEQ ID NO. 477]** |
| Antisense strand siRNA: GAUGUUAUAAUGUAUCGGGtt **[SEQ ID NO. 478]** |
| **Target sequence 3:** AACATCACCAGCATCGTGCCT **[SEQ ID NO. 479]** |
| Position in gene sequence: 35 |
| GC content: 52.4% |
| |
| Antisense strand siRNA: AGGCACGAUGCUGGUGAUGtt **[SEQ ID NO. 481]** |
| **Target sequence 4:** AAGCC61ATGCCTGCTGCCAC **[SEQ ID NO. 482]** |
| Position in gene sequence: 57 |
| GC content: 57.1% |
| Sense strand siRNA: GCC61AUGCCUGCUGCCACtt **[SEQ ID NO. 483]** |
| Antisense strand siRNA: GUGGCAGCAGGCAU16GGCtt **[SEQ ID NO. 484]** |
| **Target sequence 5:** AAT121TATGAGGGCACATCC **[SEQ ID NO. 485]** |
| Position in gene sequence: 121 |
| GC content: 38.1% |
| Sense strand siRNA: U121UAUGAGGGCACAUCCtt **[SEQ ID NO. 486]** |
| Antisense strand siRNA: GGAUGUGCCCUCAUA121Att **[SEQ ID NO. 487]** |
| **Target sequence 6:** AATACCAGGTCCTGGCTACTG **[SEQ ID NO. 488]** |
| Position in gene sequence: 144 |
| GC content: 52.4% |
| Sense strand siRNA: UACCAGGUCCUGGCUACUGtt **[SEQ ID NO. 489]** |
| Antisense strand siRNA: CAGUAGCCAGGACCUGGUAtt **[SEQ ID NO. 490]** |
| **Target sequence 7:** AATTGGACCCCTCATC181TC **[SEQ ID NO. 491]** |
| Position in gene sequence: 171 |
| GC content: 42.9% |
| Sense strand siRNA: UUGGACCCCUCAUC181Uctt [SEQ ID NO. 492] |
| Antisense strand siRNA: GA181GAUGAGGGGUCCAAtt [SEQ ID NO. 493] |
| **Target sequence 8:** AACTACTACAACCGGGTA241 [SEQ ID NO. 494] |
| Position in gene sequence: 232 |
| GC content: 38.1% |
| Sense strand siRNA: CUACUACAACCGGGUA241tt [SEQ ID NO. 495] |
| Antisense strand siRNA: 142UACCCGGUUGUAGUAGtt [SEQ ID NO. 496] |
| **Target sequence 9:** AACCGGGTA241 TATGGAGAA [SEQ ID NO. 497] |
| Position in gene sequence: 241 |
| GC content: 38.1 % |
| Sense strand siRNA: CCGGGUA241UAUGGAGAAtt [SEQ ID NO. 498] |
| Antisense strand siRNA: UUCUCCAUA142UACCCGGtt [SEQ ID NO. 499] |
| **Target sequence 10:** AATTCATGCGAGTCTGGATCT **[SEQ ID NO. 500]** |
| Position in gene sequence: 260 |
| GC content: 42.9% |
| Sense strand siRNA: UUCAUGCGAGUCUGGAUCUtt **[SEQ ID NO. 501]** |
| Antisense strand siRNA: AGAUCCAGACUCGCAUGAAtt **[SEQ ID NO. 502]** |
| **Target sequence 11:** AAACACTCATTATCAGCAAGT **[SEQ ID NO. 503]** |
| Position in gene sequence: 290 |
| GC content: 33.3% |
| Sense strand siRNA: ACACUCAUUAUCAGCAAGUtt **[SEQ ID NO. 504]** |
| Antisense strand siRNA: ACUUGCUGAUAAUGAGUGUtt **[SEQ ID NO. 505]** |
| **Target sequence 12:** AAGTCC301TCAAGTATGTTC **[SEQ ID NO. 506]** |
| Position in gene sequence: 307 |
| GC content: 33.3% |
| Sense strand siRNA: GUCC301UCAAGUAUGUUCtt **[SEQ ID NO. 507]** |
| Antisense strand siRNA: GAACAUACUUGA103GGACtt **[SEQ ID NO. 508]** |
| **Target sequence 13:** AAGTATGTTCCACATAATGAA **[SEQ ID NO. 509]** |
| Position in gene sequence: 318 |
| GC content: 28.6% |
| Sense strand siRNA: GUAUGUUCCACAUAAUGAAtt **[SEQ ID NO. 510]** |
| Antisense strand siRNA: UUCAUUAUGUGGAACAUACtt **[SEQ ID NO. 511]** |
| **Target sequence 14:** AATGAAGCACAATCATTACAG **[SEQ ID NO. 512]** |
| Position in gene sequence: 333 |
| GC content: 33.3% |
| Sense strand siRNA: UGAAGCACAAUCAUUACAGtt **[SEQ ID NO. 513]** |
| Antisense strand siRNA: CUGUAAUGAUUGUGCUUCAtt **[SEQ ID NO. 514]** |
| **Target sequence 15:** AAGCACAATCATTACAGCTCT **[SEQ ID NO. 515]** |
| Position in gene sequence: 337 |
| GC content: 38.1% |
| Sense strand siRNA: GCACAAUCAUUACAGCUCUtt **[SEQ ID NO. 516]** |
| Antisense strand siRNA: AGAGCUGUAAUGAUUGUGCtt **[SEQ ID NO. 517]** |
| **Target sequence 16:** AATCATTACAGCTCTCGATTC **[SEQ ID NO. 518]** |
| Position in gene sequence: 343 |
| GC content: 38.1% |
| Sense strand siRNA: UCAUUACAGCUCUCGAUUCtt **[SEQ ID NO. 519]** |
| Antisense strand siRNA: GAAUCGAGAGCUGUAAUGAtt **[SEQ ID NO. 520]** |
| **Target sequence 17:** AAACTT361 GGGCTGCAGTGC **[SEQ ID NO. 521]** |
| Position in gene sequence: 370 |
| GC content: 47.6% |
| Sense strand siRNA: ACUU361GGGCUGCAGUGCtt **[SEQ ID NO. 522]** |
| Antisense strand siRNA: GCACUGGAGCCC163AAGUtt **[SEQ ID NO. 523]** |
| **Target sequence 18:** AAAGGCATCATATTTAACAAC **[SEQ ID NO. 524]** |
| Position in gene sequence: 406 |
| GC content: 28.6% |
| Sense strand siRNA: AGGCAUGAUAUUUAACAACtt **[SEQ ID NO. 525]** |
| Antisense strand siRNA: GUUGUUAAAUAUGAUGCCUtt **[SEQ ID NO. 526]** |
| **Target sequence 19:** AACAACAATCCAGAGCTC421 **[SEQ ID NO. 527]** |
| Position in gene sequence: 421 |
| GC content: 38.1% |
| Sense strand siRNA: CAACAAUCCAGAGCUC421tt **[SEQ ID NO. 528]** |
| Antisense strand siRNA: 124GAGCUCUGGAUUGUUGtt **[SEQ ID NO. 529]** |
| **Target sequence 20:** AACAATCCAGAGCTC421TGG **[SEQ ID NO. 530]** |
| Position in gene sequence: 424 |
| GC content: 42.9% |
| Sense strand siRNA: CAAUCCAGAGCUC421UGGtt **[SEQ ID NO. 531]** |
| Antisense strand siRNA: CCA124GAGCUCUGGAUUGtt **[SEQ ID NO. 532]** |
| **Target sequence 21:** AATCCAGAGCTC421TGGAAA **[SEQ ID NO. 533]** |
| Position in gene sequence: 427 |
| GC content: 38.1% |
| Sense strand siRNA: UCCAGAGCUC421UGGAAAtt **[SEQ ID NO. 534**] |
| Antisense strand siRNA: UUUCCA124GAGCUCUGGAtt **[SEQ ID NO. 535]** |
| **Target sequence 22:** AAAACAACTCGACCCTTCTTT **[SEQ ID NO. 536]** |
| Position in gene sequence: 445 |
| GC content: 38.1% |
| Sense strand siRNA: AACAACUCGACCCUUCUUUtt **[SEQ ID NO. 537]** |
| Antisense strand siRNA: AAAGAAGGGUCGAGUUGUUtt **[SEQ ID NO. 538]** |
| **Target sequence 23:** AACAACTCGACCCTTCTTTAT **[SEQ ID NO. 539]** |
| Position in gene sequence: 447 |
| GC content: 38.1% |
| Sense strand siRNA: CAACUCGACCCUUCUUUAUtt **[SEQ ID NO. 540]** |
| Antisense strand siRNA: AUAAAGAAGGGUCGAGUUGtt **[SEQ ID NO. 541]** |
| **Target sequence 24:** AACTCGACCCTTCTTTATGAA **[SEQ ID NO. 542]** |
| Position in gene sequence: 450 |
| GC content: 38.1% |
| Sense strand siRNA: CUCGACCCUUCUUUAUGAAtt **[SEQ ID NO. 543]** |
| Antisense strand siRNA: UUCAUAAAGAAGGGUCGAGtt **[SEQ ID NO. 544]** |
| **Target sequence 25:** AAAGCTCTGTCAGGCCCCGGC **[SEQ ID NO. 545]** |
| Position in gene sequence: 469 |
| GC content: 66.7% |
| Sense strand siRNA: AGCUCUGUCAGGCCCCGGCtt **[SEQ ID NO. 546]** |
| Antisense strand siRNA: GCCGGGGCCUGACAGAGCUtt **[SEQ ID NO. 547]** |
| **Target sequence 26:** AATCCCTCAAAACACATCTGG **[SEQ ID NO. 548]** |
| Position in gene sequence: 521 |
| GC content: 42.9% |
| Sense strand siRNA: UCCCUCAAAACACAUCUGGtt **[SEQ ID NO. 549]** |
| Antisense strand siRNA: CCAGAUGUGUUUUGAGGGAtt **[SEQ ID NO. 550]** |
| **Target sequence 27:** AAAACACATCTGGACAGGTTG **[SEQ ID NO. 551]** |
| Position in gene sequence: 529 |
| GC content: 42.9% |
| Sense strand siRNA: AACACAUCUGGACAGGUUGtt **[SEQ ID NO. 552]** |
| Antisense strand siRNA: CAACCUGUCCAGAUGUGUUtt **[SEQ ID NO. 553]** |
| **Target sequence 28:** AACACATCTGGACAGGTTGGA **[SEQ ID NO. 554]** |
| Position in gene sequence: 531 |
| GC content: 47.6% |
| Sense strand siRNA: CACAUCUGGACAGGUUGGAtt **[SEQ ID NO. 555]** |
| Antisense strand siRNA: UCCAACCUGUCCAGAUGUGtt **[SEQ ID NO. 556]** |
| **Target sequence 29:** AAT541GAATCGGGCTATGTG **[SEQ ID NO. 557]** |
| Position in gene sequence: 562 |
| GC content: 38.1% |
| Sense strand siRNA: U541 GAAUCGGGCUAUGUGtt **[SEQ ID NO. 558]** |
| Antisense strand siRNA: CACAUAGCCCGAUUC145Att **[SEQ ID NO. 559]** |
| **Target sequence 30:** AATCGGGCTATGTGGACGTGT **[SEQ ID NO. 560]** |
| Position in gene sequence: 569 |
| GC content: 52.4% |
| Sense strand siRNA: UCGGGCUAUGUGGACGUGUtt **[SEQ ID NO. 561]** |
| Antisense strand siRNA: ACACGUCCACAUAGCCCGAtt **[SEQ ID NO. 562]** |
| **Target sequence 31:** AAC601ACGCTCTTCTTGAGG **[SEQ ID NO. 563]** |
| Position in gene sequence: 625 |
| GC content: 42.9% |
| Sense strand siRNA: C601ACGCUCUUCUUGAGGtt **[SEQ ID NO. 564]** |
| Antisense strand siRNA: CCUCAAGAAGAGCGU106Gtt **[SEQ ID NO. 565]** |
| **Target sequence 32:** AAAGTGCTATCGTGGTTAAAA **[SEQ ID NO. 566]** |
| Position in gene sequence: 659 |
| GC content: 33.3% |
| Sense strand siRNA: AGUGCUAUCGUGGUUAAAAtt **[SEQ ID NO. 567]** |
| Antisense strand siRNA: UUUUAACCACGAUAGCACUtt **[SEQ ID NO. 568]** |
| **Target sequence 33:** AAAATCCAAGGTTAT661TTT **[SEQ ID NO. 569]** |
| Position in gene sequence: 676 |
| GC content: 19% |
| Sense strand siRNA: AAUCCAAGGUUAUG61UUUtt **[SEQ ID NO. 570]** |
| Antisense strand siRNA: AAA166AUAACCUUGGAUUtt **[SEQ ID NO. 571]** |
| **Target sequence 34:** AATCCAAGGTTAT661TTTGA **[SEQ ID NO. 572]** |
| Position in gene sequence: 678 |
| GC content: 23.8% |
| Sense strand siRNA: UCCAAGGUUAU661UUUGAtt **[SEQ ID NO. 573]** |
| Antisense strand siRNA: UCAAA166AUAACCUUGGAtt **[SEQ ID NO. 574]** |
| **Target sequence 35:** AAGGTTAT661TTTGATGCAT **[SEQ ID NO. 575]** |
| Position in gene sequence: 683 |
| GC content: 23.8% |
| Sense strand siRNA: GGUUAU661UUUGAUGCAUtt **[SEQ ID NO. 576]** |
| Antisense strand siRNA: AUGCAUCAAA166AUAACCtt **[SEQ ID NO. 577]** |
| **Target sequence 36:** AAGCTCTCCTCATCAAACCAG **[SEQ ID NO. 578]** |
| Position in gene sequence: 707 |
| GC content: 47.6% |
| Sense strand siRNA: GCUCUCCUCAUCAAACCAGtt **[SEQ ID NO. 579]** |
| Antisense strand siRNA: CUGGUUUGAUGAGGAGAGCtt **[SEQ ID NO. 580]** |
| **Target sequence 37:** AAACCAGACATCTTCTTTAAG **[SEQ ID NO. 581]** |
| Position in gene sequence: 721 |
| GC content: 33.3% |
| Sense strand siRNA: ACCAGACAUCUUCUUUAAGtt **[SEQ ID NO. 582]** |
| Antisense strand siRNA: CUUAAAGAAGAUGUCUGGUtt **[SEQ ID NO. 583]** |
| **Target sequence 38:** AAGATTTCTTGGCTA721TAC **[SEQ ID NO. 584]** |
| Position in gene sequence: 739 |
| GC content: 28.6% |
| Sense strand siRNA: GAUUUCUUGGCUA721UACtt **[SEQ ID NO. 585]** |
| Antisense strand siRNA: GUA127UAGCCAAGAAAUCtt **[SEQ ID NO. 586]** |
| **Target sequence 39:** AAAAAGTATGAGAAGTCTGTC **[SEQ ID NO. 587]** |
| Position in gene sequence: 760 |
| GC content: 33.3% |
| Sense strand siRNA: AAAGUAUGAGAAGUCUGUCtt **[SEQ ID NO. 588]** |
| Antisense strand siRNA: GACAGACUUCUCAUACUUUtt **[SEQ ID NO. 589]** |
| **Target sequence 40:** AAAGTATGAGAAGTCTGTCAA **[SEQ ID NO. 590]** |
| Position in gene sequence: 762 |
| GC content: 33.3% |
| Sense strand siRNA: AGUAUGAGAAGUCUGUCAAtt **[SEQ ID NO. 591]** |
| Antisense strand siRNA: UUGACAGACUUCUCAUACUtt **[SEQ ID NO. 592]** |
| **Target sequence 41:** AAGTCTGTCAAGGATTTGAAA **[SEQ ID NO. 593]** |
| Position in gene sequence: 772 |
| GC content: 33.3% |
| Sense strand siRNA: GUCUGUCAAGGAUUUGAAAtt **[SEQ ID NO. 594]** |
| Antisense strand siRNA: UUUCAAAUCCUUGACAGACtt **[SEQ ID NO. 595]** |
| **Target sequence 42:** AAGGATTTGAAAGATGCCATA **[SEQ ID NO. 596]** |
| Position in gene sequence: 781 |
| GC content: 33.3% |
| Sense strand siRNA: GGAUUUGAAAGAUGCCAUAtt **[SEQ ID NO. 597]** |
| Antisense strand siRNA: UAUGGCAUCUUUCAAAUCCtt **[SEQ ID NO. 598]** |
| **Target sequence 43:** AAAGATGCCATAGAAGTTCTG **[SEQ ID NO. 599]** |
| Position in gene sequence: 790 |
| GC content: 38.1% |
| Sense strand siRNA: AGAUGCCAUAGAAGUUCUGtt **[SEQ ID NO. 600]** |
| Antisense strand siRNA: CAGAACUUCUAUGGCAUCUtt **[SEQ ID NO. 601]** |
| **Target sequence 44:** AAGTTCTGATAGCA781GAAA **[SEQ ID NO. 602]** |
| Position in gene sequence: 803 |
| GC content: 28.6% |
| Sense strand siRNA: GUUCUGAUAGCA781GAAAtt **[SEQ ID NO. 603]** |
| Antisense strand siRNA; UUUC187UGCUAUCAGAACtt **[SEQ ID NO. 604]** |
| **Target sequence 45:** AAAAAAGACGCAGGATTTCCA **[SEQ ID NO. 605]** |
| Position in gene sequence: 821 |
| GC content: 38.1% |
| Sense strand siRNA: AAAAGACGCAGGAUUUCCAtt **[SEQ ID NO. 606]** |
| Antisense strand siRNA: UGGAAAUCCUGCGUCUUUUtt **[SEQ ID NO. 607]** |
| **Target sequence 46:** AAAAGACGCAGGATTTCCACA **[SEQ ID NO. 608]** |
| Position in gene sequence: 823 |
| GC content: 42.9% |
| Sense strand siRNA: AAGACGCAGGAUUUCCACAtt **[SEQ ID NO. 609]** |
| Antisense strand siRNA: UGUGGAAAUCCUGCGUCUUtt **[SEQ ID NO. 610]** |
| **Target sequence 47:** AAGACGCAGGATTTCCACAGA **[SEQ ID NO. 611]** |
| Position in gene sequence: 825 |
| GC content: 47.6% |
| Sense strand siRNA: GACGCAGGAUUUCCACAGAtt **[SEQ ID NO. 612]** |
| Antisense strand siRNA: UCUGUGGAAAUCCUGCGUCtt **[SEQ ID NO. 613]** |
| **Target sequence 48:** AAGAGAAACTGGAAGAATGTA **[SEQ ID NO. 614]** |
| Position in gene sequence: 845 |
| GC content: 33.3% |
| Sense strand siRNA: GAGAAACUGGAAGAAUGUAtt **[SEQ ID NO. 615]** |
| Antisense strand siRNA: UACAUUCUUCCAGUUUCUCtt **[SEQ ID NO. 616]** |
| **Target sequence 49:** AAACTGGAAGAATGTATGGAC **[SEQ ID NO. 617]** |
| Position in gene sequence: 850 |
| GC content: 38.1% |
| Sense strand siRNA: ACUGGAAGAAUGUAUGGACtt **[SEQ ID NO. 618]** |
| Antisense strand **siRNA:** GUCCAUACAUUCUUCCAGUtt **[SEQ ID NO. 619]** |
| **Target sequence 50: AAGAATGTATGGACTTTGCCA [SEQ ID NO. 620]** |
| Position in gene sequence: 857 |
| GC content: 38.1% |
| Sense strand siRNA: GAAUGUAUGGACUUUGCCAtt **[SEQ ID NO. 621]** |
| Antisense strand siRNA: UGGCAAAGUCCAUACAUUCtt **[SEQ ID NO. 622]** |
| **Target sequence 51:** AATGTATGGACTTTGCCACT8 **[SEQ ID NO. 623]** |
| Position in gene sequence: 860 |
| GC content: 38.1% |
| Sense strand siRNA: UGUAUGGACUUUGCCACU8tt **[SEQ ID NO. 624]** |
| Antisense strand siRNA: 8AGUGGCAAAGUCCAUACAtt **[SEQ ID NO. 625]** |
| **Target sequence 52:** AAACGTGGTGACCTGACAAGA **[SEQ ID NO. 626]** |
| Position in gene sequence: 901 |
| GC content: 47.6% |
| Sense strand siRNA: ACGUGGUGACCUGACAAGAtt **[SEQ ID NO. 627]** |
| Antisense strand siRNA: UCUUGUCAGGUCACCACGUtt **[SEQ ID NO. 628]** |
| **Target sequence 53:** AAGAGAGAATGTGAACCAGTG **[SEQ ID NO. 629]** |
| Position in gene sequence: 918 |
| GC content: 42.9% |
| Sense strand siRNA: GAGAGAAUGUGAACCAGUGtt **[SEQ ID NO. 630]** |
| Antisense strand siRNA: CACUGGWCACAUUCUCUCtt **[SEQ ID NO. 631]** |
| **Target sequence 54:** AATGTGAACCAGTGCATA901 **[SEQ ID NO. 632]** |
| Position in gene sequence: 925 |
| GC content: 33.3% |
| Sense strand siRNA: UGUGAACCAGUGCAUA901tt **[SEQ ID NO. 633]** |
| Antisense strand siRNA: 109UAUGCACUGGUUCACAtt **[SEQ ID NO. 634]** |
| **Target sequence 55:** AACCAGTGCATA901TTGGAA **[SEQ ID NO. 635]** |
| Position in gene sequence: 931 |
| GC content: 33.3% |
| Sense strand siRNA: CCAGUGCAUA901UUGGAAtt **[SEQ ID NO. 636]** |
| Antisense strand siRNA: UUCCAA109UAUGCACUGGtt **[SEQ ID NO. 637]** |
| **Target sequence 56:** AAATGCTGATCGCAGCTCCTG **[SEQ ID NO. 638]** |
| Position in gene sequence: 950 |
| GC content: 52.4% |
| Sense strand siRNA: AUGCUGAUCGCAGCUCCUGtt **[SEQ ID NO. 639]** |
| Antisense strand siRNA: CAGGAGCUGCGAUCAGCAUtt **[SEQ ID NO. 640]** |
| **Target sequence 57:** AAAGCACCCTAATGTTGAAGA **[SEQ ID NO. 641]** |
| Position in gene sequence: 1017 |
| GC content: 38.1% |
| Sense strand siRNA: AGCACCCUAAUGUUGAAGAtt **[SEQ ID NO. 642]** |
| Antisense strand siRNA: UCUUCAACAUUAGGGUGCUtt **[SEQ ID NO. 643]** |
| **Target sequence 58:** AATGTTGAAGAGGCAATAATA **[SEQ ID NO. 644]** |
| Position in gene sequence: 1027 |
| GC content: 28.6% |
| Sense strand siRNA: UGUUGAAGAGGCAAUAAUAtt **[SEQ ID NO. 645]** |
| Antisense strand siRNA: UAUUAUUGCCUCUUCAACAtt **[SEQ ID NO. 646]** |
| **Target sequence 59:** AAGAGGCAATAATAAAGGAAA **[SEQ ID NO. 647]** |
| Position in gene sequence: 1034 |
| GC content: 28.6% |
| Sense strand siRNA: GAGGCAAUAAUAAAGGAAAtt **[SEQ ID NO. 648]** |
| Antisense strand siRNA: UUUCCUUUAUUAUUGCCUCtt **[SEQ ID NO. 649]** |
| **Target sequence 60:** AATAATAAAGGAAATCCAGAC **[SEQ ID NO. 650]** |
| Position in gene sequence: 1041 |
| GC content: 28.6% |
| Sense strand siRNA: UAAUAAAGGAAAUCCAGACtt **[SEQ ID NO. 651]** |
| Antisense strand siRNA: GUCUGGAUUUCCUUUAUUAtt **[SEQ ID NO. 652]** |
| **Target sequence 61:** AATAAAGGAAATCCAGACTGT **[SEQ ID NO. 653]** |
| Position in gene sequence: 1044 |
| GC content: 33.3% |
| Sense strand siRNA: UAAAGGAAAUCCAGACUGUtt **[SEQ ID NO. 654]** |
| Antisense strand siRNA: ACAGUCUGGAUUUCCUUUAtt **[SEQ ID NO. 655]** |
| **Target sequence 62:** AAAGGAAATCCAGACTGTTAT **[SEQ ID NO. 656]** |
| Position in gene sequence: 1047 |
| GC content: 33.3% |
| Sense strand siRNA: AGGAAAUCCAGACUGUUAUtt **[SEQ ID NO. 65,7]** |
| Antisense strand siRNA: AUAACAGUCUGGAUUUCCUtt **[SEQ ID NO. 658]** |
| **Target sequence 63:** AAATCCAGACTGTTATT1021 **[SEQ ID NO. 659]** |
| Position in gene sequence: 1052 |
| GC content: 23.8% |
| Sense strand siRNA: AUCCAGACUGUUAUU1021tt **[SEQ ID NO. 660]** |
| Antisense strand siRNA: 1201AAUAACAGUCUGGAUtt **[SEQ ID NO. 661]** |
| **Target sequence 64:** AAAGATTGATGATATACAAAA **[SEQ ID NO. 662]** |
| Position in gene sequence: 1087 |
| GC content: 19% |
| Sense strand siRNA: AGAUUGAUGAUAUACAAAAtt **[SEQ ID NO. 663]** |
| Antisense strand siRNA: UUUUGUAUAUCAUCAAUCUtt **[SEQ ID NO. 664]** |
| **Target sequence 65:** AAAAATTAAAAGTGATGGAAA **[SEQ ID NO. 665]** |
| Position in gene sequence: 1104 |
| GC content: 19% |
| Sense strand siRNA: AAAUUAAAAGUGAUGGAAAtt **[SEQ ID NO. 666]** |
| Antisense strand siRNA: UUUCCAUCACUUUUAAUUUtt **[SEQ ID NO. 667]** |
| **Target sequence 66:** AAATTAAAAGTGATGGAAAAC **[SEQ ID NO. 668]** |
| Position in gene sequence: 1106 |
| GC content: 23.8% |
| Sense strand silzNA: AUUAAAAGUGAUGGAAAACtt **[SEQ ID NO. 669]** |
| Antisense strand siRNA: GUUUUCCAUCACUUUUAAUtt **[SEQ ID NO. 670]** |
| **Target sequence 67:** AAAAGTGATGGAAAACTTCAT **[SEQ ID NO. 671]** |
| Position in gene sequence: 1111 |
| GC content: 28.6% |
| Sense strand siRNA: AAGUGAUGGAAAACUUCAUtt **[SEQ ID NO. 672]** |
| Antisense strand siRNA: AUGAAGUUUUCCAUCACUUtt **[SEQ ID NO. 673]** |
| **Target sequence 68:** AAGTGATGGAAAACTTCATT1 **[SEQ ID NO. 674]** |
| Position in gene sequence: 1113 |
| GC content: 28.6% |
| Sense strand siRNA: GUGAUGGAAAACUUCAUU1tt **[SEQ ID NO. 675]** |
| Antisense strand siRNA: IAAUGAAGUUUUCCAUCACtt **[SEQ ID NO. 676]** |
| **Target sequence 69:** AAAACTTCATT1081TATGAG **[SEQ ID NO. 677]** |
| Position in gene sequence: 1122 |
| GC content: 19% |
| Sense strand siRNA: AACUUCAUU1081UAUGAGtt **[SEQ ID NO. 678]** |
| Antisense strand siRNA: CUCAUA1801AAUGAAGUUtt **[SEQ ID NO. 679]** |
| **Target sequence 70:** AACTTCATT1081TATGAGAG **[SEQ ID NO. 680]** |
| Position in gene sequence: 1124 |
| GC content: 23.8% |
| Sense strand siRNA: CUUCAUU1081UAUGAGAGtt **[SEQ ID NO. 681]** |
| Antisense strand siRNA,: CUCUCAUA1801AAUGAAGtt **[SEQ ID NO. 682]** |
| **Target sequence 71:** AAAGCCTTAGAAGAT1141GA **[SEQ ID NO. 683]** |
| Position in gene sequence: 1182 |
| GC content: 28.6% |
| Sense strand siRNA: AGCCUUAGAAGAU1141Gatt **[SEQ ID NO. 684]** |
| Antisense strand siRNA: UC1411AUCUUCUAAGGCUtt **[SEQ ID NO. 685]** |
| **Target sequence 72:** AAGAT1141GATGTAATCGAT **[SEQ ID NO. 686]** |
| Position in gene sequence: 1192 |
| GC content: 23.8% |
| Sense strand siRNA: GAU1141GAUGUAAUCGAUtt **[SEQ ID NO. 687]** |
| Antisense strand siRNA: AUCGAUUACAUC1411AUCtt **[SEQ ID NO. 688]** |
| **Target sequence 73:** AATCGATGGCTACCCAGTGAA **[SEQ ID NO. 689]** |
| Position in gene sequence: 1206 |
| GC content: 47.6% |
| Sense strand siRNA: UCGAUGGCUACCCAGUGAAtt **[SEQ ID NO. 690]** |
| Antisense strand siRNA: UUCACUGGGUAGCCAUCGAtt **[SEQ ID NO. 691]** |
| **Target sequence 74:** AAAAAGGGGACAAACATTATC **[SEQ ID NO. 692]** |
| Position in gene sequence: 1225 |
| GC content: 33.3% |
| Sense strand siRNA: AAAGGGGACAAACAUUAUCtt **[SEQ ID NO. 693]** |
| Antisense strand siRNA: GAUAAUGUUUGUCCCCUUUtt **[SEQ ID NO. 694]** |
| **Target sequence 75:** AAAGGGGACAAACATTATCCT **[SEQ ID NO. 695]** |
| Position in gene sequence: 1227 |
| content: 38.1% |
| strand siRNA: AGGGGACAAACAUUAUCCUtt **[SEQ ID NO. 6961** |
| strand siRNA: AGGAUAAUGUUUGUCCCCUtt **[SEQ ID NO. 697]** |
| **Target sequence 76:** AAACATTATCCTGAATATTGG **[SEQ ID NO. 698]** |
| Position in gene sequence: 1236 |
| GC content: 28.6% |
| Sense strand siRNA: ACAUUAUCCUGAAUAUUGGtt **[SEQ ID NO. 699]** |
| Antisense strand siRNA: CCAAUAUUCAGGAUAAUGUtt **[SEQ ID NO. 700]** |
| **Target sequence 77:** AATATTGGAAGG1201ATGCA **[SEQ ID NO. 701]** |
| Position in gene sequence: 1249 |
| GC content: 28.6% |
| Sense strand siRNA: UAUUGGAAGG1201AUGCAtt **[SEQ ID NO. 702]** |
| Antisense strand siRNA: UGCAU1021CCUUCCAAUAtt **[SEQ ID NO. 703]** |
| **Target sequence 78:** AAGG1201ATGCACAGACTCG **[SEQ ID NO. 704]** |
| Position in gene sequence: 1257 |
| GC content: 42.9% |
| Sense strand siRNA: GG1201AUGCACAGACUCGtt **[SEQ ID NO. 705]** |
| Antisense strand siRNA: CGAGUCUGUGCAU1021CCtt **[SEQ ID NO. 706]** |
| **Target sequence 79:** AAACCCAATGAATTTACTCTT **[SEQ ID NO. 707]** |
| position in gene sequence: 1289 |
| GC content: 28.6% |
| Sense strand siRNA: ACCCAAUGAAUUUACUCUUtt **[SEQ ID NO. 708]** |
| Antisense strand siRNA: AAGAGUAAAUUCAUUGGGUtt **[SEQ ID NO. 709]** |
| **Target sequence 80:** AATGAATTTACTCTTGAAAAT **[SEQ ID NO. 710]** |
| Position in gene sequence: 1295 |
| GC content: 19% |
| Sense strand siRNA: UGAAUUUACUCUUGAAAAUtt **[SEQ ID NO. 711]** |
| Antisense strand siRNA: AUUUUCAAGAGUAAAUUCAtt **[SEQ ID NO. 712]** |
| **Target sequence 81:** AATTTACTCTTGAAAATTTTG **[SEQ ID NO. 713]** |
| Position in gene sequence: 1299 |
| GC content: 19% |
| Sense strand siRNA: UUUACUCUUGAAAAUUUUGtt **[SEQ ID NO. 714]** |
| Antisense strand siRNA: CAAAAUUUUCAAGAGUAAAtt **[SEQ ID NO. 715]** |
| **Target sequence 82:** AVAAATTTTGCAAAG1261AAT **[SEQ ID NO. 716]** |
| Position in gene sequence: 1311 |
| GC content: 14.3% |
| Sense strand siRNA: AAUUUUGCAAAG1261AAUtt **[SEQ ID NO. 717]** |
| Antisense strand siRNA: AUU1621CUUUGCAAAAUUtt **[SEQ ID NO. 718]** |
| **Target sequence 83:** AATTTTGCAAAG1261AATGT **[SEQ ID NO. 719]** |
| Position in gene sequence: 1313 |
| GC content: 19% |
| Sense strand siRNA: UUUUGCAAAG1261AAUGUtt **[SEQ ID NO. 720]** |
| Antisense strand siRNA: ACAUU1621CUUUGCAAAAtt **[SEQ ID NO. 7211** |
| **Target sequence 84:** AAAG1261AATGTTCCTTATA **[SEQ ID NO. 722]** |
| Position in gene sequence: 1321 |
| GC content: 19% |
| Sense strand siRNA: AG1261AAUGUUCCUUAUAtt **[SEQ ID NO. 723]** |
| Antisense strand siRNA: UAUAAGGAACATJU1621Cutt **[SEQ ID NO. 724]** |
| **Target sequence 85:** AATGTTCCTTATAGGTACTTT **[SEQ ID NO. 725]** |
| Position in gene sequence: 1329 |
| GC content: 28.6% |
| Sense strand siRNA: UGUUCCUUAUAGGUACUUUtt **[SEQ ID NO. 726]** |
| Antisense strand siRNA: AAAGUACCUAUAAGGAACAtt **[SEQ ID NO. 727]** |
| **Target sequence 86:** AAAG1321TACATCGCCATGG **[SEQ ID NO. 728]** |
| Position in gene sequence: 1385 |
| GC content: 38.1% |
| Sense strand siRNA: AG1321UACAUCGCCAUGGtt **[SEQ ID NO. 729]** |
| Antisense strand siRNA: CCAUGGCGAUGUA1231CUtt **[SEQ ID NO. 730]** |
| **Target sequence 87:** AAAGCCATCCTCGTTACACTT **[SEQ ID NO. 731]** |
| Position in gene sequence: 1414 |
| GC content: 42.9% |
| Sense strand siRNA: AGCCAUCCUCGUUACACUUtt **[SEQ ID NO. 732]** |
| Antisense strand siRNA: AAGUGUAACGAGGAUGGCUtt **[SEQ ID NO. 733]** |
| **Target sequence 88:** AAGACATTGCAAGGACAGTGT **[SEQ ID NO. 734]** |
| Position in gene sequence: 1457 |
| GC content: 42.9% |
| Sense strand siRNA: GACAUUGCAAGGACAGUGUtt **[SEQ ID NO. 735]** |
| Antisense strand siRNA: ACACUGUCCUUGCAAUGUCtt **[SEQ ID NO. 736]** |
| **Target sequence 89:** AAGGACAGTGTGTTGAGAGCA **[SEQ ID NO. 737]** |
| Position in gene sequence: 1467 |
| GC content: 47.6% |
| Sense strand siRNA: GGACAGUGUGUUGAGAGCAtt **[SEQ ID NO. 738]** |
| Antisense strand siRNA: UGCUCUCAACACACUGUCCtt **[SEQ ID NO. 739]** |
| **Target sequence 90:** AAGATACACGACTTGTCCTTG **[SEQ ID NO. 740]** |
| Position in gene sequence: 1493 |
| GC content: 42,9% |
| Sense strand siRNA: GAUACACGACUUGUCCUUGtt **[SEQ ID NO.741]** |
| Antisense strand siRNA: **[SEQ ID NO. 742]** |
| **Target sequence 91:** AAAAACATGCTGGAAATGATC **[SEQ ID NO. 743]** |
| Position in gene sequence: 1533 |
| GC content: 33.3% |
| Sense strand siRNA: AAACAUGCUGGAAAUGAUCtt **[SEQ ID NO. 744]** |
| Antisense strand siRNA: GAUCAUUUCCAGCAUGUUUtt **[SEQ ID NO. 745]** |
| **Target sequence 92:** AAACATGCTGGAAATGATCTT **[SEQ ID NO. 746]** |
| Position in gene sequence: 1535 |
| GC content: 33.3% |
| Sense strand siRNA: ACAUGCUGGAAAUGAUCUUtt **[SEQ ID NO. 747]** |
| Antisense strand siRNA: AAGAUCAUUUCCAGCAUGUtt **[SEQ ID NO. 748]** |
| **Target sequence 93:** AAATGATCTTTACCCCAAGAA **[SEQ ID NO. 749]** |
| Position in gene sequence: 1546 |
| GC content: 33.3% |
| Sense strand siRNA: AUGAUCUUUACCCCAAGAAtt **[SEQ ID NO. 750]** |
| Antisense strand siRNA: UUCUUGGGGUAAAGAUCAUtt **[SEQ ID NO. 751]** |
| **Target sequence 94:** AAGAAGCTCAGACAGGTGT15 **[SEQ ID NO. 752]** |
| Position in gene sequence: 1562 |
| GC content: 42.9% |
| Sense strand siRNA: GAAGCUCAGACAGGUGU15tt **[SEQ ID NO. 753]** |
| Antisense strand siRNA: 51ACACCUGUCUGAGCUUCtt **[SEQ ID NO. 754]** |
| **Target sequence 95:** AAGCTCAGACAGGTGT1501C **[SEQ ID NO. 755]** |
| Position in gene sequence: 1565 |
| GC content: 42.9% |
| Sense strand siRNA: GCUCAGACAGGUGU1501Ctt **[SEQ ID NO. 756]** |
| Antisense strand siRNA: G1051ACACCUGUCUGAGCtt **[SEQ ID NO. 757]** |

| **Table 5: 3-alpha-Hydroxysteroiddehydrogenase** |
|---|
| **3-alpha-Hydroxysteroiddehydrogenase - BAA99542 (gi: 9186908) [SEQ ID NO. 758]** |
| 1 atggatccca aatatcagcg tgtagagcta aatgatggtc atttcatgcc cgtattggga 61 tttggcacct atgcacctcc agaggttccg aggaacagag ctgtagaggt caccaaatta 121 gcaatagaag ctggcttccg ccatattgat tctgcttatt tatacaataa tgaggagcag 181 gttggactgg ccatccgaag caagattgca gatggcagtg tgaagagaga agacatattc 241 tacacttcaa agctttggtg cactttcttt caaccacaga tggtccaacc agccttggaa 301 agctcactga aaaaacttca actggactat gttgacctct atcttcttca tttcccaatg 361 gctctcaagc caggtgagac gccactacca aaagatgaaa atggaaaagt aatattcgac 421 acagtggatc tctgtgccac atgggaggtc atggagaagt gtaaggatgc aggattggcc 481 aagtccatcg gggtgtcaaa cttcaactgc aggcagctgg agatgatcct caacaagcca 541 ggactcaagt acaagcctgt ctgcaaccag gtagaatgtc atccttacct caaccagagc 601 aaactgctgg atttctgcaa gtcaaaagac attgttctgg ttgcccacag tgctctggga 661 acccaacgac ataaactatg ggtggaccca aactccccag ttcttttgga ggacccagtt 721 ctttgtgcct tagcaaagaa acacaaacga accccagccc tgattgccct gcgctaccag 781 ctgcagcgtg gggttgtggt cctggccaag agctacaatg agcagcggat cagagagaac 841 atccaggttt ttgaattcca gttgacatca gaggatatga aagttctaga tggtctaaac 901 agaaattatc gatatgttgt catggatttt gttatggacc atcctgatta tccattttca 961 gatgaatatt ag |
| **Target sequence 1:** AAATATCAGCGTGTAGAGCTA **[SEQ ID NO. 759]** |
| Position in gene sequence: 11 |
| GC content: 38.1% |
| Sense strand siRNA: AUAUCAGCGUGUAGAGCUAtt **[SEQ ID NO. 760]** |
| Antisense strand siRNA: UAGCUCUACACGCUGAUAUtt **[SEQ ID NO. 761]** |
| **Target sequence 2:** AAATGATGGTCATTTCATGCC **[SEQ ID NO. 7621** |
| Position in gene sequence: 31 |
| GC content: 38.1% |
| Sense strand siRNA: AUGAUGGUCAUUUCAUGCCtt **[SEQ ID NO. 763]** |
| Antisense strand siRNA: GGCAUGAAAUGACCAUCAUtt **[SEQ ID NO. 764]** |
| **Target sequence 3:** AACAGAGCTGTAGAGGTCACC **[SEQ ID NO. 765]** |
| Position in gene sequence: 97 |
| GC content: 52.4% |
| Sense strand siRNA: CAGAGCUGUAGAGGUCACCtt **[SEQ ID NO. 766]** |
| Antisense strand siRNA: GGUGACCUCUACAGCUCUGtt **[SEQ ID NO. 7671** |
| **Target sequence 4:** AAATTA121GCAATAGAAGCT **[SEQ ID NO. 768]** |
| Position in gene sequence: 118 |
| GC content: 23.8% |
| Sense strand siRNA: AUUA121GCAAUAGAAGCUtt **[SEQ ID NO. 769]** |
| Antisense strand siRNA: AGCUUCUAUUGC121UAAUtt **[SEQ ID NO. 770]** |
| **Target sequence 5:** AATAGAAGCTGGCTTCCGCCA **[SEQ ID NO. 771]** |
| Position in gene sequence: 129 |
| GC content: 52.4% |
| Sense strand siRNA: UAGAAGCUGGCUUCCGCCAtt **[SEQ ID NO. 772]** |
| Antisense strand siRNA: UGGCGGAAGCCAGCUUCUAtt **[SEQ ID NO. 773]** |
| **Target sequence 6:** AAGCTGGCTTCCGCCATATTG **[SEQ ID NO. 774]** |
| Position in gene sequence: 134 |
| GC content: 52.4% |
| Sense strand siRNA: GCUGGCUUCCGCCAUAUUGtt **[SEQ ID NO. 775]** |

| **Table 5: 3-alpba-Hydroxysteroiddehydrogenase** |
|---|
| Antisense strand siRNA; CAAUAUGGCGGAAGCCAGCtt **[SEQ ID NO. 776]** |
| **Target sequence 7:** AATAATGAGGAGCAG181GTT **[SEQ ID NO. 777]** |
| Position in gene sequence: 172 |
| GC content: 33.3% |
| Sense strand siRNA: UAAUGAGGAGCAG181GUUtt **[SEQ ID NO. 778]** |
| Antisense strand siRNA: AAC181CUGCUCCUCAUUAtt **[SEQ ID NO. 779]** |
| **Target sequence 8:** AATGAGGAGCAG181GTTGGA **[SEQ ID NO. 780]** |
| Position in gene sequence: 175 |
| GC content: 42.9% |
| Sense strand siRNA: UGAGGAGCAG181GUUGGAtt **[SEQ ID NO. 781]** |
| Antisense strand siRNA: UCCAAC181CUGCUCCUCAtt **[SEQ ID NO. 782]** |
| **Target sequence 9:** AAGCAAGATTGCAGATGGCAG **[SEQ ID NO. 783]** |
| Position in gene sequence: 207 |
| GC content: 47.6% |
| Sense strand siRNA: GCAAGAUUGCAGAUGGCAGtt **[SEQ ID NO. 784]** |
| Antisense strand siRNA: CUGCCAUCUGCAAUCUUGCtt **[SEQ ID NO. 785]** |
| **Target sequence 10:** AAGATTGCAGATGGCAGTGTG **[SEQ ID NO. 786]** |
| Position in gene sequence: 211 |
| GC content: 47.6% |
| Sense strand siRNA: GAUUGCAGAUGGCAGUGUGtt **[SEQ ID NO. 787]** |
| Antisense strand siRNA: CACAGUGCCAUCUGCAAUCtt **[SEQ ID NO. 788]** |
| **Target sequence 11:** AAGAGAGAAGACATATTC241 **[SEQ ID NO. 789]** |
| Position in gene sequence: 232 |
| GC content 28,6% |
| Sense strand siRNA: GAGAGAAGACAUAUUC241tt **[SEQ ID NO. 790]** |
| Antisense strand siRNA: 142GAAUAUGUCUUCUCUCtt **[SEQ ID NO. 791]** |
| **Target sequence 12:** AAGACATATTC241 TACACTT **[SEQ ID NO. 792]** |
| Position in gene sequence: 239 |
| GC content: 23.8% |
| Sense strand siRNA: GACAUAUUC241UACACUUtt **[SEQ ID NO. 793]** |
| Antisense strand siRNA: AAGUGUA142GAAUAUGUCtt **[SEQ ID NO. 794]** |
| **Target sequence 13:** AAAGCTTTGGTGCACTTTCTT **[SEQ ID NO. 795]** |
| Position in gene sequence: 261 |
| GC content: 38.1% |
| Sense strand siRNA: AGCUUUGGUGCACWUCWtt **[SEQ ID NO. 796]** |
| Antisense strand siRNA: AAGAAAGUGCACCAAAGCUtt **[SEQ ID NO. 797]** |
| **Target sequence 14:** AACCACAGATGGTCCAACCAG **[SEQ ID NO. 798]** |
| Position in gene sequence: 284 |
| GC content: 52.4% |
| Sense strand siRNA: CCACAGAUGGUCCAACCAGtt **[SEQ ID NO. 799]** |
| Antisense strand siRNA: CUGGUUGGACCAUCUGUGGtt **[SEQ ID NO. 800]** |
| **Target sequence 15:** AACCAGCCTTGGAA301AGCT **[SEQ ID NO. 801]** |
| Position in gene sequence: 299 |
| GC content: 42.9% |
| Sense strand siRNA: CCAGCCUUGGAA301AGCUtt **[SEQ ID NO. 802]** |
| Antisense strand siRNA: AGCU103UUCCAAGGCUGGtt **[SEQ ID NO. 803]** |
| **Target sequence 16:** AA301AGCTCACTGAAAAAAC **[SEQ ID NO. 804]** |
| Position in gene sequence: 311 |
| GC content: 28.6% |

| **Table 5: 3-alpha-Hydroxysteroiddehydrogenase** |
|---|
| Sense strand siRNA: 301AGCUCACUGAAAAAACtt **[SEQ ID NO. 805]** |
| Antisense strand siRNA: GUUUUUUCAGUGAGCU103tt **[SEQ ID NO. 806]** |
| **Target sequence 17:** AAAAAACTTCAACTGGACTAT **[SEQ ID NO. 807]** |
| Position in gene sequence: 325 |
| GC content: 28.6% |
| Sense strand siRNA: AAAACUUCAACUGGACUAUtt **[SEQ ID NO. 808]** |
| Antisense strand siRNA: AUAGUCCAGUUGAAGUUUUtt **[SEQ ID NO. 809]** |
| **Target sequence 18:** AAAACTTCAACTGGACTATGT **[SEQ ID NO. 810]** |
| Position in gene sequence: 327 |
| GC content: 33.3% |
| Sense strand siRNA: AACUUCAACUGGACUAUGUtt **[SEQ ID NO. 811]** |
| Antisense strand siRNA: ACAUAGUCCAGUUGAAGUUtt **[SEQ ID NO. 812]** |
| **Target sequence 19:** AACTTCAACTGGACTATGTTG **[SEQ ID NO. 813]** |
| Position in gene sequence: 329 |
| GC content: 38.1% |
| Sense strand siRNA: CUUCAACUGGACUAUGUUGtt **[SEQ ID NO. 814]** |
| Antisense strand siRNA: CAACAUAGUCCAGUUGAAGtt **[SEQ ID NO. 815]** |
| **Target sequence 20:** AACTGGACTATGTTGACCTCT **[SEQ ID NO. 816]** |
| Position in gene sequence: 335 |
| GC content: 42.9% |
| Sense strand siRNA: CUGGACUAUGUUGACCUCUtt **[SEQ ID NO. 817]** |
| Antisense strand siRNA: AGAGGUCAACAUAGUCCAGtt **[SEQ ID NO. 818]** |
| **Target sequence 21:** AATG361GCTCTCAAGCCAGG **[SEQ ID NO. 819]** |
| Position in gene sequence: 372 |
| GC content: 47.6% |
| Sense strand siRNA: UG361GCUCUCAAGCCAGGtt **[SEQ ID NO. 820]** |
| Antisense strand siRNA: CCUCGCUUGAGAGC163Catt **[SEQ ID NO. 821]** |
| **Target sequence 22:** AAGCCAGGTGAGACGCCACTA **[SEQ ID NO. 822]** |
| Position in gene sequence: 385 |
| GC content: 57.1% |
| Sense strand siRNA: GCCAGGUGAGACGCCACUAtt **[SEQ ID NO. 823]** |
| Antisense strand siRNA: UAGUGGCGUCUCACCUGGCtt **[SEQ ID NO. 824]** |
| **Target sequence 23:** AAAAGATGAAAATGGAAAAGT **[SEQ ID NO. 825]** |
| Position in gene sequence: 408 |
| GC content: 23.8% |
| Sense strand siRNA: AAGAUGAAAAUGGAAAAGUtt **[SEQ ID NO. 826]** |
| Antisense strand siRNA: ACUUWCCAUUUUCAUCUUtt **[SEQ ID NO. 827]** |
| **Target sequence 24:** AAGATGAAAATGGAAAAGTAA **[SEQ ID NO. 828]** |
| Position in gene sequence: 410 |
| GC content: 23.8% |
| Sense strand siRNA: GAUGAAAAUGGAAAAGUAAtt **[SEQ ID NO. 829]** |
| Antisense strand siRNA: UUACUUUUCCAUUUUCAUCtt **[SEQ ID NO. 830]** |
| **Target sequence 25:** AAAATGGAAAAGTAATATTCG **[SEQ ID NO. 831]** |
| Position in gene sequence: 416 |
| GC content: 23.8% |
| Sense strand siRNA: AAUGGAAAAGUAAUAUUCGtt **[SEQ ID NO. 832]** |
| Antisense strand siRNA: CGAAUAUUACUUUUCCAUUtt **[SEQ ID NO. 833]** |
| **Target sequence 26:** AATGGAAAAGTAATATTCGAC **[SEQ ID NO. 834]** |
| Position in gene sequence: 418 |
| GC content: 28.6% |
| Sense strand siRNA: UGGAAAAGUAAUAUUCGACtt **[SEQ ID NO. 835]** |
| Antisense strand siRNA: GUCGAAUAUUACUUUUCCAtt **[SEQ ID NO. 836]** |
| **Target sequence 27:** AAAAGTAATATTCGAC421AC **[SEQ ID NO. 837]** |
| Position in gene sequence: 423 |
| GC content: 23.8% |
| Sense strand siRNA: AAGUAAUAUUCGAC421ACtt **[SEQ ID NO. 838]** |
| Antisense strand siRNA: GUI24GUCGAAUAUUACUUtt **[SEQ ID NO. 839]** |
| **Target sequence 28:** AAGTAATATTCGAC421ACAG **[SEQ ID NO. 840]** |
| Position in gene sequence: 425 |
| GC content: 28.6% |
| Sense strand siRNA: GUAAUAUUCGAC421ACAGtt **[SEQ ID NO. 841]** |
| Antisense strand siRNA: CUGU124GUCGAAUAUUACtt **[SEQ ID NO. 842]** |
| **Target sequence 29:** AATATTCGAC421ACAGTGGA **[SEQ ID NO. 843]** |
| Position in gene sequence: 429 |
| GC content: 33.3% |
| Sense strand siRNA,: UAUUCGAC421ACAGUGGAtt **[SEQ ID NO. 844]** |
| Antisense strand siRNA: UCCACUGU124GUCGAAUAtt **[SEQ ID NO. 845]** |
| **Target sequence 30:** AAGTGTAAGGATGCAGGATTG **[SEQ ID NO. 846]** |
| Position in gene sequence: 478 |
| GC content: 42.9% |
| Sense strand siRNA: GUGUAAGGAUGCAGGAUUGtt **[SEQ ID** NO. **847]** |
| antisense strand siRNA: CAAUCCUGCAUCCUUACACtt **[SEQ ID NO. 848]** |
| **Target sequence 31:** AAGGATGCAGGATTGGCC481 **[SEQ ID NO. 849]** |
| Position in gene sequence: 484 |
| GC content: 47.6% |
| Sense strand siRNA: GGAUGCAGGAUUGGCC481tt **[SEQ ID NO. 850]** |
| Antisense strand siRNA: 184GGCCAAUCCUGCAUCCtt **[SEQ ID NO. 851]** |
| **Target sequence 32:** AAGTCCATCGGGGTGTCAAAC **[SEQ ID NO. 852]** |
| Position in gene sequence: 505 |
| GC content: 52.4% |
| Sense strand siRNA: GUCCAUCGGGGUGUCAAACtt **[SEQ ID NO. 853]** |
| Antisense strand siRNA: GUUUGACACCCCGAUGGACtt **[SEQ ID NO. 854]** |
| **Target sequence 33:** AAACTTCAACTGCAGGCAGCT **[SEQ ID NO. 855]** |
| Position in gene sequence: 522 |
| GC content: 47.6% |
| Sense strand siRNA: ACUUCAACUGCAGGCAGCUtt **[SEQ ID NO. 856]** |
| Antisense strand siRNA: AGCUGCCUGCAGUUGAAGUtt **[SEQ ID NO. 857]** |
| **Target sequence 34:** AACTGCAGGCAGCTGGAGATG **[SEQ ID NO. 858]** |
| Position in gene sequence: 529 |
| GC content: 57.1% |
| Sense strand siRNA: CUGCAGGCAGCUGGAGAUGtt **[SEQ ID NO. 859]** |
| Antisense strand siRNA: CAUCUCCAGCUGCCUGCAGtt **[SEQ ID NO. 560]** |
| **Target sequence 35:** AACAAGCCA541GGACTCAAG **[SEQ ID NO. 861]** |
| Position in gene sequence: 556 |
| GC content: 42.9% |
| Sense strand siRNA: CAAGCCA541GGACUCAAGtt **[SEQ ID NO. 862]** |
| Antisense strand siRNA: CUUGAGUCC145UGGCUUGtt **[SEQ ID NO. 863]** |
| **Target sequence 36:** AAGCCA541GOACTCAAGTAC **[SEQ** ID **NO. 864]** |
| Position in gene sequence: 559 |
| GC content: 42.9% |
| Sense strand siRNA: GCCA541 GGACUCAAGUACtt **[SEQ TD NO. 865]** |
| Antisense strand siRNA: GUACUUGAGUCC145UGGCtt **[SEQ ID NO. 866]** |
| **Target sequence 37:** AAGTACAAGCCTGTCTGCAAC **[SEQ ID NO. 867]** |
| Position in gene sequence: 574 |
| GC content: 47.6% |
| Sense strand siRNA: GUACAAGCCUGUCUGCAACtt **[SEQ ID NO. 868]** |
| Antisense strand siRNA: GUUGCAGACAGGCUUGUACtt **[SEQ ID NO. 869]** |
| **Target sequence 38:** AAGCCTGTCTGCAACCAGGTA **[SEQ ID NO. 870]** |
| Position in gene sequence: 580 |
| GC content: 52.4% |
| Sense strand siRNA: GCCUGUCUGCAACCAGGUAtt **[SEQ ID NO. 871]** |
| Antisense strand siRNA: UACCUGGUUGCAGACAGGCtt **[SEQ ID NO. 872]** |
| **Target sequence 39:** AACCAGGTAGAATGTCATCCT **[SEQ ID NO. 873]** |
| Position in gene sequence: 592 |
| GC content: 42.9% |
| Sense strand siRNA: CCAGGUAGAAUGUCAUCCUtt **[SEQ ID NO. 874]** |
| Antisense strand siRNA: AGGAUGACAUUCUACCUGGtt **[SEQ ID NO. 875]** |
| **Target sequence 40:** AATGTCATCCTTACCTCAACC **[SEQ ID NO. 876]** |
| Position in gene sequence: 602 |
| GC content: 42.9% |
| Sense strand siltNA: UGUCAUCCUUACCUCAACCtt **[SEQ ID NO. 877]** |
| Antisense strand siRNA: GGUUGAGGUAAGGAUGACAtt **[SEQ ID NO. 878]** |
| **Target sequence 41:** AACCAGAGC601AAACTGCTG **[SEQ ID NO. 879]** |
| Position in gene sequence: 619 |
| GC content: 42.9% |
| Sense strand siRNAs: CCAGAGC601AAACUGCUGtt **[SEQ ID NO. 880]** |
| Antisense strand siRNA: CAGCAGUUU106GCUCUGGtt **[SEQ ID NO. 881]** |
| **Target sequence 42:** AAACTGCTGGATTTCTGCAAG **[SEQ ID NO. 882]** |
| Position in gene sequence: 631 |
| GC content: 42.9% |
| Sense strand siRNA: ACUGCUGGAUUUCUGCAAGtt **[SEQ ID NO. 883]** |
| Antisense strand siRNA: CUUGCAGAAAUCCAGCAGUtt **[SEQ ID NO. 884]** |
| **Target sequence 43:** AAGTCAAAAGACATTGTTCTG **[SEQ ID NO. 885]** |
| Position in gene sequence: 649 |
| GC content: 33.3% |
| Sense strand siRNA: GUCAAAAGACAUUGUUCUGtt **[SEQ ID NO. 886]** |
| Antisense strand siRNA: CAGAACAAUGUCUUUUGACtt **[SEQ ID NO. 887]** |
| **Target sequence 44:** AAAAGACATTGTTCTGGTTGC **[SEQ ID NO. 888]** |
| Position in gene sequence: 654 |
| GC content: 38.1% |
| Sense strand siRNA: AAGACAUUGUUCUGGUUGCtt **[SEQ ID NO. 889]** |
| Antisense strand siRNA: GCAACCAGAACAAUGUCUUtt **[SEQ ID NO. 890]** |
| **Target sequence 45:** AAGACATTGTTCTGGTTGCCC **[SEQ ID NO. 891]** |
| Position in gene sequence: 656 |
| GC content: 47.6% |
| Sense strand siRNA: GACAUUGUUCUGGUUGCCCtt **[SEQ ID NO. 892]** |
| Antisense strand siRNA: GGGCAACCAGAACAAUGUCtt **[SEQ ID NO. 893]** |
| **Target sequence 46:** AACGACATAAACTATGGGTGG **[SEQ ID NO. 894]** |
| Position in gene sequence: 698 |
| GC content: 42.9% |
| Sense strand siRNA: CGACAUAAACUAUGGGUGGtt **[SEQ ID NO. 895]** |
| Antisense strand siRNA: CCACCCAUAGUUUAUGUCGtt **[SEQ ID NO. 896]** |
| **Target sequence 47:** AAACTATGGGTGGACCCAAAC **[SEQ ID NO. 897]** |
| Position in gene sequence: 706 |
| GC content: 47.6% |
| Sense strand siRNA: ACUAUGGGUGGACCCAAACtt **[SEQ ID NO. 898]** |
| Antisense strand siRNA: GUUUGGGUCCACCCAUAGUtt **[SEQ ID NO. 899]** |
| **Target sequence 48:** AAACTCCGCAGTTCTITTGGA **[SEQ ID NO. 900]** |
| Position in gene sequence: 723 |
| GC content: 42.9% |
| Sense strand siRNA: ACUCCCCAGUUCUUUUGGAtt **[SEQ ID NO. 901]** |
| Antisense strand siRNA: UCCAAAAGAACUGGGGAGUtt **[SEQ ID NO. 902]** |
| **Target sequence 49:** AAAGAAACACAAACGAACCCC **[SEQ ID NO. 903]** |
| Position in gene sequence: 771 |
| GC content: 42.9% |
| Sense strand siRNA: AGAAACACAAACGAACCCCtt **[SEQ ID NO. 904]** |
| Antisense strand siRNA: GGGGUUCGUUUGUGUUUCUtt **[SEQ ID NO. 905]** |
| **Target sequence 50:** AAACACAAACGAACCCCAGCC **[SEQ ID NO. 906]** |
| Position in gene sequence: 775 |
| GC content: 52.4% |
| Sense strand siRNA: ACACAAACGAACCCCAGCCtt **[SEQ ID NO. 907]** |
| Antisense strand siRNA: GGCUGGGGUUCGUUUGUGUtt **[SEQ ID NO. 908]** |
| **Target sequence 51:** AAACGAACCCCAGCCCTGATT **[SEQ ID NO. 909]** |
| Position in gene sequence: 781 |
| GC content: 52.4% |
| Sense strand siRNA: ACGAACCCCAGCCCUGAUUtt **[SEQ ID NO. 910]** |
| Antisense strand siRNA: AAUCAGGGCUGGGGUUCGUtt **[SEQ ID NO. 911]** |
| **Target sequence 52:** AACCCCAGCCCTGATTGCCCT **[SEQ ID NO. 912]** |
| Position in gene sequence: 786 |
| GC content: 61.9% |
| Sense strand siRNA: CCCCAGCCCUGAUUGCCCUtt **[SEQ ID NO. 913]** |
| Antisense strand siRNA: AGGGCAAUCAGGGCUGGGGtt **[SEQ ID NO. 914]** |
| **Target sequence 53:** AAGAGCTACAATGAGCAGCGG **[SEQ ID NO. 915]** |
| Position in gene sequence: 847 |
| GC content: 52.4% |
| Sense strand siRNA: GAGCUACAAUGAGCAGCGGtt **[SEQ ID NO. 916]** |
| Antisense strand siRNA: CCGCUGCUCAUUGUAGCUCtt **[SEQ ID NO. 917]** |
| **Target sequence 54:** AATGAGCAGCGGATCAGAGAG **[SEQ ID NO. 918]** |
| Position in gene sequence: 856 |
| GC content: 52.4% |
| Sense strand siRNA: UGAGCAGCGGAUCAGAGAGtt **[SEQ ID NO. 919]** |
| Antisense strand siRNA: CUCUCUGAUCCGCUGCUCAtt **[SEQ ID NO. 920]** |
| **Target sequence 55:** AAC841ATCCAGGTTTTTGAA **[SEQ ID NO. 921]** |
| Position in gene sequence: 877 |
| GC content: 28.6% |
| Sense strand siRNA: C841AUCCAGGUUUUUGAAtt **[SEQ ID NO. 922]** |
| Antisense strand siRNA: UUCAAAAACCUGGAU148Gtt **[SEQ ID NO. 923]** |
| **Target sequence 56:** AATTCCAGTTGACATCAGAGG **[SEQ ID NO. 924]** |
| Position in gene sequence: 896 |
| GC content: 42.9% |
| Sense strand siRNA: UUCCAGUUGACAUCAGAGGtt **[SEQ ID NO. 925]** |
| Antisense strand siRNA: CCUCUGAUGUCAACUGGAAtt **[SEQ ID NO. 926]** |
| **Target sequence 57:** AAAGTTCTAGATGGTCTAAAC **[SEQ ID NO. 927]** |
| Position in gene sequence: 922 |
| GC content: 33.3% |
| Sense strand siRNA: AGUUCUAGAUGGUCUAAACtt **[SEQ ID NO. 928]** |
| Antisense strand siRNA: GUUUAGACCAUCUAGAACUtt **[SEQ ID NO. 929]** |
| **Target sequence 58:** AAAC901AGAAATTATCGATA **[SEQ ID NO. 930]** |
| Position in gene sequence: 939 |
| GC content: 19% |
| Sense strand siRNA: AC901AGAAAUUAUCGAUAtt **[SEQ ID NO. 931]** |
| Antisense strand siRNA: UAUCGAUAAUUUCU109GUtt **[SEQ ID NO. 932]** |
| **Target sequence 59:** AAATTATCGATATGTTGTCAT **[SEQ ID NO. 933]** |
| Position in gene sequence: 948 |
| GC content: 23.8% |
| Sense strand siRNA: AUUAUCGAUAUGUUGUCAUtt **[SEQ ID NO. 934]** |
| Antisense strand siRNA: AUGACAACAUAUCGAUAAUtt **[SEQ ID NO. 935]** |

| **Table 6: 3-beta-Hydroxysteroiddehydrogenase** |
|---|
| **3-beta-Hydroxysteroiddehydrogenase-DEHUHS (gi: 65959) [SEQ ID NO. 936]** |
| 1 atgacgggct ggagctgcct tgtgacagga gcaggagggt ttctgggaca gaggatcatc 61 cgcctcttgg tgaaggagaa ggagctgaag gagatcaggg tcttggacaa ggccttcgga 121 ccagaattga gagaggaatt ttctaaactc cagaacaaga ccaagctgac agtgctggaa 181 ggagacattc tggatgagcc attcctgaag agagcctgcc aggacgtctc ggtcatcatc 241 cacaccgcct gtatcattga tgtcttcggt gtcactcaca gagagtctat catgaatgtc 301 aatgtgaaag gtacccagct cctgttagag gcctgtgtcc aagctagtgt gccagtcttc 361 atctacacca gtagcataga ggtagccggg cccaactcct acaaggaaat catccagaat 421 ggccatgaag aagagcctct ggaaaacaca tggcccgctc catacccaca cagcaaaaag 481 cttgctgaga aggctgtact ggcggctaac gggtggaatc tgaaaaacgg cggcaccctg 541 tacacttgtg ccttacgacc catgtatatc tatggggaag gaagccgatt cctttctgct 601 agtataaacg aggccctgaa caacaatggg atcctgtcaa gtgttggaaa gttctccact 661 gttaacccag tctatgttgg caatgtggcc tgggcccaca ttctggcctt gagggccctg 721 caggacccca agaaggcccc aagcatccga ggacagttct actatatctc agatgacacg 781 cctcaccaaa gctatgataa ccttaattac accctgagca aagagttcgg cctccgcctt 841 gattccagat ggagctttcc tttatccctg atgtattgga ttggcttcct gctggaaata 901 gtgagcttcc tactcaggcc aatttacacc tatcgaccgc ccttcaaccg ccacatagtc 961 acattgtcaa atagcgtatt caccttctct tataagaagg ctcagcgaga tctggcgtat 1021 aagccactct acagctggga ggaagccaag cagaaaacgg tggagtgggt tggttccctt 1081 gtggaccggc acaaggagac cctgaagtcc aagactcagt ga |
| **Target sequence 1:** AAGGAGAAGGAGCTGAAGGAG **[SEQ ID NO. 937]** |
| Position in gene sequence: 75 |
| GC content: 52.4% |
| Sense strand siRNA: GGAGAAGGAGCUGAAGGAGtt **[SEQ ID NO. 938]** |
| Antisense strand siRNA: CUCCUUCAGCUCCUUCUCCtt **[SEQ ID NO. 939]** |
| **Target sequence 2:** AAGGAGCTGAAGGAGATCAGG **[SEQ ID NO. 940]** |
| Position in gene sequence: 81 |
| GC content: 52.4% |
| Sense strand siRNA: GGAGCUGAAGGAGAUCAGGtt **[SEQ ID NO. 941]** |
| Antisense strand siRNA: CCUGAUCUCCUUCAGCUCCtt **[SEQ ID NO. 942]** |
| **Target sequence 3:** AAGGAGATCAGGGTCTTGGAC **[SEQ ID NO. 943]** |
| Position in gene sequence: 90 |
| GC content: 52.4% |
| Sense strand siRNA: GGAGAUCAGGGUCUUGGACtt **[SEQ ID NO. 944]** |
| Antisense strand siRNA: GUCCAAGACCCUGAUCUCCtt **[SEQ ID NO. 945]** |
| **Target sequence 4:** AAGGCCTTCGGA121CCAGAA **[SEQ ID NO. 946]** |
| Position in gene sequence: 111 |
| GC content: 47.6% |
| Sense strand siRNA: GGCCUUCGGA121CCAGAAtt **[SEQ ID NO. 947]** |
| Antisense strand siRNA: UUCUGG121UCCGAAGGCCtt **[SEQ ID,NO. 948]** |
| **Target sequence 5:** AATTGAGAGAGGAATTTTCTA **[SEQ ID NO. 949]** |
| Position in gene sequence: 130 |
| GC content: 28.6% |
| Sense strand siRNA: UUGAGAGAGGAAUUUUCUAtt **[SEQ ID NO. 950]** |
| Antisense strand siRNA: UAGAAAAUUCCUCUCUCAAtt **[SEQ ID NO. 951]** |
| **Target sequence 6:** AATTTTCTAAACTCCAGAACA **[SEQ ID NO. 952]** |
| Position in gene sequence: 142 |
| GC content: 28.6% |
| Sense strand siRNA: UUUUCUAAACUCCAGAACAtt **[SEQ ID NO. 953]** Antisense strand siRNA: UGUUCUGGAGUUUAGAAAAtt **[SEQ ID NO. 954]** |
| **Target sequence 7:** AAACTCCAGAACAAGACCAAG **[SEQ ID NO. 955]** |
| Position in gene sequence: 150 |
| GC content: 42.9% |
| Sense strand siRNA: ACUCCAGAACAAGACCAAGtt **[SEQ ID NO. 956]** |
| Antisense strand siRNA: CUUGGUCUUGUUCUGGAGUtt **[SEQ ID NO. 957]** |
| **Target sequence 8:** AACAAGACCAAGCTGACAGTG **[SEQ ID NO. 958]** |
| Position in gene sequence: 159 |
| GC content: 47.6% |
| Sense strand siRNA: CAAGACCAAGCUGACAGUGtt **[SEQ ID NO. 959]** |
| Antisense strand siRNA: CACUGUCAGCUUGGUCUUGtt **[SEQ ID NO. 960]** |
| **Target sequence 9:** AAGACCAAGCTGACAGTGCTG **[SEQ ID NO. 961]** |
| Position in gene sequence: 162 |
| GC content: 52.4% |
| Sense strand siRNA: GACCAAGCUGACAGUGCUGtt **[SEQ ID NO. 962]** |
| Antisense strand siRNA: CAGCACUGUGAGCUUGGUCtt **[SEQ ID NO. 963]** |
| **Target sequence 10:** AAGCTGACAGTGCTGGAA181 **[SEQ ID NO. 964]** |
| Position in gene sequence: 168 |
| GC content: 42.9% |
| Sense strand siRNA: GCUGACAGUGCUGGAA181tt **[SEQ ID NO. 965]** |
| Antisense strand siRNA: 181UUCCAGCACUGUCAGCtt **[SEQ ID NO. 966]** |
| **Target sequence 11:** AA181GGAGACATTCTGGATG **[SEQ ID NO. 967]** |
| Position in gene sequence: 184 |
| GC content: 38.1% |
| Sense strand siRNA: 181GGAGACAUUCUGGAUGtt **[SEQ ID NO. 968]** |
| Antisense strand siRNA: CAUCCAGAAUGUCUCC181tt **[SEQ ID NO. 969]** |
| **Target sequence 12:** AAGAGAGCCTGCCAGGACGTC **[SEQ ID NO. 970]** |
| Position in gene sequence: 216 |
| GC content: 61.9% |
| Sense strand siRNA: GAGAGCCUGCCAGGACGUCtt **[SEQ ID NO. 971]** |
| Antisense strand siRNA: GACGUCCUGGCAGGCUCUCtt **[SEQ ID NO. 972]** |
| **Target sequence 13:** AATGTC301AATGTGAAAGGT **[SEQ ID NO. 973]** |
| Position in gene sequence: 306 |
| GC content: 28.6% |
| Sense strand siRNA: UGUC301AAUGUGAAAGGUtt **[SEQ ID NO. 974]** |
| Antisense strand siRNA: ACCUUUCACAUU103GACAtt **[SEQ ID NO. 975]** |
| **Target sequence 14:** AATGTGAAAGGTACCCAGCTC **[SEQ ID NO. 976]** |
| Position in gene sequence: 315 |
| GC content: 47.6% |
| Sense strand siRNA: UGUGAAAGGUACCCAGCUCtt **[SEQ ID NO. 977]** |
| Antisense strand siRNA: GAGCUGGGUACCUUUCACAtt **[SEQ ID NO. 978]** |
| **Target sequence 15:** AAAGGTACCCAGCTCCTGTTA **[SEQ ID NO. 979]** |
| Position in gene sequence: 321 |
| GC content: 47.6% |
| Sense strand siRNA: AGGUACCCAGCUCCUGUUAtt **[SEQ ID NO. 980]** |
| Antisense strand siRNA: UAACAGGAGCUGGGUACCUtt **[SEQ ID NO. 981]** |
| **Target sequence 16:** AAGCTAGTGTGCCAGTCTTC3 **[SEQ ID NO. 982]** |
| Position in gene sequence: 355 |
| GC content: 47.6% |
| Sense strand siRNA: GCUAGUGUGCCAGUCUUC3tt **[SEQ ID NO. 983]** |
| Antisense strand siRNA: 3GAAGACUGGCACACUAGCtt **[SEQ ID NO. 984]** |
| **Target sequence 17:** AACTCCTACAAGGAAATCATC **[SEQ ID NO. 985]** |
| Position in gene sequence: 411 |
| GC content: 38.1% |
| Sense strand siRNA: CUCCUACAAGGAAAUCAUCtt **[SEQ ID NO. 986]** |
| Antisense strand siRNA: GAUGAUUUCCUUGUAGGAGtt **[SEQ ID NO. 987]** |
| **Target sequence 18:** AAGGAAATCATCCAGAAT421 **[SEQ ID NO. 988]** |
| Position in gene sequence: 420 |
| GC content: 28.6% |
| Sense strand siRNA: GGAAAUCAUCCAGAAU421tt **[SEQ ID NO. 989]** |
| Antisense strand siRNA: 124AUUCUGGAUGAUUUCCtt **[SEQ ID NO. 990]** |
| **Target sequence 19:** AAATCATCCAGAAT421 GGCC **[SEQ ID NO. 991]** |
| Position in gene sequence: 424 |
| GC content: 38.1% |
| Sense strand siRNA: AUCAUCCAGAAU421 GGCCtt **[SEQ ID NO. 992]** |
| Antisense strand siRNA: GGCC124AUUCUGGAUGAUtt **[SEQ ID NO. 993]** |
| **Target sequence 20:** AAT421 GGCCATGAAGAAGAG **[SEQ ID NO. 994]** |
| Position in gene sequence: 435 |
| GC content: 38.1% |
| Sense strand siRNA: U421GGCCAUGAAGAAGAGtt **[SEQ ID NO. 995]** |
| Antisense strand siRNA: CUCUUCUUCAUGGCC124Att **[SEQ ID NO. 996]** |
| **Target sequence 21:** AAGAAGAGCCTCTGGAAAACA **[SEQ ID NO. 997]** |
| Position in gene sequence: 448 |
| GC content: 42.9% |
| Sense strand siRNA: GAAGAGCCUCUGGAAAACAtt **[SEQ ID NO. 998]** |
| Antisense strand siRNA: UGUUUUCCAGAGGCUCUUCtt **[SEQ ID NO. 999]** |
| **Target sequence 22:** AAGAGCCTCTGGAAAACACAT **[SEQ ID NO. 1000]** |
| Position in gene sequence: 451 |
| GC content: 42.9% |
| Sense strand siRNA: GAGCCUCUGGAAAACACAUtt **[SEQ ID NO. 1001]** |
| Antisense strand siRNA: AUGUGUUUUCCAGAGGCUCtt **[SEQ ID NO. 1002]** |
| **Target sequence 23:** AAAACACATGGCCCGCTCCAT **[SEQ ID NO. 1003]** |
| Position in gene sequence: 463 |
| GC content: 52.4% |
| Sense strand siRNA: AACACAUGGCCCGCUCCAUtt **[SEQ ID NO. 1004]** |
| Antisense strand siRNA: AUGGAGCGGGCCAUGUGUUtt **[SEQ ID NO. 1005]** |
| **Target sequence 24:** AACACATGGCCCGCTCCATAC **[SEQ ID NO. 1006]** |
| Position in gene sequence: 465 |
| GC content: 57.1% |
| Sense strand siRNA: CACAUGGCCCGCUCCAUACtt **[SEQ ID NO. 1007]** |
| Antisense strand siRNA: GUAUGGAGCGGGCCAUGUGtt **[SEQ ID NO.1008]** |
| **Target sequence 25:** AAAAAG481CTTGCTGAGAAG **[SEQ ID NO. 1009]** |
| Position in gene sequence: 495 |
| GC content: 33.3% |
| Sense strand siRNA: AAAG481CUUGCUGAGAAGtt **[SEQ ID NO. 1010]** |
| Antisense strand siRNA: CUUCUCAGCAAG184CUUUtt **[SEQ ID NO. 1011]** |
| **Target sequence 26:** AAAG481CTTGCTGAGAAGGC **[SEQ ID NO.1012]** |
| Position in gene sequence: 497 |
| GC content: 42.9% |
| Sense strand siRNA: AG481CUUGCUGAGAAGGCtt **[SEQ ID NO. 1013]** |
| Antisense strand siRNA: GCCUUCUCAGCAAG184CUtt **[SEQ ID NO. 1014]** |
| **Target sequence 27:** AAGGCTGTACTGGCGGCTAAC **[SEQ ID NO. 1015]** |
| Position in gene sequence: 513 |
| GC content: 57.1% |
| Sense strand siRNA: GGCUGUACUGGCGGCUAACtt **[SEQ ID NO. 1016]** |
| Antisense strand siRNA: GUUAGCCGCCAGUACAGCCtt **[SEQ ID NO. 1017]** |
| **Target sequence 28:** AACGGGTGGAATCTGAAAAAC **[SEQ ID NO.1018]** |
| Position in gene sequence: 531 |
| GC content: 42.9% |
| Sense strand siRNA: CGGGUGGAAUCUGAAAAACtt **[SEQ ID NO. 1019]** |
| Antisense strand siRNA: GUUUUUCAGAUUCCACCCGtt **[SEQ ID NO. 1020]** |
| **Target sequence 29:** AATCTGAAAAACGGCGGCACC **[SEQ ID NO. 1021]** |
| Position in gene sequence: 540 |
| GC content: 52.4% |
| Sense strand siRNA: UCUGAAAAACGGCGGCACCtt **[SEQ ID NO. 1022]** |
| Antisense strand siRNA: GGUGCCGCCGUUUUUCAGAtt **[SEQ ID NO. 1023]** |
| **Target sequence 30:** AAAAACGGCGGCACCCTG541 **[SEQ ID NO. 1024]** |
| Position in gene sequence: 546 |
| GC content: 52.4% |
| Sense strand siRNA: AAACGGCGGCACCCUG541tt **[SEQ ID NO. 1025]** |
| Antisense strand siRNA: 145CAGGGUGCCGCCGUUUtt **[SEQ ID NO.1026]** |
| **Target sequence 31:** AAACGGCGGCACCCTG541TA **[SEQ ID NO. 1027]** |
| Position in gene sequence: 548 |
| GC content: 52.4% |
| Sense strand siRNA: ACGGCGGCACCCUG541UAtt **[SEQ ID NO. 1028]** |
| Antisense strand siRNA: UA145CAGGGUGCCGCCGUtt **[SEQ ID NO. 1029]** |
| **Target sequence 32:** AAGGAAGCCGATTCCTTTCTG **[SEQ ID NO. 1030]** |
| Position in gene sequence: 604 |
| GC content: 47.6% |
| Sense strand siRNA: GGAAGCCGAUUCCUUUCUGtt **[SEQ ID NO. 1031]** |
| Antisense strand siRNA: CAGAAAGGAAUCGGCUUCCtt **[SEQ ID NO. 1032]** |
| **Target sequence 33:** AAGCCGATTCCTTTCTGCT60 **[SEQ ID NO. 1033]** |
| Position in gene sequence: 608 |
| GC content: 42.9% |
| Sense strand siRNA: GCCGAUUCCUUUCUGCU60tt **[SEQ ID NO. 1034]** |
| Antisense strand siRNA: 06AGCAGAAAGGAAUCGGCtt **[SEQ ID NO. 1035]** |
| **Target sequence 34:** AAACGAGGCCCTGAACAACAA **[SEQ ID NO. 1036]** |
| Position in gene sequence: 635 |
| GC content: 47.6% |
| Sense strand siRNA: ACGAGGCCCUGAACAACAAtt **[SEQ ID NO. 1037]** |
| Antisense strand siRNA: UUGUUGUUCAGGGCCUCGUtt **[SEQ ID NO. 1038]** |
| **Target sequence 35:** AACAACAATGGGATCCTGTCA **[SEQ ID NO. 1039]** |
| Position in gene sequence: 648 |
| GC content: 42.9% |
| Sense strand siRNA: CAACAAUGGGAUCCUGUCAtt **[SEQ ID NO. 1040]** |
| Antisense strand siRNA: UGACAGGAUCCCAUUGUUGtt **[SEQ ID NO. 1041]** |
| **Target sequence 36:** AACAATGGGATCCTGTCAAGT **[SEQ ID NO. 1042]** |
| Position in gene sequence: 651 |
| GC content: 42.9% |
| Sense strand siRNA: CAAUGGGAUCCUGUCAAGUtt **[SEQ ID NO. 1043]** |
| Antisense strand siRNA: ACUUGACAGGAUCCCAUUGtt **[SEQ ID NO. 1044]** |
| **Target sequence 37:** AATGGGATCCTGTCAAGTGTT **[SEQ ID NO. 1045]** |
| Position in gene sequence: 654 |
| GC content: 42.9% |
| Sense strand siRNA: UGGGAUCCUGUCAAGUGUUtt **[SEQ ID NO. 1046]** |
| Antisense strand siRNA: AACACUUGACAGGAUCCCAtt **[SEQ ID NO. 1047]** |
| **Target sequence 38:** AAGTGTTGGAAAGTTCTCCAC **[SEQ ID NO. 1048]** |
| Position in gene sequence: 668 |
| GC content: 42.9% |
| Sense strand siRNA: GUGUUGGAAAGUUCUCCACtt **[SEQ ID NO. 1049]** |
| Antisense strand siRNA: GUGGAGAACUUUCCAACACtt **[SEQ ID NO. 1050]** |
| **Target sequence 39:** AAAGTTCTCCACT661GTTAA **[SEQ ID NO. 1051]** |
| Position in gene sequence: 677 |
| GC content: 28.6% |
| Sense strand siRNA: AGUUCUCCACU661GUUAAtt **[SEQ ID NO. 1052]** |
| Antisense strand siRNA: UUAAC166AGUGGAGAACUtt **[SEQ ID NO. 1053]** |
| **Target sequence 40:** AACCCAGTCTATGTTGGCAAT **[SEQ ID NO. 1054]** |
| Position in gene sequence: 696 |
| GC content: 42.9% |
| Sense strand siRNA: CCCAGUCUAUGUUGGCAAUtt **[SEQ ID NO. 1055]** |
| Antisense strand siRNA: AUUGCCAACAUAGACUGGGtt **[SEQ ID NO. 1056]** |
| **Target sequence 41:** AATGTGGCCTGGGCCCACATT **[SEQ ID NO. 1057]** |
| Position in gene sequence: 714 |
| GC content: 57.1% |
| Sense strand siRNA: UGUGGCCUGGGCCCACAUUtt **[SEQ ID NO. 1058]** |
| Antisense strand siRNA: AAUGUGGGCCCAGGCCACAtt **[SEQ ID NO. 1059]** |
| **Target sequence 42:** AAGAAGGCCCCAAGCATCCGA **[SEQ ID NO. 1060]** |
| Position in gene sequence: 765 |
| GC content: 57.1% |
| Sense strand siRNA: GAAGGCCCCAAGCAUCCGAtt **[SEQ ID NO. 1061]** |
| Antisense strand siRNA: UCGGAUGCUUGGGGCCUUCtt **[SEQ ID NO. 1062]** |
| **Target sequence 43:** AAGGCCCCAAGCATCCGAGGA **[SEQ ID NO. 1063]** |
| Position in gene sequence: 768 |
| GC content: 61.9% |
| Sense strand siRNA: GGCCCCAAGCAUCCGAGGAtt **[SEQ ID NO. 1064]** |
| Antisense strand siRNA: UCCUCGGAUGCUUGGGGCCtt **[SEQ ID NO. 1065]** |
| **Target sequence 44:** AAGCATCCGAGGACAGTTCTA **[SEQ ID NO. 1066]** |
| Position in gene sequence: 776 |
| GC content: 47.6% |
| Sense strand siRNA: GCAUCCGAGGACAGUUCUAtt **[SEQ ID NO. 1067]** |
| Antisense strand siRNA: UAGAACUGUCCUCGGAUGCtt **[SEQ ID NO. 1068]** |
| **Target sequence 45:** AAAGCTATGATAACCTTAATT **[SEQ ID NO. 1069]** |
| Position in gene sequence: 826 |
| GC content: 23.8% |
| Sense strand siRNA: AGCUAUGAUAACCUUAAUUtt **[SEQ ID NO. 1070]** |
| Antisense strand siRNA: AAUUAAGGUUAUCAUAGCUtt **[SEQ ID NO. 1071]** |
| **Target sequence 46:** AACCTTAATTACACCCTGAGC **[SEQ ID NO. 1072]** |
| Position in gene sequence: 837 |
| GC content: 42.9% |
| Sense strand siRNA: CCUUAAUUACACCCUGAGCtt **[SEQ ID NO. 1073]** |
| Antisense strand siRNA: GCUCAGGGUGUAAUUAAGGtt **[SEQ ID NO. 1074]** |
| **Target sequence 47:** AATTACACCCTGAGCAAAGAG **[SEQ ID NO. 1075]** |
| Position in gene sequence: 843 |
| GC content: 42.9% |
| Sense strand siRNA: UUACACCCUGAGCAAAGAGtt **[SEQ ID NO. 1076]** |
| Antisense strand siRNA: CUCUUUGCUCAGGGUGUAAtt **[SEQ ID NO. 1077]** |
| **Target sequence 48:** AAAGAGTTCGGCCTCCGCCTT **[SEQ ID NO. 1078]** |
| Position in gene sequence: 858 |
| GC content: 57.1% |
| Sense strand siRNA: AGAGUUCGGCCUCCGCCUUtt **[SEQ ID NO. 1079]** |
| Antisense strand siRNA: AAGGCGGAGGCCGAACUCUtt **[SEQ ID NO. 1080]** |
| **Target sequence 49:** AAATA901GTGAGCTTCCTAC **[SEQ ID NO. 1081]** |
| Position in gene sequence: 937 |
| GC content: 33.3% |
| Sense strand siRNA: AUA901GUGAGCUUCCUACtt **[SEQ ID NO. 1082]** |
| Antisense strand siRNA: GUAGGAAGCUCAC109UAUtt **[SEQ ID NO. 1083]** |
| **Target sequence 50:** AATTTACACCTATCGACCGCC **[SEQ ID NO. 1084]** |
| Position in gene sequence: 965 |
| GC content: 47.6% |
| Sense strand siRNA: UUUACACCUAUCGACCGCCtt **[SEQ ID NO.1085]** |
| Antisense strand siRNA: GGCGGUCGAUAGGUGUAAAtt **[SEQ ID NO._{.} 1086]** |
| **Target sequence 51:** AACCGCCACATAGTC961ACA **[SEQ ID NO.1087]** |
| Position in gene sequence: 990 |
| GC content: 42.9% |
| Sense strand siRNA: CCGCCACAUAGUC961ACAtt **[SEQ ID NO. 1088]** |
| Antisense strand siRNA: UGU169GACUAUGUGGCGGtt **[SEQ ID NO. 1089]** |
| **Target sequence 52:** AAATAGCGTATTCACCTTCTC **[SEQ ID NO. 1090]** |
| Position in gene sequence: 1016 |
| GC content: 38.1% |
| Sense strand siRNA: AUAGCGUAUUCACCUUCUCtt **[SEQ ID NO. 1091]** |
| Antisense strand siRNA: GAGAAGGUGAAUACGCUAUtt **[SEQ ID NO. 1092]** |
| **Target sequence 53:** AAGAAGGCTCAGCGAGATCTG **[SEQ ID NO. 1093]** |
| Position in gene sequence: 1041 |
| GC content: 52.4% |
| Sense strand siRNA: GAAGGCUCAGCGAGAUCUGtt **[SEQ ID NO. 1094]** |
| Antisense strand siRNA: CAGAUCUCGCUGAGCCUUCtt **[SEQ ID NO. 1095]** |
| **Target sequence 54:** AAGGCTCAGCGAGATCTGGCG **[SEQ ID NO. 1096]** |
| Position in gene sequence: 1044 |
| GC content: 61.9% |
| Sense strand siRNA: GGCUCAGCGAGAUCUGGCGtt **[SEQ ID NO. 1097]** |
| Antisense strand siRNA: CGCCAGAUCUCGCUGAGCCtt **[SEQ ID NO. 1098]** |
| **Target sequence 55:** AAGCCACTCTACAGCTGGGAG **[SEQ ID NO. 1099]** |
| Position in gene sequence: 1072 |
| GC content: 57.1% |
| Sense strand siRNA: GCCACUCUACAGCUGGGAGtt **[SEQ ID NO. 1100]** |
| Antisense strand siRNA: CUCCCAGCUGUAGAGUGGCtt **[SEQ ID NO. 1101]** |
| **Target sequence 56:** AAGCCAAGCAGAAAACGGTGG **[SEQ ID NO. 1102]** |
| Position in gene sequence: 1094 |
| GC content: 52.4% |
| Sense strand siRNA: GCCAAGCAGAAAACGGUGGtt **[SEQ ID NO. 1103]** |
| Antisense strand siRNA: CCACCGUUUUCUGCUUGGCtt **[SEQ ID NO. 1104]** |
| **Target sequence 57:** AAGCAGAAAACGGTGGAGTGG **[SEQ ID NO. 1105]** |
| Position in gene sequence: 1099 |
| GC content: 52.4% |
| Sense strand siRNA: GCAGAAAACGGUGGAGUGGtt **[SEQ ID NO. 1106]** |
| Antisense strand siRNA: CCACUCCACCGUUUUCUGCtt **[SEQ ID NO. 1107]** |
| **Target sequence 58:** AAAACGGTGGAGTGGGTTGGT **[SEQ ID NO. 1108]** |
| Position in gene sequence: 1105 |
| GC content: 52.4% |
| Sense strand siRNA: AACGGUGGAGUGGGUUGGUtt **[SEQ ID NO. 1109]** |
| Antisense strand siRNA: ACCAACCCACUCCACCGUUtt **[SEQ ID NO. 1110]** |
| **Target sequence 59:** AACGGTGGAGTGGGTTGGTTC **[SEQ ID NO. 1111]** |
| Position in gene sequence: 1107 |
| GC content: 57.1% |
| Sense strand siRNA: CGGUGGAGUGGGUUGGUUCtt **[SEQ ID NO. 1112]** |
| Antisense strand siRNA: GAACCAACCCACUCCACCGtt **[SEQ ID NO. 1113]** |
| **Target sequence 60:** AAGGAGACCCTGAAGTCCAAG **[SEQ ID NO. 1114]** |
| Position in gene sequence: 1148 |
| GC content: 52.4% |
| Sense strand siRNA: GGAGACCCUGAAGUCCAAGtt **[SEQ ID NO. 1115]** |
| Antisense strand siRNA: CUUGGACUUCAGGGUCUCCtt **[SEQ ID NO. 1116]** |

| **Table 7: 3-beta-Hydroxysteroiddehydrogenase-4-5-isomerase** |
|---|
| **3-beta-Hydroxysteroiddehydrogenase-4-5-isomerase - S45679 (gi: 257052) [SEQ ID NO. 1117]** |
| 1 atgacgggct ggagctgcct tgtgacagga gcaggagggt ttctgggaca gaggatcatc 61 cgcctcttgg tgaaggagaa ggagctgaag gagatcaggg tcttggacaa ggccttcgga 121 ccagaattga gagaggaatt ttctaaactc cagaacaaga ccaagctgac agtgctggaa 181 ggagacattc tggatgagcc attcctgaag agagcctgcc aggacgtctc ggtcatcatc 241 cacaccgcct gtatcattga tgtcttcggt gtcactcaca gagagtctat catgaatgtc 301 aatgtgaaag gtacccagct cctgttagag gcctgtgtcc aagctagtgt gccagtcttc 361 atctacacca gtagcataga ggtagccggg cccaactcct acaaggaaat catccagaat 421 ggccatgaag aagagcctct ggaaaacaca tggcccgctc catacccaca cagcaaaaag 481 cttgctgaga aggctgtact ggcggctaac gggtggaatc tgaaaaacgg cggcaccctg 541 tacacttgtg ccttacgacc catgtatatc tatggggaag gaagccgatt cctttctgct 601 agtataaacg aggccctgaa caacaatggg atcctgtcaa gtgttggaaa gttctccact 661 gttaacccag tctatgttgg caatgtggcc tgggcccaca ttctggcctt gagggccctg 721 caggacccca agaaggcccc aagcatccga ggacagttct actatatctc agatgacacg 781 cctcaccaaa gctatgataa ccttaattac accctgagca aagagttcgg cctccgcctt 841 gattccagat ggagctttcc tttatccctg atgtattgga ttggcttcct gctggaaata 901 gtgagcttcc tactcaggcc aatttacacc tatcgaccgc ccttcaaccg ccacatagtc 961 acattgtcaa atagcgtatt caccttctct tataagaagg ctcagcgaga tctggcgtat 1021 aagccactct acagctggga ggaagccaag cagaaaacgg tggagtgggt tggttccctt 1081 gtggaccggc acaaggagac cctgaagtcc aagactcagt ga |
| **Target sequence 1:** AAGGAGAAGGAGCTGAAGGAG **[SEQ ID NO. 1118]** |
| Position in gene sequence: 76 |
| GC content: 52,4% |
| Sense strand siRNA: GGAGAAGGAGCUGAAGGAGtt **[SEQ ID NO. 1119]** |
| Antisense strand siRNA: CUCCUUCAGCUCCUUCUCCtt **[SEQ ID NO. 1120]** |
| **Target sequence 2:** AAGGAGCTGAAGGAGATCAGG **[SEQ ID NO. 1121]** |
| Position in gene sequence: 82 |
| GC content: 52.4% |
| Sense strand siRNA: GGAGCUGAAGGAGAUCAGGtt **[SEQ ID NO. 1122]** |
| Antisense strand siRNA: CCUGAUCUCCUUCAGCUCCtt **[SEQ ID NO. 1123]** |
| **Target sequence 3:** AAGGAGATCAGGGTCTTGGAC **[SEQ ID NO. 1124]** |
| Position in gene sequence: 91 |
| GC content: 52.4% |
| Sense strand siRNA: GGAGAUCAGGGUCUUGGACtt **[SEQ ID NO. 1125]** |
| Antisense strand siRNA: GUCCAAGACCCUGAUCUCCtt **[SEQ ID NO. 1126]** |
| **Target sequence 4:** AAGGCCTTCGGA121CCAGAA **[SEQ ID NO. 1127]** |
| Position in gene sequence: 112 |
| GC content: 47.6% |
| Sense strand siRNA: GGCCUUCGGA121CCAGAAtt **[SEQ ID NO. 1128]** |
| Antisense strand siRNA: UUCUGG121UCCGAAGGCCtt **[SEQ ID NO. 1129]** |
| **Target sequence 5:** AATTGAGAGAGGAATTTTCTA **[SEQ ID NO. 1130]** |
| Position in gene sequence: 131 |
| GC content: 28.6% |
| Sense strand siRNA: UUGAGAGAGGAAUUUUCUAtt **[SEQ ID NO. 1131]** |
| Antisense strand siRNA: UAGAAAAUUCCUCUCUCAAtt **[SEQ ID NO. 1132]** |
| **Target sequence 6:** AATTTTCTAAACTCCAGAACA **[SEQ ID NO. 1133]** |
| Position in gene sequence: 143 |
| GC content: 28.6% |
| Sense strand siRNA: UUUUCUAAACUCCAGAACAtt **[SEQ ID NO. 1134]** |
| Antisense strand siRNA: UGUUCUGGAGUUUAGAAAAtt **[SEQ ID NO. 1135]** |
| **Target sequence 7:** AAACTCCAGAACAAGACCAAG **[SEQ ID NO. 1136]** |
| Position in gene sequence: 151 |
| GC content: 42.9% |
| Sense strand siRNA: ACUCCAGAACAAGACCAAGtt **[SEQ ID NO. 1137]** |
| Antisense strand siRNA: CUUGGUCUUGUUCUGGAGUtt **[SEQ ID NO. 1138]** |
| **Target sequence 8:** AACAAGACCAAGCTGACAGTG **[SEQ ID NO. 1139]** |
| Position in gene sequence: 160 |
| GC content: 47.6% |
| Sense strand siRNA: CAAGACCAAGCUGACAGUGtt **[SEQ ID NO. 1140]** |
| Antisense strand siRNA: CACUGUCAGCUUGGUCUUGtt **[SEQ ID NO. 1141]** |
| **Target sequence 9:** AAGACCAAGCTGACAGTGCTG **[SEQ ID NO. 1142]** |
| Position in gene sequence: 163 |
| GC content: 52.4% |
| Sense strand siRNA: GACCAAGCUGACAGUGCUGtt **[SEQ ID NO. 1143]** |
| Antisense strand siRNA: CAGCACUGUCAGCUUGGUCtt **[SEQ ID NO. 1144]** |
| **Target sequence 10:** AAGCTGACAGTGCTGGAA181 **[SEQ ID NO. 1145]** |
| Position in gene sequence: 169 |
| GC content: 42.9% |
| Sense strand siRNA: GCUGACAGUGCUGGAA181tt **[SEQ ID NO. 1146]** |
| Antisense strand siRNA: 181UUCCAGCACUGUCAGCtt **[SEQ ID NO. 1147]** |
| **Target sequence 11:** AA181GGAGACATTCTGGATG **[SEQ ID NO. 1148]** |
| Position in gene sequence: 185 |
| GC content: 38.1% |
| Sense strand siRNA: 181GGAGACAUUCUGGAUGtt **[SEQ ID NO. 1149]** |
| Antisense strand siRNA: CAUCCAGAAUGUCUCC181tt **[SEQ ID NO. 1150]** |
| **Target sequence 12:** AAGAGAGCCTGCCAGGACGTC **[SEQ ID NO. 1151]** |
| Position in gene sequence: 217 |
| GC content: 61.9% |
| Sense strand siRNA: GAGAGCCUGCCAGGACGUCtt **[SEQ ID NO. 1152]** |
| Antisense strand siRNA: GACGUCCUGGCAGGCUCUCtt **[SEQ ID NO. 1153]** |
| **Target sequence 13:** AATGTC301AATGTGAAAGGT **[SEQ ID NO. 1154]** |
| Position in gene sequence: 307 |
| GC content: 28.6% |
| Sense strand siRNA: UGUC301AAUGUGAAAGGUtt **[SEQ ID NO. 1155]** |
| Antisense strand siRNA: ACCUUUCACAUU103GACAtt **[SEQ ID NO. 1156]** |
| **Target sequence 14:** AATGTGAAAGGTACCCAGCTC **[SEQ ID NO. 1157]** |
| Position in gene sequence: 316 |
| GC content: 47.6% |
| Sense strand siRNA: UGUGAAAGGUACCCAGCUCtt **[SEQ ID NO. 1158]** |
| Antisense strand siRNA: GAGCUGGGUACCUUUCACAtt **[SEQ ID NO. 1159]** |
| **Target sequence 15:** AAAGGTACCCAGCTCCTGTTA **[SEQ ID NO. 1160]** |
| Position in gene sequence: 322_{.} |
| GC content: 47.6% |
| Sense strand siRNA: AGGUACCCAGCUCCUGUUAtt **[SEQ ID NO. 1161]** |
| Antisense strand siRNA: UAACAGGAGCUGGGUACCUtt **[SEQ ID NO. 1162]** |
| **Target sequence 16:** AAGCTAGTGTGCCAGTCTTC3 **[SEQ ID NO. 1163]** |
| Position in gene sequence: 356 |
| GC content: 47.6% |
| Sense strand siRNA: GCUAGUGUGCCAGUCUUC3tt **[SEQ ID NO. 1164]** |
| Antisense strand siRNA: 3GAAGACUGGCACACUAGCtt **[SEQ ID NO. 1165]** |
| **Target sequence 17:** AACTCCTACAAGGAAATCATC **[SEQ ID NO. 1166]** |
| Position in gene sequence: 412 |
| GC content: 38.1% |
| Sense strand siRNA: CUCCUACAAGGAAAUCAUCtt **[SEQ ID NO. 1167]** |
| Antisense strand siRNA: GAUGAUUUCCUUGUAGGAGtt **[SEQ ID NO.1168]** |
| **Target sequence 18:** AAGGAAATCATCCAGAAT421 **[SEQ ID NO. 1169]** |
| Position in gene sequence: 421 |
| GC content: 28.6% |
| Sense strand siRNA: GGAAAUCAUCCAGAAU421tt **[SEQ ID NO. 1170]** |
| Antisense strand siRNA: 124AUUCUGGAUGAUUUCCtt **[SEQ ID NO. 1171]** |
| **Target sequence 19:** AAATCATCCAGAAT421 GGCC **[SEQ ID NO. 1172]** |
| Position in gene sequence: 425 |
| GC content: 38.1% |
| Sense strand siRNA: AUCAUCCAGAAU421GGCCtt **[SEQ ID NO. 1173]** |
| Antisense strand siRNA: GGCC124AUUCUGGAUGAUtt **[SEQ ID NO. 1174]** |
| **Target sequence** 20: AAT421GGCCATGAAGAAGAG **[SEQ ID NO. 1175]** |
| Position in gene sequence: 436 |
| GC content: 38.1% |
| Sense strand siRNA: U421GGCCAUGAAGAAGAGtt **[SEQ ID NO.1176]** |
| Antisense strand siRNA: CUCUUCUUCAUGGCC124Att **[SEQ ID NO. 1177]** |
| **Target sequence 21:** AAGAAGAGCCTCTGGAAAACA **[SEQ ID NO. 1178]** |
| Position in gene sequence: 449 |
| GC content: 42.9% |
| Sense strand siRNA: GAAGAGCCUCUGGAAAACAtt **[SEQ ID NO. 1179]** |
| Antisense strand siRNA: UGUUUUCCAGAGGCUCUUCtt **[SEQ ID NO. 1180]** |
| **Target sequence 22:** AAGAGCCTCTGGAAAACACAT **[SEQ ID NO. 1181]** |
| Position in gene sequence: 452 |
| GC content: 42.9% |
| Sense strand siRNA: GAGCCUCUGGAAAACACAUtt **[SEQ ID NO. 1182]** |
| Antisense strand siRNA: AUGUGUUUUCCAGAGGCUCtt **[SEQ ID NO. 1183]** |
| **Target sequence 23:** AAAACACATGGCCCGCTCCAT **[SEQ ID NO. 1184]** |
| Position in gene sequence: 464 |
| GC content: 52.4% |
| Sense strand siRNA: AACACAUGGCCCGCUCCAUtt **[SEQ ID NO. 1185]** |
| Antisense strand siRNA: AUGGAGCGGGCCAUGUGUUtt **[SEQ ID NO. 1186]** |
| **Target sequence 24:** AACACATGGCCCGCTCCATAC **[SEQ ID NO. 1187]** |
| Position in gene sequence: 466 |
| GC content: 57.1 % |
| Sense strand siRNA: CACAUGGCCCGCUCCAUACtt **[SEQ ID NO. 1188]** |
| Antisense strand siRNA: GUAUGGAGCGGGCCAUGUGtt **[SEQ ID NO. 1189]** |
| **Target sequence 25:** AAAAAG481CTTGCTGAGAAG **[SEQ ID NO. 1190]** |
| Position in gene sequence: 496 |
| GC content: 33.3% |
| Sense strand siRNA: AAAG481CUUGCUGAGAAGtt **[SEQ ID NO. 1191]** |
| Antisense strand siRNA: CUUCUCAGCAAG184CUUUtt **[SEQ ID NO. 1192]** |
| **Target sequence 26:** AAAG481CTTGCTGAGAAGGC **[SEQ ID NO. 1193]** |
| Position in gene sequence: 498 |
| GC content: 42.9% |
| Sense strand siRNA: AG481CUUGCUGAGAAGGCtt **[SEQ ID NO. 1194]** |
| Antisense strand siRNA: GCCUUCUCAGCAAG184Cutt **[SEQ ID NO. 1195]** |
| **Target sequence 27:** AAGGCTGTACTGGCGGCTAAC **[SEQ ID NO. 1196]** |
| Position in gene sequence: 514 |
| GC content: 57.1% |
| Sense strand siRNA: GGCUGUACUGGCGGCUAACtt **[SEQ ID NO. 1197]** |
| Antisense strand siRNA: GUUAGCCGCCAGUACAGCCtt **[SEQ ID NO. 1198]** |
| **Target sequence 28:** AACGGGTGGAATCTGAAAAAC **[SEQ ID NO. 1199]** |
| Position in gene sequence: 532 |
| GC content: 42.9% |
| Sense strand siRNA: CGGGUGGAAUCUGAAAAACtt **[SEQ ID NO. 1200]** |
| Antisense strand siRNA: GUUUUUCAGAUUCCACCCGtt **[SEQ ID NO. 1201]** |
| **Target sequence 29:** AATCTGAAAAACGGCGGCACC **[SEQ ID NO. 1202]** |
| Position in gene sequence: 541 |
| GC content: 52.4% |
| Sense strand siRNA: UCUGAAAAACGGCGGCACCtt **[SEQ ID NO. 1203]** |
| Antisense strand siRNA: GGUGCCGCCGUUUUUCAGAtt **[SEQ ID NO. 1204]** |
| **Target sequence 30:** AAAAACGGCGGCACCCTG541 **[SEQ ID NO. 1205]** |
| Position in gene sequence: 547 |
| GC content: 52.4% |
| Sense strand siRNA: AAACGGCGGCACCCUG541tt **[SEQ ID NO. 1206]** |
| Antisense strand siRNA: 145CAGGGCTGCCGCCGUUUtt **[SEQ ID NO. 1207]** |
| **Target sequence 31:** AAACGGCGGCACCCTG541TA **[SEQ ID NO. 1208]** |
| Position in gene sequence: 549 |
| GC content: 52.4% |
| Sense strand siRNA: ACGGCGGCACCCUG541Uatt **[SEQ ID NO. 1209]** |
| Antisense strand siRNA: UA145CAGGGUGCCGCCGUtt **[SEQ ID NO. 1210]** |
| **Target sequence 32:** AAGGAAGCCGATTCCTTTCTG **[SEQ ID NO. 1211]** |
| Position in gene sequence: 605 |
| GC content: 47.6% |
| Sense strand siRNA: GGAAGCCGAUUCCUUUCUGtt **[SEQ ID NO. 1212]** |
| Antisense strand siRNA: CAGAAAGGAAUCGGCUUCCtt **[SEQ ID NO. 1213]** |
| **Target sequence 33:** AAGCCGATTCCTTTCTGCT60 **[SEQ ID NO. 1214]** |
| Position in gene sequence: 609 |
| GC content: 42.9% |
| Sense strand siRNA: GCCGAUUCCUUUCUGCU60tt **[SEQ ID NO. 1215]** |
| Antisense strand siRNA: 06AGCAGAAAGGAAUCGGCtt **[SEQ ID NO. 1216]** |
| **Target sequence 34:** AAACGAGGCCCTGAACAACAA **[SEQ ID NO. 1217]** |
| Position in gene sequence: 636 |
| GC content: 47.6% |
| Sense strand siRNA: ACGAGGCCCUGAACAACAAtt **[SEQ ID NO. 1218]** |
| Antisense strand siRNA: UUGUUGUUCAGGGCCUCGUtt **[SEQ ID NO. 1219]** |
| **Target sequence 35:** AACAACAATGGGATCCTGTCA **[SEQ ID NO. 1220]** |
| Position in gene sequence: 649 |
| GC content: 42.9% |
| Sense strand siRNA: CAACAAUGGGAUCCUGUCAtt **[SEQ ID NO. 122]** |
| Antisense strand siRNA: UGACAGGAUCCCAUUGUUGtt **[SEQ ID NO. 1222]** |
| **Target sequence 36:** AACAATGGGATCCTGTCAAGT **[SEQ ID NO. 1223]** |
| Position in gene sequence: 652 |
| GC content: 42.9% |
| Sense strand siRNA: CAAUGGGAUCCUGUCAAGUtt **[SEQ ID NO. 1224]** |
| Antisense strand siRNA: ACUUGACAGGAUCCCAUUGtt **[SEQ ID NO. 1225]** |
| **Target sequence 37:** AATGGGATCCTGTCAAGTGTT **[SEQ ID NO. 1226]** |
| Position in gene sequence: 655 |
| GC content: 42.9% |
| Sense strand siRNA: UGGGAUCCUGUCAAGUGUUtt **[SEQ ID NO. 1227]** |
| Antisense strand siRNA: AACACULTGACAGGAUCCCAtt **[SEQ ID NO. 1228]** |
| **Target sequence 38:** AAGTGTTGGAAAGTTCTCCAC **[SEQ ID NO. 1229]** |
| Position in gene sequence: 669 |
| GC content: 42.9% |
| Sense strand siRNA: GUGUUGGAAAGUUCUCCACtt **[SEQ ID NO. 1230]** |
| Antisense strand siRNA: GUGGAGAACUUUCCAACACtt **[SEQ ID NO. 1231]** |
| **Target sequence 39:** AAAGTTCTCCACT661GTTAA **[SEQ ID NO. 1232]** |
| Position in gene sequence: 678 |
| GC content: 28.6% |
| Sense strand siRNA: AGUUCUCCACU661GUUAAtt **[SEQ ID NO. 1233]** |
| Antisense strand siRNA: UUAAC166AGUGGAGAACUtt **[SEQ ID NO. 1234**] |
| **T**ar**get sequence 40:** AACCCAGTCTATGTTGGCAAT **[SEQ ID NO. 1235]** |
| Position in gene sequence: 697 |
| GC content: 42.9% |
| Sense strand siRNA: CCCAGUCUAUGUUGGCAAUtt **[SEQ ID NO. 1236]** |
| Antisense strand siRNA: AUUGCCAACAUAGACUGGGtt [**SEQ ID NO. 1237**] |
| **Target sequence 41:** AATGTGGCCTGGGCCCACATT **[SEQ ID NO. 1238]** |
| Position in gene sequence: 715 |
| GC content: 57.1% |
| Sense strand siRNA: UGUGGCCUGGGCCCACAUUtt **[SEQ ID NO. 1239]** |
| Antisense strand siRNA: AAUGUGGGCCCAGGCCACAtt **[SEQ ID NO. 1240]** |
| **Target sequence 42:** AAGAAGGCCCCAAGCATCCGA **[SEQ ID NO. 1241]** |
| Position in gene sequence: 766 |
| GC content: 57.1% |
| Sense strand siRNA: GAAGGCCCCAAGCAUCCGAtt **[SEQ ID NO. 124.2]** |
| Antisense strand siRNA: UCGGAUGCUUGGGGCCUUCtt [**SEQ ID NO. 1243]** |
| **Target sequence 43:** AAGGCCCCAAGCATCCGAGGA **[SEQ ID NO. 1244]** |
| Position in gene sequence: 769 |
| GC content: 61.9% |
| Sense strand siRNA: GGCCCCAAGCAUCCGAGGAtt **[SEQ ID NO. 1245]** |
| Antisense strand siRNA: UCCUCGGAUGCUUGGGGCCtt **(SEQ ID NO. 1246]** |
| **Target sequence 44:** AAGCATCCGAGGACAGTTCTA **[SEQ ID NO. 1247]** |
| Position in gene sequence: 777 |
| GC content: 47.6% |
| Sense strand siRNA: GCAUCCGAGGACAGUUCUAtt **[SEQ ID NO. 1248]** |
| Antisense strand siRNA: UAGAACUGUCCUCGGAUGCtt **[SEQ ID NO. 1249]** |
| **Target sequence 45:** AAAGCTATGATAACCTTAATT **[SEQ ID NO. 1250]** |
| Position in gene sequence: 827 |
| GC content: 23.8% |
| Sense strand siRNA: AGCUAUGAUAACCUUAAUUtt **[SEQ ID NO. 1251]** |
| Antisense strand siRNA: AAUUAAGGUUAUCAUAGCUtt **[SEQ ID NO. 1252]** |
| **Target sequence 46:** AACCTTAATTACACCCTGAGC **[SEQ ID NO. 1253]** |
| Position in gene sequence: 838 |
| GC content: 42.9% |
| Sense strand siRNA: CCUUAAUUACACCCUGAGCtt **[SEQ ID NO. 1254]** |
| Antisense strand siRNA: GCUCAGGGUGUAAUUAAGGtt **[SEQ ID NO. 1255]** |
| **Target sequence 47:** AATTACACCCTGAGCAAAGAG **[SEQ ID NO. 1256]** |
| Position in gene sequence: 844 |
| GC content: 42.9% |
| Sense strand siRNA: UUACACCCUGAGCAAAGAGtt **[SEQ ID NO. 1257]** |
| Antisense strand siRNA: CUCUUUGCUCAGGGUGUAAtt **[SEQ ID NO. 1258]** |
| **Target sequence 48:** AAAGAGTTCGGCCTCCGCCTT **[SEQ ID NO. 1259]** |
| Position in gene sequence: 859 |
| GC content: 57.1% |
| Sense strand siRNA: AGAGUUCGGCCUCCGCCUUtt **[SEQ ID NO. 1260]** |
| Antisense strand siRNA: AAGGCGGAGGCCGAACUCUtt **[SEQ ID NO. 1261]** |
| **Target sequence 49:** AAATA901GTGAGCTTCCTAC **[SEQ ID NO. 1262]** |
| Position in gene sequence: 938 |
| GC content: 33.3% |
| Sense strand siRNA: AUA901GUGAGCUUCCUACtt **[SEQ ID NO. 1263]** |
| Antisense strand siRNA: GUAGGAAGCUCAC109UAUtt **[SEQ ID NO. 1264]** |
| **Target sequence 50**: AATTTACACCTATCGACCGCC **[SEQ ID NO. 1265]** |
| Position in gene sequence: 966 |
| GC content: 47.6% |
| Sense strand siRNA: UUUACACCUAUCGACCGCCtt **[SEQ ID NO. 1266]** |
| Antisense strand siRNA: GGCGGUCGAUAGGUGUAAAtt **[SEQ ID NO. 1267]** |
| **Target sequence 51:** AACCGCCACATAGTC961ACA **[SEQ ID NO.1268]** |
| Position in gene sequence: 991 |
| GC content: 42.9% |
| Sense strand siRNA: CCGCCACAUAGUC961ACAtt **[SEQ ID NO. 1269]** |
| Antisense strand siRNA: UGU169GACUAUGUGGCGGtt **[SEQ ID NO. 1270]** |
| **Target sequence 52:** AAATAGCGTATTCACCTTCTC **[SEQ ID NO. 1271]** |
| Position in gene sequence: 1017 |
| GC content: 38.1% |
| Sense strand siRNA: AUAGCGUAUUCACCUUCUCtt **[SEQ ID NO. 1272]** |
| Antisense strand siRNA: GAGAAGGUGAAUACGCUAUtt **[SEQ ID NO. 1273]** |
| **Target sequence 53:** AAGAAGGCTCAGCGAGATCTG **[SEQ ID NO. 1274]** |
| Position in gene sequence: 1042 |
| GC content: 52.4% |
| Sense strand siRNA: GAAGGCUCAGCGAGAUCUGtt **[SEQ ID NO. 1275**] |
| Antisense strand siRNA: CAGAUCUCGCUGAGCCUUCtt **[SEQ ID NO. 1276]** |
| **Target sequence 54:** AAGGCTCAGCGAGATCTGGCG **[SEQ ID NO. 1277]** |
| Position in gene sequence: 1045 |
| GC content: 61.9% |
| Sense strand siRNA: GGCUCAGCGAGAUCUGGCGtt **[SEQ ID NO. 1278]** |
| Antisense strand siRNA: CGCCAGAUCUCGCUGAGCCtt **[SEQ ID NO. 1279]** |
| **Target sequence 55:** AAGCCACTCTACAGCTGGGAG **[SEQ ID NO. 1280]** |
| Position in gene sequence: 1073 |
| GC content: 57.1% |
| Sense strand siRNA: GCCACUCUACAGCUGGGAGtt **[SEQ ID NO. 1281]** |
| Antisense strand siRNA: CUCCCAGCUGUAGAGUGGCtt **[SEQ ID NO. 1282**] |
| **Target sequence 56:** AAGCCAAGCAGAAAACGGTGG **[SEQ ID NO. 1283]** |
| Position in gene sequence: 1095 |
| GC content: 52.4% |
| Sense strand siRNA: GCCAAGCAGAAAACGGUGGtt **[SEQ ID NO.1284]** |
| Antisense strand siRNA: CCACCGUUUUCUGCUUGGCtt **[SEQ ID NO. 1285]** |
| **Target sequence 57:** AAGCAGAAAACGGTGGAGTGG **[SEQ ID NO. 1286]** |
| Position in gene sequence: 1100 |
| GC content: 52.4% |
| Sense strand siRNA: GCAGAAAACGGUGGAGUGGtt **[SEQ ID NO. 1287]** |
| Antisense strand siRNA: CCACUCCACCGUUUUCUGCtt **[SEQ ID NO. 1288**] |
| **Target sequence 58:** AAAACGGTGGAGTGGGTTGGT **[SEQ ID NO. 1289]** |
| Position in gene sequence: 1106 |
| GC content: 52.4% |
| Sense strand siRNA: AACGGUGGAGUGGGUUGGUtt **[SEQ ID NO. 1290]** |
| Antisense strand siRNA: ACCAACCCACUCCACCGUUtt **[SEQ ID NO. 1291**] |
| **Target sequence 59:** AACGGTGGAGTGGGTTGGTTC **[SEQ ID NO. 1292]** |
| Position in gene sequence: 1108 |
| GC content: 57.1 % |
| Sense strand siRNA: CGGUGGAGUGGGUUGGUUCtt **[SEQ ID NO. 1293]** |
| Antisense strand siRNA: GAACCAACCCACUCCACCGtt **[SEQ ID NO. 1294]** |
| **Target sequence 60:** AAGGAGACCCTGAAGTCCAAG **[SEQ ID NO. 1295]** |
| Position in gene sequence: 1149 |
| GC content: 52.4% |
| Sense strand siRNA: GGAGACCCUGAAGUCCAAGtt **[SEQ ID NO. 1296]** |
| Antisense strand siRNA: CUUGGACUUCAGGGUCUCCtt **[SEQ ID NO. 1297]** |

| **Table 8: 17-beta-Hydroxysteroidoxidoreductase** |
|---|
| **17-beta-Hydroxysteroidoxidoreductase - P14061 (gi: 118554) [SEQ ID NO. 1298]** |
| 1 atggcccgca ccgtggtgct catcaccggc tgttcctcgg gcatcggcct gcacttggcc 61 gtacgtctgg cttcagatcc atcccagagc ttcaaagtgt atgccacgtt gagggacctg 121 aaaacacagg gccggctgtg ggaggcggcc cgggccctgg catgccctcc gggatccctg 181 gagacgttgc agctggacgt aagggactca aaatccgtgg ccgctgcccg ggaacgcgtg 241 actgagggcc gcgtggacgt gctggtgtgt aacgcaggcc tgggcctgct ggggccgctg 301 gaggcgctgg gggaggacgc cgtggcctct gtgctggacg tgaatgtagt agggactgtg 361 cggatgctgc aggccttcct gccagacatg aagaggcgcg gttcgggacg cgtgttggtg 421 accgggagcg tgggaggatt gatggggctg cctttcaatg acgtttattg cgccagcaag 481 ttcgcgctcg aaggcttatg cgagagtctg gcggttctgc tgctgccctt tggggtccac 541 ttgagcctga tcgagtgcgg cccagtgcac accgccttca tggagaaggt gttgggcagc 601 ccagaggagg tgctggaccg cacggacatc cacaccttcc accgcttcta ccaatacctc 661 gcccacagca agcaagtctt tcgcgaggcg gcgcagaacc ctgaggaggt ggcggaggtc 721 ttcctcaccg ctttgcgcgc cccgaagccg accctgcgct acttcaccac cgagcgcttc 781 ctgcccctgc tgcggatgcg cctggacgac cccagcggct ccaactacgt caccgccatg 841 caccgggaag tgttcggcga cgttccggca aaggccgagg ctggggccga ggctgggggc 901 ggggccgggc ctggggcaga ggacgaggcc gggcgcagtg cggtggggga ccctgagctc 961 ggcgatcctc cggccgcccc gcagtaa |
| **Target sequence 1:** AAAGTGTATGCCACGTTGAGG **[SEQ ID NO. 1299]** |
| Position in gene sequence: 97 |
| GC content: 47.6% |
| Sense strand siRNA: AGUGUAUGCCACGUUGAGGtt **[SEQ ID NO. 1300]** |
| Antisense strand siRNA: CCUCAACGUGGCAUACACUtt **[SEQ ID NO. 1301]** |
| **Target sequence 2:** AAAACACAGGGCCGGCTGTGG **[SEQ ID NO. 1302]** |
| Position in gene sequence: 127 |
| GC content: 61.9% |
| Sense strand siRNA: AACACAGGGCCGGCUGUGGtt **[SEQ ID NO. 1303]** |
| Antisense strand siRNA: CCACAGCCGGCCCUGUGUUtt **[SEQ ID NO. 1304]** |
| **Target sequence 3:** AACACAGGGCCGGCTGTGGGA **[SEQ ID NO. 1305]** |
| Position in gene sequence: 129 |
| GC content: 66.7% |
| Sense strand siRNA: CACAGGGCCGGCUGUGGGAtt **[SEQ ID NO. 1306]** |
| Antisense strand siRNA: UCCCACAGCCGGCCCUGUGtt **[SEQ ID NO. 1307]** |
| **Target sequence 4:** AAGGGACTCAAAATCCGTGGC **[SEQ ID NO.1308]** |
| Position in gene sequence: 210 |
| GC content: 52.4% |
| Sense strand siRNA: GGGACUCAAA.AUCCGUGGCtt **[SEQ ID NO. 1309**] |
| Antisense strand siRNA: GCCACGGAUUUUGAGUCCCtt **[SEQ ID NO. 1310]** |
| **Target sequence 5:** AAAATCCGTGGCCGCTGCCCG **[SEQ ID NO. 1311]** |
| Position in gene sequence: 219 |
| GC content: 66.7% |
| Sense strand siRNA: AAUCCGUGGCCGCUGCCCGtt **[SEQ ID NO. 1312]** |
| Antisense strand siRNA: CGGGCAGCGGCCACGGAUUtt **[SEQ ID NO. 1313]** |
| **Target sequence 6:** AATCCGTGGCCGCTGCCCGGG **[SEQ ID NO.1314]** |
| Position in gene sequence: 221 |
| GC content: 76.2% |
| Sense strand siRNA: UCCGUGGCCGCUGCCCGGGtt **[SEQ ID NO. 1315]** |
| Antisense strand siRNA: CCCGGGCAGCGGCCACGGAtt **[SEQ ID NO. 1316]** |
| T**arget sequence 7:** AACGCGTG241ACTGAGGGCC **[SEQ ID NO. 1317]** |
| Position in gene sequence: 242 |
| GC content: 57.1% |
| Sense strand siRNA: CGCGUG241ACUGAGGGCCtt **[SEQ ID NO. 1318]** |
| Antisense strand siRNA: GGCCCUCAGU142CACGCGtt **[SEQ ID NO. 1319]** |
| **Target sequence 8:** AACGCAGGCCTGGGCCTGCTG **[SEQ ID NO. 1320]** |
| Position in gene sequence: 283 |
| GC content: 71.4% |
| Sense strand siRNA: CGCAGGCCUGGGCCUGCUGtt **[SEQ ID NO. 1321]** |
| Antisense strand siRNA: CAGCAGGCCCAGGCCUGCGtt **[SEQ ID NO. 1322]** |
| **Target sequence 9:** AATGTAGTAGGGACTGTG361 **[SEQ ID NO. 1323]** |
| Position in gene sequence: 358 |
| GC content: 38.1% |
| Sense strand siRNA: UGUAGUAGGGACUGUG361tt **[SEQ ID NO. 1324]** |
| Antisense strand siRNA: 163CACAGUCCCUACUACAtt **[SEQ ID NO. 1325]** |
| **Target sequence 10:** AAGAGGCGCGGTTCGGGACGC **[SEQ ID NO. 1326]** |
| Position in gene sequence: 409 |
| GC content: 71.4% |
| Sense strand siRNA: GAGGCGCGGUUCGGGACGCtt **[SEQ ID NO. 1327]** |
| Antisense strand siRNA: GCGUCCCGAACCGCGCCUCtt **[SEQ ID NO. 1328]** |
| **Target sequence 11:** AATGACGTTTATTGCGCCAGC **[SEQ ID NO. 1329]** |
| Position in gene sequence: 478 |
| GC content: 47.6% |
| Sense strand siRNA: UGACGUUUAUUGCGCCAGCtt **[SEQ ID NO. 1330]** |
| Antisense strand siRNA: GCUGGCGCAAUAAACGUCAtt **[SEQ ID NO. 1331]** |
| **Target sequence 12:** AAG481TTCGCGCTCGAAGGC **[SEQ ID NO. 1332]** |
| Position in gene sequence: 499 |
| GC content: 52.4% |
| Sense strand siRNA: G481UUCGCGCUCGAAGGCtt **[SEQ ID NO. 1333]** |
| Antisense strand siRNA: GCCUUCGAGCGCGAA184Ctt **[SEQ ID NO. 1334]** |
| **Target sequence 13:** AAGGCTTATGCGAGAGTCTGG **[SEQ ID NO. 1335]** |
| Position in gene sequence: 515 |
| GC content: 52.4% |
| Sense strand siRNA: GGCUUAUGCGAGAGUCUGGtt **[SEQ ID NO. 1336]** |
| Antisense strand siRNA: CCAGACUCUCGCAUAAGCCtt **[SEQ ID NO. 1337]** |
| **Target sequence 14:** AAGGTGTTGGGCAGC601 CCA **[SEQ ID NO. 1338]** |
| Position in gene sequence: 613 |
| GC content: 52.4% |
| Sense strand siRNA: GGUGUUGGGCAGC601CCAtt **[SEQ ID NO. 1339]** |
| Antisense strand siRNA: UGG106GCUGCCCAACACCtt **[SEQ ID NO. 1340]** |
| **Target sequence 15:** AATACCTC661GCCCACAGCA **[SEQ ID NO. 1341]** |
| Position in gene sequence: 683 |
| GC content: 47.6% |
| Sense strand siRNA: UACCUC661GCCCACAGCAtt **[SEQ ID NO. 1342**] |
| Antisense strand siRNA: UGCUGUGGGC156GAGGUAtt **[SEQ ID NO. 1343]** |
| **Target sequence 16:** AAGCAAGTCTTTCGCGAGGCG **[SEQ ID NO. 1344]** |
| Position in gene sequence: 703 |
| GC content: 57.1% |
| Sense strand siRNA: GCAAGUCUUUCGCGAGGCGtt **[SEQ ID NO. 1345]** |
| Antisense strand siRNA: CGCCUCGCGAAAGACUUGCtt **[SEQ ID NO. 1346]** |
| **Target sequence 17:** AAGTCTTTCGCGAGGCGGCGC **[SEQ ID NO. 1347]** |
| Position in gene sequence: 707 |
| GC content: 66.7% |
| Sense strand siRNA: GUCUUUCGCGAGGCGGCGCtt **[SEQ ID NO. 1348]** |
| Antisense strand siRNA: GCGCCGCCUCGCGAAAGACtt **[SEQ ID NO. 1349**] |
| **Target sequence 18:** AACCCTGAGGAGGTGGCGGAG **[SEQ ID NO. 1350**] |
| Position in gene sequence: 730 |
| GC content: 66.7% |
| Sense strand siRNA: CCCUGAGGAGGUGGCGGAGtt **[SEQ ID NO. 1351]** |
| Antisense strand siRNA: CUCCGCCACCUCCUCAGGGtt **[SEQ ID NO. 1352]** |
| **Target sequence 19:** AAGCCGACCCTGCGCTACTTC [**SEQ ID NO. 1353]** |
| Position in gene sequence: 781 |
| GC content: 61.9% |
| Sense strand siRNA: GCCGACCCUGCGCUACUUCtt **[SEQ ID NO. 1354]** |
| Antisense strand siRNA: GAAGUAGCGCAGGGUCGGCtt **[SEQ ID NO. 1355]** |
| **Target sequence 20:** AACTACGTCACCGCCATG841 **[SEQ ID NO. 1356]** |
| Position in gene sequence: 862 |
| GC content: 47.6% |
| Sense strand siRNA: CUACGUCACCGCCAUG841tt **[SEQ ID NO. 1357]** |
| Antisense strand siRNA: 148CAUGGCGGUGACGUAGtt **[SEQ ID NO. 1358]** |
| **Target sequence 21:** AAGTGTTCGGCGACGTTCCGG **[SEQ ID NO. 1359]** |
| Position in gene sequence: 890 |
| GC content: 61.9% |
| Sense strand siRNA: GUGUUCGGCGACGUUCCGGtt **[SEQ ID NO. 1360]** |
| Antisense strand siRNA: CCGGAACGUCGCCGAACACtt **[SEQ ID NO. 1361]** |
| **Target sequence 22:** AAAGGCCGAGGCTGGGGCCGA **[SEQ ID NO. 1362]** |
| Position in gene sequence: 912 |
| GC content: 71.4% |
| Sense strand siRNA: AGGCCGAGGCUGCTGGCCGAtt **[SEQ ID NO. 1363]** |
| Antisense strand siRNA: UCGGGCCCAGCCUCGGCCUtt **[SEQ ID NO. 1364]** |

| **Table 9: Steroid Sulfatase** |
|---|
| **Steroid Sulfatase - AAA60598 (gi: 338607) [SEQ ID NO. 1365]** |
| 1 ggaaatggca ccttgggatg agctgtcaca gcaagactga cttctgtcac caccctttac 61 atcacggctt caattatttc tatgggatct ctttgaccaa tctgagagac tgcaagcccg 121 gagagggcag tgtcttcacc acgggcttca agaggctggt cttcctcccc ctgcagatcg 181 tcggggtcac cctccttacc cttgctgcac tcaattgtct ggggctactc cacgtgcctc 241 taggcgtttt tttcagcctt ctcttcctag cagccctaat cctgaccctt ttcttgggct 301 tccttcatta cttccggccc ctgaactgct tcatgatgag gaactacgag atcattcagc 361 agcccatgtc ctatgacaat ctcacccaga ggctaacggt ggaggcggcc cagttcatac 421 agcg |
| **Target sequence 1:** AAATGGCACCTTGGGATGAGC **[SEQ ID NO. 1366]** |
| Position in gene sequence: 4 |
| GC content: 52.4% |
| Sense strand siRNA: AUGGCACCUUGGGAUGAGCtt **[SEQ ID NO. 1367]** |
| Antisense strand siRNA: GCUCAUCCCAAGGUGCCAUtt **[SEQ ID NO.1368]** |
| **Target sequence 2:** AAGACTGACTTCTGTCACCAC **[SEQ ID NO. 1369]** |
| Position in gene sequence: 34 |
| GC content: 47.6% |
| Sense strand siRNA: GACUGACUUCUGUCACCACtt **[SEQ ID NO. 1370]** |
| Antisense strand siRNA: GUGGUGACAGAAGUCAGUCtt **[SEQ ID NO. 1371]** |
| **Target sequence 3:** AATTATTTCTATGGGATCTCT **[SEQ ID NO. 1372]** |
| Position in gene sequence: 75 |
| GC content: 28.6% |
| Sense strand siRNA: UUAWUCUAUGGGAUCUCUtt **[SEQ ID NO.1373]** |
| Antisense strand siRNA: AGAGAUCCCAUAGAAAUAAtt **[SEQ ID NO. 1374]** |
| **Target sequence 4:** AATCTGAGAGACTGCAAGCCC **[SEQ ID NO. 1375]** |
| Position in gene sequence: 102 |
| GC content: 52.4% |
| Sense strand siRNA: UCUGAGAGACUGCAAGCCCtt **[SEQ ID NO. 1376]** |
| Antisense strand siRNA: GGGCUUGCAGUCUCUCAGAtt **[SEQ ID NO. 1377]** |
| **Target sequence 5:** AAGCCCG121GAGAGGGCAGT **[SEQ ID NO. 1378]** |
| Position in gene sequence: 117 |
| GC content: 57.1% |
| Sense strand siRNA: GCCCG121GAGAGGGCAGUtt **[SEQ ID NO. 1379]** |
| Antisense strand siRNA: ACUGCCCUCUC121CGGGCtt **[SEQ ID NO. 1380]** |
| **Target sequence 6:** AAGAGGCTGGTCTTCCTCCCC **[SEQ ID NO. 1381]** |
| Position in gene sequence: 156 |
| GC content: 61.9% |
| Sense strand siRNA: GAGGCUGGUCUUCCUCCCCtt **[SEQ ID NO. 1382]** |
| Antisense strand siRNA: GGGGAGGAAGACCAGCCUCtt **[SEQ ID NO. 13831** |
| **Target sequence 7:** AATTGTCTGGGGCTACTCCAC **[SEQ ID NO. 1384]** |
| Position in gene sequence: 222 |
| GC content: 52.4% |
| Sense strand siRNA: UUGUCUGGGGCUACUCCACtt **[SEQ ID NO. 1385]** |
| Antisense strand siRNA: GUGGAGUAGCCCCAGACAAtt **[SEQ ID NO. 1386]** |
| **Target sequence 8:** AATCCTGACCCTTTTCTTGGG **[SEQ ID NO.1387]** |
| Position in gene sequence: 290 |
| GC content: 47.6% |
| Sense strand siRNA: UCCUGACCCWUUCUUGGGtt **[SEQ ID NO. 1388]** |
| Antisense strand siRNA: CCCAAGAAAAGGGUCAGGAtt **[SEQ ID NO. 1389]** |
| **Target sequence 9:** AACTGCTTCATGATGAGGAAC **[SEQ ID NO. 1390]** |
| Position in gene sequence: 339 |
| GC content: 42.9% |
| Sense strand siRNA: CUGCUUCAUGAUGAGGAACtt **[SEQ ID NO. 1391]** |
| Antisense strand siRNA: GUUCCUCAUCAUGAAGCAGtt **[SEQ ID NO. 1392]** |
| **Target sequence 10:** AACTACGAGATCATTCAGC36 **[SEQ ID NO. 1393]** |
| Position in gene sequence: 357 |
| GC content: 38.1% |
| Sense strand siRNA: CUACGAGAUCAUUCAGC36tt **[SEQ ID NO. 1394]** |
| Antisense strand siRNA: 63GCUGAAUGAUCUCGUAGtt **[SEQ ID NO. 1395]** |
| **Target sequence 11:** AATCTCACCCAGAGGCTAACG **[SEQ ID NO. 1396]** |
| Position in gene sequence: 396 |
| GC content: 52.4% |
| Sense strand siRNA: UCUCACCCAGAGGCUAACGtt **[SEQ ID NO. 1397]** |
| Antisense strand siRNA: CGUUAGCCUCUGGGUGAGAtt **[SEQ ID NO. 1398]** |
| **Target sequence 12:** AACGGTGGAGGCGGCCCAGTT **[SEQ ID NO.1399]** |
| Position in gene sequence: 413 |
| GC content: 66.7% |
| Sense strand siRNA: CGGUGGAGGCGGCCCAGUUtt **[SEQ ID NO. 1400]** |
| antisense strand siRNA: AACUGGGCCGCCUCCACCGtt **[SEQ ID NO. 1401]** |

The present invention will now be described with reference to the following clauses
1. A method of reducing hair loss in a mammal comprising the step of:
   contacting a plurality of the mammal's hair cells with double-stranded ribonucleic acid, wherein one strand comprises at least a partial nucleic acid sequence of an androgen signal transduction pathway protein, and wherein the double-stranded ribonucleic acid interferes with the translation of mRNA of the androgen signal transduction protein.
2. A method of reducing hair loss in mammal comprising the step of:
   administering to the mammal a composition comprising a double-stranded ribonucleic acid, wherein one strand comprises at least a partial nucleic acid sequence of an androgen signal transduction pathway protein, and wherein the double-stranded ribonucleic acid interferes with the translation of mRNA of the androgen signal transduction protein; and a pharmaceutically acceptable carrier.
3. A method of treating alopecia comprising the step of:
   administering to a mammal a composition comprising a double-stranded ribonucleic acid, wherein one strand comprises at least a partial nucleic acid sequence of an androgen signal transduction pathway protein, and wherein the double-stranded ribonucleic acid interferes with the translation of mRNA of the androgen signal transduction protein;
      and a pharmaceutically acceptable carrier.
4. A method of inducing hair growth comprising the step of:
   increasing the duration of anagen of a hair cell of a mammal by contacting the hair cell with a double-stranded ribonucleic acid, wherein one strand comprises at least a partial nucleic acid sequence of an androgen signal transduction pathway protein.
5. A method of promoting hair growth or decreasing hair loss, said method comprising the steps of:
   a) providing an agent comprising at least one member selected from the group consisting of a small inhibitory ribonucleic acid comprising at least a partial nucleic acid sequence of an androgen signal transduction pathway protein and physiologically compatible salts thereof; and
   b) applying said agent to hair and scalp in an amount sufficient for said promoting of said hair growth or said decreasing of said hair loss.
6. The method of clause 1 , further comprising the step of c) leaving said agent on said hair and scalp for at least 24 hours.
7. An inhibitory nucleic acid comprising:
   a double-stranded ribonucleic acid, wherein one strand comprises at least a partial nucleic acid sequence of an androgen signal transduction pathway protein, and wherein the double-stranded ribonucleic acid inhibits the translation of a mRNA encoding the nucleic acid sequence of the androgen signal transduction pathway protein.
8. A vector comprising:
   a double-stranded ribonucleic acid, wherein one strand comprises at least a partial nucleic acid sequence of an androgen signal transduction pathway protein, and wherein the double-stranded ribonucleic acid inhibits the translation of a mRNA encoding the nucleic acid sequence of the androgen signal transduction pathway protein.
9. A cell comprising:
   a double-stranded ribonucleic acid, wherein one strand comprises at least a partial nucleic acid sequence of an androgen signal transduction pathway protein, and wherein the double-stranded ribonucleic acid inhibits the translation of a mRNA encoding the nucleic acid sequence of the androgen signal transduction pathway protein.
10. Any one of clauses 1-9, wherein the androgen signal transduction pathway protein is selected from the group consisting of isozymes I and II of 5-α reductase, the androgen receptor, aromatase, 3-α-hydroxysteroiddehydrogenase, 3-β-hydroxysteroiddehydrogenase ,3-β-hydroxysteroiddehydrogenase-4-5-isomerase, 17-β-hydroxysteroidoxidoreductase, and steroid sulfatase.
11. Any one of clauses 1 -9, wherein the partial nucleic acid sequence is selected from SEQ. ID Nos 1-1400 in Tables 1-9.
12. Any one of clauses 1-6, further comprising the step of: administering a second therapeutic agent.
13. The method of clause 12, wherein the second therapeutic agent is selected from the group consisting of 17β-N-t butylcarbamoyl-4-aza-5α-androst-1-en-3-one, 6-amino-1,2-dihydro-1-hydroxy-2-imino-4-piperodinopyrimidine, a combination thereof, and a prodrug thereof.
14. A method of treating hair loss in a mammal comprising reducing the levels of dihydroxy testosterone in the mammal by contacting the mammal with an effective amount of inhibitory nucleic acid specific for 5-α-reductase, wherein the effective amount of inhibitory nucleic acid specific for 5-α-reductase inhibits the expression of 5-α reductase protein.
15. A method of reducing testosterone metabolites in a cell comprising contacting the cell with an amount of inhibitory nucleic acid specific for 5-α-reductase sufficient to reduce the levels of 5-α reductase protein in the cell.
16. The method of clause 15, wherein the testosterone metabolites comprise dihydroxy testosterone.
17. The method of clause 15, wherein the cell is a hair cell.
18. The method of clause 15, wherein the inhibitory nucleic acid comprises a nucelotide sequence selected from SEQ. ID. Nos 1-91.

## Claims

1. A double-stranded ribonucleic acid, wherein one strand comprises a sequence transcribed from the partial nucleic acid sequence of an androgen signal transduction pathway protein set forth in SEQ ID NO: 147, and wherein the double-stranded ribonucleic acid interferes with the translation of mRNA of the androgen signal transduction protein for use in a therapeutic method of reducing hair loss in a mammal.

2. A vector, preferably a viral vector, encoding a double-stranded ribonucleic acid, wherein one strand comprises a sequence transcribed from the partial nucleic acid sequence of an androgen signal transduction pathway protein set forth in SEQ ID NO: 147, and wherein the double-stranded ribonucleic acid interferes with the translation of mRNA of the androgen signal transduction protein for use in a therapeutic method of reducing hair loss in a mammal.

3. The double-stranded riboncuelic acid of claim 1 or vector of claim 2 for use in treating alopecia.

4. The non-therapeutic use of a double-stranded ribonucleic acid, wherein one strand comprises the partial nucleic acid sequence of an androgen signal transduction pathway protein set forth in SEQ ID NO: 147, and wherein the double-stranded ribonucleic acid interferes with the translation of mRNA of the androgen signal transduction protein to reduce hair loss in a mammal or to promote hair growth in a mammal.

5. The non-therapeutic use of a vector, preferably a viral vector, encoding a double-stranded ribonucleic acid, wherein one strand comprises the partial nucleic acid sequence of an androgen signal transduction pathway protein set forth in SEQ ID NO:147, and wherein the double-stranded ribonucleic acid interferes with the translation of mRNA of the androgen signal transduction protein to reduce hair loss in a mammal or to promote hair growth in a mammal.

6. The double-stranded ribonucleic acid or vector or use of any one of the preceding claims wherein one of the strands comprises the sequence set forth in any one of SEQ ID NOs: 149, 150, 152, 153, 155, 156, 158, 159, 161, 162, 164, 165, 167, 168, 170, 171, 173, 174, 176, 177, 179, 180, 182, 183, 185, 186, 188, 189, 191, 192, 194, 195, 197, 198, 200, 201, 203, 204, 206, 207, 209, 210, 212, 213, 215, 216, 218, 219, 221, 222, 224, 225, 227, 228, 230, 231, 233, 234, 236, 237, 239, 240, 242, 243, 245, 246, 248, 249, 251, 252, 254, 255, 257, 258, 260, 261, 263, 264, 266, 267, 269, 270, 272, 273, 275, 276, 278, 279, 281, 282, 284, 285, 287, 288, 290, 291, 293, 294, 296, 297, 299, 300, 302, 303, 305, 306, 308, 309, 311, 312, 314, 315, 317, 318, 320, 321, 323, 324, 326, 327, 329, 330, 332, 333, 335, 336, 338, 339, 341, 342, 344, 345, 347, 348, 350, 351, 353, 354, 356, 357, 359, 360, 362, 363, 365, 366, 368, 369, 371, 372, 374, 375, 377, 378, 380, 381, 383, 384, 386, 387, 389, 390, 392, 393, 395, 396, 398, 399, 401, 402, 404, 405, 407, 408, 410, 411, 413, 414, 416, 417, 419, 420, 422, 423, 425, 426, 428, 429, 431, 432, 434, 435, 437, 438, 440, 441, 443, 444, 446, 447, 449, 450, 452, 453, 455, 456, 458, 459, 461, 462, 464, 465, 467, 468, 470, and 471.

7. The double-stranded ribonucleic acid of claim 1, 3, or 6 or vector of claim 2, 3, or 6, **characterized in that** the double-stranded ribonucleic acid or vector is to be administered with a second therapeutic agent.

8. The double-stranded ribonucleic acid or vector of claim 7, wherein the second therapeutic agent is selected from the group consisting of 17β-N-t butylcarbamoyl-4-aza-5α-androst-1-en-3-one, 6-amino-1,2-dihydro-1-hyrdroxy-2-imino-4-peperodinopyrimidine, a combination thereof, and a prodrug thereof.
